# EUROPEAN PATENT SPECIFICATION

(11) **EP 2 143 806 B1**
(45) Date of publication and mention of the grant of the patent: **29.07.2015**
(21) Application number: 08739639.6
(22) Date of filing: 26.03.2008
(51) Int. Cl.: C12Q 1/68, C12N 15/09, G01N 33/53

(54) **METHOD OF MEASURING DNA METHYLATION**
VERFAHREN ZUR MESSUNG VON DNA-METHYLIERUNG
PROCÉDÉ DE MESURE DE MÉTHYLATION D'ADN

(30) Priority: 26.03.2007 JP 2007078660
(43) Date of publication of application: 13.01.2010
(73) Proprietor: Sumitomo Chemical Company, Limited, Tokyo 104-8260 (JP)
(72) Inventor: TOMIGAHARA, Yoshitaka, Toyonaka-shi Osaka 560-0013 (JP); TARUI, Hirokazu, Toyonaka-shi Osaka 561-0802 (JP)
(74) Representative: Duckworth, Timothy John
(86) International application number: PCT/JP2008/056526
(87) International publication number: WO 2008/117888

(56) References cited:
- EP-A2- 1 914 320
- WO-A1-89/04876
- WO-A1-2006/056478
- WO-A2-2004/094675
- WO-A2-2007/081791
- MAKI ET AL: "Nanowire-transistor based ultra-sensitive DNA methylation detection" BIOSENSORS AND BIOELECTRONICS, ELSEVIER BV, NL LNKD- DOI:10.1016/J.BIOS.2007.08.017, vol. 23, no. 6, 20 December 2007 (2007-12-20), pages 780-787, XP022396668 ISSN: 0956-5663
- SALZBERG A ET AL: "Identification of methylated sequences in genomic DNA of adult Drosophila melanogaster" BIOCHEMICAL AND BIOPHYSICAL RESEARCH COMMUNICATIONS, ACADEMIC PRESS INC. ORLANDO, FL, US LNKD- DOI:10.1016/J.BBRC.2004.07.134, vol. 322, no. 2, 17 September 2004 (2004-09-17), pages 465-469, XP004599163 ISSN: 0006-291X
- CHENG LIANG ET AL: "Precise microdissection of human bladder carcinomas reveals divergent tumor subclones in the same tumor." CANCER 1 JAN 2002 LNKD- PUBMED:11815965, vol. 94, no. 1, 1 January 2002 (2002-01-01), pages 104-110, XP007913481 ISSN: 0008-543X
- KUMAR R.R. ET AL.: 'Immunoaffinity chromatography to isolate methylated DNA using immobilizes anti-5-methyl cytosine antibody' BIOTECHNOL. TECH. vol. 5, no. 6, 1991, pages 469 - 470, XP008083955
- DEOBAGKAR D.N. ET AL.: 'Separation of 5-methylcytosine-rich DNA using immobilized antibody' ENZYME MICROB. TECHNOL. vol. 8, no. 2, 1986, pages 97 - 100, XP023666146

## Description

### TECHNICAL FIELD

The present invention relates to a method of measuring the content of methylated DNA in a DNA region of interest in a genomic DNA contained in a biological specimen, and so on.

### BACKGROUND ART

As a method for evaluating the methylation state of DNA in an objective DNA region in a genomic DNA contained in a biological specimen, for example, there is known a method of measuring the content of methylated DNA in an objective DNA region in a genomic DNA (see, for example, Nucleic Acids Res., 1994, Aug 11; 22(15): 2990-7, and Proc. Natl. Acad. Sci. U.S.A., 1997, Mar 18; 94(6): 2284-9 for reference). In such a measuring method, first, it is necessary to extract DNA containing the objective DNA region from a DNA sample derived from a genomic DNA, and the extracting operation is complicated.

As a method of measuring the content of methylated DNA in an objective region of extracted DNA, for example, (1) a method of amplifying an objective region by subjecting the DNA to a chain reaction for DNA synthesis by DNA polymerase after modification of the DNA with a sulfite or the like (Polymerase Chain Reaction; hereinafter also referred to as PCR), and (2) a method of amplifying an objective region by subjecting the DNA to PCR after digestion of the DNA using a methylation sensitive restriction enzyme are known. Both of these methods require time and labor for DNA modification for detection of methylation, subsequent purification of the product, preparation of a reaction system for PCR, and checking of DNA amplification.

### DISCLOSURE OF THE INVENTION

It is an object of the present invention to provide a method of measuring the content of methylated DNA in an objective DNA region in a genomic DNA contained in a biological specimen in a simple and convenient manner.

That is, the present invention provides:
1. A method (hereinafter, also referred to as the present measuring method) of detecting or quantifying methylated DNA in a target DNA region possessed by genomic DNA contained in a biological specimen, consisting of:
   (1) First step of separating double-stranded DNA contained in a DNA sample derived from the genomic DNA contained in the biological specimen into single-stranded DNA,
   (2) Second step of mixing (i) the single-stranded DNA separated in First step, (ii) a methylated DNA antibody, and (iii) an oligonucleotide (hereinafter, also referred to as the present oligonucleotide) capable of binding with the single-stranded DNA without inhibiting binding between the methylated DNA antibody and methylated DNA in a target DNA region, thereby forming a complex of the single-stranded DNA containing methylated DNA in the target DNA region, the methylated DNA antibody, and the oligonucleotide, and immobilizing the methylated DNA antibody or the oligonucleotide contained in the complex to a support followed by separating the complex simultaneously or after the formation; and
   (3) Third step of detecting or quantifying either the methylated DNA antibody or the oligonucleotide contained in the separated complex, according to an identification function available for detection possessed by the methylated DNA antibody or the oligonucleotide,
      wherein when the methylated DNA antibody contained in the complex is bound to a support said third step consists of detecting or quantifying said oligonucleotide, and wherein when the oligonucleotide contained in the complex is bound to a support said third step consists of detecting or quantifying said methylated DNA antibody,
      thereby detecting or quantifying methylated DNA in the target DNA region contained in the biological specimen;
2. The method according to the above 1., wherein as the oligonucleotide that is mixed in Second step, and does not inhibit binding between the methylated DNA antibody and a methylated base existing in single-stranded DNA containing the target DNA region, two or more kinds of oligonucleotides are used;
3. The method according to the above 1., wherein the complex formed in Second step, or a bound body of the single-stranded DNA separated in First step and the oligonucleotide not inhibiting binding between the methylated DNA antibody and a methylated base existing in single-stranded DNA containing the target DNA region arising during formation of the complex in Second step is formed in a reaction system containing a bivalent positive ion;
4. The method according to the above 3., wherein the bivalent positive ion is a magnesium ion;
5. The method according to any one of the above 1. to 4., comprising as a separating operation of the complex in Second step, a step of making the methylated DNA antibody contained in the formed complex be bound to a support;
6. The method according to any one of the above 1. to 4., comprising as a separating operation of the complex in Second step, a step of making the oligonucleotide contained in the formed complex be bound to a support;
7. The method according to any one of the above 1. to 6., additionally comprising, between immediately after end of First step and immediately before start of Third step, a step of digesting the single-stranded DNA separated in First step by at least one kind of methylation sensitive restriction enzyme capable of digesting single-stranded DNA;
8. The method according to any one of the above 1. to 6., additionally comprising, between immediately after end of First step and immediately before start of Third step, (i) a step of mixing the single-stranded DNA separated in First step, and a masking oligonucleotide having a recognition sequence of at least one kind of methylation sensitive restriction enzyme as its part, and (ii) digesting a mixture obtained by the previous step (single-stranded DNA existing therein in which DNA is not methylated in the target DNA region) by the methylation sensitive restriction enzyme;
9. The method according to the above 7., wherein the at least one kind of methylation sensitive restriction enzyme capable of digesting single-stranded DNA is HhaI which is a methylation sensitive restriction enzyme capable of digesting single-stranded DNA;
10. The method according to the above 8., wherein the at least one kind of methylation sensitive restriction enzyme is HpaII or HhaI which is a methylation sensitive restriction enzyme;
11. The method according to any one of the above 1. to 10., wherein the methylated DNA antibody is a methyl cytosine antibody;
12. The method according to any one of the above 1. to 11., wherein the biological specimen is serum or plasma of a mammal;
13. The method according to any one of the above 1. to 11., wherein the biological specimen is blood or a bodily fluid of a mammal;
14. The method according to any one of the above 1. to 11., wherein the biological specimen is a cell lysate or a tissue lysate;
15. The method according to any one of the above 1. to 14., wherein the DNA sample derived from the genomic DNA contained in the biological specimen is a DNA sample preliminarily subjected to a digestion treatment by a restriction enzyme whose recognition cleaving site excludes a target DNA region possessed by the genomic DNA;
16. The method according to any one of the above 1. to 15., wherein the DNA sample derived from the genomic DNA contained in the biological specimen is a DNA sample preliminarily subjected to a digestion treatment by at least one kind of methylation sensitive restriction enzyme;
17. The method according to any one of the above 1. to 15., wherein the DNA sample derived from the genomic DNA contained in the biological specimen is a DNA sample preliminarily subjected to a digestion treatment by at least one kind of methylation sensitive restriction enzyme after addition of the masking oligonucleotide;
18. The method according to the above 16. or 17., wherein the at least one kind of methylation sensitive restriction enzyme is HpaII or HhaI which is a methylation sensitive restriction enzyme;
19. The method according to any one of the above 1. to 18., wherein the DNA sample derived from the genomic DNA contained in the biological specimen is a preliminarily purified DNA sample;
20. The method according to any one of the above 1. to 19., wherein the target DNA region possessed by the genomic DNA is a DNA region having a cleaving site recognized by at least one kind of methylation sensitive restriction enzyme;
21. The method according to any one of the above 1. to 20., wherein a counter oligonucleotide is added in separating double-stranded DNA contained in a DNA sample derived from the genomic DNA into single-stranded DNA in First step;
22. The method according to any one of the above 1. to 21., wherein separation of double-stranded DNA contained in a DNA sample derived from the genomic DNA into single-stranded DNA in First step is conducted in a reaction system containing a bivalent positive ion or a magnesium ion; and so on.

### BRIEF DESCRIPTION OF THE DRAWINGS

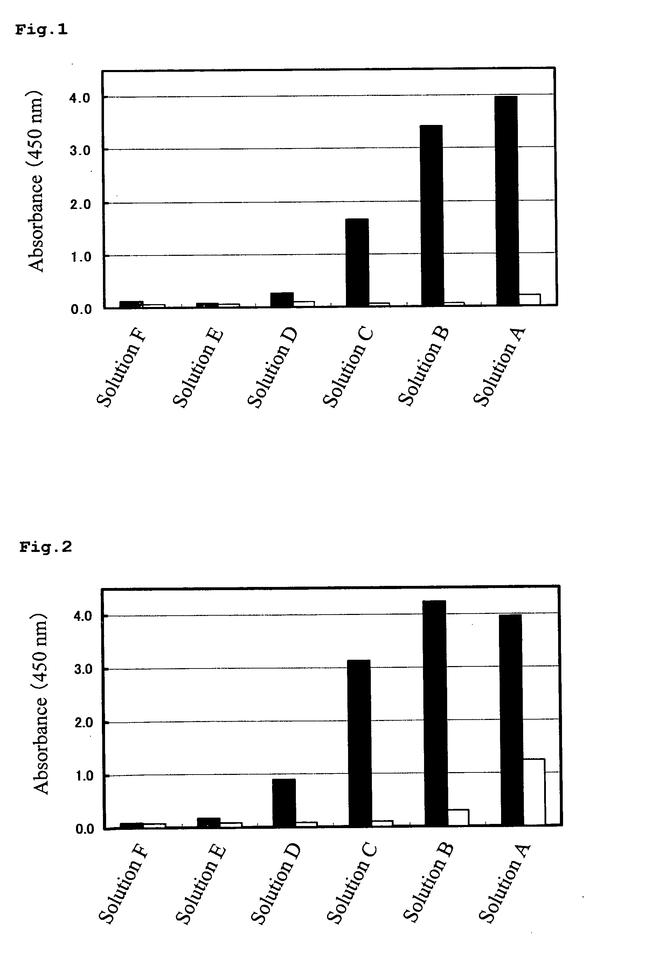

Fig. 1 shows results of Example 1 carried out using 1 µg/mL of a methylated cytosine antibody. For each of Solution A (2.5 pmol/100 µL solution in TE buffer), Solution B (0.25 pmol/100 µL solution in TE buffer), Solution C (0.025 pmol/100 µL solution in TE buffer), Solution D (0.0025 pmol/100 µL solution in TE buffer), Solution E (0.00025 pmol/100 µL solution in TE buffer), and Solution F (0 pmol/100 µL solution in TE buffer (negative control solution)), a residual amount of a methylated oligonucleotide, and a residual amount of an unmethylated oligonucleotide measured by absorbance (450 nm) are shown in this order from the left-hand.
Fig. 2 shows results of Example 1 carried out using 10 µg/mL of a methylated cytosine antibody. For each of Solution A (2.5 pmol/100 µL solution in TE buffer), Solution B (0.25 pmol/100 µL solution in TE buffer), Solution C (0.025 pmol/100 µL solution in TE buffer), Solution D (0.0025 pmol/100 µL solution in TE buffer), Solution E (0.00025 pmol/100 µL solution in TE buffer), and Solution F (0 pmol/100 µL solution in TE buffer (negative control solution)), a residual amount of a methylated oligonucleotide, and a residual amount of an unmethylated oligonucleotide measured by absorbance (450 nm) are shown in this order from the left-hand.

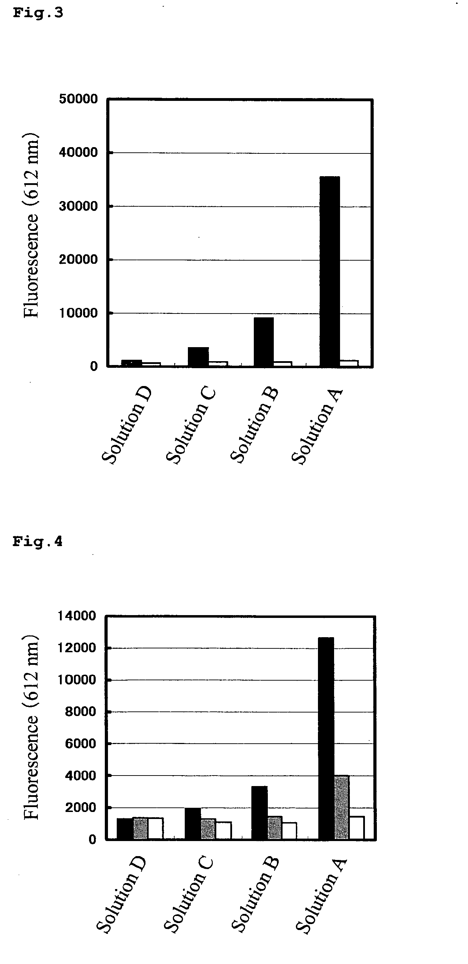

Fig. 3 shows results of Example 2. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (formation amount of a complex) of a methylated oligonucleotide UBC-M8 having the nucleotide sequence of SEQ ID NO: 25, and a residual amount (formation amount of a complex) of an unmethylated oligonucleotide UBC-UM8 having the nucleotide sequence of SEQ ID NO: 26 measured by fluorescence (612 nm) are shown in this order from the left-hand.
Fig. 4 shows results of Group A (no treatment group) in Example 3. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC-M having the nucleotide sequence of SEQ ID NO: 28, a residual amount (complex formation amount) of a partially methylated oligonucleotide UBC-HM having the nucleotide sequence of SEQ ID NO: 29, and a residual amount (complex formation amount) of an unmethylated oligonucleotide UBC-UM having the nucleotide sequence of SEQ ID NO: 30 measured by fluorescence (612 nm) are shown in this order from the left-hand.

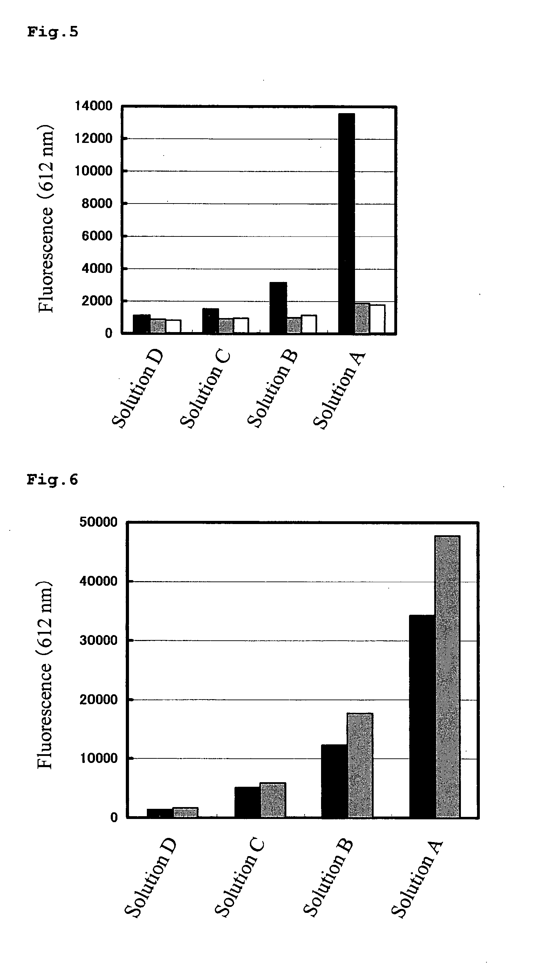

Fig. 5 shows results of Group B (Hha treatment group) in Example 3. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC-M having the nucleotide sequence of SEQ ID NO: 28, a residual amount (complex formation amount) of a partially methylated oligonucleotide UBC-HM having the nucleotide sequence of SEQ ID NO: 29, and a residual amount (complex formation amount) of an unmethylated oligonucleotide UBC-UM having the nucleotide sequence of SEQ ID NO: 30 measured by fluorescence (612 nm) are shown in this order from the left-hand.
Fig. 6 shows results of Group A (no treatment group) in Example 4. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC86-M having the nucleotide sequence of SEQ ID NO: 33, and a residual amount (complex formation amount) of a partially methylated oligonucleotide UBC118-HM having the nucleotide sequence of SEQ ID NO: 34 measured by fluorescence (612 nm) are shown in this order from the left-hand.

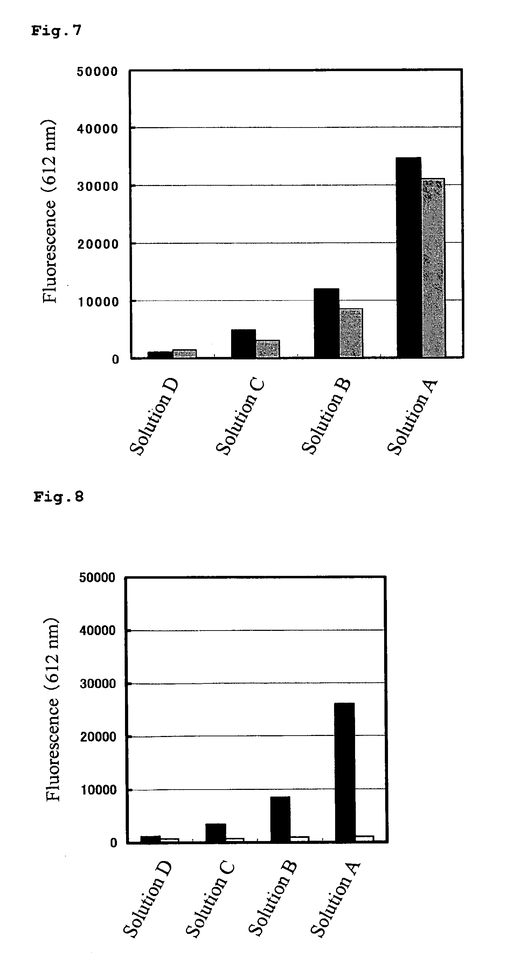

Fig. 7 shows results of Group B (Hha treatment group) in Example 4. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC86-M having the nucleotide sequence of SEQ ID NO: 33, and a residual amount (complex formation amount) of a partially methylated oligonucleotide UBC118-HM having the nucleotide sequence of SEQ ID NO: 34 measured by fluorescence (612 nm) are shown in this order from the left-hand.
Fig. 8 shows results of experiments carried out by using a 5'-end biotin-labeled oligonucleotide UBC having the nucleotide sequence of SEQ ID NO: 43 in Example 5. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution, and a complex formation amount in an unmethylated oligonucleotide mixed solution measured by fluorescence (612 nm) are shown in this order from the left-hand.

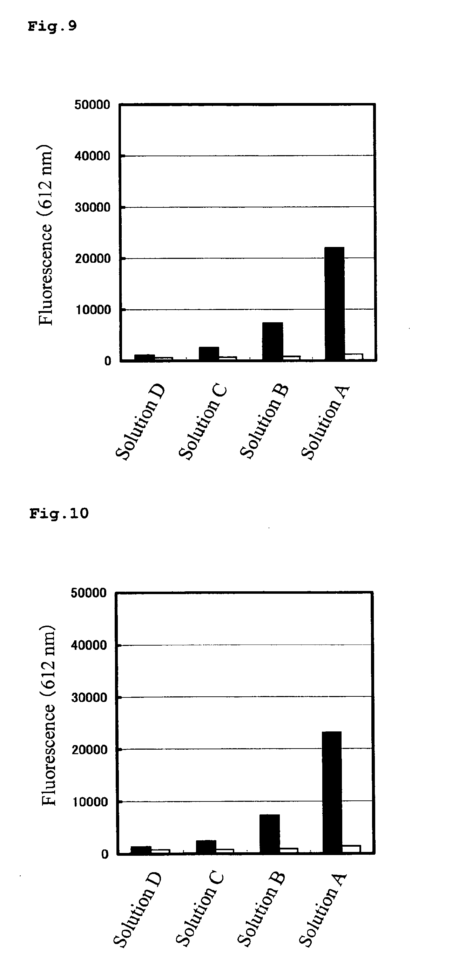

Fig. 9 shows results of experiments carried out by using a 5'-end biotin-labeled oligonucleotide FBN having the nucleotide sequence of SEQ ID NO: 44 in Example 5. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution, and a complex formation amount in an unmethylated oligonucleotide mixed solution measured by fluorescence (612 nm) are shown in this order from the left-hand.
Fig. 10 shows results of experiments carried out by using a 5'-end biotin-labeled oligonucleotide GPR having the nucleotide sequence of SEQ ID NO: 45 in Example 5. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution, and a complex formation amount in an unmethylated oligonucleotide mixed solution measured by fluorescence (612 nm) are shown in this order from the left-hand.

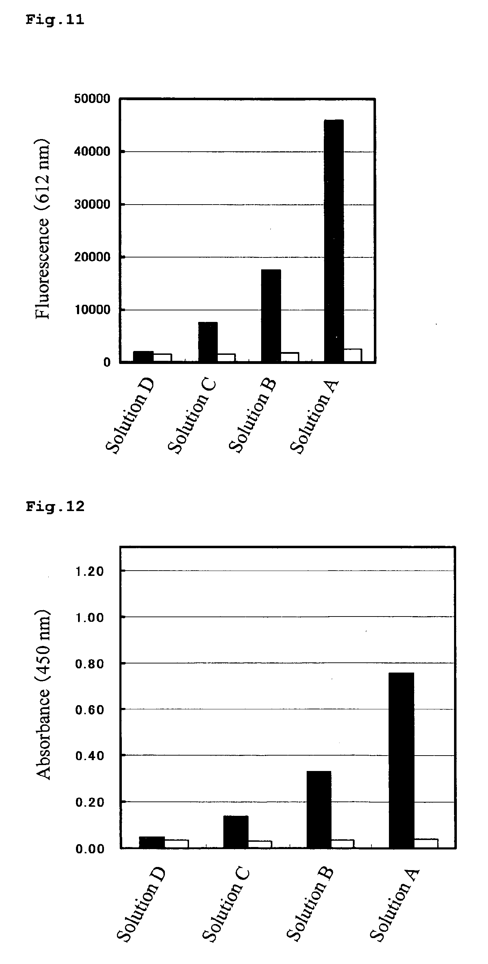

Fig. 11 shows results of experiments using a mixed solution of a 5'-end biotin-labeled oligonucleotide UBC having the nucleotide sequence of SEQ ID NO: 43, a 5'-end biotin-labeled oligonucleotide FBN having the nucleotide sequence of SEQ ID NO: 44, and a 5'-end biotin-labeled oligonucleotide GPR having the nucleotide sequence of SEQ ID NO: 45 in Example 5. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution, and a complex formation amount in an unmethylated oligonucleotide mixed solution measured by fluorescence (612 nm) are shown in this order from the left-hand.
Fig. 12 shows results of experiments using a methylated oligonucleotide UBC-M8 having the nucleotide sequence of SEQ ID NO: 46, and an unmethylated oligonucleotide UBC-U8 having the nucleotide sequence of SEQ ID NO: 49 in Example 6. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC-M8 having the nucleotide sequence of SEQ ID NO: 46, and a residual amount (complex formation amount) of an unmethylated oligonucleotide UBC-U8 having the nucleotide sequence of SEQ ID NO: 49 measured by absorbance (450 nm) are shown in this order from the left-hand.

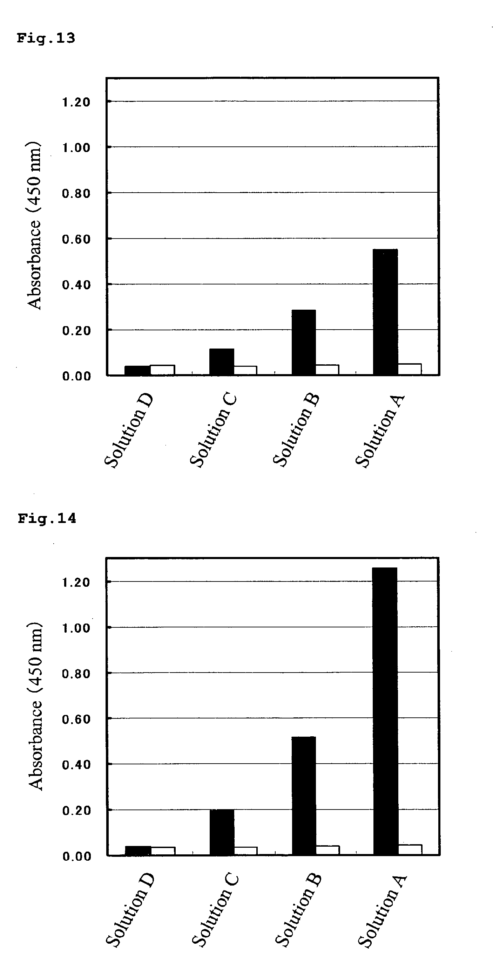

Fig. 13 shows results of experiments using a methylated oligonucleotide GPR-M8 having the nucleotide sequence of SEQ ID NO: 47, and an unmethylated oligonucleotide GPR-U8 having the nucleotide sequence of SEQ ID NO: 50 in Example 6. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide GPR-M8 having the nucleotide sequence of SEQ ID NO: 47, and a residual amount (complex formation amount) of an unmethylated oligonucleotide GPR-U8 having the nucleotide sequence of SEQ ID NO: 50 measured by absorbance (450 nm) are shown in this order from the left-hand.
Fig. 14 shows results of experiments using a methylated oligonucleotide FBN-M8 having the nucleotide sequence of SEQ ID NO: 48, and an unmethylated oligonucleotide FBN-U8 having the nucleotide sequence of SEQ ID NO: 51 in Example 6. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide FBN-M8 having the nucleotide sequence of SEQ ID NO: 48, and a residual amount (complex formation amount) of an unmethylated oligonucleotide FBN-U8 having the nucleotide sequence of SEQ ID NO: 51 measured by absorbance (450 nm) are shown in this order from the left-hand.

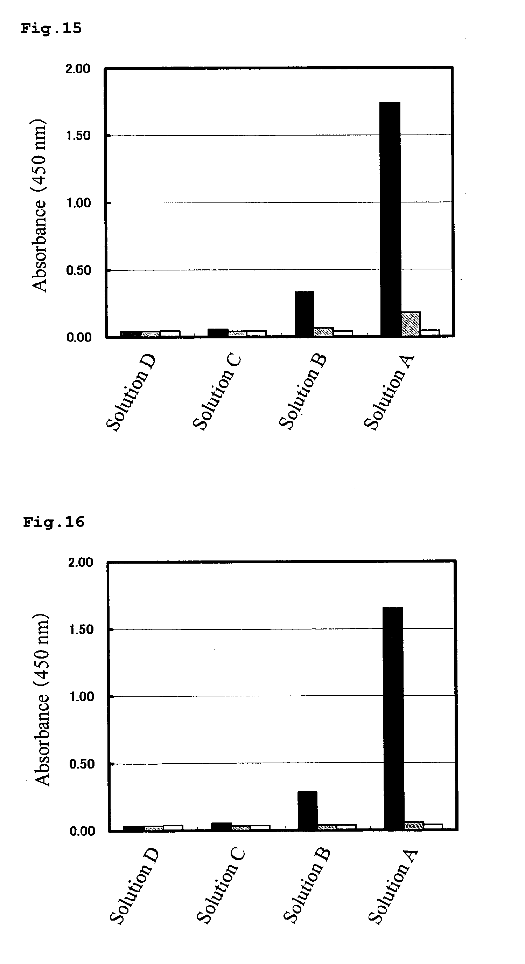

Fig. 15 shows results of Group A (no treatment group) in Example 7. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC-M having the nucleotide sequence of SEQ ID NO: 55, a residual amount (complex formation amount) of a partially methylated oligonucleotide UBC-HM having the nucleotide sequence of SEQ ID NO: 56, and a residual amount (complex formation amount) of an unmethylated oligonucleotide UBC-UM having the nucleotide sequence of SEQ ID NO: 57 measured by fluorescence (612 nm) are shown in this order from the left-hand.
Fig. 16 shows results of Group B (Hha treatment group) in Example 7. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide UBC-M having the nucleotide sequence of SEQ ID NO: 55, a residual amount (complex formation amount) of a partially methylated oligonucleotide UBC-HM having the nucleotide sequence of SEQ ID NO: 56, and a residual amount (complex formation amount) of an unmethylated oligonucleotide UBC-UM having the nucleotide sequence of SEQ ID NO: 57 measured by fluorescence (612 nm) are shown in this order from the left-hand.

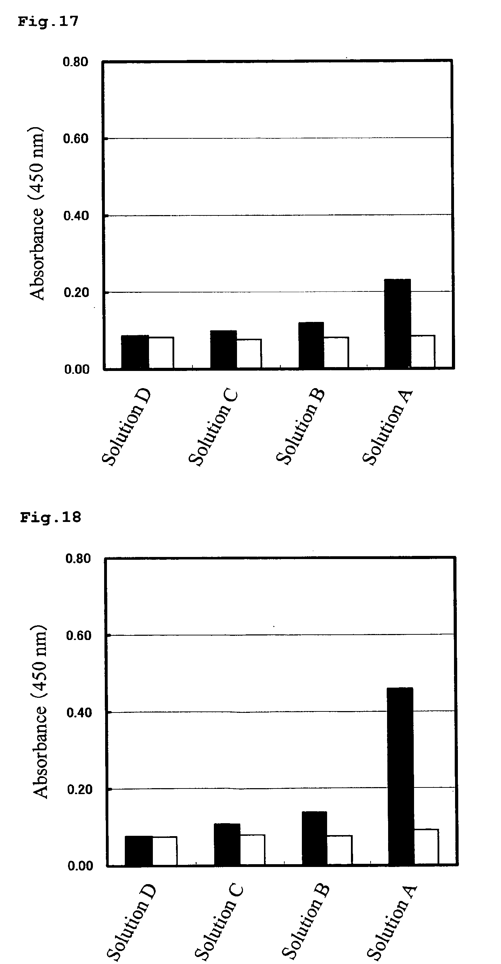

Fig. 17 shows results of experiments using an oligonucleotide UBC labeled with FITC at 5'-end having the nucleotide sequence of SEQ ID NO: 62 in Example 8. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount of a methylated oligonucleotide UBC/GPR/FBN-M having the nucleotide sequence of SEQ ID NO: 60, and a complex formation amount of an unmethylated oligonucleotide UBC/GPR/FBN-UM having the nucleotide sequence of SEQ ID NO: 61 measured by absorbance (450 nm) are shown in this order from the left-hand.
Fig. 18 shows results of experiments using an oligonucleotide GPR labeled with FITC at 5'-end having the nucleotide sequence of SEQ ID NO: 63 in Example 8. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount of a methylated oligonucleotide UBC/GPR/FBN-M having the nucleotide sequence of SEQ ID NO: 60, and a complex formation amount of an unmethylated oligonucleotide UBC/GPR/FBN-UM having the nucleotide sequence of SEQ ID NO: 61 measured by absorbance (450 nm) are shown in this order from the left-hand.

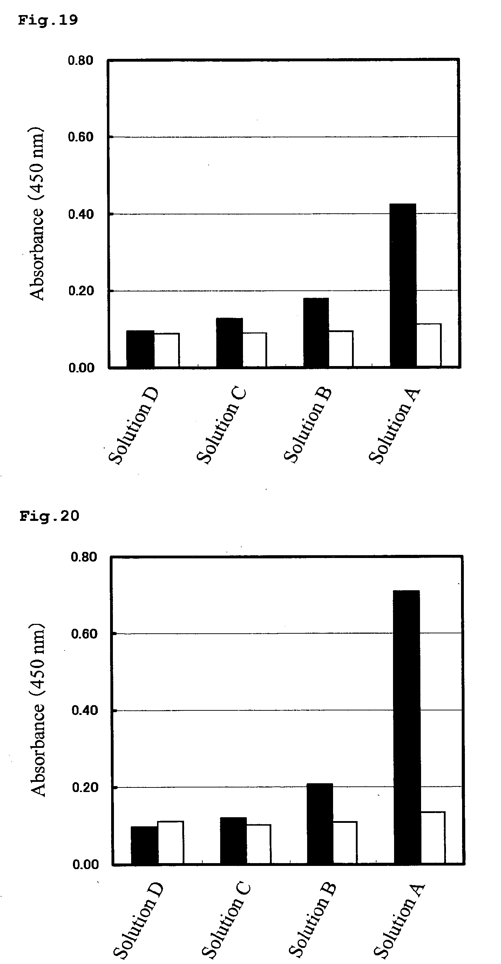

Fig. 19 shows results of experiments using an oligonucleotide FBN labeled with FITC at 5'-end having the nucleotide sequence of SEQ ID NO: 64 in Example 8. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount of a methylated oligonucleotide UBC/GPR/FBN-M having the nucleotide sequence of SEQ ID NO: 60, and a complex formation amount of an unmethylated oligonucleotide UBC/GPR/FBN-UM having the nucleotide sequence of SEQ ID NO: 61 measured by absorbance (450 nm) are shown in this order from the left-hand.
Fig. 20 shows results of experiments using a mixed solution of an oligonucleotide UBC labeled with FITC at 5'-end having the nucleotide sequence of SEQ ID NO: 62, an oligonucleotide GPR labeled with FITC at 5'-end having the nucleotide sequence of SEQ ID NO: 63, and an oligonucleotide FBN labeled with FITC at 5'-end having the nucleotide sequence of SEQ ID NO: 64 in Example 8. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount of a methylated oligonucleotide UBC/GPR/FBN-M having the nucleotide sequence of SEQ ID NO: 60, and a complex formation amount of an unmethylated oligonucleotide UBC/GPR/FBN-UM having the nucleotide sequence of SEQ ID NO: 61 measured by absorbance (450 nm) are shown in this order from the left-hand.

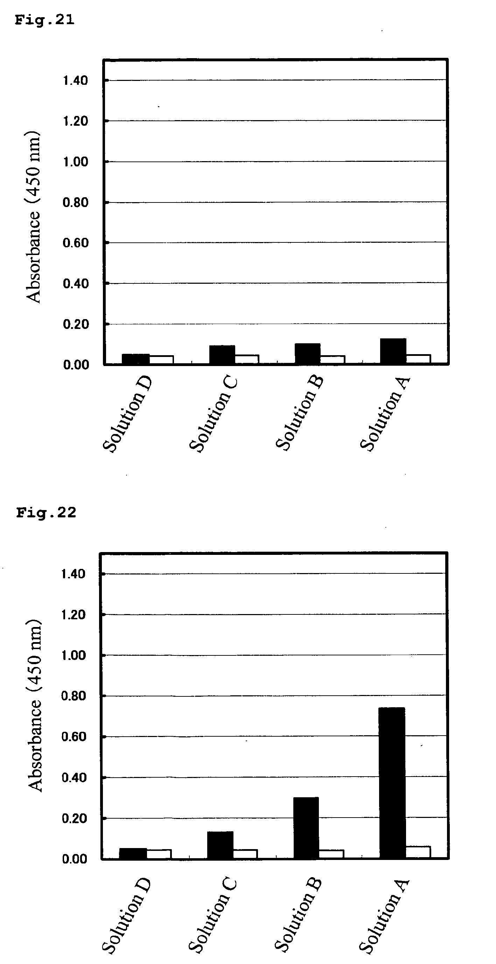

Fig. 21 shows results of experiments using an oligonucleotide UBC labeled with FITC at 5'-end having the nucleotide sequence of SEQ ID NO: 71 in Example 9. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution M0, and a complex formation amount in an unmethylated oligonucleotide mixed solution U0 measured by absorbance (450 nm) are shown in this order from the left-hand.
Fig. 22 shows results of experiments using an oligonucleotide FBN labeled with FITC at 5'-end having the nucleotide sequence of SEQ ID NO: 73 in Example 9. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution M0, and a complex formation amount in an unmethylated oligonucleotide mixed solution U0 measured by absorbance (450 nm) are shown in this order from the left-hand.

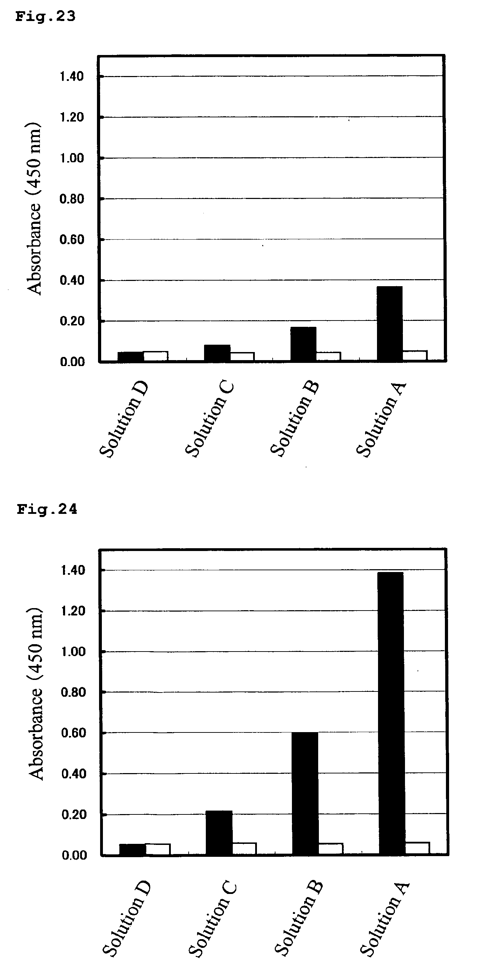

Fig. 23 shows results of experiments using an oligonucleotide GPR labeled with FITC at 5'-end having the nucleotide sequence of SEQ ID NO: 72 in Example 9. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution M0, and a complex formation amount in an unmethylated oligonucleotide mixed solution U0 measured by absorbance (450 nm) are shown in this order from the left-hand.
Fig. 24 shows results of experiments using a mixed solution of an oligonucleotide UBC labeled with FITC at 5'-end having the nucleotide sequence of SEQ ID NO: 71, an oligonucleotide GPR labeled with FITC at 5'-end having the nucleotide sequence of SEQ ID NO: 72, and an oligonucleotide FBN labeled with FITC at 5'-end having the nucleotide sequence of SEQ ID NO: 73 in Example 9. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a complex formation amount in a methylated oligonucleotide mixed solution M0, and a complex formation amount in an unmethylated oligonucleotide mixed solution U0 measured by absorbance (450 nm) are shown in this order from the left-hand.

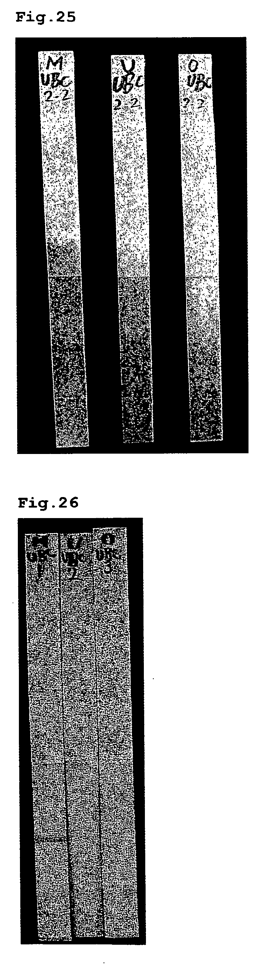

Fig. 25 shows results of Example 10. From the left-hand of the drawing, a chromatostrip (M) tested and developed using a solution of a methylated oligonucleotide UBC-M8 having the nucleotide sequence of SEQ ID NO: 74, a chromatostrip (U) tested and developed using a solution of an unmethylated oligonucleotide UBC-U8 having the nucleotide sequence of SEQ ID NO: 75, and a chromatostrip (O) tested and developed using a TE buffer solution are shown.
Fig. 26 shows results obtained by using an oligonucleotide UBC labeled with FITC at 5'-end having the nucleotide sequence of SEQ ID NO: 84 as a 5'-end FITC-labeled oligonucleotide in Example 11. From the left-hand of the drawing, a chromatostrip (M) tested and developed using a solution of a methylated oligonucleotide UBC-M8 having the nucleotide sequence of SEQ ID NO: 78, a chromatostrip (U) tested and developed using a solution of an unmethylated oligonucleotide UBC-U8 having the nucleotide sequence of SEQ ID NO: 81, and a chromatostrip (O) tested and developed using a TE buffer solution are shown.

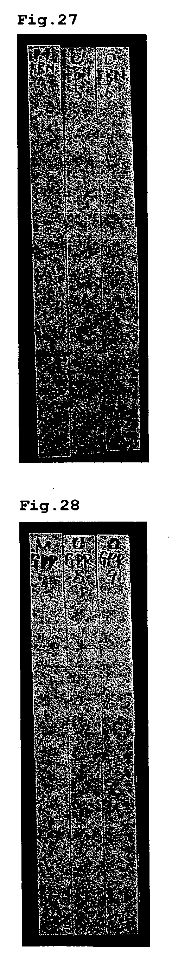

Fig. 27 shows results obtained by using an oligonucleotide FBN labeled with FITC at 5'-end having the nucleotide sequence of SEQ ID NO: 85 as a 5'-end FITC-labeled oligonucleotide in Example 11. From the left-hand of the drawing, a chromatostrip (M) tested and developed using a solution of a methylated oligonucleotide FBN-M8 having the nucleotide sequence of SEQ ID NO: 79, a chromatostrip (U) tested and developed using a solution of an unmethylated oligonucleotide FBN-U8 having the nucleotide sequence of SEQ ID NO: 82, and a chromatostrip (O) tested and developed using a TE buffer solution are shown.
Fig. 28 shows results obtained by using an oligonucleotide GPR labeled with FITC at 5'-end having the nucleotide sequence of SEQ ID NO: 86 as a 5'-end FITC-labeled oligonucleotide in Example 11. From the left-hand of the drawing, a chromatostrip (M) tested and developed using a solution of a methylated oligonucleotide GPR-M8 having the nucleotide sequence of SEQ ID NO: 80, a chromatostrip (U) tested and developed using a solution of an unmethylated oligonucleotide GPR-U8 having the nucleotide sequence of SEQ ID NO: 83, and a chromatostrip (O) tested and developed using a TE buffer solution are shown.

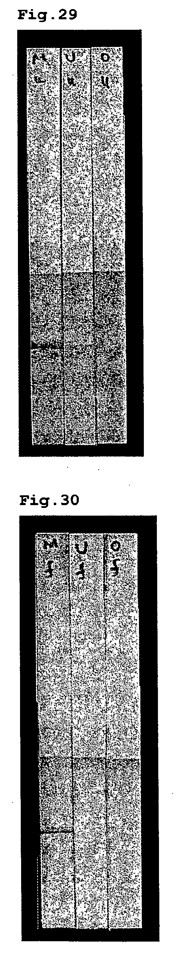

Fig. 29 shows results obtained by using an oligonucleotide UBC labeled with FITC at 5'-end having the nucleotide sequence of SEQ ID NO: 94 as a 5'-end FITC-labeled oligonucleotide in Example 12. From the left-hand of the drawing, a chromatostrip (M) tested and developed using a solution of a methylated oligonucleotide UBC-M8 having the nucleotide sequence of SEQ ID NO: 88, a chromatostrip (U) tested and developed using a solution of an unmethylated oligonucleotide UBC-U8 having the nucleotide sequence of SEQ ID NO: 91, and a chromatostrip (O) tested and developed using a TE buffer solution are shown.
Fig. 30 shows results obtained by using an oligonucleotide FBN labeled with FITC at 5'-end having the nucleotide sequence of SEQ ID NO: 95 as a 5'-end FITC-labeled oligonucleotide in Example 12. From the left-hand of the drawing, a chromatostrip (M) tested and developed using a solution of a methylated oligonucleotide FBN-M8 having the nucleotide sequence of SEQ ID NO: 89, a chromatostrip (U) tested and developed using a solution of an unmethylated oligonucleotide FBN-U8 having the nucleotide sequence of SEQ ID NO: 92, and a chromatostrip (O) tested and developed using a TE buffer solution are shown.

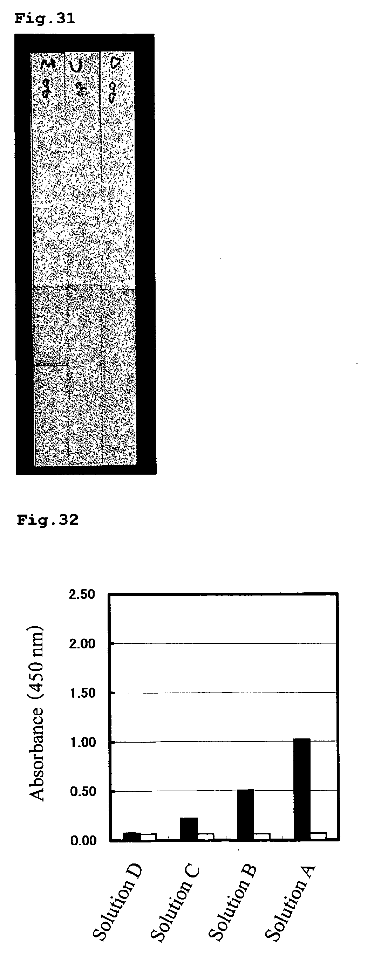

Fig. 31 shows results obtained by using an oligonucleotide GPR labeled with FITC at 5'-end having the nucleotide sequence of SEQ ID NO: 96 as a 5'-end FITC-labeled oligonucleotide in Example 12. From the left-hand of the drawing, a chromatostrip (M) tested and developed using a solution of a methylated oligonucleotide GPR-M8 having the nucleotide sequence of SEQ ID NO: 90, a chromatostrip (U) tested and developed using a solution of an unmethylated oligonucleotide GPR-U8 having the nucleotide sequence of SEQ ID NO: 93, and a chromatostrip (O) tested and developed using a TE buffer solution are shown.
Fig. 32 shows results of experiments using Buffer 1 in Example 13. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide FBN-M8 having the nucleotide sequence of SEQ ID NO: 98, and a residual amount (complex formation amount) of an unmethylated oligonucleotide FBN-U8 having the nucleotide sequence of SEQ ID NO: 99 measured by absorbance (450 nm) are shown in this order from the left-hand.

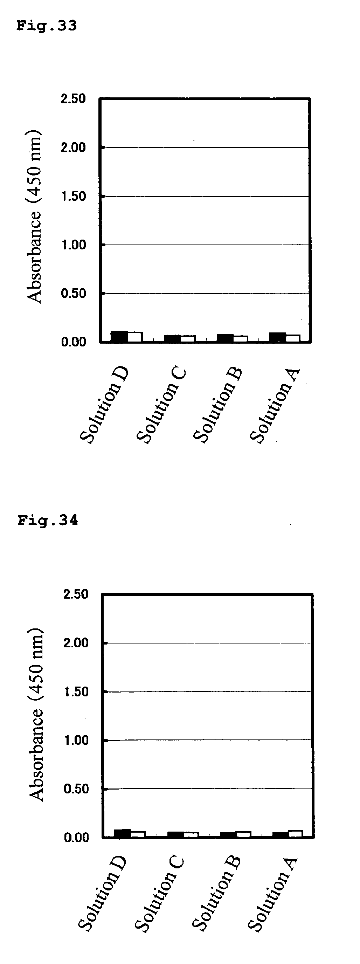

Fig. 33 shows results of experiments using Buffer 2 in Example 13. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide FBN-M8 having the nucleotide sequence of SEQ ID NO: 98, and a residual amount (complex formation amount) of an unmethylated oligonucleotide FBN-U8 having the nucleotide sequence of SEQ ID NO: 99 measured by absorbance (450 nm) are shown in this order from the left-hand.
Fig. 34 shows results of experiments using Buffer 3 in Example 13. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide FBN-M8 having the nucleotide sequence of SEQ ID NO: 98, and a residual amount (complex formation amount) of an unmethylated oligonucleotide FBN-U8 having the nucleotide sequence of SEQ ID NO: 99 measured by absorbance (450 nm) are shown in this order from the left-hand.

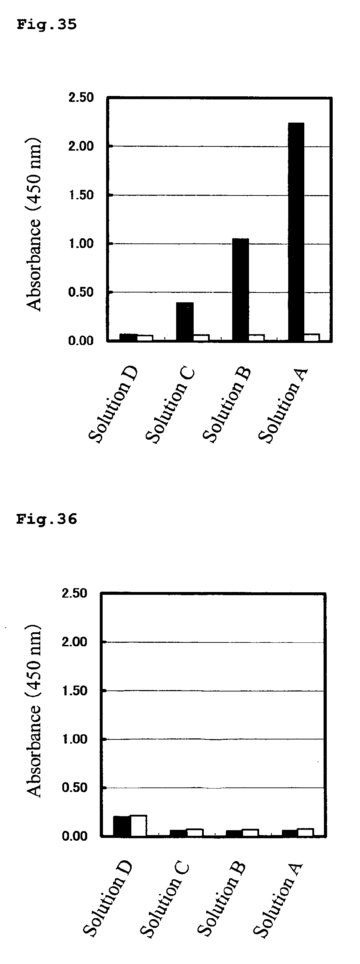

Fig. 35 shows results of experiments using Buffer 4 in Example 13. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide FBN-M8 having the nucleotide sequence of SEQ ID NO: 98, and a residual amount (complex formation amount) of an unmethylated oligonucleotide FBN-U8 having the nucleotide sequence of SEQ ID NO: 99 measured by absorbance (450 nm) are shown in this order from the left-hand.
Fig. 36 shows results of experiments using Buffer 5 in Example 13. For each of Solution A (0.1 pmol/100 µL solution in TE buffer), Solution B (0.01 pmol/100 µL solution in TE buffer), Solution C (0.001 pmol/100 µL solution in TE buffer), and Solution D (0 pmol/100 µL solution in TE buffer (negative control solution)), a residual amount (complex formation amount) of a methylated oligonucleotide FBN-M8 having the nucleotide sequence of SEQ ID NO: 98, and a residual amount (complex formation amount) of an unmethylated oligonucleotide FBN-U8 having the nucleotide sequence of SEQ ID NO: 99 measured by absorbance (450 nm) are shown in this order from the left-hand.

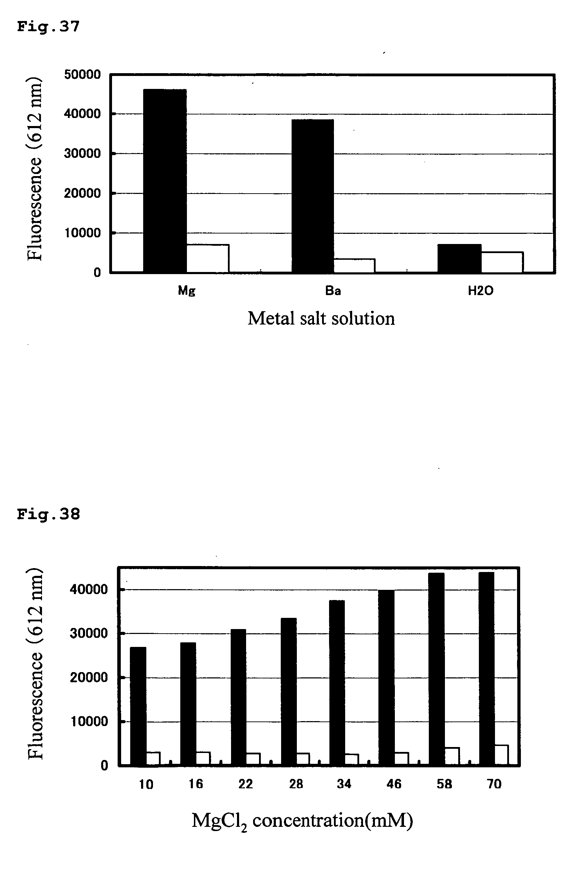

Fig. 37 shows results of Example 14. For each of Metal salt solution Mg (100 mM MgCl₂ aqueous solution), Metal salt solution Ba (100 mM BaCl₂ aqueous solution), and Metal salt solution H₂O (ultrapure water), a residual amount (complex formation amount) of a methylated oligonucleotide UBC-M8 having the nucleotide sequence of SEQ ID NO: 102, and a residual amount (complex formation amount) of an unmethylated oligonucleotide UBC-U8 having the nucleotide sequence of SEQ ID NO: 103 measured by fluorescence (612 nm) are shown in this order from the left-hand.
Fig. 38 shows results of Example 15. For each of 10, 16, 22, 28, 34, 46, 58, 70 mM MgCl₂ solutions (final concentration), a residual amount (complex formation amount) of a methylated oligonucleotide FBN-M8 having the nucleotide sequence of SEQ ID NO: 105, and a residual amount (complex formation amount) of an unmethylated oligonucleotide FBN-U8 having the nucleotide sequence of SEQ ID NO: 106 measured by fluorescence (612 nm) are shown in this order from the left-hand.

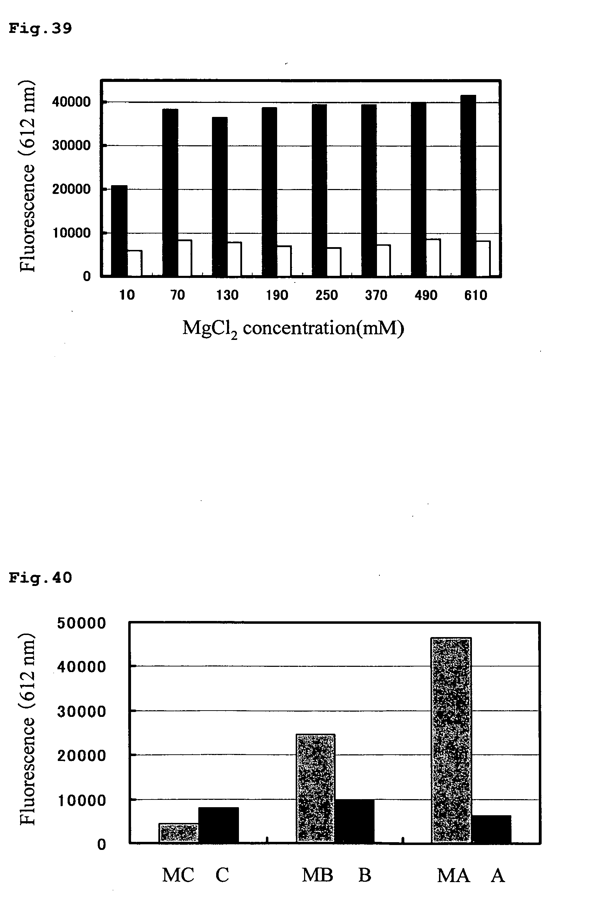

Fig. 39 shows results of Example 16. For each of 10, 70, 130, 190, 250, 370, 490, 610 mM MgCl₂ solutions (final concentration), a residual amount (complex formation amount) of a methylated oligonucleotide FBN-M8 having the nucleotide sequence of SEQ ID NO: 108, and a residual amount (complex formation amount) of an unmethylated oligonucleotide FBN-U8 having the nucleotide sequence of SEQ ID NO: 109 measured by fluorescence (612 nm) are shown in this order from the left-hand.
Fig. 40 shows results of Example 17. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB (1 ng/10 uL solution in TE buffer), Solution B (1 ng/10 µL solution in TE buffer), Solution MC (0 ng/10 µL solution in TE buffer (negative control solution)), and Solution C (0 ng/10 µL solution in TE buffer (negative control solution)).

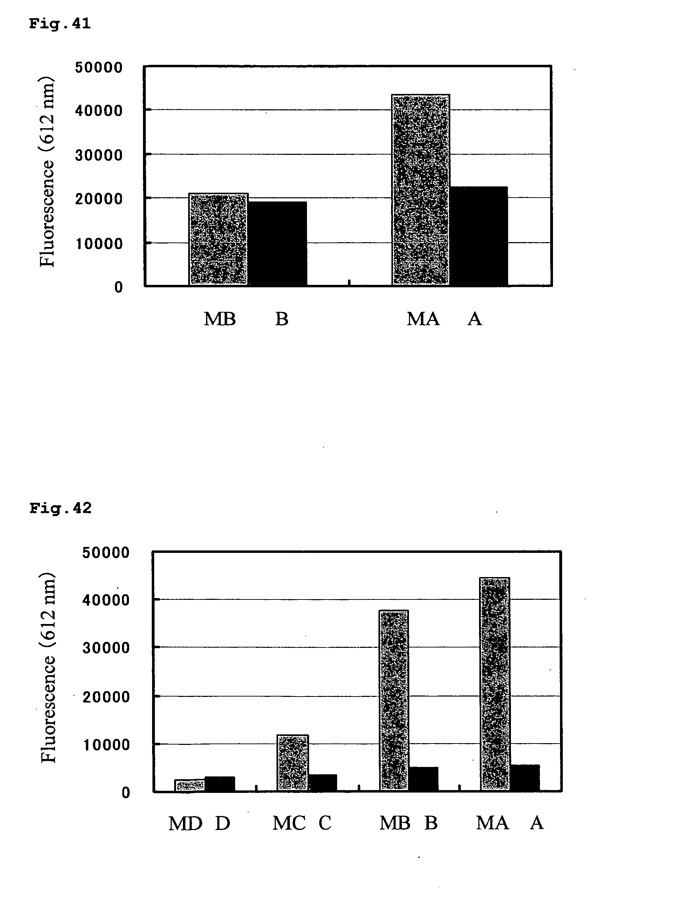

Fig. 41 shows results of Example 18. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB (0 ng/0 µL solution in TE buffer (negative control solution)) and Solution B (0 ng/10 µL solution in TE buffer (negative control solution)).
Fig. 42 shows results of Example 19. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB (1 ng/10 µL solution in TE buffer), Solution B (1 ng/10 µL solution in TE buffer), Solution MC (0.1 ng/10 µL solution in TE buffer), Solution C (0.1 ng/10 µL solution in TE buffer), Solution MD (0 ng/10 µL solution in TE buffer (negative control solution)), and Solution D (0 ng/10 µL solution in TE buffer (negative control solution)).

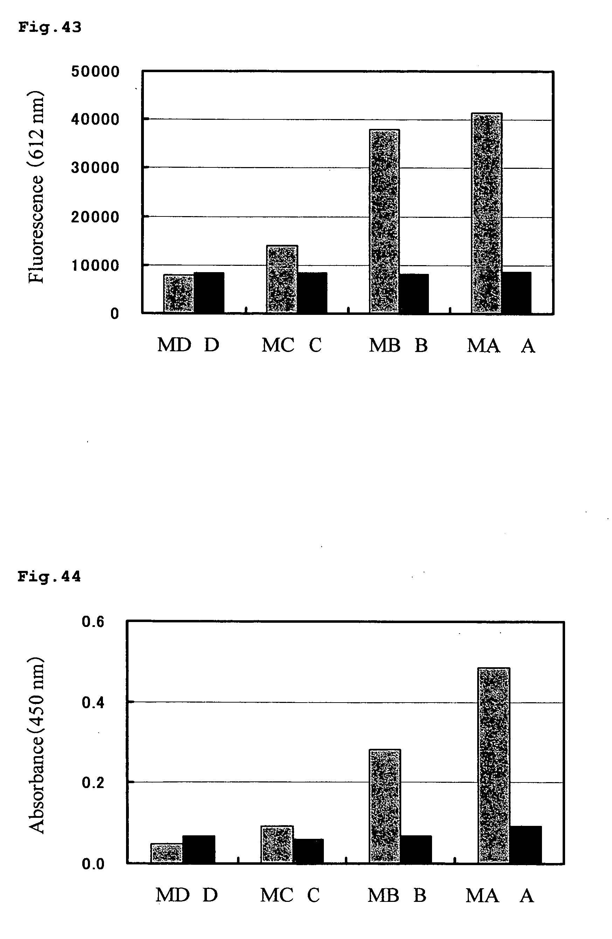

Fig. 43 shows results of Example 20. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB.(1 ng/10 µL solution in TE buffer), Solution B (1 ng/10 µL solution in TE buffer), Solution MC (0.1 ng/10 µL solution in TE buffer), Solution C (0.1 ng/10 µL solution in TE buffer), Solution MD (0 ng/10 µL solution in TE buffer (negative control solution)), and Solution D (0 ng/10 µL solution in TE buffer (negative control solution)).
Fig. 44 shows results of Example 21. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB (1 ng/10 µL solution in TE buffer), Solution B (1 ng/10 µL solution in TE buffer), Solution MC (0.1 ng/10 µL solution in TE buffer), Solution C (0.1 ng/10 µL solution in TE buffer), Solution MD (0 ng/10 µL solution in TE buffer (negative control solution)), and Solution D (0 ng/10 µL solution in TE buffer (negative control solution)).

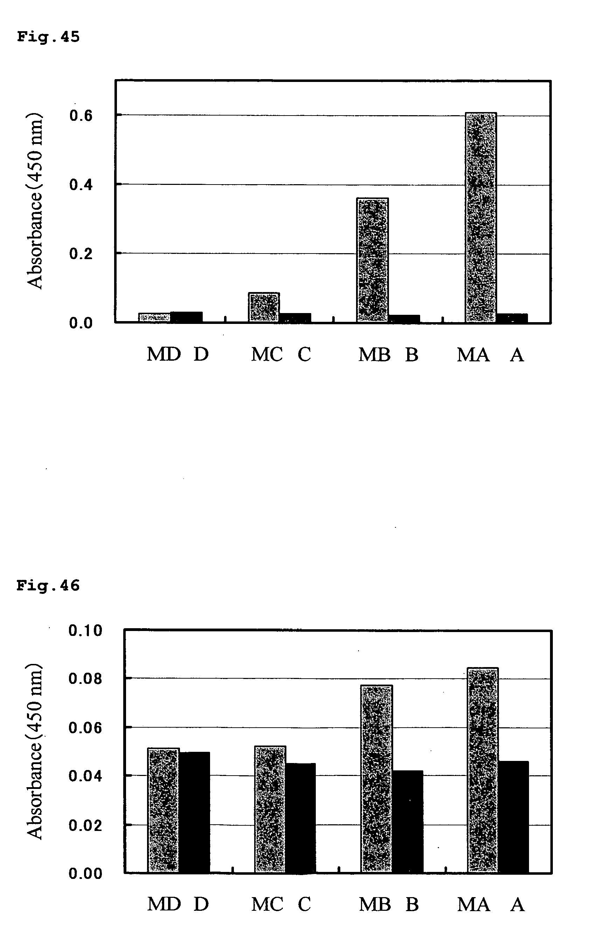

Fig. 45 shows results of Example 22. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB (1 ng/10 µL solution in TE buffer), Solution B (1 ng/10 µL solution in TE buffer), Solution MC (0.1 ng/10 µL solution in TE buffer), Solution C (0.1 ng/10 µL solution in TE buffer), Solution MD (0 ng/10 µL solution in TE buffer (negative control solution)), and Solution D (0 ng/10 µL solution in TE buffer (negative control solution)).
Fig. 46 shows results of Example 23. From the left hand, a formation amount of a complex measured by fluorescence (excitation 340 nm/detection 612 nm) is shown for each of Solution MA (10 ng/10 µL solution in TE buffer), Solution A (10 ng/10 µL solution in TE buffer), Solution MB (1 ng/10 µL solution in TE buffer), Solution B (1 ng/10 µL solution in TE buffer), Solution MC (0.1 ng/10 µL solution in TE buffer), Solution C (0.1 ng/10 µL solution in TE buffer), Solution MD (0 ng/10 µL solution in TE buffer (negative control solution)), and Solution D (0 ng/10 µL solution in TE buffer (negative control solution)).

### BEST MODE FOR CARRYING OUT THE INVENTION

It is known that DNA methylation abnormality occurs in various diseases (for example, cancer), and it is believed that the degree of various diseases can be measured by detecting this DNA methylation abnormality.

For example, when there is a region where methylation occurs at 100% in a specimen derived from a diseased organism, and the present measuring method is executed for the region, the amount of methylated DNA would increase. For example, when there is a region where methylation does not occur at 100% in a specimen derived from a diseased organism, and the present measuring method is executed for the region, the amount of methylated DNA would be approximately 0. For example, when there is a region where the methylation rate is low in in a specimen derived from a healthy organism, and a region where the methylation rate is high in a specimen derived from a diseased organism, and the present measuring method is executed for the region, the amount of methylated DNA would be approximately 0 for a healthy subject, and a significantly higher value than that of a healthy subject would be exhibited by a disease subject, so that the "degree of disease" can be determined based on this difference in value. The "degree of disease" used herein has the same meaning as those commonly used in this field of art, and concretely means, for example, malignancy when the biological specimen is a cell, and means, for example, abundance of disease cells in the tissue when the biological specimen is a tissue. Further, when the biological specimen is plasma or serum, it means the probability that the individual has a disease. Therefore, the present measuring method makes it possible to diagnose various diseases by examining methylation abnormality.

As the "biological specimen" in the present invention, for example, a cell lysate, a tissue lysate (here the term "tissue" is used in a broad sense including blood, lymph node and so on) or biological samples including bodily sections such as plasma, serum and lymph, bodily secretions (urine, milk and so on) and the like and a genomic DNA obtained by extracting these biological samples, in mammals can be recited. As a biological specimen, for example, samples derived from microorganisms, viruses and the like can be recited, and in such a case, "a genomic DNA" in the present measuring method also means genomic DNA of microorganisms, viruses and the like.

When the specimen derived from a mammal is blood, use of the present measuring method in a regular health check or a simple examination is expected.

For obtaining a genomic DNA from a specimen derived from a mammal, for example, DNA may be extracted using a commercially available DNA extraction kit.

When blood is used as a specimen, plasma or serum is prepared from blood in accordance with a commonly used method, and using the prepared plasma or serum as a specimen, free DNA (including DNA derived from cancer cells such as gastric cancer cells) contained in the specimen is analyzed. This enables analysis of DNA derived from cancer cells such as gastric cancer cells while avoiding DNA derived from hemocytes, and improves the sensitivity of detection of cancer cells such as gastric cancer cells and a tissue containing the same.

Usually, a gene (a genomic DNA) consists of four kinds of bases. In these bases, such a phenomenon is known that only cytosine is methylated, and such methylation modification of DNA is limited to cytosine in a nucleotide sequence represented by 5'-CG-3' (C represents cytosine, and G represents guanine. Hereinafter, the nucleotide sequence is also referred to as "CpG"). The site to be methylated in cytosine is its position 5. In DNA replication prior to cell division, only cytosine in "CpG" of a template chain is methylated immediately after replication, however, cytosine in "CpG" of a newly-generated strand is immediately methylated by the action of methyltransferase. Therefore, the methylation state of DNA will be passed to new two sets of DNA even after DNA replication. The term "methylated DNA" in the present invention means DNA occurring by such methylation modification.

The term "CpG pair" in the present invention means double-stranded oligonucleotide in which a nucleotide sequence represented by CpG and a CpG that is complement with this are base-paired.

The term "objective DNA region" (hereinafter, also referred to as an "objective region") used in the present invention means a DNA region for which presence or absence of methylation of cytosine included in the region is to be examined, and has a recognition site of at least one kind of methylation sensitive restriction enzyme. A DNA region containing at least one cytosine in a nucleotide sequence represented by CpG which is present in a nucleotide sequence of a promoter region, an untranslated region, or a translated region (coding region) of a useful protein gene such as Lysyl oxidase, HRAS-like suppressor, bA305P22.2.1, Gamma filamin, HAND1, Homologue of RIKEN 2210016F16, FLJ32130, PPARG angiopoietin-related protein, Thrombomodulin, p53-responsive gene 2, Fibrillin2, Neurofilament3, disintegrin and metalloproteinase domain 23, G protein-coupled receptor 7, G-protein coupled somatostatin and angiotensin-like peptide receptor, Solute carrier family 6 neurotransmitter transporter noradrenalin member 2 and so on can be recited.

To be more specific, when the useful protein gene is a Lysyl oxidase gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Lysyl oxidase gene derived from human, and a promoter region located 5' upstream of the same can be recited, and more concretely, the nucleotide sequence of SEQ ID NO: 1 (corresponding to a nucleotide sequence represented by base No. 16001 to 18661 in the nucleotide sequence described in Genbank Accession No. AF270645) can be recited. In the nucleotide sequence of SEQ ID NO: 1, ATG codon encoding methionine at amino terminal of Lysyl oxidase protein derived from human is represented in base No. 2031 to 2033, and a nucleotide sequence of the above exon 1 is represented in base No. 1957 to 2661. Cytosine in the nucleotide sequence represented by CpG which is present in the nucleotide sequence of SEQ ID NO: 1, in particular, cytosine in CpG which is present in a region where CpGs are densely present in the nucleotide sequence of SEQ ID NO: 1 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1560, 1574, 1600, 1623, 1635, 1644, 1654, 1661, 1682, 1686, 1696, 1717, 1767, 1774, 1783, 1785, 1787, 1795 and so on in the nucleotide sequence of SEQ ID NO: 1 can be recited.

To be more specific, when the useful protein gene is a HRAS-like suppressor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HRAS-like suppressor gene derived from human, and a promoter region located 5' upstream of the same can be recited, and more concretely, the nucleotide sequence of SEQ ID NO: 2 (corresponding to a nucleotide sequence represented by base No. 172001 to 173953 in the nucleotide sequence described in Genbank Accession No. AC068162) can be recited. In the nucleotide sequence of SEQ ID NO: 2, the nucleotide sequence of exon 1 of a HRAS-like suppressor gene derived from human is represented in base No. 1743 to 1953. Cytosine in the nucleotide sequence represented by CpG which is present in the nucleotide sequence of SEQ ID NO: 2, in particular, cytosine in CpG which is present in a region where CpGs are densely present in the nucleotide sequence of SEQ ID NO: 2 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1316, 1341, 1357, 1359, 1362, 1374, 1390, 1399, 1405, 1409, 1414, 1416, 1422, 1428, 1434, 1449, 1451, 1454, 1463, 1469, 1477, 1479, 1483, 1488, 1492, 1494, 1496, 1498, 1504, 1510, 1513, 1518, 1520 and so on in the nucleotide sequence of SEQ ID NO: 2 can be recited.

To be more specific, when the useful protein gene is a bA305P22.2.1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a bA305P22.2.1 gene derived from human, and a promoter region located 5' upstream of the same can be recited, and more concretely, the nucleotide sequence of SEQ ID NO: 3 (corresponding to a nucleotide sequence represented by base No. 13001 to 13889 in the nucleotide sequence described in Genbank Accession No. AL121673) can be recited. In the nucleotide sequence of SEQ ID NO: 3, ATG codon encoding methionine at amino terminal of bA305P22.2.1 protein derived from human is represented in base No. 849 to 851, and a nucleotide sequence of the above exon 1 is represented in base No. 663 to 889. Cytosine in the nucleotide sequence represented by CpG which is present in the nucleotide sequence of SEQ ID NO: 3, in particular, cytosine in CpG which is present in a region where CpGs are densely present in the nucleotide sequence of SEQ ID NO: 3 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 335, 337, 351, 363, 373, 405, 424, 427, 446, 465, 472, 486 and so on in the nucleotide sequence of SEQ ID NO: 3 can be recited.

To be more specific, when the useful protein gene is a Gamma filamin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Gamma filamin gene derived from human, and a promoter region located 5' upstream of the same can be recited, and more concretely, the nucleotide sequence of SEQ ID NO: 4 (corresponding to a complementary sequence to a nucleotide sequence represented by base No. 63528 to 64390 in the nucleotide sequence described in Genbank Accession No. AC074373) can be recited. In the nucleotide sequence of SEQ ID NO: 4, ATG codon encoding methionine at amino terminal of Gamma filamin protein derived from human is represented in base No. 572 to 574, and a nucleotide sequence of the above exon 1 is represented in base No. 463 to 863. Cytosine in the nucleotide sequence represented by CpG which is present in the nucleotide sequence of SEQ ID NO: 4, in particular, cytosine in CpG which is present in a region where CpGs are densely present in the nucleotide sequence of SEQ ID NO: 4 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 333, 337, 350, 353, 360, 363, 370, 379, 382, 384, 409, 414, 419, 426, 432, 434, 445, 449, 459, 472, 474, 486, 490, 503, 505 and so on in the nucleotide sequence of SEQ ID NO: 4 can be recited.

To be more specific, when the useful protein gene is a HAND1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HAND1 gene derived from human, and a promoter region located 5' upstream of the same can be recited, and more concretely, the nucleotide sequence of SEQ ID NO: 5 (corresponding to a complementary sequence to a nucleotide sequence represented by base No. 24303 to 26500 in the nucleotide sequence described in Genbank Accession No. AC026688) can be recited. In the nucleotide sequence of SEQ ID NO: 5, ATG codon encoding methionine at amino terminal of HAND1 protein derived from human is represented in base No. 1656 to 1658, and a nucleotide sequence of the above exon 1 is represented in base No. 1400 to 2198. Cytosine in the nucleotide sequence represented by CpG which is present in the nucleotide sequence of SEQ ID NO: 5, in particular, cytosine in CpG which is present in a region where CpGs are densely present in the nucleotide sequence of SEQ ID NO: 5 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1153, 1160, 1178, 1187, 1193, 1218, 1232, 1266, 1272, 1292, 1305, 1307, 1316, 1356, 1377, 1399, 1401, 1422, 1434 and so on in the nucleotide sequence of SEQ ID NO: 5 can be recited.

To be more specific, when the useful protein gene is a Homologue of RIKEN 2210016F16 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Homologue of RIKEN 2210016F16 gene derived from human, and a promoter region located 5' upstream of the same can be recited, and more concretely, the nucleotide sequence of SEQ ID NO: 6 (corresponding to a complementary nucleotide sequence to a nucleotide sequence represented by base No. 157056 to 159000 in the nucleotide sequence described in Genbank Accession No. AL354733) can be recited. In the nucleotide sequence of SEQ ID NO: 6, a nucleotide sequence of exon 1 of a Homologue of a RIKEN 2210016F16 gene derived from human is represented in base No. 1392 to 1945. Cytosine in the nucleotide sequence represented by CpG which is present in the nucleotide sequence of SEQ ID NO: 6, in particular, cytosine in CpG which is present in a region where CpGs are densely present in the nucleotide sequence of SEQ ID NO: 6 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1172, 1175, 1180, 1183, 1189, 1204, 1209, 1267, 1271, 1278, 1281, 1313, 1319, 1332, 1334, 1338, 1346, 1352, 1358, 1366, 1378, 1392, 1402, 1433, 1436, 1438 and so on in the nucleotide sequence of SEQ ID NO: 6 can be recited.

To be more specific, when the useful protein gene is a FLJ32130 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a FLJ32130 gene derived from human, and a promoter region located 5' upstream of the same can be recited, and more concretely, the nucleotide sequence of SEQ ID NO: 7 (corresponding to a complementary nucleotide sequence to a nucleotide sequence represented by base No. 1 to 2379 in the nucleotide sequence described in Genbank Accession No. AC002310) can be recited. In the nucleotide sequence of SEQ ID NO: 7, ATG codon encoding methionine at amino terminal of FLJ32130 protein derived from human is represented in base No. 2136 to 2138, and a nucleotide sequence assumed to be the above exon 1 is represented in base No. 2136 to 2379. Cytosine in the nucleotide sequence represented by CpG which is present in the nucleotide sequence of SEQ ID NO: 7, in particular, cytosine in CpG which is present in a region where CpGs are densely present in the nucleotide sequence of SEQ ID NO: 7 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1714, 1716, 1749, 1753, 1762, 1795, 1814, 1894, 1911, 1915, 1925, 1940, 1955, 1968 and so on in the nucleotide sequence of SEQ ID NO: 7 can be recited.

To be more specific, when the useful protein gene is a PPARG angiopoietin-related protein gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a PPARG angiopoietin-related protein gene derived from human, and a promoter region located 5' upstream of the same can be recited, and more concretely, the nucleotide sequence of SEQ ID NO: 8 can be recited. In the nucleotide sequence of SEQ ID NO: 8, ATG codon encoding methionine at amino terminal of PPARG angiopoietin-related protein derived from human is represented in base No. 717 to 719, and a nucleotide sequence of the 5' side part of the above exon 1 is represented in base No. 1957 to 2661. Cytosine in the nucleotide sequence represented by CpG which is present in the nucleotide sequence of SEQ ID NO: 8, in particular, cytosine in CpG which is present in a region where CpGs are densely present in the nucleotide sequence of SEQ ID NO: 8 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 35, 43, 51, 54, 75, 85, 107, 127, 129, 143, 184, 194, 223, 227, 236, 251, 258 and so on in the nucleotide sequence of SEQ ID NO: 8 can be recited.

To be more specific, when the useful protein gene is a Thrombomodulin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Thrombomodulin gene derived from human, and a promoter region located 5' upstream of the same can be recited, and more concretely, the nucleotide sequence of SEQ ID NO: 9 (corresponding to a nucleotide sequence represented by base No. 1 to 6096 in the nucleotide sequence described in Genbank Accession No. AF495471) can be recited. In the nucleotide sequence of SEQ ID NO: 9, ATG codon encoding methionine at amino terminal of Thrombomodulin protein derived from human is represented in base No. 2590 to 2592, and a nucleotide sequence of the above exon 1 is represented in base No. 2048 to 6096. Cytosine in the nucleotide sequence represented by CpG which is present in the nucleotide sequence of SEQ ID NO: 9, in particular, cytosine in CpG which is present in a region where CpGs are densely present in the nucleotide sequence of SEQ ID NO: 9 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1551, 1571, 1579, 1581, 1585, 1595, 1598, 1601, 1621, 1632, 1638, 1645, 1648, 1665, 1667, 1680, 1698, 1710, 1724, 1726, 1756 and so on in the nucleotide sequence of SEQ ID NO: 9 can be recited.

To be more specific, when the useful protein gene is a p53-responsive gene 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a p53-responsive gene 2 gene derived from human, and a promoter region located 5' upstream of the same can be recited, and more concretely, the nucleotide sequence of SEQ ID NO: 10 (corresponding to a complementary sequence to a nucleotide sequence represented by base No. 113501 to 116000 in the nucleotide sequence described in Genbank Accession No. AC009471) can be recited. In the nucleotide sequence of SEQ ID NO: 10, a nucleotide sequence of exon 1 of a p53-responsive gene 2 gene derived from human is represented in base No. 1558 to 1808. Cytosine in the nucleotide sequence represented by CpG which is present in the nucleotide sequence of SEQ ID NO: 10 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1282, 1284, 1301, 1308, 1315, 1319, 1349, 1351, 1357, 1361, 1365, 1378, 1383 and so on in the nucleotide sequence of SEQ ID NO: 10 can be recited.

To be more specific, when the useful protein gene is a Fibrillin2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Fibrillin2 gene derived from human, and a promoter region located 5' upstream of the same can be recited, and more concretely, the nucleotide sequence of SEQ ID NO: 11 (corresponding to a complementary sequence to a nucleotide sequence represented by base No. 118801 to 121000 in the nucleotide sequence described in Genbank Accession No. AC113387) can be recited. In the nucleotide sequence of SEQ ID NO: 11, a nucleotide sequence of exon 1, of a Fibrillin2 gene derived from human is represented in base No. 1091 to 1345. Cytosine in the nucleotide sequence represented by CpG which is present in the nucleotide sequence of SEQ ID NO: 11 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 679, 687, 690, 699, 746, 773, 777, 783, 795, 799, 812, 823, 830, 834, 843 and so on in the nucleotide sequence of SEQ ID NO: 11 can be recited.

To be more specific, when the useful protein gene is a Neurofilament3 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Neurofilament3 gene derived from human, and a promoter region located 5' upstream of the same can be recited, and more concretely, the nucleotide sequence of SEQ ID NO: 12 (corresponding to a complementary sequence to a nucleotide sequence represented by base No. 28001 to 30000 in the nucleotide sequence described in Genbank Accession No. AF106564) can be recited. In the nucleotide sequence of SEQ ID NO: 12, a nucleotide sequence of exon 1 of a Neurofilament3 gene derived from human is represented in base No. 614 to 1694. Cytosine in the nucleotide sequence represented by CpG which is present in the nucleotide sequence of SEQ ID NO: 12 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 428, 432, 443, 451, 471, 475, 482, 491, 499, 503, 506, 514, 519, 532, 541, 544, 546, 563, 566, 572, 580 and so on in the nucleotide sequence of SEQ ID NO: 12 can be recited.

To be more specific, when the useful protein gene is a disintegrin and metalloproteinase domain 23 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a disintegrin and metalloproteinase domain 23 gene derived from human, and a promoter region located 5' upstream of the same can be recited, and more concretely, the nucleotide sequence of SEQ ID NO: 13 (corresponding to a nucleotide sequence represented by base No. 21001 to 23300 in the nucleotide sequence described in Genbank Accession No. AC009225) can be recited. In the nucleotide sequence of SEQ ID NO: 13, a nucleotide sequence of exon 1 of a disintegrin and metalloproteinase domain 23 gene derived from human is represented in base No. 1194 to 1630. Cytosine in the nucleotide sequence represented by CpG which is present in the nucleotide sequence of SEQ ID NO: 13 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 998, 1003, 1007, 1011, 1016, 1018, 1020, 1026, 1028, 1031, 1035, 1041, 1043, 1045, 1051, 1053, 1056, 1060, 1066, 1068, 1070, 1073, 1093, 1096, 1106, 1112, 1120, 1124, 1126 and so on in the nucleotide sequence of SEQ ID NO: 13 can be recited.

To be more specific, when the useful protein gene is a G protein-coupled receptor 7 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G protein-coupled receptor 7 gene derived from human, and a promoter region located 5' upstream of the same can be recited, and more concretely, the nucleotide sequence of SEQ ID NO: 14 (corresponding to a nucleotide sequence represented by base No. 75001 to 78000 in the nucleotide sequence described in Genbank Accession No. AC009800) can be recited. In the nucleotide sequence of SEQ ID NO: 14, a nucleotide sequence of exon 1 of a G protein-coupled receptor 7 gene derived from human is represented in base No. 1666 to 2652. Cytosine in the nucleotide sequence represented by CpG which is present in the nucleotide sequence of SEQ ID NO: 14 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1480, 1482, 1485, 1496, 1513, 1526, 1542, 1560, 1564, 1568, 1570, 1580, 1590, 1603, 1613, 1620 and so on in the nucleotide sequence of SEQ ID NO: 14 can be recited.

To be more specific, when the useful protein gene is a G-protein coupled somatostatin and angiotensin-like peptide receptor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G-protein coupled somatostatin and angiotensin-like peptide receptor gene derived from human, and a promoter region located 5' upstream of the same can be recited, and more concretely, the nucleotide sequence of SEQ ID NO: 15 (corresponding to a complementary sequence to a nucleotide sequence represented by base No. 57001 to 60000 in the nucleotide sequence described in Genbank Accession No. AC008971) can be recited. In the nucleotide sequence of SEQ ID NO: 15, a nucleotide sequence of exon 1 of a G-protein coupled somatostatin and angiotensin-like peptide receptor gene derived from human is represented in base No. 776 to 2632. Cytosine in the nucleotide sequence represented by CpG which is present in the nucleotide sequence of SEQ ID NO: 15 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 470, 472, 490, 497, 504, 506, 509, 514, 522, 540, 543, 552, 566, 582, 597, 610, 612 and so on in the nucleotide sequence of SEQ ID NO: 15 can be recited.

To be more specific, when the useful protein gene is a Solute carrier family 6 neurotransmitter transporter noradrenalin member 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Solute carrier family 6 neurotransmitter transporter noradrenalin member 2 gene derived from human, and a promoter region located 5' upstream of the same can be recited, and more concretely, the nucleotide sequence of SEQ ID NO: 16 (corresponding to a complementary sequence to a nucleotide sequence represented by base No. 78801 to 81000 in the nucleotide sequence described in Genbank Accession No. AC026802) can be recited. In the nucleotide sequence of SEQ ID NO: 16, a nucleotide sequence of exon 1 of a Solute carrier family 6 neurotransmitter transporter noradrenalin member 2 gene derived from human is represented in base No. 1479 to 1804. Cytosine in the nucleotide sequence represented by CpG which is present in the nucleotide sequence of SEQ ID NO: 16 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1002, 1010, 1019, 1021, 1051, 1056, 1061, 1063, 1080, 1099, 1110, 1139, 1141, 1164, 1169, 1184 and so on in the nucleotide sequence of SEQ ID NO: 16 can be recited.

Detection in the phrase "detection or quantification of methylated DNA" in the present measuring method means that whether methylated DNA is present in a target DNA region can be determined according to whether a methylated DNA antibody or the present oligonucleotide is detected, and detecting a methylated DNA antibody or the present oligonucleotide indicates presence of methylated DNA in a target DNA region in the specimen, and not detecting a methylated DNA antibody or the present oligonucleotide indicates that the abundance of methylated DNA in a target DNA region in the specimen is less than the detection limit.

Quantification in the phrase "detection or quantification of methylated DNA" in the present measuring method means an amount of methylated DNA in a target DNA region in a specimen estimated from the quantified amount of a methylated DNA antibody or the present oligonucleotide, and for example, an amount of methylated DNA in a target region contained in 1 mL of serum when the specimen is 1 mL of serum.

In First step of the present measuring method, double-stranded DNA contained in a DNA sample derived from genomic DNA contained in a biological specimen is separated to a single-stranded state. Concretely, for example, by adding an annealing buffer to double-stranded DNA, a mixture is obtained. Then, the obtained mixture is boiled at 95°C for about 30 seconds, and then rapidly cooled on ice-cooled water for several minutes. For example, free DNA contained in blood or the like can be single-stranded DNA. Therefore, when genomic DNA contained in a biological specimen is single-stranded DNA, this operation is not required.

The term "methylated DNA antibody" in Second step of the present measuring method means an antibody that binds to a methylated base in DNA as its antigen. For example, an antibody having a property of recognizing and binding to cytosine methylated at position 5 in single-stranded DNA can be recited, and more concretely, a methyl cytosine antibody can be recited.

A methylated DNA antibody can be prepared by an usual immunological technique from a methylated base as an antigen. Concretely, it can be obtained by selecting from antibodies prepared against an antigen such as 5-methyl cytidine, 5-methyl cytosine or DNA containing 5-methyl cytosine based on specific binding to methyl cytosine in DNA as an index.

As an antibody obtainable by immunizing an animal against an antigen, an antibody of IgG fraction (polyclonal antibody), and an antibody produced by a single clone (monoclonal antibody) are recited. In the present measuring method, since an antibody capable of specifically recognizing a methylated base is desired, it is preferable to use a monoclonal antibody.

As a method of preparing a monoclonal antibody, a procedure based on a cell fusion method can be recited. For example, in the cell fusion method, a hybridoma is prepared by allowing cell fusion between a pancreatic cell (B cell) derived from an immunized mouse and a myeloma cell, and an antibody produced by the prepared hybridoma is selected for preparation of a methyl cytosine antibody (monoclonal antibody). When a monoclonal antibody is prepared by a cell fusion method, it is not necessary to purify an antigen, and for example, a mixture of 5-methyl cytidine, 5-methyl cytosine or DNA or the like containing 5-methyl cytosine may be administered as an antigen to an animal used for immunization. As an administration method, 5-methyl cytidine, 5-methyl cytosine or DNA or the like containing 5-methyl cytosine is directly administered to a mouse for production of an antibody. When an antibody is difficult to be produced, an antigen bound to a support may be used for immunization. Also, by thoroughly mixing an adjuvant solution (prepared, for example, by mixing liquid paraffin and Aracel A, and mixing killed tubercle bacilli as an adjuvant) and an antigen, and immunizing via liposome incorporating the same, immunity of an antigen can be improved. Also a method involving adding equivalent amounts of a solution containing an antigen and an adjuvant solution, fully emulsifying them, and subcutaneously or intraperitoneally injecting the resultant mixture to a mouse, and a method of adding killed Bordetella pertussis as an adjuvant after mixing well with alum water are known. A mouse may be boosted intraperitoneally or intravenously after an appropriate term from initial immunization. When the amount of an antigen is small, a solution in which the antigen is suspended may be directly injected into a mouse spleen to effect immunization.

After exenterating a spleen and peeling an adipose tissue off after several days from the final immunization, a spleen cell suspension is prepared. The spleen cell is fused, for example, with an HGPRT-deficient myeloma cell to prepare a hybridoma. As a cell fusion agent, any means capable of efficiently fusing a spleen cell (B cell) and a myeloma cell is applicable, and for example, a method of using a hemagglutinating virus of Japan (HVJ), polyethyleneglycol (PEG) and the like are recited. Cell fusion may be conducted by a method using a high voltage pulse.

After the cell fusion operation, cells are cultured in an HAT medium, a clone of a hybridoma in which a spleen cell and a myeloma cell are fused is selected, and the cell is allowed to grow until screening becomes possible. In a method of detecting an antibody for selecting a hybridoma that produces an intended antibody, or a method of measuring a titer of an antibody, an antigen-antibody reaction system may be used. Concretely, as a method of measuring an antibody against a soluble antigen, a radioisotope immune assay (RIA), an enzyme-linked immunosorbent assay (ELISA) and the like can be recited.

Considering the property of the methylated DNA antibody (one antibody binds to one methylated base (cytosine)), it is desired to select a region where a number of methylated bases (cytosine), namely CpG are present as a target DNA region, and thus an improvement in quantification accuracy and detection sensitivity is expected.

The term "the present oligonucleotide" in Second step of the present measuring method means an oligonucleotide that does not inhibit binding between a methylated DNA antibody and methylated DNA in a target DNA region, but is able to bind with the single-stranded DNA, and for example, an oligonucleotide having a nucleotide sequence capable of binding with single-stranded DNA containing a target DNA region, and not inhibiting binding to a methylated base (cytosine) in single-stranded DNA containing a target DNA region of a methylated DNA antibody when it binds with single-stranded DNA containing a target DNA region.

The phrase "nucleotide sequence capable of binding with single-stranded DNA containing a target DNA region" means a nucleotide sequence required for forming a bound body (double-strand) with single-stranded DNA containing a target DNA region, namely, a nucleotide sequence containing a nucleotide sequence complementary to part of a nucleotide sequence of a target DNA region, or a nucleotide sequence containing a nucleotide sequence complementary to part of a nucleotide sequence of a DNA region which is located further 5'-end side from 5'-end of a target DNA region, or a nucleotide sequence containing a nucleotide sequence complementary to part of a nucleotide sequence of a DNA region which is located further 3'-end side from 3'-end of a target DNA region.

The wording "not inhibiting binding between a methylated DNA antibody and methylated DNA in a target DNA region" means that the present oligonucleotide has such a nucleotide sequence that complementary binding between the present oligonucleotide and the single-stranded DNA does not occur in an occupied space required for a methylated DNA antibody to bind with methylated single-stranded DNA. In other words, it appears that a methylated DNA antibody occupies not only a methylated base (cytosine) to which it directly binds, but also the space surrounding the methylated base (cytosine) to bind to a methylated base (cytosine). Therefore, the present oligonucleotide may be any one insofar as it does not complementarily bind with the single-stranded DNA in a space occupied by a methylated DNA antibody to bind with methylated DNA. The present oligonucleotide to be bound to the single-stranded DNA is not necessarily one kind, but two or more kinds may be used insofar as binding of a methylated DNA antibody is not inhibited. Use of plural present oligonucleotides will improve quantification accuracy and detection sensitivity.

As a preferred embodiment of forming a complex formed in Second step of the present measuring method, or a bound body of single-stranded DNA separated in First step, a methylated DNA antibody and an oligonucleotide not inhibiting binding between a methylated DNA antibody and a methylated base present in single-stranded DNA containing a target DNA region arising in the course of forming a complex in Second step, formation in a reaction system containing a bivalent positive ion can be recited. More preferably, the bivalent positive ion is a magnesium ion. Here, the reaction system containing a bivalent positive ion" means a reaction system containing a bivalent positive ion in an annealing buffer used for forming a complex formed in Second step of the present measuring method, or a bound body of single-stranded DNA separated in First step, a methylated DNA antibody and an oligonucleotide not inhibiting binding between a methylated DNA antibody and a methylated base present in single-stranded DNA containing a target DNA region arising in the course of forming a complex in Second step, and concretely, for example, it preferably contains a salt composed of a magnesium ion (for example, MgOAc₂, MgCl₂ and so on) in a concentration ranging from 1 mM to 600 mM.

"Formation of a complex" in Second step of the present measuring method means formation of a mixture in the condition that single-stranded DNA separated in First step, the present oligonucleotide, and a methylated DNA antibody are bound.

"Separating a complex" in Second step of the present measuring method is enabled by binding and immobilizing the complex to a support. The material and form of the support are not particularly limited as far as a complex can bind thereto. For example, any form suited for use purpose may be employed, including the forms of tube, test plate, filter, disc, bead and so on. As the material, those used as supports for a usual immune measuring method, for example, synthetic resins such as polystyrene, polypropylene, polyacrylamide, polymethylmethacrylate, polysulfone, polyacrylonitrile and nylon, or the synthetic resins incorporating a reactive functional group such as a sulfonic group, or an amino group can be recited. Also, glass, polysaccharides or derivatives thereof (cellulose, nitrocellulose and so on), silica gel, porous ceramics, metal oxides and the like may be used.

As a method of binding and immobilizing a complex to a support for separating the complex, the following two approaches are recited: immobilizing a methylated DNA antibody to a support, and immobilizing the present oligonucleotide to a support. Therefore, the phrase "separate a complex at the same time or after formation of the complex" in Second step of the present measuring method concretely means "at the same time or after formation of a complex, by allowing single-stranded DNA containing methylated DNA in a target DNA region, the present oligonucleotide, and a methylated DNA antibody immobilizable to a support to bind simultaneously or sequentially, the methylated DNA antibody immobilizable to a support contained in the complex is immobilized to a support, and the complex is separated", or "at the same time or after formation of a complex, by allowing single-stranded DNA containing methylated DNA in a target DNA region, the present oligonucleotide immobilizable to a support, and a methylated DNA antibody to bind simultaneously or sequentially, the present oligonucleotide immobilizable to a support contained in the complex is immobilized to a support, and the complex is separated". Here, when single-stranded DNA containing methylated DNA in a target DNA region is bound to a support via a methylated DNA antibody, the present oligonucleotide is detected or quantified according to its function (as will be described later), and when single-stranded DNA containing methylated DNA in a target DNA region is bound to a support via the present oligonucleotide, a methylated DNA antibody is detected or quantified according to its function (as will be described later).

When a methylated DNA antibody is used as an object immobilizable to a support, it suffices that a methylated DNA antibody is eventually immobilized to a support while it forms a complex with single-stranded DNA containing methylated DNA in a target DNA region and the present oligonucleotide, and
(1) a methylated DNA antibody may be immobilized to a support prior to binding between the single-stranded DNA and the methylated DNA antibody, or
(2) a methylated DNA antibody may be immobilized to a support after binding between the single-stranded DNA and the methylated DNA antibody.

One exemplary concrete method for immobilizing a methylated DNA antibody to a support involves immobilizing a biotinylated methylated DNA antibody obtained by biotinylating a methylated DNA antibody to a streptavidin-coated support (for example, a PCR tube coated with streptavidin, magnetic beads coated with streptavidin, a chromatostrip partially coated with streptavidin and so on).

Also there is a method of letting a molecule having an active functional group such as an amino group, a thiol group, or an aldehyde group covalently bind to a methylated DNA antibody, and letting the resultant bound body covalently bind to a support made of glass, a polysaccharide derivative, silica gel or the synthetic resin or thermostable plastic whose surface is activated by a silane coupling agent or the like. The above described covalent bonding may be achieved, for example, by covalently coupling the molecule having an active functional group to a methylated DNA antibody using a spacer formed by serially connecting five triglycerides, a cross linker or the like.

A methylated DNA antibody may be directly immobilized to a support, or an antibody against a methylated DNA antibody (secondary antibody) may be immobilized to a support, and a methylated antibody may be bound to the secondary antibody to achieve immobilization to a support.

When the present oligonucleotide is used as an object immobilizable to a support, it suffices that the present oligonucleotide is eventually immobilized to a support while it forms a complex with single-stranded DNA containing methylated DNA in a target DNA region and a methylated DNA antibody, and
(1) the present oligonucleotide may be immobilized to a support prior to binding between the single-stranded DNA and the present oligonucleotide, or
(2) the present oligonucleotide may be immobilized to a support after binding between the single-stranded DNA and the present oligonucleotide.

One exemplary concrete method for immobilizing the present oligonucleotide to a support involves immobilizing a biotinylated oligonucleotide obtained by biotinylating 5'-end or 3'-end of the present oligonucleotide to a streptavidin-coated support (for example, a PCR tube coated with streptavidin, magnetic beads coated with streptavidin, a chromatostrip partially coated with streptavidin and so on).

Also there is a method of letting a molecule having an active functional group such as an amino group, a thiol group, or an aldehyde group covalently bind to 5'-end or 3'-end of the present oligonucleotide, and letting it be covalently bind to a support made of glass, a polysaccharide derivative, silica gel or the synthetic resin or thermostable plastic whose surface is activated by a silane coupling agent or the like. The above described covalent bonding may be achieved, for example, by covalently coupling the molecule having an active functional group to a methylated DNA antibody using a spacer formed by serially connecting five triglycerides, a cross linker or the like. Furthermore, a direct chemical synthesis method on a support made of glass or silicone from an end side of the present oligonucleotide may be employed.

To "separate a complex at the same time or after formation of the complex" in Second step of the present measuring method, when "a methylated DNA antibody immobilizable to a support is immobilized to a support at the same time or after formation of a complex by binding of single-stranded DNA containing methylated DNA in a target DNA region, the present oligonucleotide, and a methylated DNA antibody immobilizable to a support", concretely, for example, it may be executed in the following manner using a "biotin-labeled biotinylated methylated DNA antibody" as a methylated DNA antibody immobilizable to a support.
(a) An appropriate amount of a biotinylated methylated DNA antibody is added (for example, a 4 µg/mL solution is added in an amount of 100 µL/well) to an avidin-coated plate, and then left still, for example, for about 2 hours at room temperature, to promote immobilization between the biotinylated methylated DNA antibody and streptavidin. Thereafter, the remaining solution is removed and washing is performed. A washing buffer (for example, a 0.05% Tween 20-containing phosphate buffer (1 mM KH₂PO₄, 3 mM Na₂HPO·7H₂O, 154 mM NaCl, pH 7.4)) is added, for example, in a proportion of, for example, 300 µL/well, and the solution is removed. This washing operation is repeated several times, to leave the biotinylated methylated cytosine antibody immobilized on a support on wells.
(b) Single-stranded DNA obtained by separating double-stranded DNA contained in a DNA sample derived from genomic DNA contained in a biological specimen, the present oligonucleotide, and an annealing buffer (for example, 33 mM Tris-Acetate pH 7.9, 66 mM KOAc, 10 mM MgOAc₂, 0.5 mM Dithothreitol) are mixed, and heated at 95°C for several minutes, for example. Thereafter, in order to form a bound body of single-stranded DNA containing a target DNA region and the present oligonucleotide, the mixture is rapidly cooled to a temperature lower by about 10 to 20°C than the Tm value of the present oligonucleotide, and the reaction is kept at this temperature for several minutes, for example, and then the reaction is returned to room temperature (the bound body formed in this stage includes a bound body of single-stranded DNA containing methylated DNA in a target DNA region and the present oligonucleotide, as well as a bound body of single-stranded DNA not containing methylated DNA in a target DNA region and the present oligonucleotide).
(c) The formed bound body of single-stranded DNA and the present oligonucleotide is added to an avidin-coated plate to which a biotinylated methylated DNA antibody is immobilized, and then left still at room temperature for about 3 hours, to promote formation of a complex of the biotinylated methylated DNA antibody, single-stranded DNA containing methylated DNA in a target DNA region among the single-stranded DNA, and the present oligonucleotide (formation of a complex)(in this stage, the bound body of unmethylated single-stranded DNA containing a target DNA region and the present oligonucleotide does not form a complex). Thereafter, the remaining solution is removed and washing is performed. A washing buffer (for example, a 0.05% Tween 20-containing phosphate buffer (1 mM KH₂PO₄, 3 mM Na₂HPO·7H₂O, 154 mM NaCl, pH 7.4)) is added, for example, in a proportion of 300 µL/well, and the solution is removed. This washing operation is repeated several times, to leave the complex on wells (separation of a complex).

The annealing buffer used in (b) is not limited to the annealing buffer described above as far as it is suited for binding between the present oligonucleotide and single-stranded DNA containing a target DNA region. Use of a buffer in which a bivalent ion, preferably a magnesium ion is dissolved in a concentration of 1 to 600 mM will improve the binding stability.

The washing operation in (a) and (c) is important for removing an unimmobilized methylated DNA antibody suspended in the solution, unmethylated single-stranded DNA that does not bind with a methylated DNA antibody and hence is suspended in the solution, and single-stranded DNA in regions other than the target region that does not form a complex with the present oligonucleotide, or DNA digested by a restriction enzyme as will be described later and suspended in the solution, from the reaction solution. The washing buffer is not limited to the washing buffer described above, insofar as it is suited for removing the free methylated DNA antibody, single-stranded DNA and so on suspended in the solution and the like, and a DELFIA buffer (available from Perkin Elmer, Tris-HCl pH 7.8 with Tween 80), a TE buffer and the like may be used.

As described above, in the above (a) to (c), after binding between the single-stranded DNA containing the target DNA region, and the present oligonucleotide, a complex is formed by causing a biotinylated methylated DNA antibody to bind with the resultant bound body, however, the order is not limited to this. In other words, after binding between the single-stranded DNA containing the target DNA region and a biotinylated methylated DNA antibody, the present oligonucleotide may be caused to bind with the resultant bound body to form a complex. For example, by adding single-stranded DNA obtained by separating double-stranded DNA contained in a DNA sample derived from genomic DNA contained in a biological specimen, to a biotinylated methylated DNA antibody immobilized to a streptavidin-coated support, a bound body of a biotinylated methylated DNA antibody immobilized to a support and the single-stranded DNA is formed (the bound body formed in this stage includes not only a bound body of single-stranded DNA containing methylated DNA in a target DNA region and a methylated antibody, but also a bound body of methylated single-stranded DNA other than in a target DNA region and a methylated antibody). Thereafter, the present oligonucleotide may be added to this, to form and separate a complex with a bound body having single-stranded DNA containing methylated DNA in a target DNA region in the bound body (in this stage, the bound body of single-stranded DNA containing methylated DNA in regions other than the target DNA region and a methylated antibody does not form a complex).

The operations of (a) to (c) may be conducted using a chromatostrip. In that case, the operations are conducted in the following manner. For example, first, an appropriate amount of a biotinylated methylated DNA antibody is developed by a chromatostrip partially coated with streptavidin. By this operation, a biotinylated methylated DNA antibody is immobilized to the part coated with streptavidin. Then, a bound body of the single-stranded DNA and the present oligonucleotide obtained in (b) (the bound body formed in this stage includes not only a bound body of single-stranded DNA containing methylated DNA in a target DNA region and the present oligonucleotide, but also a bound body of single-stranded DNA not containing methylated DNA in a target DNA region and the present oligonucleotide) is developed by the chromatostrip. By these operations, a complex of single-stranded DNA obtained by separating double-stranded DNA contained in a DNA sample derived from genomic DNA contained in a biological specimen, the present oligonucleotide, and a biotinylated methylated DNA antibody is immobilized to a part coated with streptavidin (formation and selection of a complex)(in this stage, the bound body of single-stranded DNA not containing methylated DNA in a target DNA region and the present oligonucleotide does not form a complex). The order of formation of a complex is not limited to the order of these operations. For example, after forming a complex of single-stranded DNA containing methylated DNA in a target DNA region, the present oligonucleotide, and a biotinylated methylated DNA antibody, the complex may be developed by a chromatostrip and immobilized to the part coated with streptavidin. In these operations, unnecessary components can be removed by developing the solution by a chromatostrip, and the washing operation can be omitted. Of course, the washing operation (development of a chromatostrip by a washing buffer (for example, a 0.05% Tween 20-containing phosphate buffer (1 mM KH₂PO₄, 3 mM Na₂HPO·7H₂O, 154 mM NaCl, pH 7.4)) may be conducted between operations without causing any problem.

Alternatively, to "separate a complex at the same time or after formation of the complex" in Second step of the present measuring method, when "a methylated DNA antibody immobilizable to a support is immobilized to a support at the same time or after formation of a complex by binding of single-stranded DNA containing methylated DNA in a target DNA region, the present oligonucleotide, and a methylated DNA antibody immobilizable to a support", concretely, for example, it may be executed in the following manner using a "biotin-labeled biotinylated methylated DNA antibody" as a methylated DNA antibody immobilizable to a support.
(a) By adding an annealing buffer (for example, 33 mM Tris-Acetate pH 7.9, 66 mM KOAc, 10 mM MgOAc₂, 0.5 mM Dithothreitol) and the biotinylated present oligonucleotide to a DNA sample derived from genomic DNA contained in a biological specimen, a mixture is obtained. Then the obtained mixture is heated at 95°C, for example, for several minutes to separate double-stranded DNA contained in a DNA sample derived from genomic DNA contained in a biological specimen into single-stranded DNA. Then, in order to form a bound body of single-stranded DNA containing a target DNA region and the biotinylated present oligonucleotide, the mixture is rapidly cooled to a temperature lower by about 10 to 20°C than the Tm value of the biotinylated present oligonucleotide, and kept at this temperature, for example, for several minutes, and then returned to room temperature (the bound body formed in this stage includes a bound body of single-stranded DNA containing methylated DNA in a target DNA region and the present oligonucleotide, as well as a bound body of single-stranded DNA not containing methylated DNA in a target DNA region and the present oligonucleotide).
(b) By adding the mixture obtained in the above (a) to a streptavidin-coated support and keeping the support at 37°C, for example, for several minutes, the bound body of single-stranded DNA containing a target DNA region and the biotinylated present oligonucleotide is immobilized to a streptavidin-coated support. Then the remaining solution is removed and washing is performed. A washing buffer (for example, a 0.05% Tween 20-containing phosphate buffer (1 mM KH₂PO₄, 3 mM Na₂HPO·7H₂O, 154 mM NaCl, pH 7.4)) is added, for example, in a proportion of 300 µL/well, and the solution is removed. This washing operation is repeated several times, to leave the bound body of the biotinylated present oligonucleotide and single-stranded DNA containing a target DNA region immobilized on a support on wells (also in this stage, the bound body includes a bound body of single-stranded DNA containing methylated DNA in a target DNA region and the present oligonucleotide, as well as a bound body of single-stranded DNA not containing methylated DNA in a target DNA region and the present oligonucleotide).
(c) An appropriate amount of a methylated DNA antibody (for example, a 4 µg/mL solution is added in an amount of 100 µL/well) is added to wells, and then left still at room temperature, for example, for about 3 hours, to promote formation of a complex of a methylated DNA antibody, single-stranded DNA containing methylated DNA in a target DNA region in the single-stranded DNA, and the biotinylated present oligonucleotide (formation of a complex)(in this stage, the bound body of single-stranded DNA not containing methylated DNA in a target DNA region and the present oligonucleotide does not form a complex). Thereafter, the remaining solution is removed and washing is performed. A washing buffer (for example, a 0.05% Tween 20-containing phosphate buffer (1 mM KH₂PO₄, 3 mM Na₂HPO·7H₂O, 154 mM NaCl, pH 7.4)) is added, for example, in a proportion of 300 µL/well, and the solution is removed. This washing operation is repeated several times, to leave the complex on wells (separation of a complex).

The annealing buffer used in (a) is not limited to the annealing buffer described above as far as it is suited for binding between the present oligonucleotide and single-stranded DNA containing a target DNA region. Use of a buffer in which a bivalent ion, preferably a magnesium ion is dissolved in a concentration of 1 to 600 mM will improve the binding stability.

The washing operation in (b) and (c) is important for removing an unimmobilized methylated DNA antibody suspended in the solution, unmethylated single-stranded DNA that does not bind with a methylated DNA antibody and hence is suspended in the solution, or DNA digested by a restriction enzyme as will be described later and suspended in the solution from the reaction solution. The washing buffer is not limited to the washing buffer described above, insofar as it is suited for removing the free methylated DNA antibody, single-stranded DNA and so on suspended in the solution and the like, and a DELFIA buffer (available from Perkin Elmer, Tris-HCl pH 7.8 with Tween 80), a TE buffer and the like may be used.

As described above, in the above (a) to (c), after binding between the single-stranded DNA containing the target DNA region and the biotinylated present oligonucleotide, the biotinylated present oligonucleotide contained in the resultant bound body is caused to bind with a streptavidin-coated support to form a complex, however, the order is not limited to this. In other words, after binding between the biotinylated present oligonucleotide and a streptavidin-coated support, the single-stranded DNA containing the target DNA region is caused to bind with the resultant bound body, to form a complex. For example, by adding double-stranded DNA contained in a DNA sample derived from genomic DNA contained in a biological specimen to the biotinylated present oligonucleotide immobilized to a streptavidin-coated support, a mixture of the biotinylated present oligonucleotide immobilized to a support and the double-stranded DNA is obtained. For separating the double-stranded DNA contained in the obtained mixture to obtain single-stranded DNA, the mixture is heated at 95°C, for example, for several minutes. For forming a bound body of the obtained single-stranded DNA and the biotinylated present oligonucleotide, the mixture is rapidly cooled to a temperature lower by about 10 to 20°C than the Tm value of the biotinylated present oligonucleotide, and kept at this temperature, for example, for several minutes. Then the operation of (c) may be conducted to form and separate a complex (in this stage, the bound body of single-stranded DNA not containing methylated DNA in a target DNA region and the present oligonucleotide does not form a complex).

The operations of (a) to (c) may be conducted using a chromatostrip. In that case, the operations are conducted in the following manner. For example, first, a solution containing a bound body of single-stranded DNA obtained by separating double-stranded DNA contained in a DNA sample derived from genomic DNA contained in a biological specimen and the biotinylated present oligonucleotide is developed by a chromatostrip partially coated with streptavidin. By this operation, the bound body of single-stranded DNA obtained by separating double-stranded DNA contained in a DNA sample derived from genomic DNA contained in a biological specimen and the biotinylated present oligonucleotide is immobilized to the part coated with streptavidin (the bound body formed in this stage includes not only a bound body of single-stranded DNA containing methylated DNA in a target DNA region and a methylated antibody, but also a bound body of methylated single-stranded DNA other than in a target DNA region and a methylated antibody).

Then an appropriate amount of a methylated DNA antibody is developed by the chromatostrip. By this operation, a complex of single-stranded DNA obtained by separating double-stranded DNA contained in a DNA sample derived from genomic DNA contained in a biological specimen, the biotinylated present oligonucleotide, and a methylated DNA antibody is immobilized to part coated with streptavidin (formation and selection of a complex)(in this stage, the bound body of single-stranded DNA not containing methylated DNA in a target DNA region and the present oligonucleotide does not form a complex). The order of formation of a complex is not limited to the order of these operations. For example, after forming a complex of single-stranded DNA containing methylated DNA in a target DNA region, the biotinylated present oligonucleotide, and a methylated DNA antibody, the complex may be developed by a chromatostrip and immobilized to the part coated with streptavidin. In these operations, unnecessary components can be removed by developing the solution by a chromatostrip, and a washing operation can be omitted. Of course, a washing operation (development of a chromatostrip by a washing buffer (for example, a 0.05% Tween 20-containing phosphate buffer (1 mM KH₂PO₄, 3 mM Na₂HPO·7H₂O, 154 mM NaCl, pH 7.4)) may be conducted between operations without causing any problem.

The identification function" in Third step of the present measuring method is a function based on labeling used for detection or quantification, and concretely, in the case of a methylated DNA antibody, a fluorescent or a chromogenic property of a methylated DNA antibody labeled with europium, gold colloid, latex beads, a radioisotope, a fluorescent substance (FITC or the like), horseradish Peroxidase (HRP), alkaline phosphatase, biotin or the like can be recited, and the own property of an antibody (the property that the antibody itself binds to a secondary antibody) and the like are also recited as this function. On the other hand, in the case of the present oligonucleotide, a fluorescent or chromogenic property of the present oligonucleotide labeled at its 5'-end or 3'-end with europium, gold colloid, latex beads, a radioisotope, a fluorescent substance (FITC or the like), horseradish Peroxidase (HRP), alkaline phosphatase or the like can be recited. For detection or quantification based on such a function, for example, measurement by a radiation detector or a spectrophotometer, or visual check may be used.

In the case of a methylated DNA antibody, as described above, the own property of an antibody may be considered as a function, and even when the antibody itself is not labeled, a methylated DNA antibody can be indirectly detected or quantified by using a secondary antibody to which a detectable or quantifiable function is imparted.

When single-stranded DNA containing a target DNA region is bound to a support via a methylated DNA antibody, the present oligonucleotide is detected or quantified according to its function, whereas when single-stranded DNA containing a target DNA region is bound to a support via the present oligonucleotide, a methylated DNA antibody is detected or quantified according to its function.

When a methylated DNA antibody is detected or quantified according to its function, concretely when the own property of an antibody is used as a function, for example, the operation may be executed in the following manner. A secondary antibody against a methylated DNA antibody (for example, an Eu-N1-labeled mouse IgG antibody: available from Perkin Elmer) is added to a complex, and left still at room temperature for about an hour, to promote binding of the secondary antibody to the complex. Then, Enhancement Solution (available from Perkin Elmer) is added and mixed, and left still, for example, for about 45 minutes at room temperature. Thereafter, the fluorescence (excitation 340 nm/fluorescence 612 nm) is measured by a fluorescent detector to detect or quantify a methylated DNA antibody.

When an antibody to which FITC is bound is used as a secondary antibody, a methylated DNA antibody may be detected or quantified by measuring the fluorescence of FITC by a known method.

Also, for example, when biotin is not used for immobilization of the present oligonucleotide, a biotinylated methylated DNA antibody may be used for detection or quantification. When a biotinylated methylated DNA antibody is detected or quantified, for example, a biotinylated methylated DNA antibody can be detected or quantified by adding and mixing HRP-labeled streptavidin to a biotinylated methylated DNA antibody, and measuring the activity of HRP by a known method after formation and separation of a bound body of the biotinylated methylated DNA antibody and HRP-labeled streptavidin.

When the present oligonucleotide is detected or quantified according to its function, concretely, for example, a complex is formed using FITC-labeled oligonucleotide labeled with FITC as the present oligonucleotide required for formation of a complex, according to the method as described above, and the complex is separated. For the obtained complex, the fluorescence of FITC is measured by a known method, to detect or quantify the present oligonucleotide. Also a secondary antibody against FITC (for example, an HRP-labeled anti-FITC antibody: available from Jackson ImmunoResearch Laboratories) is added, and left still for about 2 hours at room temperature, to promote binding of a secondary antibody to a complex. Thereafter, an appropriate substrate (for example, Substrate Reagent Pack #DY999: available from R&D SYSTEMS) is added and mixed, and left still, for example, for 5 to 20 minutes at room temperature. Then Stop Solution (1 N H₂SO₄) is added. In 30 minutes after addition, the absorbance at 450 nm is measured by an absorbance detector, and thus the present oligonucleotide may be detected or quantified.

When biotin is not used for immobilization of a methylated DNA antibody, the biotinylated present oligonucleotide may be used for detection or quantification. When the biotinylated present oligonucleotide is detected or quantified, for example, HRP-labeled streptavidin is added or mixed to the biotinylated present oligonucleotide to form and separate a bound body of the biotinylated present oligonucleotide and HRP-labeled streptavidin, and then the activity of HRP is measured by a known method to detect or quantify a biotinylated methylated DNA antibody.

The present invention also provides a modified method additionally comprising a step of digesting the single-stranded DNA separated in First step by at least one kind of methylation sensitive restriction enzyme capable of digesting single-stranded DNA, between immediately after end of First step of the present measuring method and immediately before start of Third step (Modified method 1), or between immediately after end of First step of the present measuring method and immediately before start of Third step (Modified method 2).

The "methylation-sensitive restriction enzyme" in the modified methods (Modified method 1 and Modified method 2) present invention, for example, a restriction enzyme or the like that does not digest a recognition sequence containing methylated cytosine, but digests only a recognition sequence containing unmethylated cytosine. In other words, in the case of DNA wherein cytosine contained in a recognition sequence inherently recognizable by the methylation sensitive restriction enzyme is methylated, the DNA will not be cleaved even when the methylation sensitive restriction enzyme is caused to act on the DNA. On the other hand, in the case of DNA wherein cytosine contained in a recognition sequence inherently recognizable by the methylation sensitive restriction enzyme is not methylated, the DNA will be cleaved when the methylation sensitive restriction enzyme is caused to act on the DNA. Concrete examples of such methylation sensitive restriction enzymes include HpaII, BstUI, NarI, SacII, and HhaI. The aforementioned methylation sensitive restriction enzyme will not cleave double-stranded DNA containing a CpG pair in a hemimethyl state (namely, double-stranded DNA wherein cytosine in one strand is methylated and cytosine in the other strand is not methylated in the above CpG pair) and this is already revealed by Gruenbaum et al. (Nucleic Acid Research, 9, 2509-2515). The wording "methylation sensitive restriction enzyme capable of digesting single-stranded DNA" means, for example, a restriction enzyme or the like capable of digesting only a recognition sequence containing unmethylated cytosine without digesting a recognition sequence containing methylated cytosine in single-stranded DNA. Specifically, some methylation sensitive restriction enzymes digest single-stranded DNA. For example, HhaI or the like is recited.

As a fear in a digestion treatment with a methylation sensitive restriction enzyme, the possibility that the recognition sequence containing unmethylated cytosine may not be completely digested (so called "DNA remaining undigested") is recited. When such a possibility is problematic, the target DNA region has at least one recognition site of a methylation sensitive restriction enzyme, and as many as possible recognition sites would be preferred because it is possible to suppress the "DNA remaining undigested" as much as possible by presence of abundant recognition sites of a methylation sensitive restriction enzyme.

As described above, as to the target DNA region, from the view point of the property of a methylated DNA antibody (one antibody binds to one methylated base (cytosine)), a region where a number of methylated bases (cytosine, CpG) are present is desirably selected as a target DNA region, and from the view point of minimizing "DNA remaining undigested", a region where a number of recognition sites of a methylation sensitive restriction enzyme are present is desirably selected.

The term "masking oligonucleotide" in Modified method 2 means an oligonucleotide having a nucleotide sequence complementary to a nucleotide sequence of a recognition site of a methylation sensitive restriction enzyme, and is an oligonucleotide that forms a double strand by complementary binding with at least one site (even every site is possible, and one or more sites may be concurrently contained in the following 8 to 30 bases depending on the nucleotide sequence of the target region) of several recognition sequences of a methylation sensitive restriction enzyme contained in a target DNA region in single-stranded DNA (namely, the site is made into a double-stranded state), thereby enabling a methylation sensitive restriction enzyme that uses only double-stranded DNA as a substrate to digest the site, and improving digestion efficiency at the site for a methylation sensitive restriction enzyme capable of digesting single-stranded DNA (a methylation sensitive restriction enzyme capable of digesting single-stranded DNA also digests double-stranded DNA, and digestion efficiency thereof is higher with respect to double-stranded DNA than with respect to single-stranded DNA), and is a nucleotide not inhibiting formation of a bound body between single-stranded DNA containing DNA of a target region and the present oligonucleotide.

As a masking oligonucleotide, more concretely, oligonucleotides having a nucleotide sequence complementary to a nucleotide sequence having a length of 8 to 30 bases containing one specific site (one or more sites may be concurrently contained in the following 8 to 30 bases depending on the nucleotide sequence of the target) among several recognition sequences of a methylation sensitive restriction enzyme contained in a target DNA region in single-stranded DNA, and not inhibiting formation of a bound body of single-stranded DNA containing DNA of a target region and the present oligonucleotide can be recited.

The masking oligonucleotide to be mixed with single-stranded DNA (plus strand) comprising a target DNA region contained in a DNA sample derived from genomic DNA may be one kind or plural kinds. When plural kinds are used, many of recognition sites of a methylation sensitive restriction enzyme in single-stranded DNA comprising a target DNA region become a double-stranded DNA state, and "DNA remaining undigested" as described above by a methylation sensitive restriction enzyme can be minimized. However, since a methylated DNA antibody is no longer able to bind to the site where a masking oligonucleotide forms double-stranded DNA, it is not appropriate to use a masking oligonucleotide to too much recognition sites of a methylation sensitive restriction enzyme, and it is preferred to use appropriate kinds. If a masking oligonucleotide is used for every recognition site of a methylation sensitive restriction enzyme contained in a target DNA region, binding between a methylated base (cytosine) and a methylated DNA antibody in the recognition site is inhibited, and the probability of formation of a desired complex can be impaired (of course, a methylated DNA antibody binds to CpG other than the recognition site, however when a methylated DNA antibody is detected or quantified, the detection sensitivity and quantification accuracy are impaired). For example, it is particularly useful to use a masking oligonucleotide designed in accordance with a site intended not to be digested when it is methylated and intended to be digested when it is not methylated among several recognition sequences of a methylation sensitive restriction enzyme contained in a target DNA region (for example, the site that is methylated at 100% in a diseased patient specimen, but is not methylated at 100% in a healthy specimen).

In these Modified methods (Modified method 1 and Modified method 2), by additionally conducting the step of digesting the single-stranded DNA separated in First step by at least one kind of methylation sensitive restriction enzyme capable of digesting single-stranded DNA between after First step and immediately before Third step, it is possible to minimize (exclude) formation of a complex having single-stranded DNA (plus strand) not containing methylated DNA in a target DNA region, so that the detection sensitivity and quantification accuracy of methylated DNA in a target DNA region are improved.

In Modified method 1, the step of digesting the single-stranded DNA separated in First step by at least one kind of methylation sensitive restriction enzyme capable of digesting single-stranded DNA executed between immediately after end of First step and immediately before start of Third step may be concretely executed in the following manner. To 10 µL of a solution containing single-stranded DNA separated in First step, 3 µL of an optimum 10 × buffer, 15 U of a methylation sensitive enzyme (HhaI) capable of digesting a single strand, and an appropriate amount of BSA or the like as necessary are added, and then the resultant mixture is added with sterile ultrapure water to a liquid volume of 30 µL, and then incubated at 37°C, for example, for an hour to overnight. As a result, an HhaI recognition site unmethylated in a target DNA region will be digested (a treatment solution). For example, when this step is executed before formation of a complex, the treatment solution may be directly used for formation of a complex for executing formation of a complex and/or a washing operation at the time of separation. In executing this step after separation of a complex, since it is necessary to remove single-stranded DNA (digested and generated single-stranded DNA since a recognition site of the methylation sensitive restriction enzyme is not methylated) suspended in the treatment solution from the treatment solution, it is necessary to conduct a washing operation similar to that executed in formation of a complex and/or separation again after end of the step.

In Modified method 2, (i) a step of mixing single-stranded DNA separated in First step, a masking oligonucleotide having a recognition sequence of at least one kind of methylation sensitive restriction enzyme as its part, and (ii) digesting the mixture obtained by the previous step (single-stranded DNA in which DNA is not methylated in a target DNA region that is present therein) by the methylation sensitive restriction enzyme additionally executed between immediately after end of First step and immediately before start of Third step may be executed, for example, in the following manner. To 10 µL of a solution containing single-stranded DNA separated in First step, 3 µL of an optimum 10 × buffer, 15 U of a methylation sensitive enzyme (HhaI, HhaI or the like), about 10 pmol of a masking oligonucleotide for one specific site among recognition sequences of a methylation sensitive enzyme, and an appropriate amount of BSA or the like as necessary are added, and then the resultant mixture is added with sterile ultrapure water to a liquid volume of 30 µL, and then incubated at 37°C, for example, for an hour to overnight. As a result, the site unmethylated in a target DNA region will be digested (a treatment solution). For example, when this operation is executed before formation of a complex, the treatment solution may be directly used for formation of a complex for executing formation of a complex and/or a washing operation at the time of separation. In executing this operation after separation of a complex, since it is necessary to remove single-stranded DNA (digested and generated single-stranded DNA since a recognition site of the methylation sensitive restriction enzyme is not methylated) suspended in the treatment solution from the treatment solution, it is necessary to conduct a washing operation similar to that executed in formation of a complex and/or separation again after end of the step.

In the present measuring method, in one preferred embodiment, "a DNA sample derived from genomic DNA contained in a biological specimen" is a DNA sample that is preliminarily digested with a restriction enzyme whose recognition cleaving site excludes the target DNA region possessed by the genomic DNA. In forming a bound body of single-stranded DNA separated in First step and the present oligonucleotide, it is expected that the shorter the single-stranded DNA is, the better the operability is as well as the more easily a complex is formed insofar as methylated DNA is contained in a target DNA region. To shorten the single-stranded DNA, it is efficient to make it short when it is in former genomic DNA. Therefore, it is preferred to conduct a digestion treatment using a restriction enzyme whose recognition cleaving site excludes a target DNA region on a DNA sample derived from genomic DNA contained in a biological specimen as a pretreatment. As a method of the digestion treatment by a restriction enzyme whose recognition cleaving site excludes a target DNA region, a generally known restriction enzyme treatment method may be used. When the above DNA sample is a DNA sample preliminarily purified, a digestion treatment may be executed using a generally used amount of a restriction enzyme, whereas when a biological specimen is a tissue lysate, a cell lysate or the like, a digestion treatment may be conducted using an amount of 500 times or more the DNA amount of a restriction enzyme.

In one preferred embodiment, "a DNA sample derived from genomic DNA contained in a biological specimen" is a DNA sample that is digested with at least one kind of methylation sensitive restriction enzyme. By preliminarily digesting a biological specimen itself with a methylation sensitive restriction enzyme, it is possible to improve the accuracy of detection or quantification of methylated DNA in Third step. This method is useful for eliminating "DNA remaining undigested" as described above. As a method of digesting a biological specimen itself with a methylation sensitive restriction enzyme, when genomic DNA contained in a biological specimen exists in the condition of double-stranded DNA, a generally known restriction enzyme treatment may be conducted using a methylation sensitive restriction enzyme. On the other hand, when genomic DNA contained in a biological specimen exists in the condition of single-stranded DNA or when genomic DNA contained in a biological specimen exists in the condition of combination of double-stranded DNA and single-stranded DNA, a digestion treatment may be conducted by the method according to Modified method 1 or Modified method 2. Concretely, for example, to 10 µL of a specimen solution, 5 µL of an optimum 10 × buffer, 15 U of a methylation sensitive enzyme (HhaI) capable of digesting single-stranded DNA, and an appropriate amount of BSA or the like as necessary are added, and then the resultant mixture is added with sterile ultrapure water to a liquid volume of 50 µL, and then incubated at 37°C, for example, for an hour to overnight. Alternatively, to 10 µL of a specimen solution, 5 µL of an optimum 10 × buffer, 15 U of a methylation sensitive restriction enzyme (HpaII, HhaI or the like), about 15 pmol of a masking oligonucleotide for one specific site among recognition sequences of the methylation sensitive enzyme, and then the resultant mixture is added with sterile ultrapure water to a liquid volume of 50 µL, and then incubated at 37°C, for example, for an hour to overnight. When the biological specimen is a specimen preliminarily purified, a digestion treatment may be executed using a generally used amount of a restriction enzyme, whereas when a biological specimen is a tissue lysate, or a cell lysate, a digestion treatment may be conducted using a large excess of a methylation sensitive restriction enzyme, for example, an amount of 500 times or more DNA amount of a methylation sensitive restriction enzyme.

In a preferred embodiment in separating double-stranded DNA contained in a DNA sample derived from genomic DNA into single-stranded DNA in First step, a counter oligonucleotide may be added. The counter oligonucleotide is an oligonucleotide obtained by dividing a nucleotide sequence same as that of a target DNA region into shorter oligonucleotides. It is designed to have a length of usually 10 to 100 bases, and more preferably 20 to 50 bases. Preferably, a counter oligonucleotide is designed not to contain a nucleotide sequence that complementary binds to a nucleotide sequence on the aforementioned oligonucleotide required for complementary binding between the aforementioned methylated single-stranded DNA containing a target DNA region and the aforementioned oligonucleotide. The counter oligonucleotide is added in large excess relative to genomic DNA, and is added so as to prevent a complementary strand (minus strand) of a target DNA region and a single strand (plus strand) of a target DNA region from re-binding by complementation when binding with an immobilized methylated DNA antibody is caused after making a target DNA region into single strand (plus strand). Preferably, a counter oligonucleotide is added in an amount of at least 10 times, usually 100 times or more relative to a target DNA region.

"Adding a counter oligonucleotide in separating double-stranded DNA contained in a DNA sample derived from genomic DNA into single-stranded DNA in First step" is conducted, for the purpose of mixing a DNA sample derived from genomic DNA contained in a biological specimen with a counter oligonucleotide, to form a double strand between a complementary strand of a target DNA region and a counter oligonucleotide, thereby making a target DNA region into a single strand. When a target DNA region is in a single strand state, it is intended to facilitate binding between (ii) a methylated DNA antibody, and (iii) an oligonucleotide capable of binding with the single-stranded DNA without inhibiting binding between the methylated DNA antibody and methylated DNA in a target DNA region in Second step. The counter oligonucleotide is not designed on a nucleotide sequence where (iii) an oligonucleotide capable of binding with the single-stranded DNA without inhibiting binding between the methylated DNA antibody and methylated DNA in a target DNA region complementarily binds with the methylated DNA.

That is, a counter oligonucleotide is mixed with (i) single-stranded DNA separated in First step, (ii) a methylated DNA antibody, and (iii) an oligonucleotide capable of binding with the single-stranded DNA without inhibiting binding between the methylated DNA antibody and methylated DNA in a target DNA region, to facilitate formation of a complex of methylated single-stranded DNA containing a target DNA region, a methylated DNA antibody, and the oligonucleotide, and may be added in separating single-stranded DNA in First step or added in forming the complex in Second step.

When a counter oligonucleotide is added, for example, in First step, it may be added in First step for separating double-stranded DNA contained in a DNA sample derived from genomic DNA contained in a biological specimen into single-stranded DNA. Concretely, the DNA sample and the counter oligonucleotide are added to 5 µL of a buffer (330 mM Tris-Acetate pH 7.9, 660 mM KOAc, 100 mM MgOAc₂, 5 mM Dithiothreitol), 5 µL of a 100 mM MgCl₂ solution, and 5 µL of a 1 mg/mL BSA solution, and the resultant mixture is added with sterile ultrapure water to a liquid volume of 50 µL, and mixed, heated at 95°C for 10 minutes, rapidly cooled to 70°C, kept at this temperature for 10 minutes, then cooled to 50°C, kept at this temperature for 10 minutes, and then kept at 37°C for 10 minutes and returned to room temperature.

In a preferred embodiment in adding a counter oligonucleotide, the binding is caused in a reaction system containing a bivalent positive ion. More preferably, a bivalent positive ion is a magnesium ion. Here, the "reaction system containing a bivalent positive ion" means such a reaction system that contains a bivalent positive ion in an annealing buffer used for binding between the single-stranded DNA (plus strand) and the single-stranded immobilized oligonucleotide, and concretely, it is preferred to contain, for example, a salt composed of a magnesium ion (for example, MgOAc₂, MgCl₂ and so on) in a concentration of 1 mM to 600 mM.

As a method of detecting or quantifying minor substances contained in a biological sample such as blood or urine, immunological measuring methods are generally used. Among such immunological measuring methods, a so-called immunochromatography using chromatography is widely used in various situations including, for example, clinical examinations in hospitals, assays in laboratories and so on because of its simple operation and short time required for assay. In recent years, a so-called hybrid chromatography has been utilized in which labeled DNA (gene) is developed on a chromatostrip, and target DNA (gene) is detected by hybridization using a probe capable of capturing the target DNA (gene). Also this method is now coming to be widely used in situations including, for example, clinical examinations in hospitals, assays in laboratories and so on because of its simple operation and short time required for assay. The present measuring method conceptually enables a combined method of the immunochromatography and the hybrid chromatography. In the present measuring method, since the order is not particularly limited in terms of formation of a complex and separation of a complex, various methods are possible. Concretely, the following methods may be executed.

Method 1: A sample immediately after end of First step is added with the biotinylated present oligonucleotide, to cause formation of a bound body of single-stranded DNA containing a target DNA region and the biotinylated present oligonucleotide (the bound body formed in this stage includes not only a bound body of single-stranded DNA containing methylated DNA in a target DNA region and the present oligonucleotide, but also a bound body of single-stranded DNA not containing methylated DNA in a target DNA region and the present oligonucleotide), and added with a methylated antibody having an identification function, to cause formation of a complex of single-stranded DNA containing methylated DNA in a target DNA region, the biotinylated present oligonucleotide, and a methylated DNA antibody having an identification function (in this stage, the bound body of single-stranded DNA not containing methylated DNA in a target DNA region and the present oligonucleotide does not form a complex). Upon dropping (introduction) of the obtained sample into an introduction part of a chromatostrip, the complex migrates in a development part by a capillary phenomenon, and is trapped in the part preliminarily coated with streptavidin. Thereafter, by detecting or quantifying a methylated DNA antibody contained in the separated complex, according to its identification function, methylated DNA in a target DNA region can be detected or quantified.

Method 2: A sample immediately after end of First step is added with the biotinylated present oligonucleotide, to cause formation of a bound body of single-stranded DNA containing a target DNA region and the biotinylated present oligonucleotide (the bound body formed in this stage includes not only a bound body of single-stranded DNA containing methylated DNA in a target DNA region and the present oligonucleotide, but also a bound body of single-stranded DNA not containing methylated DNA in a target DNA region and the present oligonucleotide). Upon dropping (introduction) of the obtained sample into an introduction part of a chromatostrip, the bound body migrates in a development part by a capillary phenomenon, and is trapped in the part preliminarily coated with streptavidin (also in this stage, the bound body includes not only a bound body of single-stranded DNA containing methylated DNA in a target DNA region and the present oligonucleotide, but also a bound body of single-stranded DNA not containing methylated DNA in a target DNA region and the present oligonucleotide). Then, upon dropping (introduction) of a methylated antibody having an identification function into an introduction part, it migrates in a development part and binds to methylated cytosine of the bound body, to form a complex of single-stranded DNA containing methylated DNA in a target DNA region, the biotinylated present oligonucleotide, and a methylated DNA antibody having an identification function (in this stage, the bound body of single-stranded DNA not containing methylated DNA in a target DNA region and the present oligonucleotide does not form a complex). By detecting or quantifying a methylated DNA antibody contained in the obtained complex according to its identification function, methylated DNA in a target DNA region can be detected or quantified.

Method 3: Upon dropping (introduction) of the biotinylated present oligonucleotide into an introduction part of a chromatostrip, the oligonucleotide migrates in a development part by a capillary phenomenon, and is trapped in the part preliminarily coated with streptavidin. Then, upon dropping (introduction) of a sample immediately after end of First step into an introduction part, it migrates in a development part, and is trapped by the biotinylated present oligonucleotide that has been already trapped in the condition that single-stranded DNA containing a target DNA region forms a bound body (the bound body formed in this stage includes not only a bound body of single-stranded DNA containing methylated DNA in a target DNA region and the present oligonucleotide, but also a bound body of single-stranded DNA not containing methylated DNA in a target DNA region and the present oligonucleotide). Then, upon dropping (introduction) of a methylated antibody having an identification function into an introduction part, it migrates in a development part, and binds to methylated cytosine of the bound body, to form a complex of single-stranded DNA containing methylated DNA in a target DNA region, the biotinylated present oligonucleotide, and a methylated DNA antibody having an identification function (in this stage, the bound body of single-stranded DNA not containing methylated DNA in a target DNA region and the present oligonucleotide does not form a complex). By detecting or quantifying a methylated DNA antibody contained in the obtained complex according to its identification function, methylated DNA in a target DNA region can be detected or quantified.

Method 4: Upon dropping (introduction) of the biotinylated present oligonucleotide into an introduction part of a chromatostrip, the oligonucleotide migrates in a development part by a capillary phenomenon, and is trapped in the part preliminarily coated with streptavidin. A sample immediately after end of First step is added with a methylated DNA antibody having an identification function, to form a bound body of single-stranded DNA having methylated cytosine (in which single-stranded DNA containing a target DNA region and single-stranded DNA other than the target are present) and a methylated DNA antibody having an identification function (the bound body formed in this stage includes not only a bound body of single-stranded DNA containing methylated DNA in a target DNA region and a methylated antibody, but also a bound body of methylated single-stranded DNA other than in a target DNA region and a methylated antibody). Upon dropping (introduction) of the obtained bound body into an introduction part, it migrates in a development part, and single-stranded DNA containing methylated DNA in a target DNA region binds to the biotinylated present oligonucleotide that has been already trapped, to form a complex of single-stranded DNA containing methylated DNA in a target DNA region, the biotinylated present oligonucleotide, and a methylated DNA antibody having an identification function (in this stage, the bound body of methylated single-stranded DNA other than in a target DNA region and a methylated antibody does not form a complex). By detecting or quantifying a methylated DNA antibody contained in the obtained complex according to its identification function, methylated DNA in a target DNA region can be detected or quantified.

Method 5: A sample immediately after end of First step is added with the present oligonucleotide having an identification function, to form a bound body of single-stranded DNA containing a target DNA region and the present oligonucleotide having an identification function (the bound body formed in this stage includes not only a bound body of single-stranded DNA containing methylated DNA in a target DNA region and the present oligonucleotide, but also a bound body of single-stranded DNA not containing methylated DNA in a target DNA region and the present oligonucleotide), and then added with a biotinylated methylated antibody, to form a complex of single-stranded DNA containing methylated DNA in a target DNA region, the present oligonucleotide having an identification function, and a biotinylated methylated DNA antibody (in this stage, the bound body of single-stranded DNA not containing methylated DNA in a target DNA region and the present oligonucleotide does not form a complex). Upon dropping (introduction) of the obtained sample into an introduction part of a chromatostrip, the complex migrates in a development part by a capillary phenomenon, and is trapped in the part preliminarily coated with streptavidin. Then by detecting or quantifying the present oligonucleotide contained in the obtained complex according to its identification function, methylated DNA in a target DNA region can be detected or quantified.

Method 6: A sample immediately after end of First step is added with a biotinylated methylated DNA antibody, to form a bound body of single-stranded DNA having methylated cytosine (in which single-stranded DNA containing a target DNA region and single-stranded DNA other than the target are present) and a biotinylated methylated DNA antibody (the bound body formed in this stage includes not only a bound body of single-stranded DNA containing methylated DNA in a target DNA region and a methylated antibody, but also a bound body of methylated single-stranded DNA other than in a target DNA region and a methylated antibody). Upon dropping (introduction) of the obtained sample into an introduction part of a chromatostrip, the bound body migrates in a development part by a capillary phenomenon, and is trapped in the part preliminarily coated with streptavidin (also in this stage, the bound body includes not only a bound body of single-stranded DNA containing methylated DNA in a target DNA region and a methylated antibody, but also a bound body of methylated single-stranded DNA other than in a target DNA region and a methylated antibody). Then, upon dropping (introduction) of the present oligonucleotide having an identification function into an introduction part, it migrates in a development part, and binds only to methylated single-stranded DNA containing a target DNA region of a bound body, to form a complex of single-stranded DNA containing methylated DNA in a target DNA region, the present oligonucleotide having an identification function, and a biotinylated methylated DNA antibody (in this stage, the bound body of methylated single-stranded DNA other than in a target DNA region and a methylated antibody does not form a complex). By detecting or quantifying the present oligonucleotide contained in the obtained complex according to its identification function, methylated DNA in a target DNA region can be detected or quantified.

Method 7: Upon dropping (introduction) of a biotinylated methylated DNA antibody into an introduction part of a chromatostrip, the methylated DNA antibody migrates in a development part by a capillary phenomenon, and is trapped in the part preliminarily coated with streptavidin. Then, upon dropping (introduction) of a sample immediately after end of First step into an introduction part, it migrates in a development part, and single-stranded DNA having methylated cytosine is trapped as a bound body by a methylated DNA antibody that has been already trapped (the bound body formed in this stage includes not only a bound body of single-stranded DNA containing methylated DNA in a target DNA region and a methylated antibody, but also a bound body of methylated single-stranded DNA other than in a target DNA region and a methylated antibody). Then, upon dropping (introduction) of the present oligonucleotide having an identification function, it migrates in a development part, and binds only to methylated single-stranded DNA containing a target DNA region of the bound body, to form a complex of single-stranded DNA containing methylated DNA in a target DNA region, the present oligonucleotide having an identification function, and a biotinylated methylated DNA antibody (in this stage, the bound body of methylated single-stranded DNA other than in a target DNA region and a methylated antibody does not form a complex). By detecting or quantifying the present oligonucleotide contained in the obtained complex according to its identification function, methylated DNA in a target DNA region can be detected or quantified.

Method 8: Upon dropping (introduction) of a biotinylated methylated DNA antibody into an introduction part of a chromatostrip, the methylated DNA antibody migrates in a development part by a capillary phenomenon, and is trapped in the part preliminarily coated with streptavidin. A sample immediately after end of First step is added with the present oligonucleotide having an identification function, to form a bound body of single-stranded DNA containing a target DNA region and the present oligonucleotide having an identification function (the bound body formed in this stage includes not only a bound body of single-stranded DNA containing methylated DNA in a target DNA region and the present oligonucleotide, but also a bound body of single-stranded DNA not containing methylated DNA in a target DNA region and the present oligonucleotide). Then, upon dropping (introduction) of the obtained bound body into an introduction part, it migrates in a development part, and only single-stranded DNA containing methylated DNA in a target DNA region of the bound body binds with a methylated DNA antibody that has been already trapped, to form a complex of single-stranded DNA containing methylated DNA in a target DNA region, the present oligonucleotide having an identification function, and a biotinylated methylated DNA antibody (in this stage, the bound body of single-stranded DNA not containing methylated DNA in a target DNA region and the present oligonucleotide does not form a complex). By detecting or quantifying the present oligonucleotide contained in the obtained complex according to its identification function, methylated DNA in a target DNA region can be detected or quantified.

Also a plurality of detection sites may be provided on a single chromatostrip (the present oligonucleotides capable of trapping different target DNA regions are immobilized to a support), and each target DNA region may be sequentially detected or quantified, and by enabling one detection site to trap a plurality of target DNA regions, or by immobilizing a number of the present oligonucleotides capable of trapping a plurality of target DNA regions on one detection site, it is possible to dramatically improve the detection sensitivity. Also, using a number of the present oligonucleotides having an identification function capable of trapping a plurality of target DNA regions so as to allow formation of a complex with a plurality of target DNA regions will also improve the detection sensitivity dramatically. Further, designing a number of the present oligonucleotides in a single target region, and using these on the side of a support or on the side of detection will also improve the detection sensitivity dramatically.

The restriction enzyme, the present oligonucleotide, or the methylated DNA antibody which may be used in the present measuring method as described above is useful as a reagent in a detection kit. The present measuring method also provides a detection kit containing such a restriction enzyme, the present oligonucleotide, or a methylated DNA antibody and so on as a regent, and a detection chip including a support on which the present oligonucleotide, or a methylated DNA antibody and so on is immobilized, and the scope of the present measuring method implies use in the form of a detection kit or a detection chip as described above utilizing a substantial principle of the method.

### Examples

In the following, the present invention will be explained in detail by way of examples, which are not intended to limit the present invention.

### Example 1

Methylated oligonucleotides M1, M2, M3, and M4 having the nucleotide sequences of SEQ ID NOs: 17, 18, 19, and 20, and unmethylated oligonucleotides U1, U2, U3, and U4 having the nucleotide sequences of SEQ ID NOs: 21, 22, 23, and 24 labeled with biotin at 5'-end were synthesized, and the following solutions in TE buffer (10 mM Tris, 1 mM EDTA, pH 8.0) were prepared for each oligonucleotide.
Solution A: 2.5 pmol/100 µL solution in TE buffer
Solution B: 0.25 pmol/100 µL solution in TE buffer
Solution C: 0.025 pmol/100 µL solution in TE buffer
Solution D: 0.0025 pmol/100 µL solution in TE buffer
Solution E: 0.00025 pmol/100 µL solution in TE buffer
Solution F: 0 pmol/100 µL solution in TE buffer (negative control solution)

Then, for each solution (Solution A to Solution F), a methylated oligonucleotide mixed solution M0 in which equivalent amounts of respective solutions of the methylated oligonucleotides M1, M2, M3, and M4 are mixed, and a methylated oligonucleotide mixed solution U0 in which equivalent amounts of respective solutions of the unmethylated oligonucleotides U1, U2, U3, and U4 are mixed were prepared.
<5'-end biotin-labeled methylated oligonucleotides>
N represents methylated cytosine.
M1: 5'-ANGAANGTANGGANGC-3'(SEQ ID NO: 17)
M2: 5'-TNGATNGTTNGGTNGC-3'(SEQ ID NO: 18)
M3: 5'-GNGAGNGTGNGGGNGC-3' (SEQ ID NO: 19)
M4: 5'-CNGACNGTCNGGCNGC-3' (SEQ ID NO: 20)
<5'-end biotin-labeled unmethylated oligonucleotides>
U1: 5'-ACGAACGTACGGACGC-3' (SEQ ID NO: 21)
U2: 5'-TCGATCGTTCGGTCGC-3' (SEQ ID NO: 22)
U3: 5'-GCGAGCGTGCGGGCGC-3' (SEQ ID NO: 23)
U4: 5'-CCGACCGTCCGGCCGC-3' (SEQ ID NO: 24)

For each of the obtained solutions, the following treatment was executed (each solution was prepared in duplicate).

Each 100 µL of a 5'-end biotin-labeled oligonucleotide mixed solution (Solution A to Solution F of M0 and U0) prepared above was added to a 96-well plate coated with streptavidin, subjected to a plate shaker for about 10 seconds, and left still for about 30 minutes at room temperature, and thus the 5'-end biotin-labeled methylated oligonucleotide, or the 5'-end biotin-labeled unmethylated oligonucleotide was immobilized to the plate. After being left still, the solution in the plate was removed by decantation, and then each well in the plate was washed three times with 300 µL of a washing buffer [0.05% Tween 20-containing phosphate buffer (1 mM KH₂PO₄, 3 mM Na₂HPO·7H₂O, 154 mM NaCl, pH 7.4)].

After adding each of the wells with 100 µL of a methylated cytosine antibody [available from Aviva Systems Biology, a 1 or 10 µg/mL solution in a 0.1% BSA-containing phosphate buffer (1 mM KH₂PO₄, 3 mM Na₂HPO·7H₂O, 154 mM NaCl, pH 7.4)], the reaction was left still for 2 hours at room temperature. After being left still, each well was washed three times with 300 µL of a washing buffer [0.05% Tween 20-containing phosphate buffer (1 mM KH₂PO₄, 3 mM Na₂HPO·7H₂O, 154 mM NaCl, pH 7.4)].

Then, after adding each of the wells with 100 µL of an HRP-labeled mouse IgG antibody [available from Santa Cruz Biotechnology, 0.05 µg/100 µL solution in a 0.1% BSA-containing phosphate buffer (1 mM KH₂PO₄, 3 mM Na₂HPO·7H₂O, 154 mM NaCl, pH 7.4)], the reaction was left still for 2 hours at room temperature. After being left still, each well was washed three times with 300 µL of a washing buffer [0.05% Tween 20-containing phosphate buffer (1 mM KH₂PO₄, 3 mM Na₂HPO·7H₂O, 154 mM NaCl, pH 7.4)], and 100 µL of a substrate (available from R&D, #DY999) was added and mixed to each of the wells to start a reaction.

After being left still for about 20 minutes at room temperature, each well was added with 50 µL of a stop solution (1 N H₂SO₄ aqueous solution) to stop the reaction. The absorbance at 450 nm of the sample obtained within 30 minutes after stop of the reaction was measured.

The results are shown in Fig. 1 and Fig. 2. It was revealed that the immobilized 5'-end biotin-labeled methylated oligonucleotide M0 is detected and quantified with very good sensitivity compared with the immobilized 5'-end biotin-labeled unmethylated oligonucleotide U0. It was also suggested that use of too much methylated antibody (10 µg/mL, Fig. 2) results in occurrence of non-specific binding in Solution A and Solution B containing a large amount of the immobilized 5'-end biotin-labeled unmethylated oligonucleotide.

These demonstrate that a methylated DNA fragment can be detected and quantified in the present measuring method using a methylated cytosine antibody.

### Example 2

A methylated oligonucleotide UBC-M8 having the nucleotide sequence of SEQ ID NO: 25, and an unmethylated oligonucleotide UBC-UM8 having the nucleotide sequence of SEQ ID NO: 26 were synthesized, and the following solutions in TE buffer were prepared for each oligonucleotide.
Solution A: 0.1 pmol/10 µL solution in TE buffer
Solution B: 0.01 pmol/10 µL solution in TE buffer
Solution C: 0.001 pmol/10 µL solution in TE buffer
Solution D: 0 pmol/10 µL solution in TE buffer (negative control solution)

<Methylated oligonucleotide> N represents methylated cytosine.
UBC-M8: 5'-ANGAANGTANGGANGCTNGATNGTTNGGTNGCCTGTCTGACTACAACATCCAGA-3' (SEQ ID NO: 25)

<Unmethylated oligonucleotide>
UBC-U8: 5'-ACGAACGTACGGACGCTCGATCGTTCGGTCGCCTGTCTGACTACAACATCCAGA-3' (SEQ ID NO: 26)

Also, a 5'-end biotin-labeled oligonucleotide B1 having the nucleotide sequence of SEQ ID NO: 27 was synthesized, and a 0.002 µM solution in TE buffer was prepared.

### <5'-end biotin-labeled oligonucleotide>

B1: 5'-TCTGGATGTTGTAGTCAGACAG-3' (SEQ ID NO: 27)

For each solution of the obtained methylated oligonucleotide, or unmethylated oligonucleotide, the following treatment was executed (each solution was prepared singly).

To a PCR tube were added 10 µL of the oligonucleotide solution prepared above, 50 µL of the 5'-end biotin-labeled oligonucleotide solution, 10 µL of a buffer (330 mM Tris-Acetate pH 7.9, 660 mM KOAc, 100 mM MgOAc₂, 5 mM Dithiothreitol), and the mixture was added with sterile ultrapure water to a liquid volume of 100 µL, and mixed. Then the PCR tube was heated at 95°C for 10 minutes, rapidly cooled to 70°C, and kept at that temperature for 10 minutes. Then the PCR tube was cooled to 50°C and kept at this temperature for 10 minutes, and then kept at 37°C for 10 minutes, and then returned to room temperature to promote formation of a bound body of a 5'-end biotin-labeled oligonucleotide and an oligonucleotide.

Every obtained mixture was transferred to a 96-well plate coated with streptavidin, and left still for about 30 minutes at room temperature, to immobilize the bound body of a 5'-end biotin-labeled oligonucleotide and an oligonucleotide onto the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each of the wells was added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. After being left still, each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each of the wells was added with 100 µL of an Eu-N1 labeled mouse IgG antibody [available from Perkin Elmer, a 0.25 µg/mL solution in a 0.1% BSA-containing phosphate buffer (1 mM KH₂PO₄, 3 mM Na₂HPO·7H₂O, 154 mM NaCl, pH 7.4)] and left still for an hour at room temperature. After being left still, each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 3 minutes at room temperature, the fluorescence of the obtained sample was measured with excitation 340 nm/fluorescence 612 nm.

The result is shown in Fig. 3. It was revealed that the methylated oligonucleotide UBC-M8 is selected with higher accuracy and detected and quantified with higher sensitivity by an immobilized 5'-end biotin-labeled oligonucleotide B1 compared with the unmethylated oligonucleotide UBC-U8.

These demonstrate that it is possible to form and separate a complex of a methylated cytosine antibody, a methylated DNA fragment, and an immobilized 5'-end biotin-labeled oligonucleotide, and to detect and quantify a methylated cytosine antibody in the complex according to its identification function, and to detect and quantify a methylated DNA fragment by the present measuring method.

### Example 3

A methylated oligonucleotide UBC-M having the nucleotide sequence of SEQ ID NO: 28, a partially methylated oligonucleotide UBC-HM having the nucleotide sequence of SEQ ID NO: 29, and an unmethylated oligonucleotide UBC-UM having the nucleotide sequence of SEQ ID NO: 30 were synthesized, and the following solutions in TE buffer were prepared for each oligonucleotide.
Solution A: 0.1 pmoL/10 µL solution in TE buffer
Solution B: 0.01 pmoL/10 µL solution in TE buffer
Solution C: 0.001 pmoL/10 µL solution in TE buffer
Solution D: TE buffer solution (negative control solution)

<Methylated oligonucleotide> N represents methylated cytosine.

<Partially methylated oligonucleotide> N represents methylated cytosine.

### <Unmethylated oligonucleotide>

For each obtained solution, the following treatment was executed (each solution was prepared singly).

Group A (no treatment group): After adding 10 µL of an oligonucleotide solution prepared as described above, 5 µL of a buffer (330 mM Tris-Acetate pH 7.9, 660 mM KOAc, 100 mM MgOAc₂, 5 mM Dithiothreitol), and 5 µL of BSA (Bovine serum albumin 1 mg/mL) to a PCR tube, 5 pmol of an oligonucleotide MA having the nucleotide sequence of SEQ ID NO: 31 was added as a masking oligonucleotide, and the resultant mixture was added with sterile ultrapure water to a liquid volume of 50 µL, and mixed.

Group B (HhaI treatment group): After adding 10 µL of an oligonucleotide solution prepared as described above, 5 U of HhaI, 5 µL of a buffer (330 mM Tris-Acetate pH 7.9, 660 mM KOAc, 100 mM MgOAc₂, 5 mM Dithiothreitol), and 5 µL of BSA (Bovine serum albumin 1 mg/mL) to a PCR tube, 5 pmol of an oligonucleotide MA having the nucleotide sequence of SEQ ID NO: 31 was added as a masking oligonucleotide, and the resultant mixture was added with sterile ultrapure water to a liquid volume of 50 µL, and mixed.

### <Masking oligonucleotide>

MA: 5'-GATGGCGCTCTG-3' (SEQ ID NO: 31)

After incubating each reaction solution at 37°C for 15 hours, 50 µL of a 0.002 µM solution of the 5'-end biotin-labeled oligonucleotide B1 having the nucleotide sequence of SEQ ID NO: 32 in TE buffer was added to this and mixed.

### <5'-end biotin-labeled oligonucleotide>

B1: 5'-TCTGGATGTTGTAGTCAGACAG-3' (SEQ ID NO: 32)

Then the PCR tube was heated at 95°C for 10 minutes, rapidly cooled to 70°C, and kept at this temperature for 10 minutes. Then the tube was cooled to 50°C, kept at this temperature for 10 minutes, and then kept at 37°C for 10 minutes, and returned to room temperature, to promote formation of a bound body of a 5'-end biotin-labeled oligonucleotide and an oligonucleotide.

Every obtained mixture was transferred to a 96-well plate coated with streptavidin, and left still for about 30 minutes at room temperature, to immobilize the bound body of a 5'-end biotin-labeled oligonucleotide and an oligonucleotide onto the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each of the wells was added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. After being left still, the solution was removed by decantation from each well, and each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each of the wells was added with 100 µL of an Eu-N1 labeled mouse IgG antibody [available from Perkin Elmer, a 0.25 µg/mL solution in a 0.1% BSA-containing phosphate buffer (1 mM KH₂PO₄, 3 mM Na₂HPO·7H₂O, 154 mM NaCl, pH 7.4)] and left still for an hour at room temperature. After being left still, the solution was removed by decantation from each well, and each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 45 minutes at room temperature, the fluorescence of the obtained sample was measured with excitation 340 nm/fluorescence 612 nm.

The results are shown in Fig. 4 and Fig. 5. In the case of Group A (no treatment group) (Fig. 4), it was revealed that in the methylated oligonucleotide UBC-M where all of three sites among three sites of CG sequences are methylated, it is possible to form and separate a complex of an immobilized 5'-end biotin-labeled oligonucleotide B1 and a methylated cytosine antibody, and to detect and quantify the complex with very high sensitivity by a biotin label in the complex. On the other hand, in a partially methylated oligonucleotide UBC-HM where only one site among three sites of CG sequences is methylated, it was revealed that a complex of an immobilized 5'-end biotin-labeled oligonucleotide B1 and a methylated cytosine antibody was formed and separated, and detected and quantified with a sensitivity of about 1/3 of that in the UBC-M. In the unmethylated oligonucleotide UBC-UM where no site among three sites of CG sequences is methylated, since a complex of an immobilized 5'-end biotin-labeled oligonucleotide B1 and a methylated cytosine antibody was not formed and separated, a value approximately equal to a background value (data of Solution D) was exhibited. In the case of Group B (HhaI treatment group) (Fig. 5), it was revealed that in the methylated oligonucleotide UBC-M where all of three sites among three sites of CG sequences are methylated, since it is not digested by a methylation sensitive restriction enzyme (HhaI), a complex of an immobilized 5'-end biotin-labeled oligonucleotide B1 and a methylated cytosine antibody is formed and separated, and detected and quantified with excellent sensitivity by a biotin label in the complex as is the case with Group A (no treatment group). In a partially methylated oligonucleotide UBC-HM where only one site among three sites of CG sequences is methylated, a methylated CG sequence is lost by digestion by a methylation sensitive restriction enzyme (HhaI). Therefore, a complex is not formed and separated, and the value was approximately equal to the background value (data of Solution D). In the unmethylated oligonucleotide UBC-UM where no site among three sites of CG sequences is methylated, since a complex is not formed and separated, the value was approximately equal to the background value as is the case with A treatment group (no treatment group).

From the above, it was revealed that by a digesting treatment with a methylation sensitive restriction enzyme, a partially methylated DNA fragment can be digested, and by forming and separating a complex of a methylated cytosine antibody, a methylated DNA fragment, and an immobilized 5'-end biotin-labeled oligonucleotide, and detecting and quantifying the methylated cytosine antibody in the complex according to its identification function, it is possible to detect and quantify a methylated DNA fragment.

### Example 4

A methylated oligonucleotide UBC86-M having the nucleotide sequence of SEQ ID NO: 33, and a partially methylated oligonucleotide UBC118-HM having the nucleotide sequence of SEQ ID NO: 34 were synthesized, and the following solutions in TE buffer were prepared for each oligonucleotide.
Solution A: 0.1 pmoL/10 µL solution in TE buffer
Solution B: 0.01 pmoL/10 µL solution in TE buffer
Solution C: 0.001 pmoL/10 µL solution in TE buffer
Solution D: TE buffer solution (negative control solution)

<Methylated oligonucleotide> N represents methylated cytosine.

<Partially methylated oligonucleotide> N represents methylated cytosine.

For each obtained solution, the following treatment was executed (each solution was prepared singly).

Group A (no treatment group): After adding 10 µL of an oligonucleotide solution prepared as described above, 5 µL of a buffer (330 mM Tris-Acetate pH 7.9, 660 mM KOAc, 100 mM MgOAc₂, 5 mM Dithiothreitol), and 5 µL of BSA (Bovine serum albumin 1 mg/mL) to a PCR tube, 5 pmol of an oligonucleotide MA having the nucleotide sequence of SEQ ID NO: 35 was added as a masking oligonucleotide, and the resultant mixture was added with sterile ultrapure water to a liquid volume of 50 µL, and mixed.

Group B (HhaI treatment group): After adding 10 µL of an oligonucleotide solution prepared as described above, 5 U of HhaI, 5 µL of a buffer (330 mM Tris-Acetate pH 7.9, 660 mM KOAc, 100 mM MgOAc₂, 5 mM Dithiothreitol), and 5 µL of BSA (Bovine serum albumin 1 mg/mL) to a PCR tube, 5 pmol of an oligonucleotide MA having the nucleotide sequence of SEQ ID NO: 35 was added as a masking oligonucleotide, and the resultant mixture was added with sterile ultrapure water to a liquid volume of 50 µL, and mixed.

### <Masking oligonucleotide>

MA: 5'-GATGGCGCTCTG-3' (SEQ ID NO: 35)

After incubating each reaction solution at 37°C for 16 hours, 50 µL of a 0.002 µM solution of the 5'-end biotin-labeled oligonucleotide B1 having the nucleotide sequence of SEQ ID NO: 36 in TE buffer was added to this and mixed.

### <5'-end biotin-labeled oligonucleotide>

B1: 5'-TCTGGATGTTGTAGTCAGACAG-3' (SEQ ID NO: 36)

Then the PCR tube was heated at 95°C for 10 minutes, rapidly cooled to 70°C, and kept at this temperature for 10 minutes. Then the tube was cooled to 50°C, kept at this temperature for 10 minutes, and then kept at 37°C for 10 minutes, and returned to room temperature, to promote formation of a bound body of a 5'-end biotin-labeled oligonucleotide and an oligonucleotide.

Every obtained mixture was transferred to a 96-well plate coated with streptavidin, and left still for about 30 minutes at room temperature, to immobilize the bound body of a 5'-end biotin-labeled oligonucleotide and an oligonucleotide onto the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each of the wells was added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. After being left still, the solution was removed by decantation from each well, and each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each of the wells was added with 100 µL of an Eu-N1 labeled mouse IgG antibody [available from Perkin Elmer, a 0.25 µg/mL solution in a 0.1% BSA-containing phosphate buffer (1 mM KH₂PO₄, 3 mM Na₂HPO·7H₂O, 154 mM NaCl, pH 7.4)] and left still for an hour at room temperature. After being left still, the solution was removed by decantation from each well, and each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 45 minutes at room temperature, the fluorescence of the obtained sample was measured with excitation 340 nm/fluorescence 612 nm.

The results are shown in Fig. 6 and Fig. 7. In the case of Group A (no treatment group) (Fig. 6), it was revealed that in the methylated oligonucleotide UBC86-M where all of five sites among five sites of CG sequences are methylated, a complex of an immobilized 5'-end biotin-labeled oligonucleotide and a methylated cytosine antibody can be formed and separated, and detected and quantified with high sensitivity by a biotin label in the complex. On the other hand, it was revealed that in the partially methylated oligonucleotide UBC118-HM where 12 sites among 13 sites of CG sequences are methylated, sensitivity of detection and quantification was about 1.4 times that in UBC86-M. In the case of Group B (HhaI treatment group) (Fig. 7), it was revealed that in the methylated oligonucleotide UBC86-M where all of five sites among five sites of CG sequences are methylated, since it is not digested by a methylation sensitive restriction enzyme (HhaI), a complex of an immobilized 5'-end biotin-labeled oligonucleotide and a methylated cytosine antibody is formed and separated, and detected and quantified with excellent sensitivity by a biotin label in the complex as is the case with Group A (no treatment group). On the other hand, in the partially methylated oligonucleotide UBC118-HM where 12 sites among 13 sites of CG sequences are methylated, only two methylated sites are left as a result of digestion by a methylation sensitive restriction enzyme (HhaI). Therefore, the detection and quantification result of UBC118-HM was inferior to that of UBC86-M.

From the above, it was revealed that by a digesting treatment with a methylation sensitive restriction enzyme, a partially methylated DNA fragment can be digested, and by forming and separating a complex of a methylated cytosine antibody, a methylated DNA fragment, and an immobilized 5'-end biotin-labeled oligonucleotide, and detecting and quantifying the methylated cytosine antibody in the complex according to its identification function, it is possible to detect and quantify a methylated DNA fragment.

### Example 5

A methylated oligonucleotide UBC-M8 having the nucleotide sequence of SEQ ID NO: 37, a methylated oligonucleotide FBN-M8 having the nucleotide sequence of SEQ ID NO: 38, a methylated oligonucleotide GPR-M8 having the nucleotide sequence of SEQ ID NO: 39, an unmethylated oligonucleotide UBC-U8 having the nucleotide sequence of SEQ ID NO: 40, an unmethylated oligonucleotide FBN-U8 having the nucleotide sequence of SEQ ID NO: 41, and an unmethylated oligonucleotide GPR-U8 having the nucleotide sequence of SEQ ID NO: 42 were synthesized, and the following solutions in TE buffer were prepared for each of methylated oligonucleotides and unmethylated oligonucleotides.
Solution A: 0.3 pmol/10 µL solution in TE buffer
Solution B: 0.03 pmol/10 µL solution in TE buffer
Solution C: 0.003 pmol/10 µL solution in TE buffer
Solution D: 0 pmol/10 µL solution in TE buffer (negative control solution)

Then, for each solution (Solution A to Solution D), a methylated oligonucleotide mixed solution M0 in which equivalent amounts of respective solutions of the methylated oligonucleotides UBC-M8, FBN-M8, and GPR-M8 are mixed, and a methylated oligonucleotide mixed solution U0 in which equivalent amounts of respective solutions of the unmethylated oligonucleotides UBC-U8, FBN-U8, and GPR-U8 are mixed were prepared.

<Methylated oligonucleotides> N represents methylated cytosine.
UBC-M8: 5'-ANGAANGTANGGANGCTNGATNGTTNGGTNGCCTGTCTGACTACAACATCCAGA-3' (SEQ ID NO: 37)
FBN-M8: 5'-ANGAANGTANGGANGCTNGATNGTTNGGTNGCCAGCCGACGAAGGGCTTATTAG-3' (SEQ ID NO: 38)
GPR-M8: 5'-ANGAANGTANGGANGCTNGATNGTTNGGTNGCCTGCCGAGAACGAGGCGTTGTC-3' (SEQ ID NO: 39)

### <Unmethylated oligonucleotides>

UBC-U8: 5'-ACGAACGTACGGACGCTCGATCGTTCGGTCGCCTGTCTGACTACAACATCCAGA-3' (SEQ ID NO: 40)
FBN-U8: 5'-ACGAACGTACGGACGCTCGATCGTTCGGTCGCCAGCCGACGAAGGGCTTATTAG-3' (SEQ ID NO: 41)
GPR-U8 : 5'-ACGAACGTACGGACGCTCGATCGTTCGGTCGCCTGCCGAGAACGAGGCGTTGTC-3' (SEQ ID NO: 42)

0.1 pmol/50 µL solutions of 5'-end biotin-labeled oligonucleotides UBC, FBN, and GPR having the nucleotide sequences of SEQ ID NO: 43, SEQ ID NO: 44, and SEQ ID NO: 45 in TE buffer were prepared (a UBC-B solution, an FBN-B solution, and a GPR-B solution, respectively). Also, a biotin-labeled oligonucleotide solution (biotin-labeled oligonucleotide mixed solution) in TE buffer in which equivalent amounts of 5'-end biotin-labeled oligonucleotides UBC, FBN, and GPR having the nucleotide sequences of SEQ ID NO: 43, SEQ ID NO: 44, and SEQ ID NO: 45 (each 0.1 pmol/50 µL) are mixed were prepared.

### <5'-end biotin-labeled oligonucleotides>

UBC: 5'-TCTGGATGTTGTAGTCAGACAG-3' (SEQ ID NO: 43)
FBN: 5'-CTAATAAGCCCTTCGTCGGCT-3' (SEQ ID NO: 44)
GPR: 5'-GACAACGCCTCGTTCTCGG-3' (SEQ ID NO: 45)

For each of solutions (Solution A to Solution D) of the methylated oligonucleotide mixed solution M0, and the unmethylated oligonucleotide mixed solution U0, the following treatment was executed (each solution was prepared in quadruplicate).

After adding 10 µL of an oligonucleotide solution prepared as described above, and 10 µL of a buffer (330 mM Tris-Acetate pH 7.9, 660 mM KOAc, 100 mM MgOAc₂, 5 mM Dithothreitol) to a PCR tube, 50 µL of each solution of the 5'-end biotin-labeled oligonucleotide (UBC-B solution, FBN-B solution, GPR-B solution, and biotin-labeled oligonucleotide mixed solution) was added to each reaction solution, and the resultant mixture was added with sterile ultrapure water to a liquid volume of 100 µL, and mixed.

Then the PCR tube was heated at 95°C for 10 minutes, rapidly cooled to 70°C, and kept at this temperature for 10 minutes. Further, the tube was cooled to 50°C and kept at this temperature for 10 minutes, and then kept at 37°C for 10 minutes, and returned to room temperature, to promote formation of a bound body of a 5'-end biotin-labeled oligonucleotide and an oligonucleotide.

Every obtained mixture in solution was transferred to a 96-well plate coated with streptavidin, and left still for about 30 minutes at room temperature, to immobilize the bound body of a 5'-end biotin-labeled oligonucleotide and an oligonucleotide onto the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each of the wells was added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Thereafter, each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each of the wells was added with 100 µL of an Eu-N1 labeled mouse IgG antibody [available from Perkin Elmer, a 0.25 µg/mL solution in a 0.1% BSA-containing phosphate buffer (1 mM KH₂PO₄, 3 mM Na₂HPO·7H₂O, 154 mM NaCl, pH 7.4)] and left still for an hour at room temperature. After being left still, each of the wells was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 45 minutes at room temperature, the fluorescence of the obtained sample was measured with excitation 340 nm/fluorescence 612 nm.

The results are shown in Fig. 8 to Fig. 11. In the methylated oligonucleotide mixed solution M0, it was revealed that a complex is formed and separated between the immobilized 5'-end biotin-labeled oligonucleotide UBC (Fig. 8), FBN (Fig. 9), GPR (Fig. 10) and the oligonucleotides in which the foregoing three kinds are mixed (Fig. 11) and a methylated cytosine antibody, and detection and quantification with high sensitivity are enabled by a biotin label in the complex. It was revealed that especially when three kinds of oligonucleotides are mixed (Fig. 11), detection and quantification with better sensitivity than that in detection by single oligonucleotide (Fig. 8 to Fig. 10) are realized. In the unmethylated oligonucleotide mixed solution U0, since a complex is not formed between the immobilized 5'-end biotin-labeled oligonucleotide UBC (Fig. 8), FBN (Fig. 9), GPR (Fig. 10), and the oligonucleotides in which the foregoing three kinds are mixed (Fig. 11) and a methylated cytosine antibody, the value approximately equal to the background value was exhibited in every case.

From the above, it was revealed that by forming and separating a complex of a methylated cytosine antibody, a methylated DNA fragment, and an immobilized 5'-end biotin-labeled oligonucleotide, and detecting and quantifying the methylated cytosine antibody in the complex according to its identification function, it is possible to detect and quantify a methylated DNA fragment. It was also revealed that by using a plurality of immobilized 5'-end biotin-labeled oligonucleotides, detection and quantification with high sensitivity are realized compared with the case where one kind of immobilized 5'-end biotin-labeled oligonucleotide is used (that is, by not only using one target DNA region, but also using a plurality of target DNA regions concurrently).

### Example 6

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

The synthetically obtained biotin-labeled methylated cytosine antibody solution was added to a 96-well plate coated with streptavidin in an amount of 100 µL/well, and immobilized to wells by leaving the solution still for about an hour at room temperature. After being left still, the solution was removed from each well by decantation, and then each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

A methylated oligonucleotide UBC-M8 having the nucleotide sequence of SEQ ID NO: 46, a methylated oligonucleotide FBN-M8 having the nucleotide sequence of SEQ ID NO: 47, a methylated oligonucleotide GPR-M8 having the nucleotide sequence of SEQ ID NO: 48, an unmethylated oligonucleotide UBC-U8 having the nucleotide sequence of SEQ ID NO: 49, an unmethylated oligonucleotide FBN-U8 having the nucleotide sequence of SEQ ID NO: 50, and an unmethylated oligonucleotide GPR-U8 having the nucleotide sequence of SEQ ID NO: 51 were synthesized, and the following solutions were prepared for each oligonucleotide.
Solution A: 0.1 pmoL/10 µL solution in TE buffer
Solution B: 0.01 pmoL/10 µL solution in TE buffer
Solution C: 0.001 pmoL/10 µL solution in TE buffer
Solution D: 0 pmoL/10 µL solution in TE buffer (negative control solution)

<Methylated oligonucleotides> N represents methylated cytosine.
UBC-M8: 5'-ANGAANGTANGGANGCTNGATNGTTNGGTNGCCTGTCTGACTACAACATCCAGA-3' (SEQ ID NO: 46)
GPR-M8: 5'-ANGAANGTANGGANGCTNGATNGTTNGGTNGCCTGCCGAGAACGAGGCGTTGTC-3' (SEQ ID NO: 47)
FBN-M8 : 5'-ANGAANGTANGGANGCTNGATNGTTNGGTNGCCAGCCGACGAAGGGCTTATTAG-3' (SEQ ID NO: 48)

### <Unmethylated oligonucleotides>

UBC-UM8: 5'-ACGAACGTACGGACGCTCGATCGTTCGGTCGCCTGTCTGACTACAACATCCAGA-3' (SEQ ID NO: 49)
GPR-UM8: 5'-ACGAACGTACGGACGCTCGATCGTTCGGTCGCCTGCCGAGAACGAGGCGTTGTC-3' (SEQ ID NO: 50)
FBN-UM8: 5'-ACGAACGTACGGACGCTCGATCGTTCGGTCGCCAGCCGACGAAGGGCTTATTAG-3' (SEQ ID NO: 51)

Then an oligonucleotide UBC having the nucleotide sequence of SEQ ID NO: 52 labeled with FITC at 5'-end, an oligonucleotide GPR having the nucleotide sequence of SEQ ID NO: 53 labeled with FITC at 5'-end, and an oligonucleotide FBN having the nucleotide sequence of SEQ ID NO: 54 labeled with FITC at 5'-end were synthesized, and 1 pmol/50 µL solutions in TE buffer were prepared (UBC-FITC solution, FBN-FITC solution, and GPR-FITC solution, respectively).

### <5'-end FITC-labeled oligonucleotides>

UBC: 5'-TCTGGATGTTGTAGTCAGACAG-3' (SEQ ID NO: 52)
GPR: 5'-GACAACGCCTCGTTCTCGG-3' (SEQ ID NO: 53)
FBN: 5'-CTAATAAGCCCTTCGTCGGCT-3' (SEQ ID NO: 54)

For each obtained solution, the following treatment was executed (each solution was prepared singly).

Preparation of UBC mixed solution: A PCR tube was added with 10 µL of an oligonucleotide solution prepared above (UBC-M8 or UBC-U8 solution), 50 µL of a 5'-end FITC-labeled oligonucleotide solution (UBC-FITC solution) prepared above, and 5 µL of a buffer (330 mM Tris-Acetate pH 7.9, 660 mM KOAc, 100 mM MgOAc₂, 5 mM Dithothreitol), and the resultant mixture was added with sterile ultrapure water to a liquid volume of 100 µL, and mixed.

Preparation of FBN mixed solution: A PCR tube was added with 10 µL of an oligonucleotide solution prepared above (FBN-M8 or FBN-U8 solution), 50 µL of a 5'-end FITC-labeled oligonucleotide solution (FBN-FITC solution) prepared above, and 5 µL of a buffer (330 mM Tris-Acetate pH 7.9, 660 mM KOAc, 100 mM MgOAc₂, 5 mM Dithothreitol), and the resultant mixture was added with sterile ultrapure water to a liquid volume of 100 µL, and mixed.

Preparation of GPR mixed solution: A PCR tube was added with 10 µL of an oligonucleotide solution prepared above (GPR-M8 or GPR-U8 solution), 50 µL of a 5'-end FITC-labeled oligonucleotide solution (GPR-FITC solution) prepared above, and 5 µL of a buffer (330 mM Tris-Acetate pH 7.9, 660 mM KOAc, 100 mM MgOAc₂, 5 mM Dithothreitol), and the resultant mixture was added with sterile ultrapure water to a liquid volume of 100 µL, and mixed.

Each PCR tube was heated at 95°C for 10 minutes, rapidly cooled to 70°C, and kept at this temperature for 10 minutes. Then the tube was cooled to 50°C and kept at this temperature for 10 minutes, and kept at 37°C for 10 minutes, and returned to room temperature, to promote formation of a bound body of a 5'-end FITC-labeled oligonucleotide and a methylated or unmethylated oligonucleotide.

The whole of the solution in the PCR tube containing a bound body of a 5'-end FITC-labeled oligonucleotide and a methylated or unmethylated oligonucleotide was transferred to a plate in which a biotin-labeled methylated cytosine antibody preliminarily prepared is immobilized, and left still for about an hour at room temperature. After being left still, the solution in the plate was removed by decantation, and then 200 µL of a washing buffer [0.05% Tween 20-containing phosphate buffer (1 mM KH₂PO₄, 3 mM Na₂HPO·7H₂O, 154 mM NaCl, pH 7.4)] was added to each well in the plate, and then the buffer was removed from each well by decantation. This operation was repeated another two times.

Then, after adding 100 µL of an HRP-labeled FITC antibody solution [available from Jackson ImmunoResearch Laboratories, a 0.01 µg/100 µL solution in a 0.1% BSA-containing phosphate buffer (1 mM KH₂PO₄, 3 mM Na₂HPO·7H₂O, 154 mM NaCl, pH 7.4)] to each of the wells, the plate was left still for an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, 100 µL of a substrate (available from R&D, #DY999) was added and mixed to each well to start a reaction.

Then after being left still for about 5 minutes at room temperature, each well was added and mixed with 50 µL of a stop solution (1 N H₂SO₄ aqueous solution) to stop the reaction. The absorbance at 450 nm of the sample obtained within 30 minutes after stop of the reaction was measured.

The results are shown in Fig. 12 to Fig. 14. It was revealed that in every case of UBC (Fig. 12), GPR (Fig. 13), and FBN (Fig. 14), in methylated fragments UBC-M8, GPR-M8 and FBN-M8, by forming and separating a complex of an immobilized methylated cytosine antibody and a 5'-end FITC-labeled oligonucleotide, and detecting and quantifying the 5'-end FITC-labeled oligonucleotide in the complex according to its identification function, the methylated oligonucleotides UBC-M8, GPR-M8 and FBN-M8 can be detected and quantified with high sensitivity in any concentration. In the unmethylated fragments UBC-U8, GPR-U8 and FBN-MU, since a complex of an immobilized methylated cytosine antibody and a 5'-end FITC-labeled oligonucleotide is not formed and separated, a value approximately equal to the background value was exhibited in any case.

From the above, it was revealed that by forming and separating a complex of an immobilized methylated cytosine antibody, a methylated DNA fragment, and a 5'-end FITC-labeled oligonucleotide, and detecting and quantifying the 5'-end FITC-labeled oligonucleotide in the complex according to its identification function, it is possible to detect and quantify a methylated DNA fragment.

### Example 7

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

The synthetically obtained biotin-labeled methylated cytosine antibody solution was added to a 96-well plate coated with streptavidin in an amount of 100 µL/well, and immobilized to wells by leaving the solution still for about an hour at room temperature. After being left still, the solution was removed from each well by decantation, and then 300 µL of DELFIA Wash Concentrate (available from Perkin Elmer, Tris-HCl pH 7.8 with Tween 80) x25 diluted with sterile ultrapure water was added, and the buffer was removed from each well by decantation. This operation was repeated another two times.

A methylated oligonucleotide UBC-M having the nucleotide sequence of SEQ ID NO: 55, a partially methylated oligonucleotide UBC-HM having the nucleotide sequence of SEQ ID NO: 56, and an unmethylated oligonucleotide UBC-UM having the nucleotide sequence of SEQ ID NO: 57 were synthesized, and the following solutions were prepared for each oligonucleotide.
Solution A: 0.1 pmoL/10 µL solution in TE buffer
Solution B: 0.01 pmoL/10 µL solution in TE buffer
Solution C: 0.001 pmoL/10 µL solution in TE buffer
Solution D: 0 pmoL/10 µL solution in TE buffer (negative control solution)

<Methylated oligonucleotide> N represents methylated cytosine.

<Partially methylated oligonucleotide> N represents methylated cytosine.

### <Unmethylated oligonucleotide>

For each of the obtained solutions, the following treatment was executed (each solution was prepared in duplicate).

Group A (no treatment group): To 10 µL of the obtained solution, 5 µL of a buffer (330 mM Tris-Acetate pH 7.9, 660 mM KOAc, 100 mM MgOAc₂, 5 mM Dithothreitol), and 5 µL of BSA (Bovine serum albumin 1 mg/ml) were added, and then 10 pmol of an oligonucleotide CA1 having the nucleotide sequence of SEQ ID NO: 58 was added as a masking oligonucleotide, and the mixture was further added with sterile ultrapure water to a liquid volume of 50 µL, and mixed.

Group B (HhaI treatment group): To 10 µL of the obtained solution, 6 U of HhaI, 5 µL of a buffer (330 mM Tris-Acetate pH 7.9, 660 mM KOAc, 100 mM MgOAc₂, 5 mM Dithothreitol), and 5 µL of 10 x BSA (Bovine serum albumin 1 mg/ml) were added, and then 10 pmol of an oligonucleotide CA1 having the nucleotide sequence of SEQ ID NO: 58 was added as a masking oligonucleotide, and the mixture was further added with sterile ultrapure water to a liquid volume of 50 µL, and mixed.

### <Masking oligonucleotide>

CA1: 5'-GATGGCGCTCTG-3' (SEQ ID NO: 58)

After incubating each reaction solution at 37°C for 3 hours, 50 µL of a 1 pmol/50 µL solution in TE buffer of an oligonucleotide UBC having the nucleotide sequence of SEQ ID NO: 59 preliminarily synthesized and labeled with FITC at 5'-end was added to the PCR tube.

### <5'-end FITC-labeled oligonucleotide>

UBC: 5'-TCTGGATGTTGTAGTCAGACAG-3' (SEQ ID NO: 59)

Then the PCR tube was heated at 95°C for 10 minutes, rapidly cooled to 70°C, and kept at this temperature for 10 minutes. Then the tube was cooled to 50°C and kept at this temperature for 10 minutes, and kept at 37°C for 10 minutes, and returned to room temperature, to promote formation of a bound body of a 5'-end FITC-labeled oligonucleotide and an oligonucleotide.

The whole of the solution in the PCR tube containing a bound body of a 5'-end FITC-labeled oligonucleotide and an oligonucleotide was transferred to a plate in which a biotin-labeled methylated cytosine antibody preliminarily prepared is immobilized, and left still for about an hour at room temperature. After being left still, the solution in the plate was removed by decantation, and then 300 µL of DELFIA Wash Concentrate (available from Perkin Elmer, Tris-HCl pH 7.8 with Tween 80) x25 diluted with sterile ultrapure water was added to each well in the plate, and then the buffer was removed from each well by decantation. This operation was repeated another two times.

Then, after adding 100 µL of an HRP-labeled FITC antibody solution [available from Jackson ImmunoResearch Laboratories, a 0.01 µg/100 µL solution in a 0.1% BSA-containing phosphate buffer (1 mM KH₂PO₄, 3 mM Na₂HPO·7H₂O, 154 mM NaCl, pH 7.4)] to each of the wells, the plate was left still for an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, 100 µL of a substrate (available from R&D, #DY999) was added and mixed to each well to start a reaction.

Then after being left still for about 10 minutes at room temperature, each well was added and mixed with 50 µL of a stop solution (1 N H₂SO₄ aqueous solution) to stop the reaction. The absorbance at 450 nm of the sample obtained within 30 minutes after stop of the reaction was measured.

The results are shown in Fig. 15 and Fig. 16. In the case of Group A (no treatment group) (Fig. 15), it was revealed that in the methylated oligonucleotide UBC-M where all of three sites among three sites of CG sequences are methylated, a complex of a 5'-end FITC-labeled oligonucleotide UBC and an immobilized methylated cytosine antibody is formed and separated, and detected and quantified with high sensitivity. On the other hand, in a partially methylated oligonucleotide UBC-HM where only one site among three sites of CG sequences is methylated, a complex of a 5'-end FITC-labeled oligonucleotide UBC and an immobilized methylated cytosine antibody is formed and separated, however, detection and quantification results were inferior to those of UBC-M possibly due to the lower complex forming rate than that of UBC-M because only one site is methylated. In the unmethylated oligonucleotide UBC-UM where no site among three sites of CG sequences is methylated, a complex is not formed and separated, so that a value approximately equal to the background value (data of Solution D) was exhibited. In the case of Group B (HhaI treatment group) (Fig. 16), in the methylated oligonucleotide UBC-M where all of three sites among three sites of CG sequences are methylated, it was revealed that a complex of a 5'-end FITC-labeled oligonucleotide UBC and an immobilized methylated cytosine antibody is formed and separated, and detected and quantified with sensitivity as good as the value in Group A (no treatment group) because it is not digested by a methylation sensitive restriction enzyme (HhaI). In a partially methylated oligonucleotide UBC-HM where only one site among three sites of CG sequences is methylated, methylated CG sequences are lost due to digestion by the methylation sensitive restriction enzyme (HhaI). Therefore, a complex is not formed and separated, so that a value approximately the same with the background value (data of Solution D) was exhibited. In the unmethylated oligonucleotide UBC-UM where no site among three sites of CG sequences is methylated, a value approximately the same with the background value was exhibited as is the case with A treatment group (no treatment group) because a complex is not formed and separated.

From the above, it was revealed that a partially methylated DNA fragment can be digested by treatment using a methylation sensitive restriction enzyme, and a methylated DNA fragment can be detected and quantified by forming and separating a complex of an immobilized methylated cytosine antibody, a methylated DNA fragment, and a 5'-end FITC-labeled oligonucleotide, and detecting and quantifying the 5'-end FITC-labeled oligonucleotide in the complex according to its identification function.

### Example 8

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

The synthetically obtained biotin-labeled methylated cytosine antibody solution was added to a 96-well plate coated with streptavidin in an amount of 100 µL/well, and immobilized to wells by leaving the solution still for about an hour at room temperature. After being left still, the solution was removed from each well by decantation, and then each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

A methylated oligonucleotide UBC/GPR/FBN-M having the nucleotide sequence of SEQ ID NO: 60, and an unmethylated oligonucleotide UBC/GPR/FBN-UM having the nucleotide sequence of SEQ ID NO: 61 were synthesized, and the following solutions were prepared for each oligonucleotide.
Solution A: 0.1 pmoL/10 µL solution in TE buffer
Solution B: 0.01 pmoL/10 µL solution in TE buffer
Solution C: 0.001 pmoL/10 µL solution in TE buffer
Solution D: 0 pmoL/10 µL solution in TE buffer (negative control solution)

<Methylated oligonucleotide> N represents methylated cytosine.
UBC/GPR/FBN-M: 5'-CTGTCTGACTACAACATCCAGANGCCGAGAACGAGGCGTTGTCNGAGCCGACGAAGGGCTTA TTAG-3' (SEQ ID NO: 60)

### <Unmethylated oligonucleotide>

UBC/GPR/FBN-UM: 5'-CTGTCTGACTACAACATCCAGACGCCGAGAACGAGGCGTTGTCCGAGCCGACGAAGGGCTTA TTAG-3' (SEQ ID NO: 61)

Then, 1 pmol/50 µL solutions of oligonucleotides UBC, FBN and GPR labeled with FITC at 5'-end having the nucleotide sequences of SEQ ID NO: 62, SEQ ID NO: 63 and SEQ ID NO: 64 in TE buffer (UBC-FITC solution, FBN-FITC solution and GPR-FITC solution) were prepared. Also an FITC-labeled oligonucleotide solution (FITC-labeled oligonucleotide mixed solution) in which solutions of 5'-end FITC-labeled oligonucleotides UBC, FBN and GPR having the nucleotide sequences of SEQ ID NO: 62, SEQ ID NO: 63 and SEQ ID NO: 64 in TE buffer are mixed (each 1 pmol/50 µL) was prepared.

### <5'-end FITC-labeled oligonucleotides>

UBC: 5'-TCTGGATGTTGTAGTCAGACAG-3' (SEQ ID NO: 62)
GPR: 5'-GACAACGCCTCGTTCTCGG-3' (SEQ ID NO: 63)
FBN: 5'-CTAATAAGCCCTTCGTCGGCT-3' (SEQ ID NO: 64)

The following treatment was executed on each obtained solution.

To a new PCR tube, 10 µL of a methylated oligonucleotide solution or an unmethylated oligonucleotide solution prepared as described above (each solution was prepared in octuplicate), 10 µL of a buffer (330 mM Tris-Acetate pH 7.9, 660 mM KOAc, 100 mM MgOAc₂, 5 mM Dithothreitol), and 50 µL of a 5'-end FITC-labeled oligonucleotide solution prepared as described above (UBC-FITC solution, FBN-FITC solution, GPR-FITC solution, and FITC-labeled oligonucleotide mixed solution) were added, and the resultant mixture was added with sterile ultrapure water to a liquid volume of 100 µL, and mixed.

Then the PCR tube was heated at 95°C for 10 minutes, rapidly cooled to 70°C, and kept at this temperature for 10 minutes. Then the tube was cooled to 50°C and kept at this temperature for 10 minutes, and kept at 37°C for 10 minutes, and returned to room temperature, to promote formation of a bound body of a 5'-end FITC-labeled oligonucleotide and an oligonucleotide.

The whole of the solution in the PCR tube containing a bound body of a 5'-end FITC-labeled oligonucleotide and an oligonucleotide was transferred to a plate in which a biotin-labeled methylated cytosine antibody preliminarily prepared is immobilized, and left still for about 2 hours at room temperature. After being left still, the solution in the plate was removed by decantation, and then each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, after adding 100 µL of an HRP-labeled FITC antibody solution [available from Jackson ImmunoResearch Laboratories, a 0.01 µg/100 µL solution in a 0.1% BSA-containing phosphate buffer (1 mM KH₂PO₄, 3 mM Na₂HPO·7H₂O, 154 mM NaCl, pH 7.4)] to each of the wells, the plate was left still for about an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, 100 µL of a substrate (available from R&D, #DY999) was added and mixed to each well to start a reaction.

Then after being left still for about 5 minutes at room temperature, each well was added and mixed with 50 µL of a stop solution (1 N H₂SO₄ aqueous solution) to stop the reaction. The absorbance at 450 nm of the sample obtained within 30 minutes after stop of the reaction was measured.

The results are shown in Fig. 17 to Fig. 20. In the methylated oligonucleotide UBC/GPR/FBN-M, and the unmethylated oligonucleotide UBC/GPR/FBN-U, the 5'-end FITC-labeled oligonucleotides UBC, GPR and FBN are oligonucleotides capable of forming a double strand (respectively having a complementary nucleotide sequence in the oligonucleotide). In every case where the 5'-end FITC-labeled oligonucleotides UBC (Fig. 17), GPR (Fig. 18) and FBN (Fig. 19), and the mixture of the 5'-end FITC-labeled oligonucleotides (Fig. 20) were used, it was revealed that in the methylated fragment UBC/GPR/FBN-M, by forming and selecting a complex of an immobilized methylated cytosine antibody and a 5'-end FITC-labeled oligonucleotide and detecting and quantifying the 5'-end FITC-labeled oligonucleotide in the complex according to its identification function, it is possible to detect and quantify the methylated oligonucleotide/GPR/FBN-M with excellent sensitivity in any concentration. In an unmethylated fragment UBC/GPR/FBN-U, since a complex of an immobilized methylated cytosine antibody and a 5'-end FITC-labeled oligonucleotide is not formed and separated, a value approximately equal to the background value was exhibited. Further, it was revealed that by using a plurality of 5'-end FITC-labeled oligonucleotides (Fig. 20), detection and quantification with higher sensitivity are realized compared with the case using only one kind of (single) immobilized 5'-end biotin-labeled oligonucleotide is used (Fig. 17 to Fig. 19).

From the above, it was revealed that by forming and separating a complex of an immobilized methylated cytosine antibody, a methylated DNA fragment, and a 5'-end FITC-labeled oligonucleotide, and detecting and quantifying the 5'-end FITC-labeled oligonucleotide in the complex according to its identification function, it is possible to detect and quantify a methylated DNA fragment. Further, by using a plurality of 5'-end FITC-labeled oligonucleotides for one target DNA region (in other words, by using a plurality of the present oligonucleotides concurrently for one target DNA region), detection and quantification with better sensitivity are realized compared with the case where only one kind of (single) 5'-end FITC-labeled oligonucleotide is used.

### Example 9

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

The synthetically obtained biotin-labeled methylated cytosine antibody solution was added to a 96-well plate coated with streptavidin in an amount of 100 µL/well, and immobilized to wells by leaving the solution still for about an hour at room temperature. After being left still, the solution was removed from each well by decantation, and then each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

A methylated oligonucleotide UBC-M8 having the nucleotide sequence of SEQ ID NO: 65, a methylated oligonucleotide FBN-M8 having the nucleotide sequence of SEQ ID NO: 66, a methylated oligonucleotide GPR-M8 having the nucleotide sequence of SEQ ID NO: 67, an unmethylated oligonucleotide UBC-U8 having the nucleotide sequence of SEQ ID NO: 68, an unmethylated oligonucleotide FBN-U8 having the nucleotide sequence of SEQ ID NO: 69, and an unmethylated oligonucleotide GPR-U8 having the nucleotide sequence of SEQ ID NO: 70 were synthesized, and the following solutions were prepared for each of methylated oligonucleotides and unmethylated oligonucleotides.
Solution A: 0.3 pmol/10 µL solution in TE buffer
Solution B: 0.03 pmol/10 µL solution in TE buffer
Solution C: 0.003 pmol/10 µL solution in TE buffer
Solution D: TE buffer solution (negative control solution)

Then, for each solution (Solution A to Solution D), a methylated oligonucleotide mixed solution M0 in which equivalent amounts of respective solutions of the methylated oligonucleotides UBC-M8, FBN-M8, and GPR-M8 are mixed, and a methylated oligonucleotide mixed solution U0 in which equivalent amounts of respective solutions of the unmethylated oligonucleotides UBC-U8, FBN-U8, and GPR-U8 are mixed were prepared.

<Methylated oligonucleotides> N represents methylated cytosine.
UBC-M8: 5'-ANGAANGTANGGANGCTNGATNGTTNGGTNGCCTGTCTGACTACAACATCCAGA-3' (SEQ ID NO: 65)
FBN-M8: 5'-ANGAANGTANGGANGCTNGATNGTTNGGTNGCCAGCCGACGAAGGGCTTATTAG-3' (SEQ ID NO: 66)
GPR-M8: 5'-ANGAANGTANGGANGCTNGATNGTTNGGTNGCCTGCCGAGAACGAGGCGTTGTC-3' (SEQ ID NO: 67)

### <Unmethylated oligonucleotides>

UBC-U8: 5'-ACGAACGTACGGACGCTCGATCGTTCGGTCGCCTGTCTGACTACAACATCCAGA-3' (SEQ ID NO: 68)
FBN-U8: 5'-ACGAACGTACGGACGCTCGATCGTTCGGTCGCCAGCCGACGAAGGGCTTATTAG-3' (SEQ ID NO: 69)
GPR-U8: 5'-ACGAACGTACGGACGCTCGATCGTTCGGTCGCCTGCCGAGAACGAGGCGTTGTC-3' (SEQ ID NO: 70)

Then, 1 pmol/50 µL solutions of oligonucleotides UBC, FBN and GPR labeled with FITC at 5'-end having the nucleotide sequences of SEQ ID NO: 71, SEQ ID NO: 72 and SEQ ID NO: 73 in TE buffer (UBC-FITC solution, FBN-FITC solution and GPR-FITC solution) were prepared. Also an FITC-labeled oligonucleotide solution (FITC-labeled oligonucleotide mixed solution) in which solutions of 5'-end FITC-labeled oligonucleotides UBC, FBN and GPR having the nucleotide sequences of SEQ ID NO: 71, SEQ ID NO: 72 and SEQ ID NO: 73 in TE buffer are mixed (each 1 pmol/50 µL) was prepared.

### <5'-end FITC-labeled oligonucleotides>

UBC: 5'-TCTGGATGTTGTAGTCAGACAG-3' (SEQ ID NO: 71)
GPR: 5'-GACAACGCCTCGTTCTCGG-3' (SEQ ID NO: 72)
FBN: 5'-CTAATAAGCCCTTCGTCGGCT-3' (SEQ ID NO: 73)

The following treatment was executed on each obtained solution.

To a new PCR tube, 10 µL of oligonucleotide solutions prepared as described above (MA, MB, MC, MD, UA, UB, UC, UD), 10 µL of a buffer (330 mM Tris-Acetate pH 7.9, 660 mM KOAc, 100 mM MgOAc₂, 5 mM Dithothreitol), and 50 µL of a 5'-end FITC-labeled oligonucleotide solution prepared as described above (UBC-FITC solution, FBN-FITC solution, GPR-FITC solution, and FITC-labeled oligonucleotide mixed solution) were added, and further the resultant mixture was added with sterile ultrapure water to a liquid volume of 100 µL, and mixed.

Then the PCR tube was heated at 95°C for 10 minutes, rapidly cooled to 70°C, and kept at this temperature for 10 minutes. Then the tube was cooled to 50°C and kept at this temperature for 10 minutes, and kept at 37°C for 10 minutes, and returned to room temperature, to promote formation of a bound body of a 5'-end FITC-labeled oligonucleotide and an oligonucleotide.

The whole of the solution in the PCR tube containing a bound body of a 5'-end FITC-labeled oligonucleotide and an oligonucleotide was transferred to a plate in which a biotin-labeled methylated cytosine antibody preliminarily prepared is immobilized, and left still for about an hour at room temperature. After being left still, the solution in the plate was removed by decantation, then each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, after adding 100 µL of an HRP-labeled FITC antibody solution [available from Jackson ImmunoResearch Laboratories, a 0.01 µg/100 µL solution in a 0.1% BSA-containing phosphate buffer (1 mM KH₂PO₄, 3 mM Na₂HPO·7H₂O, 154 mM NaCl, pH 7.4)] to each well, the plate was left still for about an hour at room temperature. After being left still, the solution was removed from each well by decantation, and each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, 100 µL of a substrate (available from R&D, #DY999) was added and mixed to each well to start a reaction.

Then after being left still for about 5 minutes at room temperature, each well was added and mixed with 50 µL of a stop solution (1 N H₂SO₄ aqueous solution) to stop the reaction. The absorbance at 450 nm of the sample obtained within 30 minutes after stop of the reaction was measured.

The results are shown in Fig. 21 to Fig. 24. In the methylated oligonucleotide mixed solution M0, it was revealed that a complex is formed and separated between the 5'-end FITC-labeled oligonucleotide UBC (Fig. 21), FBN (Fig. 22), GPR (Fig. 23) and the oligonucleotides in which the foregoing three kinds are mixed (Fig. 24), and a methylated cytosine antibody, and detection and quantification with excellent sensitivity are realized. In particular, when three kinds of oligonucleotides are mixed (Fig. 24), it was revealed that detection and quantification with better sensitivity are realized compared with the case of detection by single oligonucleotide (Fig. 21 to Fig. 23). In the unmethylated oligonucleotide mixed solution U0, since a complex is not formed between the immobilized 5'-end biotin-labeled oligonucleotide UBC (Fig. 21), FBN (Fig. 22), GPR (Fig. 23) and the oligonucleotide in which the foregoing three kinds are mixed (Fig. 24), and a methylated cytosine antibody, a value approximately equal to the background value was exhibited in every case.

From the above, it was revealed that by forming and separating a complex of an immobilized methylated cytosine antibody, a methylated DNA fragment, and a 5'-end FITC-labeled oligonucleotide, and detecting and quantifying the 5'-end FITC-labeled oligonucleotide in the complex according to its identification function, it is possible to detect and quantify a methylated DNA fragment. It was also revealed that by using a plurality of 5'-end FITC-labeled oligonucleotides (that is, by not only using one target DNA region, but also using a plurality of target DNA regions concurrently), detection and quantification with high sensitivity are realized compared with the case where one kind of 5'-end FITC-labeled oligonucleotide is used.

### Example 10

The following experiment was conducted using a commercially available solution of a chromatostrip coated with streptavidin and a conjugate (an FITC antibody labeled with gold nano particles) in Bed-Side ICAN (TM) Legionella Detection Kit from TAKARA BIO INC.

A methylated oligonucleotide UBC-M8 having the nucleotide sequence of SEQ ID NO: 74, and an unmethylated oligonucleotide UBC-U8 having the nucleotide sequence of SEQ ID NO: 75 were synthesized, and a 0.1 pmol/µL solution in TE buffer was prepared for each oligonucleotide. Also, a TE buffer solution was prepared as a negative control.

<Methylated oligonucleotide> N represents methylated cytosine.
UBC-M8: 5'-ANGAANGTANGGANGCTNGATNGTTNGGTNGCCTGTCTGACTACAACATCCAGA-3' (SEQ ID NO: 74)
<Unmethylated oligonucleotide> UBC-U8: 5'-ACGAACGTACGGACGCTCGATCGTTCGGTCGCCTGTCTGACTACAACATCCAGA-3' (SEQ ID NO: 75)

A 0.5 pmol/µL solution of a 5'-end biotin-labeled oligonucleotide UBC having the nucleotide sequence of SEQ ID NO: 76 in TE buffer was prepared (UBC solution).

### <5'-end biotin-labeled oligonucleotide>

UBC: 5'-TCTGGATGTTGTAGTCAGACAG-3' (SEQ ID NO: 76)

Using the methylated oligonucleotide solution, and the unmethylated oligonucleotide solution, the following treatment was conducted (each solution was prepared singly).

To a PCR tube, 2 µL of an oligonucleotide solution prepared as described above (UBC-M8, a UBC-U8 solution, a TE buffer solution), 2 L of a 5'-end biotin-labeled oligonucleotide solution prepared as described above (UBC-B solution), 2 µL of a buffer (330 mM Tris-Acetate pH 7.9, 660 mM KOAc, 100 mM MgOAc₂, 5 mM Dithothreitol), and 2 µL of BSA (Bovine serum albumin 1 mg/mL) were added, followed by addition of 5 pmol of an oligonucleotide CA1 having the nucleotide sequence of SEQ ID NO: 77 as carrier DNA, and the resultant mixture was further added with sterile ultrapure water to a liquid volume of 20 µL, and mixed.

### <Carrier DNA>

CA1: 5'-GATGGCGCTCTG-3' (SEQ ID NO: 77)

Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end biotin-labeled oligonucleotide and an oligonucleotide.

The whole of the obtained reaction solution was transferred to a well of a flat-bottom plate, and a tip end of a streptavidin-coated chromatostrip was inserted to the bottom of each well, and developed (the entire solution was absorbed by the strip).

In another well of the flat-bottom plate charged with 20 µL of a methylated cytosine antibody [available from Aviva Systems Biology, 0.1 µg/20 µL solution in a 0.1% BSA-containing phosphate buffer (1 mM KH₂PO₄, 3 mM Na₂HPO·7H₂O, 154 mM NaCl, pH 7.4)], a tip end of a streptavidin-coated chromatostrip obtained as described above was inserted to the bottom of each well, and further developed.

In still another well of the flat-bottom plate charged with 20 µL of an FITC-labeled mouse IgG antibody (available from Medical & Biological Laboratories) solution [0.2 µg/20 µL in a 0.1% BSA-containing phosphate buffer (1 mM KH₂PO₄, 3 mM N₂HPO·7H₂O, 154 mM NaCl, pH 7.4)], a tip end of a streptavidin-coated chromatostrip obtained as described above was inserted to the bottom of each well, and further developed.

Next, in another well of the flat-bottom plate charged with 20 µL of a 0.1% BSA-containing phosphate buffer (1 mM KH₂PO₄, 3 mM Na₂HPO·7H₂O, 154 mM NaCl, pH 7.4) solution, a tip end of a streptavidin-coated chromatostrip obtained as described above was inserted to the bottom of each well, and further developed.

Thereafter, in another well of the flat-bottom plate charged with 20 µL of a conjugate solution (gold nano particle-labeled FITC antibody solution), a tip end of a streptavidin-coated chromatostrip obtained as described above was inserted to the bottom of each well, and further developed.

After completion of development, the streptavidin-coated chromatostrip was visually checked and presence or absence of a red-purple line was determined.

The results are shown in Fig. 25. In the case of the methylated oligonucleotide UBC-M8, a red-purple line was observed on the streptavidin-coated chromatostrip. This reveals that a complex of a methylated DNA antibody, an immobilized 5'-end biotin-labeled oligonucleotide, and a methylated DNA fragment is formed and separated. In the unmethylated oligonucleotide UBC-U8, and the negative control (TE buffer solution), a red-purple line was not observed because a complex is not formed.

From the above, it was revealed that a methylated DNA fragment can be detected and quantified on a chromatostrip by forming and separating a complex of a methylated cytosine antibody, a methylated DNA fragment and an immobilized 5'-end biotin-labeled oligonucleotide, and detecting and quantifying the methylated cytosine antibody in the complex according to its identification function.

### Example 11

The following experiment was conducted using a commercially available solution of a chromatostrip coated with streptavidin and a conjugate (an FITC antibody labeled with gold nano particles) in Bed-Side ICAN (TM) Legionella Detection Kit from TAKARA BIO INC.

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂0, 154 mM NaCl, pH 7.4)].

A methylated oligonucleotide UBC-M8 having the nucleotide sequence of SEQ ID NO: 78, a methylated oligonucleotide FBN-M8 having the nucleotide sequence of SEQ ID NO: 79, a methylated oligonucleotide GPR-M8 having the nucleotide sequence of SEQ ID NO: 80, an unmethylated oligonucleotide UBC-U8 having the nucleotide sequence of SEQ ID NO: 81, an unmethylated oligonucleotide FBN-U8 having the nucleotide sequence of SEQ ID NO: 82, and an unmethylated oligonucleotide GPR-U8 having the nucleotide sequence of SEQ ID NO: 83 were synthesized, and a 0.1 pmol/µL solution in TE buffer was prepared for each oligonucleotide. Also, a TE buffer solution was used as a negative control.

<Methylated oligonucleotides> N represents methylated cytosine.
UBC-M8: 5'-ANGAANGTANGGANGCTNGATNGTTNGGTNGCCTGTCTGACTACAACATCCAGA-3' (SEQ ID NO: 78)
FBN-M8: 5'-ANGAANGTANGGANGCTNGATNGTTNGGTNGCCAGCCGACGAAGGGCTTATTAG-3' (SEQ ID NO: 79)
GPR-M8: 5'-ANGAANGTANGGANGCTNGATNGTTNGGTNGCCTGCCGAGAACGAGGCGTTGTC-3' (SEQ ID NO: 80)

### <Unmethylated oligonucleotides>

UBC-U8: 5'-ACGAACGTACGGACGCTCGATCGTTCGGTCGCCTGTCTGACTACAACATCCAGA-3' (SEQ ID NO: 81)
FBN-U8: 5'-ACGAACGTACGGACGCTCGATCGTTCGGTCGCCAGCCGACGAAGGGCTTATTAG-3' (SEQ ID NO: 82)
GPR-U8: 5'-ACGAACGTACGGACGCTCGATCGTTCGGTCGCCTGCCGAGAACGAGGCGTTGTC-3' (SEQ ID NO: 83)

0.5 pmol/µL solutions of oligonucleotides UBC, FBN and GPR labeled with FITC at 5'-end having the nucleotide sequences of SEQ ID NO: 84, SEQ ID NO: 85 and SEQ ID NO: 86 in TE buffer (UBC-FITC solution, FBN-FITC solution and GPR-FITC solution) were prepared.

### <5'-end FITC-labeled oligonucleotides>

UBC: 5'-TCTGGATGTTGTAGTCAGACAG-3' (SEQ ID NO: 84)
FBN: 5'-CTAATAAGCCCTTCGTCGGCT-3' (SEQ ID NO: 85)
GPR: 5'-GACAACGCCTCGTTCTCGG-3' (SEQ ID NO: 86)

For each of the methylated oligonucleotide solutions, and the unmethylated oligonucleotide solutions, the following treatment was conducted (each solution was prepared singly).

Preparation of UBC mixed solution: To a PCR tube, 2 µL of an oligonucleotide solution prepared as described above (UBC-M8 or UBC-U8 solution), 2 µL of a 5'-end FITC-labeled oligonucleotide solution prepared as described above (UBC-FITC solution), 2 µL of a buffer (330 mM Tris-Acetate pH 7.9, 660 mM KOAc, 100 mM MgOAc₂, 5 mM Dithothreitol), and 2 µL of BSA (Bovine serum albumin 1 mg/mL) were added, followed by addition of 5 pmol of an oligonucleotide CA1 having the nucleotide sequence of SEQ ID NO: 87 as carrier DNA, and the resultant mixture was added with sterile ultrapure water to a liquid volume of 20 µL, and mixed.

Preparation of FBN mixed solution: To a PCR tube, 2 µL of an oligonucleotide solution prepared as described above (FBN-M8 or FBN-U8 solution), 2 µL of a 5'-end FITC-labeled oligonucleotide solution prepared as described above (FBN-FITC solution), 2 µL of a buffer (330 mM Tris-Acetate pH 7.9, 660 mM KOAc, 100 mM MgOAc₂, 5 mM Dithothreitol), and 2 µL of BSA (Bovine serum albumin 1 mg/mL) were added, followed by addition of 5 pmol of an oligonucleotide CA1 having the nucleotide sequence of SEQ ID NO: 87 as carrier DNA, and the resultant mixture was added with sterile ultrapure water to a liquid volume of 20 µL, and mixed.

Preparation of GPR mixed solution: To a PCR tube, 2 µL of an oligonucleotide solution prepared as described above (GPR-M8 or GPR-U8 solution), 2 µL of a 5'-end FITC-labeled oligonucleotide solution prepared as described above (GPR-FITC solution), 2 µL of a buffer (330 mM Tris-Acetate pH 7.9, 660 mM KOAc, 100 mM MgOAc₂, 5 mM Dithothreitol), and 2 µL of BSA (Bovine serum albumin 1 mg/mL) were added, followed by addition of 5 pmol of an oligonucleotide CA1 having the nucleotide sequence of SEQ ID NO: 87 as carrier DNA, and the resultant mixture was added with sterile ultrapure water to a liquid volume of 20 µL, and mixed.

### <Carrier DNA>

CA1: 5'-GATGGCGCTCTG-3' (SEQ ID NO: 87)

Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end FITC-labeled oligonucleotide and an oligonucleotide.

In another well of the flat-bottom plate charged with 20 µL of a biotin-labeled methylated cytosine antibody solution, a tip end of a streptavidin-coated chromatostrip was inserted to the bottom of each well, and developed (the entire solution was absorbed by the strip).

The whole of the reaction solution in which a bound body of a 5'-end FITC-labeled oligonucleotide and an oligonucleotide was formed was transferred to a well of a flat-bottom plate, and a tip end of a streptavidin-coated chromatostrip was inserted to the bottom of each well, and developed (the entire solution was absorbed by the strip).

In a still another well of the flat-bottom plate charged with 20 µL of a 0.1% BSA-containing phosphate buffer (1 mM KH₂PO₄, 3 mM Na₂HPO·7H₂O, 154 mM NaCl, pH 7.4) solution, a tip end of a streptavidin-coated chromatostrip obtained as described above was inserted to the bottom of each well, and further developed.

In another well of the flat-bottom plate charged with 20 µL of a conjugate solution (gold nano particle-labeled FITC antibody solution), a tip end of a streptavidin-coated chromatostrip obtained as described above was inserted to the bottom of each well, and further developed.

After completion of development, each streptavidin-coated chromatostrip was visually checked and presence or absence of a red-purple line was determined.

The results are shown in Fig. 26 to Fig. 28. In the case of the methylated oligonucleotides UBC-M8, FBN-M8 and GPR-M8, a red-purple line was observed on the streptavidin-coated chromatostrip. This reveals that a complex of an immobilized methylated DNA antibody, a 5'-end FITC biotin-labeled oligonucleotide, and a methylated DNA fragment is formed and separated. In the unmethylated oligonucleotides UBC-U8, FBN-M8 and GPR-M8, and the negative control (TE buffer solution), a red-purple line was not observed because a complex is not formed.

From the above, it was revealed that a methylated DNA fragment can be detected and quantified on a chromatostrip by forming and separating a complex of an immobilized methylated cytosine antibody, a methylated DNA fragment and a 5'-end FITC-labeled oligonucleotide, and detecting and quantifying the 5'-end FITC-labeled oligonucleotide in the complex according to its identification function.

### Example 12

The following experiment was conducted using a commercially available solution of a chromatostrip coated with streptavidin and a conjugate (an FITC antibody labeled with gold nano particles) in Bed-Side ICAN (TM) Legionella Detection Kit from TAKARA BIO INC.

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

A methylated oligonucleotide UBC-M8 having the nucleotide sequence of SEQ ID NO: 88, a methylated oligonucleotide FBN-M8 having the nucleotide sequence of SEQ ID NO: 89, a methylated oligonucleotide GPR-M8 having the nucleotide sequence of SEQ ID NO: 90, an unmethylated oligonucleotide UBC-U8 having the nucleotide sequence of SEQ ID NO: 91, an unmethylated oligonucleotide FBN-U8 having the nucleotide sequence of SEQ ID NO: 92, and an unmethylated oligonucleotide GPR-U8 having the nucleotide sequence of SEQ ID NO: 93 were synthesized, and a 0.2 pmol/µL solution in TE buffer was prepared for each oligonucleotide. Also, the TE buffer solution was used as a negative control.

<Methylated oligonucleotides> N represents methylated cytosine.
UBC-M8: 5'-ANGAANGTANGGANGCTNGATNGTTNGGTNGCCTGTCTGACTACAACATCCAGA-3' (SEQ ID NO: 88)
FBN-M8 : 5'-ANGAANGTANGGANGCTNGATNGTTNGGTNGCCAGCCGACGAAGGGCTTATTAG-3' (SEQ ID NO: 89)
GPR-M8: 5'-ANGAANGTANGGANGCTNGATNGTTNGGTNGCCTGCCGAGAACGAGGCGTTGTC-3' (SEQ ID NO: 90)

### <Unmethylated oligonucleotides>

UBC-U8: 5'-ACGAACGTACGGACGCTCGATCGTTCGGTCGCCTGTCTGACTACAACATCCAGA-3' (SEQ ID NO: 91)
FBN-U8: 5'-ACGAACGTACGGACGCTCGATCGTTCGGTCGCCAGCCGACGAAGGGCTTATTAG-3' (SEQ ID NO: 92)
GPR-U8 : 5'-ACGAACGTACGGACGCTCGATCGTTCGGTCGCCTGCCGAGAACGAGGCGTTGTC-3' (SEQ ID NO: 93)

0.5 pmol/µL solutions of oligonucleotides UBC, FBN and GPR labeled with FITC at 5'-end having the nucleotide sequences of SEQ ID NO: 94, SEQ ID NO: 95 and SEQ ID NO: 96 in TE buffer (UBC-FITC solution, FBN-FITC solution and GPR-FITC solution) were prepared.

### <5'-end FITC-labeled oligonucleotides>

UBC: 5'-TCTGGATGTTGTAGTCAGACAG-3' (SEQ ID NO: 94)
FBN: 5'-CTAATAAGCCCTTCGTCGGCT-3' (SEQ ID NO: 95)
GPR: 5'-GACAACGCCTCGTTCTCGG-3' (SEQ ID NO: 96).

For each of the methylated oligonucleotide solutions, and the unmethylated oligonucleotide solutions, the following treatment was conducted (each solution was prepared singly).

Preparation of UBC mixed solution: To a PCR tube, 2 µL of an oligonucleotide solution prepared as described above (UBC-M8 or UBC-U8 solution), 2 µL of a 5'-end FITC-labeled oligonucleotide solution prepared as described above (UBC-FITC solution), 1 µL of a buffer (330 mM Tris-Acetate pH 7.9, 660 mM KOAc, 100 mM MgOAc₂, 5 mM Dithothreitol), and 1 µL of BSA (Bovine serum albumin 1 mg/mL) were added, followed by addition of 5 pmol of an oligonucleotide CA1 having the nucleotide sequence of SEQ ID NO: 97 as carrier DNA, and the resultant mixture was added with sterile ultrapure water to a liquid volume of 10 µL, and mixed.

Preparation of FBN mixed solution: To a PCR tube, 2 µL of an oligonucleotide solution prepared as described above (FBN-M8 or FBN-U8 solution), 2 µL of a 5'-end FITC-labeled oligonucleotide solution prepared as described above (FBN-FITC solution), 1 µL of a buffer (330 mM Tris-Acetate pH 7.9, 660 mM KOAc, 100 mM MgOAc₂, 5 mM Dithothreitol), and 1 µL of BSA (Bovine serum albumin 1 mg/mL) were added, followed by addition of 5 pmol of an oligonucleotide CA1 having the nucleotide sequence of SEQ ID NO: 97 as carrier DNA, and the resultant mixture was added with sterile ultrapure water to a liquid volume of 10 µL, and mixed.

Preparation of GPR mixed solution: To a PCR tube, 2 µL of an oligonucleotide solution prepared as described above (GPR-M8 or GPR-U8 solution), 2 µL of a 5'-end FITC-labeled oligonucleotide solution prepared as described above (GPR-FITC solution), 1 µL of a buffer (330 mM Tris-Acetate pH 7.9, 660 mM KOAc, 100 mM MgOAc₂, 5 mM Dithothreitol), and 1 µL of BSA (Bovine serum albumin 1 mg/mL) were added, followed by addition of 5 pmol of an oligonucleotide CA1 having the nucleotide sequence of SEQ ID NO: 97 as carrier DNA, and the resultant mixture was added with sterile ultrapure water to a liquid volume of 10 µL, and mixed.

### <Carrier DNA>

CA1: 5'-GATGGCGCTCTG-3' (SEQ ID NO: 97)

Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end FITC-labeled oligonucleotide and an oligonucleotide.

The obtained solution was added with 10 µL of a biotin-labeled methylated cytosine antibody solution, and left still at room temperature for an hour.

The whole of the obtained reaction solution was transferred to a well of a flat-bottom plate, and a tip end of a streptavidin-coated chromatostrip was inserted to the bottom of each well, and developed (the entire solution was absorbed by the strip).

In another well of the flat-bottom plate charged with 20 µL of a 0.1% BSA-containing phosphate buffer (1 mM KH₂PO₄, 3 mM Na₂HPO·7H₂O, 154 mM NaCl, pH 7.4) solution, a tip end of a streptavidin-coated chromatostrip obtained as described above was inserted to the bottom of each well, and further developed.

In still another well of the flat-bottom plate charged with 20 µL of a conjugate solution (gold nano particle-labeled FITC antibody solution), a tip end of a streptavidin-coated chromatostrip obtained as described above was inserted to the bottom of each well, and further developed.

After completion of development, a streptavidin-coated chromatostrip was visually checked and presence or absence of a red-purple line was determined.

The results are shown in Fig. 29 to Fig. 31. In the case of the methylated oligonucleotides UBC-M8, FBN-M8 and GPR-M8, a red-purple line was observed on the streptavidin-coated chromatostrip. This reveals that a complex of an immobilized methylated DNA antibody, a 5'-end FITC biotin-labeled oligonucleotide, and a methylated DNA fragment is formed and separated. In the unmethylated oligonucleotides UBC-U8, FBN-M8 and GPR-M8, and the negative control (TE buffer solution), a red-purple line was not observed because a complex was not formed.

From the above, it was revealed that a methylated DNA fragment can be detected and quantified on a chromatostrip by forming and separating a complex of an immobilized methylated cytosine antibody, a methylated DNA fragment and a 5'-end FITC-labeled oligonucleotide, and detecting and quantifying the 5'-end FITC-labeled oligonucleotide in the complex according to its identification function.

### Example 13

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

The synthetically obtained biotin-labeled methylated cytosine antibody solution was added to a 96-well plate coated with streptavidin in an amount of 100 µL/well, and immobilized to wells by leaving the solution still for about an hour at room temperature. After being left still, the solution was removed from each well by decantation, and then 300 µL of DELFIA Wash Concentrate (available from Perkin Elmer, Tris-HCl pH 7.8 with Tween 80) x25 diluted with sterile ultrapure water was added to each well, and the buffer was removed from each well by decantation. This operation was repeated another two times.

A methylated oligonucleotide FBN-M8 having the nucleotide sequence of SEQ ID NO: 98, and an unmethylated oligonucleotide FBN-U8 having the nucleotide sequence of SEQ ID NO: 99 were synthesized, and the following solutions in TE buffer were prepared for each oligonucleotide.
Solution A: 0.1 pmol/10 µL solution in TE buffer
Solution B: 0.01 pmol/10 µL solution in TE buffer
Solution C: 0.001 pmol/10 µL solution in TE buffer
Solution D: 0 pmol/10 µL solution in TE buffer (negative control solution)

<Methylated oligonucleotide> N represents methylated cytosine.
FBN-M8: 5'-ANGAANGTANGGANGCTNGATNGTTNGGTNGCCAGCCGACGAAGGGCTTATTAG-3' (SEQ ID NO: 98)
<Unmethylated oligonucleotide> FBN-U8: 5'-ACGAACGTACGGACGCTCGATCGTTCGGTCGCCAGCCGACGAAGGGCTTATTAG-3' (SEQ ID NO: 99)

A 1 pmol/50 µL solution of a 5'-end FITC-labeled oligonucleotide FBN having the nucleotide sequence of SEQ ID NO: 100 in TE buffer was prepared (UBC-FITC solution).

### <5'-end FITC-labeled oligonucleotide>

FBN: 5'-CTAATAAGCCCTTCGTCGGCT-3' (SEQ ID NO: 100)

As a solution for promoting formation of a bound body of a 5'-end FITC-labeled oligonucleotide and an oligonucleotide, the following buffers were prepared.
Buffer 1: 100 mM Tris-HCl pH 7.5, 100 mM MgCl₂, 10 mM Dithothreitol
Buffer 2: 1 mM KH₂PO₄ pH 7.4, 3 mM Na₂HPO·7H₂O, 154 mM NaCl
Buffer 3: 10 mM Tris-HCl pH 8.0, 1 mM EDTA
Buffer 4: 330 mM Tris-Acetate pH 7.9, 660 mM KOAc, 100 mM MgOAc₂, 5 mM Dithothreitol
Buffer 5: ultrapure water

For each of the methylated oligonucleotide solutions and the unmethylated oligonucleotide solutions, the following treatment was executed (each solution was prepared singly).

To a PCR tube, 10 µL of an oligonucleotide solution prepared as described above (FBN-M8 or FBN-U8 solution), 50 µL of a 5'-end FITC-labeled oligonucleotide solution as described above (FBN-FITC solution), 5 µL of any one kind of a buffer selected from Buffers 1 to 5, and 5 µL of BSA (Bovine serum albumin 1 mg/mL) were added, followed by addition of 5 pmol of an oligonucleotide CA1 having the nucleotide sequence of SEQ ID NO: 101 as carrier DNA, and then the resultant mixture was added with sterile ultrapure water to a liquid volume of 50 µL, and mixed.

### <Carrier DNA>

CA1: 5'-GATGGCGCTCTG-3' (SEQ ID NO: 101)

Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end FITC-labeled oligonucleotide and an oligonucleotide.

The whole of the solution in the PCR tube containing a bound body of a 5'-end FITC-labeled oligonucleotide and an oligonucleotide was transferred to a plate in which a biotin-labeled methylated cytosine antibody preliminarily prepared is immobilized, and left still for about 2 hours at room temperature. After being left still, the solution in the plate was removed by decantation, and then 300 µL of DELFIA Wash Concentrate (available from Perkin Elmer, Tris-HCl pH 7.8 with Tween 80) ×25 diluted with sterile ultrapure water was added to each well in the plate, and then the buffer was removed from each well by decantation. This operation was repeated another two times.

Then, after adding 100 µL of an HRP-labeled FITC antibody solution [available from Jackson ImmunoResearch Laboratories, a 0.01 µg/100 µL solution in a 0.1% BSA-containing phosphate buffer (1 mM KH₂PO₄, 3 mM Na₂HPO·7H₂O, 154 mM NaCl, pH 7.4)] to each of the wells, the plate was left still for an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and the buffer was removed from each well by decantation. This operation was repeated another two times.

Then, 100 µL of a substrate (available from R&D, #DY999) was added and mixed to each well to start a reaction.

Then after being left still for about 10 minutes at room temperature, each well was added and mixed with 50 µL of a stop solution (1 N H₂SO₄ aqueous solution) to stop the reaction. The absorbance at 450 nm of the sample obtained within 30 minutes after stop of the reaction was measured.

The results are shown in Figs. 32 to 36. In the buffers containing magnesium ions (Buffer 1 and Buffer 4, Fig. 32 and Fig. 35), the methylated oligonucleotide FBN-M is likely to form a complex with the 5'-end biotin-labeled oligonucleotide FBN, and it was revealed that in a specimen using such buffers, a complex of an immobilized methylated DNA antibody, a 5'-end FITC biotin-labeled oligonucleotide, and a methylated DNA fragment is formed and separated, and detected and quantified with high sensitivity.

From the above, it was demonstrated that in forming a bound body of single-stranded DNA containing a target DNA region and the present oligonucleotide, use of a reaction system containing a magnesium ion is preferred.

### Example 14

A methylated oligonucleotide UBC-M8 having the nucleotide sequence of SEQ ID NO: 102, and an unmethylated oligonucleotide UBC-U8 having the nucleotide sequence of SEQ ID NO: 103 were synthesized, and a 0.01 pmol/10 µL solution in TE buffer was prepared for each oligonucleotide.

<Methylated oligonucleotide> N represents methylated cytosine.
UBC-M8: 5'-ANGAANGTANGGANGCTNGATNGTTNGGTNGCCTGTCTGACTACAACATCCAGA-3' (SEQ ID NO: 102)

### <Unmethylated oligonucleotide>

UBC-U8: 5'-ACGAACGTACGGACGCTCGATCGTTCGGTCGCCTGTCTGACTACAACATCCAGA-3' (SEQ ID NO: 103)

Also, a 5'-end biotin-labeled oligonucleotide B1 having the nucleotide sequence of SEQ ID NO: 104 was synthesized, and a 0.02 µM solution in TE buffer was prepared.

### <5'-end biotin-labeled oligonucleotide>

B1: 5'-TCTGGATGTTGTAGTCAGACAG-3' (SEQ ID NO: 104)

Also, for preparation of a solution that promotes formation of a bound body of a 5'-end biotin-labeled oligonucleotide and an oligonucleotide, the following metal salt aqueous solutions were prepared.
Metal salt solution Mg: 100 mM MgCl₂ aqueous solution
Metal salt solution Ba: 100 mM BaCl₂ aqueous solution
Metal salt solution H₂O: ultrapure water

For each of the methylated oligonucleotide solutions and the unmethylated oligonucleotide solutions, the following treatment was executed (each solution was prepared singly).

To a PCR tube, 10 µL of an oligonucleotide solution prepared as described above, 5 µL of the aforementioned 5'-end biotin-labeled oligonucleotide solution, 10 µL of a phosphate buffer (1 mM KH₂PO₄, 3 mM Na₂HPO·7H₂O, 154 mM NaCl, pH 7.4), and 60 µL of a metal salt solution (Metal salt solution Mg, metal salt solution Ba, or Metal salt solution H₂O) were added, and then the resultant mixture was added with sterile ultrapure water to a liquid volume of 100 µL, and mixed. Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end biotin-labeled oligonucleotide and an oligonucleotide.

By transferring the entire obtained mixture to a 96-well plate coated with streptavidin, and leaving it still for about 30 minutes at room temperature, the bound body of a 5'-end biotin-labeled oligonucleotide and an oligonucleotide was immobilized to the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each well was added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each well was added with 100 µL of an Eu-N1 labeled mouse IgG antibody [available from Perkin Elmer, a 0.25 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. After being left still, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 3 minutes at room temperature, the fluorescence of the obtained sample was measured with excitation 340 nm/fluorescence 612 nm.

The result is shown in Fig. 37. In metal salt solutions containing a bivalent positive ion (Metal salt solution Mg and Metal salt solution Ba), the methylated oligonucleotide UBC-M is likely to form a bound body with a 5'-end biotin-labeled oligonucleotide UBC, and it was revealed that in a specimen using such a metal salt solution, a complex of an immobilized methylated DNA antibody, a 5'-end FITC biotin-labeled oligonucleotide and a methylated DNA fragment is formed and separated, and detection and quantification with excellent sensitivity are realized.

From the above, it was demonstrated that in forming a bound body of single-stranded DNA containing a target DNA region and the present oligonucleotide, use of a reaction system containing a bivalent positive ion is preferred.

### Example 15

A methylated oligonucleotide FBN-M8 having the nucleotide sequence of SEQ ID NO: 105, and an unmethylated oligonucleotide FBN-U8 having the nucleotide sequence of SEQ ID NO: 106 were synthesized, and a 0.01 pmol/10 µL solution in TE buffer was prepared for each oligonucleotide.

<Methylated oligonucleotide> N represents methylated cytosine.
FBN-M8: 5'-ANGAANGTANGGANGCTNGATNGTTNGGTNGCCAGCCGACGAAGGGCTTATTAG-3' (SEQ ID NO: 105)

### <Unmethylated oligonucleotide>

FBN-U8: 5'-ACGAACGTACGGACGCTCGATCGTTCGGTCGCCAGCCGACGAAGGGCTTATTAG-3' (SEQ ID NO: 106)

Also, a 5'-end biotin-labeled oligonucleotide FBN having the nucleotide sequence of SEQ ID NO: 107 was synthesized, and a 0.02 µM solution in TE buffer was prepared (FBN-B solution).

### <5'-end biotin-labeled oligonucleotide>

FBN: 5'-CTAATAAGCCCTTCGTCGGCT-3' (SEQ ID NO: 107)

Also for preparation of a solution that promotes formation of a bound body of a 5'-end biotin-labeled oligonucleotide and an oligonucleotide, the following 0, 10, 20, 30, 40, 60, 80, 100 mM MgCl₂ aqueous solutions were prepared.

For each of the methylated oligonucleotide solutions and the unmethylated oligonucleotide solutions, the following treatment was executed (each solution was prepared singly).

To a PCR tube, 10 µL of an oligonucleotide solution prepared as described above, 5 µL of the aforementioned 5'-end biotin-labeled oligonucleotide solution, 10 µL of a buffer (330 mM Tris-Acetate pH 7.9, 660 mM KOAc, 100 mM MgOAc₂, 5 mM Dithothreitol), and 60 µL an MgCl₂ aqueous solution (0 to 1000 mM) were added, and then the resultant mixture was added with sterile ultrapure water to a liquid volume of 100 µL, and mixed.

Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end biotin-labeled oligonucleotide and an oligonucleotide.

By transferring the entire obtained mixture to a 96-well plate coated with streptavidin, and leaving it still for about 30 minutes at room temperature, the bound body of a 5'-end biotin-labeled oligonucleotide and an oligonucleotide was immobilized to the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each well as added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each well was added with 100 µL of an Eu-N1 labeled mouse IgG antibody [available from Perkin Elmer, a 0.25 µg/mL solution in a 0.1% BSA-containing phosphate buffer (1 mM KH_{2O}PO₄, 3 mM Na₂HPO·7H₂O, 1.54 mM NaCl, pH 7.4)], and left still for an hour at room temperature. After being left still, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 3 minutes at room temperature, the fluorescence of the obtained sample was measured with excitation 340 nm/fluorescence 612 nm.

The result is shown in Fig. 38. It was revealed that in buffers containing a magnesium ion in a final concentration of 10 mM to 70 mM, the methylated oligonucleotide FBN-M is likely to form a bound body with a 5'-end biotin-labeled oligonucleotide FBN, and in a specimen using such buffers, a complex of an immobilized methylated DNA antibody, a 5'-end FITC biotin-labeled oligonucleotide and a methylated DNA fragment is formed and separated, and detected and quantified with excellent sensitivity. In particular, it was expected that the methylated oligonucleotide FBN-M is more likely to form a bound body with the 5'-end biotin-labeled oligonucleotide FBN at a final concentration of a magnesium ion of 58 mM or more.

From the above, it was demonstrated that in forming a bound body of single-stranded DNA containing a target DNA region and the present oligonucleotide, use of a reaction system containing a magnesium ion is preferred.

### Example 16

A methylated oligonucleotide FBN-M8 having the nucleotide sequence of SEQ ID NO: 108, and an unmethylated oligonucleotide FBN-U8 having the nucleotide sequence of SEQ ID NO: 109 were synthesized, and a 0.01 pmol/10 µL solution in TE buffer was prepared for each oligonucleotide.

<Methylated oligonucleotide> N represents methylated cytosine.
FBN-M8: 5'-ANGAANGTANGGANGCTNGATNGTTNGGTNGCCAGCCGACGAAGGGCTTATTAG-3' (SEQ ID NO: 108)

### <Unmethylated oligonucleotide>

FBN-U8: 5'-ACGAACGTACGGACGCTCGATCGTTCGGTCGCCAGCCGACGAAGGGCTTATTAG-3' (SEQ ID NO: 109)

Also, a 5'-end biotin-labeled oligonucleotide FBN having the nucleotide sequence of SEQ ID NO: 110 was synthesized, and a 0.02 µM solution in TE buffer was prepared (FBN-B solution).

### <5'-end biotin-labeled oligonucleotide>

FBN: 5'-CTAATAAGCCCTTCGTCGGCT-3' (SEQ ID NO: 110)

Also for preparation of a solution that promotes formation of a bound body of a 5'-end biotin-labeled oligonucleotide and an oligonucleotide, the following 0, 100, 200, 300, 400, 600, 800, 1000 mM MgCl₂ aqueous solutions were prepared.

For each of the methylated oligonucleotide solutions and the unmethylated oligonucleotide solutions, the following treatment was executed (each solution was prepared singly).

To a PCR tube, 10 µL of an oligonucleotide solution prepared as described above, 5 µL of the aforementioned 5'-end biotin-labeled oligonucleotide solution, 10 µL of a buffer (330 mM Tris-Acetate pH 7.9, 660 mM KOAc, 100 mM MgOAc₂, 5 mM Dithothreitol), and 60 µL of an MgCl₂ aqueous solution (0 to 1000 mM) were added, and then the resultant mixture was added with sterile ultrapure water to a liquid volume of 100 µL, and mixed.

Then, the PCR tube was heated at 95°C for 10 minutes, rapidly cooled to 70°C, and kept at this temperature for 10 minutes. Further, the tube was cooled to 50°C, and kept at this temperature for 10 minutes, and then kept at 37°C for 10 minutes, and returned to room temperature, to promote formation of a bound body of a 5'-end biotin-labeled oligonucleotide and an oligonucleotide.

By transferring the entire obtained mixture to a 96-well plate coated with streptavidin, and leaving it still for about 30 minutes at room temperature, the bound body of a 5'-end biotin-labeled oligonucleotide and an oligonucleotide was immobilized to the plate. After being left still, the solution in the plate was removed by decantation, and then each well in the plate was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each well as added with 100 µL of a methylated cytosine antibody [available from Aviva Systems Biology, a 1 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then, each well was added with 100 µL of an Eu-N1 labeled mouse IgG antibody [available from Perkin Elmer, a 0.25 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. After being left still, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

By adding and mixing 200 µL of Enhancement Solution (available from Perkin Elmer) into each of the wells, a reaction was started. Then after being left still for about 3 minutes at room temperature, the fluorescence of the obtained sample was measured with excitation 340 nm/fluorescence 612 nm.

The result is shown in Fig. 39. It was revealed that in buffers containing a magnesium ion in a final concentration of 10 mM to 610 mM, the methylated oligonucleotide FBN-M is likely to form a bound body with the 5'-end biotin-labeled oligonucleotide FBN, and in a specimen using such buffers, a complex of an immobilized methylated DNA antibody, a 5'-end FITC biotin-labeled oligonucleotide and a methylated DNA fragment is formed and separated, and detected and quantified with excellent sensitivity. In particular, it was expected that the methylated oligonucleotide FBN-M is more likely to form a bound body with the 5'-end biotin-labeled oligonucleotide FBN at a final concentration of a magnesium ion ranging from 70 mM to 610 mM.

### Example 17

For genomic DNA derived from human blood purchased from Clontech, a DNA fragment (X, SEQ ID NO: 113, a region corresponding to the base numbers 25687390 to 25687775 shown in Genbank Accession No. NT_029419 and so on) to be used as a test sample was amplified by conducting PCR using oligonucleotide primers (PF1 and PR1) designed for PCR of SEQ ID NO: 111 and SEQ ID NO: 112 and the following reaction condition.

### <Oligonucleotide primers designed for PCR>

PF1: 5'-CTCAGCACCCAGGCGGCC-3' (SEQ ID NO: 111)
PR1: 5'-CTGGCCAAACTGGAGATCGC-3' (SEQ ID NO: 112)

### <DNA fragment>

As a reaction solution of PCR, 5 ng of genomic DNA which is a template, each 3 µL of oligonucleotide primer solutions prepared to 5 µµM, each 5 µL of 2 mM dNTPs, 5 µL of a 10 × buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold, available from ABI) were mixed, and sterile ultrapure water was added to a liquid volume of 50 µL. The reaction solution was kept at 95°C for 10 minutes, and then subjected to PCR conducting 40 cycles of incubation each including 30 seconds at 95°C, 30 seconds at 61°C, and 45 seconds at 72°C.

After conducting PCR, amplification was checked by 1.5% agarose gel electrophoresis, and a DNA fragment X was purified with Wizard SV Gel/PCR Kit (PROMEGA).

For a part of the obtained DNA fragment solution, a reaction solution was prepared by mixing 1 uL of SssI methylase (available from NEB), 10 uL of 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MX, SEQ ID NO: 114).

### <DNA fragment> (N represents 5-methyl cytosine)

For the obtained DNA fragment X, the following solutions were prepared in duplicate.
Solution A: 10 ng/10 µL solution in TE
Solution B: 1 ng/10 µL solution in TE
Solution C: TE solution (negative control solution)

For the obtained DNA fragment MX, the following solutions were prepared in duplicate.
Solution MA: 10 ng/10 µL solution in TE
Solution MB: 1 ng/10 µL solution in TE
Solution MC: TE solution (negative control solution)

Also a 5'-end biotin-labeled oligonucleotide B1 having the nucleotide sequence of SEQ ID NO: 116 capable of complementarily binding to a target DNA region X' having the nucleotide sequence of SEQ ID NO: 115 was synthesized, and a 0.02 µM solution thereof in TE buffer was prepared.

<Target DNA region> (5-methyl cytosine is also denoted by C)

### <5'-end biotin-labeled oligonucleotide>

B1: 5'Biotin-CTGGCCAAACTGGAGATCGC-3' (SEQ ID NO: 116)

Counter oligonucleotides C1 to C12 having the nucleotide sequences of SEQ ID NO: 117 to SEQ ID NO: 128 capable of complementarily binding to a minus strand of the target DNA region X' having the nucleotide sequence of SEQ ID NO: 115 were synthesized, and a solution in TE buffer wherein a concentration of each oligonucleotide is 0.01 µM was prepared.

<Target DNA region> (5-methyl cytosine is also denoted by C)

### <Counter oligonucleotides>

C1: 5'-GCCACCGCGAAAGTCACCGCCAGCACGGCG-3' (SEQ ID NO: 117)
C2: 5'-GCCAGCAGTACGCTGCTGTTGCCCAGCACG-3' (SEQ ID NO: 118)
C3: 5'-CGGGACGTCTTGCGCGGCGTCCGGTGCAGA-3' (SEQ ID NO: 119)
C4: 5'-AGGCTGAGGTGTCGGATGAAGAGGTGCATG-3' (SEQ ID NO: 120)
C5: 5'-ACCTGGAAGAATGCCACGGCCAGGTCGGCC-3' (SEQ ID NO: 121)
C6: 5'-TAGGTGATGTCCCAGCACATTTGCGGCAGC-3' (SEQ ID NO: 122)
C7: 5'-CGGCACAGCCAGTCGGGGCCGCGGAAGCGG-3' (SEQ ID NO: 123)
C8: 5'-ATGCCGAACACCTGCAGGTGCTTCACCACG-3' (SEQ ID NO: 124)
C9: 5'-ATGACTACCAGCATGTAGGCCGACGCAAAC-3' (SEQ ID NO: 125)
C10: 5'-TGGCACACCGCGATGTAGCGGTCGGCTGTC-3' (SEQ ID NO: 126)
C11: 5'-CGCGCGGGCTGTTGCAGAGTCTTGAGCGGG-3' (SEQ ID NO: 127)
C12: 5'-CAGGCGGCCGCGATCATGAGGCGCGAGCGG-3' (SEQ ID NO: 128)

For each of the solutions of the DNA fragment X and the solutions of the methylated DNA fragment MX, the following treatment was executed.

To a PCR tube, 10 µL of a DNA fragment solution prepared as described above, 10 µL of a 5'-end biotin-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

100 uL of a reaction solution of a DNA fragment prepared as described above was added to an 8-well strip coated with streptavidin (available from Perkin Elmer, a total of 12 wells), and immobilized to the well by leaving the solution still for 30 minutes at room temperature. Then the solution was removed by pipetting, and 200 uL of a washing buffer [0.05% Tween 20-containing phosphate buffer (1 mM KH₂PO₄, 3 mM Na₂HPO·7H₂O, 154 mM NaCl, pH 7.4)] was added, and the buffer was removed by decantation. This operation was repeated another two times (these correspond to Second step of the present measuring method).

A masking oligonucleotide M1 having the nucleotide sequence of SEQ ID NO: 129 capable of complementarily binding to a 5'-end biotin-labeled oligonucleotide B1 having the nucleotide sequence of SEQ ID NO: 116 was synthesized, and a 0.1 µM solution thereof in TE buffer was prepared.

### <5'-end biotin-labeled oligonucleotide>

B1: 5'Biotin-CTGGCCAAACTGGAGATCGC-3' (SEQ ID NO: 116)

### <Masking oligonucleotide>

M1: 5'-ATCTCCAGTTTGGCCAG-3' (SEQ ID NO: 129)

Each well was added with 100 µL of a methylated cytosine antibody [available from Aviva Systems Biology, a 0.5 µg/mL solution in a 0.1% BSA-containing phosphate buffer (1 mM KH₂PO₄, 3 mM Na₂HPO·7H₂O, 154 mM NaCl, pH 7.4)] and 1 µL of the aforementioned masking oligonucleotide solution, and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then each well was added with 100 µL of an Eu-N1 labeled mouse IgG antibody [available from Perkin Elmer, a 0.05 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Then, the solution was removed by decantation, and each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each well was added with 150 µL of Enhancement Solution (available from Perkin Elmer), stirred for 5 minutes, and left still for 15 minutes at room temperature. Thereafter, the fluorescence was measured at excitation 340 nm/fluorescence 612 nm (these correspond to Third step of the present measuring method).

The result is shown in Fig. 40. In Solutions MA and MB of the methylated DNA fragment MX, an increase in fluorescence intensity was observed, and the intensity was concentration-dependent. In the negative control solution MC, an increase in fluorescence intensity was not observed. In Solutions A, B and C of the unmethylated DNA fragment X, an increase in fluorescence intensity was not observed.

These demonstrate that DNA containing a methylated target DNA region can be selected and immobilized by a biotin-labeled oligonucleotide and a methyl cytosine antibody, and methylated DNA can be detected and quantified with high sensitivity.

### Example 18

For genomic DNA derived from human blood purchased from Clontech, a DNA fragment (Y, SEQ ID NO: 132, a region corresponding to the base numbers 76606 to 76726 shown in Genbank Accession No. ac009800 and so on) to be used as a test sample was amplified by conducting PCR using oligonucleotide primers (PF2 and PR2) designed for PCR of SEQ ID NO: 130 and SEQ ID NO: 131 and the following reaction condition.

### <Oligonucleotide primers designed for PCR>

PF2: 5'-TGAGCTCCGTAGGGCGTCC-3' (SEQ ID NO: 130)
PR2: 5'-GCGCCGGGTCCGGGCCC-3' (SEQ ID NO: 131)

### <DNA fragment>

As a reaction solution of PCR, 5 ng of genomic DNA which is a template, each 3 µL of oligonucleotide primer solutions prepared to 5 µM, each 5 µL of 2 mM dNTPs, 5 µL of a 10 × buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold, available from ABI) were mixed, and sterile ultrapure water was added to a liquid volume of 50 uL. The reaction solution was kept at 95°C for 10 minutes, and then subjected to PCR conducting 50 cycles of incubation each including 30 seconds at 95°C, 30 seconds at 60°C, and 45 seconds at 72°C.

After conducting PCR, amplification was checked by 1.5% agarose gel electrophoresis, and a DNA fragment Y was purified with Wizard SV Gel/PCR Kit (PROMEGA).

For a part of the obtained DNA fragment solution, a reaction solution was prepared by mixing 1 µL of SssI methylase (available from NEB), 10 µL of 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MY, SEQ ID NO: 133).

<Methylated DNA fragment> (N represents 5-methyl cytosine)

For the obtained DNA fragment Y, the following solutions were prepared in duplicate.
Solution A: 10 ng/10 µL solution in TE
Solution B: TE solution (negative control solution)

For the obtained DNA fragment MY, the following solutions were prepared in duplicate.
Solution MA: 10 ng/10 µL solution in TE
Solution MB: TE solution (negative control solution)

Also a 5'-end biotin-labeled oligonucleotide B2 having the nucleotide sequence of SEQ ID NO: 135 capable of complementarily binding to a target DNA region Y' having the nucleotide sequence of SEQ ID NO: 134 was synthesized, and a 0.02 µM solution thereof in TE buffer was prepared.

<Target DNA region> (5-methyl cytosine is also denoted by C)

### <5'-end biotin-labeled oligonucleotide>

B2: 5'Biotin-GACAACGCCTCGTTCTCGG-.3' (SEQ ID NO: 135)

Counter oligonucleotides C13, C14 and C15 having the nucleotide sequences of SEQ ID NO: 136, SEQ ID NO: 27 and SEQ ID NO: 138 capable of complementarily binding to the minus strand of a target DNA region Y' having the nucleotide sequence of SEQ ID NO: 134 were synthesized, and a solution in TE buffer wherein a concentration of each oligonucleotide is 0.01 µM was prepared.

<Target DNA region> (5-methyl cytosine is also denoted by C)

### <Counter oligonucleotides>

C13: 5'-GCGTCCCCCAAGGACGCCCTACGGAGCTCA-3' (SEQ ID NO: 136)
C14: 5'-CTCAACGAGGGCAGAGGAGCCGGCGACCTG-3' (SEQ ID NO: 137)
C15: 5'-CGCCGGGTCCGGGCCCGATGCGTTGGCGGG-3' (SEQ ID NO: 138)

For each of the solutions of the DNA fragment Y and the solutions of the methylated DNA fragment MY, the following treatment was executed.

To a PCR tube, 10 µL of a DNA fragment solution prepared as described above, 10 µL of a biotin-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

100 µL of a reaction solution of a DNA fragment prepared as described above was added to an 8-well strip coated with streptavidin (available from Perkin Elmer, a total of 8 wells), and immobilized to the well by leaving the solution still for 30 minutes at room temperature. Then the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and the buffer was removed by decantation. This operation was repeated another two times (these correspond to Second step of the present measuring method).

A masking oligonucleotide M2 having the nucleotide sequence of SEQ ID NO: 139 capable of complementarily binding to a 5'-end biotin-labeled oligonucleotide B2 having the nucleotide sequence of SEQ ID NO: 135 was synthesized, and a 0.1 µM solution thereof in TE buffer was prepared.

### <5'-end biotin-labeled oligonucleotide>

B2: 5'Biotin-GACAACGCCTCGTTCTCGG-3' (SEQ ID NO: 135)

### <Masking oligonucleotide>

M2: 5'-CCGAGAACGAGGCGTTGTCT-3' (SEQ ID NO: 139)

Each well was added with 100 µL of a methylated cytosine antibody [available from Aviva Systems Biology, a 0.5 µg/mL solution in a 0.1% BSA-containing phosphate buffer (1 mM KH₂PO₄, 3 mM Na₂HPO·7H₂O, 154 mM NaCl, pH 7.4)] and 1 µL of the aforementioned masking oligonucleotide solution, and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then each well was added with 100 µL of an Eu-N1 labeled mouse IgG antibody [available from Perkin Elmer, a 0.05 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Then, the solution was removed by decantation, and each well was washed three times with 200 µL of a washing buffer [0.05% Tween 20-containing phosphate buffer (1 mM KH₂PO₉, 3 mM Na₂HPO·7H₂O, 154 mM NaCl, pH 7.4)].

Each well was added with 150 µL of Enhancement Solution (available from Perkin Elmer), stirred for 5 minutes, and left still for 15 minutes at room temperature. Thereafter, the fluorescence was measured at excitation 340 nm/fluorescence 612 nm (these correspond to Third step of the present measuring method).

The result is shown in Fig. 41. In Solution MA of the methylated DNA fragment MY, an increase in fluorescence intensity was observed. In the negative control solution MB, an increase in fluorescence intensity was not observed. In Solutions A and B of the unmethylated DNA fragment Y, an increase in fluorescence intensity was not observed.

These demonstrate that DNA containing a methylated target DNA region can be selected and immobilized by a biotin-labeled oligonucleotide and a methyl cytosine antibody, and methylated DNA can be detected with high sensitivity.

### Example 19

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment (S, SEQ ID NO: 142, a region corresponding to the base numbers 271743 to 272083 of yeast chromosome VII shown in Genbank Accession No. NC_001139 and so on) to be used as a test sample was amplified by conducting PCR using primers (PF3 and PR3) of SEQ ID NO: 140 and SEQ ID NO: 141 and the following reaction condition.
PF3: 5'-AGGTGAGCTACGTGTGTTTGG-3' (SEQ ID NO: 140)
PR3: 5'-AGACATGTGCTCACGTACGGT-3' (SEQ ID NO: 141)

### <DNA fragment>

As a reaction solution of PCR, 10 ng of genomic DNA which is a template, each 3 µL of 5 µM of the aforementioned primer solutions, each 5 µL of 2 mM dNTPs, 5 µL of a 10 × buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold) were mixed, and sterile ultrapure water was added to a liquid volume of 50 µL. The reaction solution was kept at 95°C for 10 minutes, and then subjected to PCR conducting 40 cycles of incubation each including 20 seconds at 95°C, 30 seconds at 58°C, and 30 seconds at 72°C.

After conducting PCR, amplification was checked by 2% agarose gel electrophoresis, and a DNA fragment S was purified with Wizard SV Gel/PCR Kit (PROMEGA).

For a part of the obtained DNA fragment solution, a reaction solution was prepared by mixing 1 µL of SssI methylase (available from NEB), 10 µL of 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MS, SEQ ID NO: 143).

<Methylated DNA fragment> (N represents 5-methyl cytosine)

For the obtained DNA fragment S, the following solutions were prepared in duplicate.
Solution A: 10 ng/10 µL solution in TE
Solution B: 1 ng/10 µL solution in TE
Solution C: 0.1 ng/10 µL solution in TE
Solution D: TE solution (negative control solution)

For the obtained DNA fragment MS, the following solutions were prepared in duplicate.
Solution MA: 10 ng/10 µL solution in TE
Solution MB: 1 ng/10 µL solution in TE
Solution MC: 0.1 ng/10 µL solution in TE
Solution MD: TE solution (negative control solution)

Also a 5'-end biotin-labeled oligonucleotide B3 having the nucleotide sequence of SEQ ID NO: 145 capable of complementarily binding to a target DNA region S' having the nucleotide sequence of SEQ ID NO: 144 was synthesized, and a 0.02 µM solution thereof in TE buffer was prepared.

<Target DNA region> (5-methyl cytosine is also denoted by C)

### <5'-end biotin-labeled oligonucleotide>

B3: 5'Biotin-AGACATGTGCTCACGTACGGT-3' (SEQ ID NO: 145)

Counter oligonucleotides C16 to C25 having the nucleotide sequences of SEQ ID NO: 146 to SEQ ID NO: 155 capable of complementarily binding to a minus strand of the target DNA region S' having the nucleotide sequence of SEQ ID NO: 144 were synthesized, and a solution in TE buffer wherein a concentration of each oligonucleotide is 0.01 µM was prepared.

<Target DNA region> (5-methyl cytosine is also denoted by C)

### <Counter oligonucleotides>

C16: 5'-AGGTGAGCTACGTGTGTTTGG-3' (SEQ ID NO: 146)
C17: 5'-GCGTCGTGCACTGGCTCACTTGTACGCGCA-3' (SEQ ID NO: 147)
C18: 5'-CTTGTACGATTGGTGACCCGCCTTTTCGAC-3' (SEQ ID NO: 148)
C19: 5'-ACTGGACCGCTATGGACGTGGCGGCGGTGT-3' (SEQ ID NO: 149)
C20: 5'-GGCGGCGGCTCAATGACCTGTGGCGCCCGT-3' (SEQ ID NO: 150)
C21: 5'-TTGTGGCGTGCGATAGTCGAGCCGCCTGTC-3' (SEQ ID NO: 151)
C22: 5'-ACGTGCGCGGCCGCCCTGCTCCGTT-3' (SEQ ID NO: 152)
C23: 5'-TGACGCGATGCATAGCATGCGACCACCCAG-3' (SEQ ID NO: 153)
C24: 5'-ACTGCTGACGCTATTGGTCACGTGGTTATG-3' (SEQ ID NO: 154)
C25: 5'-CTGCTGTTGACTGCGGTGGCGTCCCGTTTC-3' (SEQ ID NO: 155)

For each of the solutions of the DNA fragment S and the solutions of the methylated DNA fragment MS, the following treatment was executed.

To a PCR tube, 10 µL of a DNA fragment solution prepared as described above, 10 µL of a biotin-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

A masking oligonucleotide M3 having the nucleotide sequence of SEQ ID NO: 156 capable of complementarily binding to a 5'-end biotin-labeled oligonucleotide B3 having the nucleotide sequence of SEQ ID NO: 145 was synthesized, and a 0.02 µM solution thereof in TE buffer was prepared.

100 µL of a reaction solution of a DNA fragment prepared as described above was added to an 8-well strip coated with streptavidin (available from Perkin Elmer), and immobilized to the well by leaving the solution still for 30 minutes at room temperature. Then the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and the buffer was removed by decantation. This operation was repeated another two times (these correspond to Second step of the present measuring method).

### <5'-end biotin-labeled oligonucleotide>

B3: 5'Biotin-AGACATGTGCTCACGTACGGT-3' (SEQ ID NO: 145)

### <Masking oligonucleotide>

M3: 5'-ACCGTACGTGAGCACATGTCT-3' (SEQ ID NO: 156)

Each well was added with 100 µL of a methylated cytosine antibody [available from Aviva Systems Biology, a 0.5 µg/mL solution in a 0.1% BSA-containing phosphate buffer (1 mM KH₂PO₄, 3 mM Na₂HPO·7H₂O, 154 mM NaCl, pH 7.4)] and 1 µL of the aforementioned masking oligonucleotide solution, and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then each well was added with 100 µL of an Eu-N1 labeled mouse IgG antibody [available from Perkin Elmer, a 0.05 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Then, the solution was removed by decantation, and each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each well was added with 150 µL of Enhancement Solution (available from Perkin Elmer), mixed and left still for about 45 minutes at room temperature. Thereafter, the fluorescence was measured at excitation 340 nm/fluorescence 612 nm (these correspond to Third step of the present measuring method).

The result is shown in Fig. 42. In Solutions MA, MB and MC of the methylated DNA fragment MS, an increase in fluorescence intensity was observed and the intensity was concentration-dependent. In the negative control solution MD, an increase in fluorescence intensity was not observed. In Solutions A, B, C and D of the unmethylated DNA fragment S, an increase in fluorescence intensity was not observed.

These demonstrate that DNA containing a methylated target DNA region can be selected and immobilized by a biotin-labeled oligonucleotide and a methyl cytosine antibody, and methylated DNA can be detected and quantified with high sensitivity.

### Example 20

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment (T, SEQ ID NO: 159, a region corresponding to the base numbers 384569 to 384685 of yeast chromosome VII shown in Genbank Accession No. NC_001139 and so on) to be used as a test sample was amplified by conducting PCR using oligonucleotide primers (PF4 and PR4) designed for PCR of SEQ ID NO: 157 and SEQ ID NO: 158 and the following reaction condition.

### <Oligonucleotide primers designed for PCR>

PF4: 5'-GGACCTGTGTTTGACGGGTAT-3' (SEQ ID NO: 157)
PR4: 5'-AGTACAGATCTGGCGTTCTCG-3' (SEQ ID NO: 158)

### <DNA fragment>

As a reaction solution of PCR, 10 ng of genomic DNA which is a template, each 3 µL of oligonucleotide primer solutions prepared to 5 µM, each 5 µL of 2 mM dNTPs, 5 µL of a 10 × buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold, available from ABI) were mixed, and sterile ultrapure water was added to a liquid volume of 50 µL.

The reaction solution was kept at 95°C for 10 minutes, and then subjected to PCR conducting 40 cycles of incubation each including 20 seconds at 95°C, 30 seconds at 58°C, and 30 seconds at 72°C.

After conducting PCR, amplification was checked by 1.5% agarose gel electrophoresis, and a DNA fragment T was purified with Wizard SV Gel/PCR Kit (PROMEGA).

For a part of the obtained DNA fragment solution, a reaction solution was prepared by mixing 1 µL of SssI methylase (available from NEB), 10 µL of 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MT, SEQ ID NO: 160).

<Methylated DNA fragment> (N represents 5-methyl cytosine)

For the obtained DNA fragment T, the following solutions were prepared.
Solution A: 10 ng/10 µL solution in TE
Solution B: 1 ng/10 µL solution in TE
Solution C: 0.1 ng/10 µL solution in TE
Solution D: TE solution (negative control solution)

For the obtained DNA fragment MT, the following solutions were prepared.
Solution MA: 10 ng/10 µL solution in TE
Solution MB: 1 ng/10 µL solution in TE
Solution MC: 0.1 ng/10 µL solution in TE
Solution MD: TE solution (negative control solution)

Also a 5'-end biotin-labeled oligonucleotide B4 having the nucleotide sequence of SEQ ID NO: 162 capable of complementarily binding to a target DNA region T' having the nucleotide sequence of SEQ ID NO: 161 was synthesized, and a 0.02 µM solution thereof in TE buffer was prepared.

### <Target DNA region> (5-methyl cytosine is also denoted by C)

### <5'-end biotin-labeled oligonucleotide>

B4: 5'Biotin-AGTACAGATCTGGCGTTCTCG-3' (SEQ ID NO: 162)

Counter oligonucleotides C26, C27, C28 and C29 having the nucleotide sequences of SEQ ID NO: 163, SEQ ID NO: 164, SEQ ID NO: 165 and SEQ ID NO: 166 capable of complementarily binding to a minus strand of the target DNA region T' having the nucleotide sequence of SEQ ID NO: 161 were synthesized, and a solution in TE buffer wherein a concentration of each oligonucleotide is 0.01 µM was prepared.

<Target DNA region> (Methyl cytosine is also denoted by C)

### <Counter oligonucleotides>

C26: 5'-GGACCTGTGTTTGACGGGTAT-3' (SEQ ID NO: 163)
C27: 5'-AACACTAAGTTGCGCAATTTGCTGT-3' (SEQ ID NO: 164)
C28: 5'-ATTGCGAAATCCGCCCGGACGATAT-3' (SEQ ID NO: 165)
C29: 5'-CACTCTTGAGCGCATGTGCCGTTTC-3' (SEQ ID NO: 166)

For each of the solutions of the DNA fragment T and the solutions of the methylated DNA fragment MT, the following treatment was executed.

To a PCR tube, 10 µL of a DNA fragment solution prepared as described above, 10 µL of a biotin-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

100 µL of a reaction solution of a DNA fragment prepared as described above was added to an 8-well strip coated with streptavidin (available from Perkin Elmer), and immobilized to the well by leaving the solution still for 30 minutes at room temperature. Then the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and the buffer was removed by decantation. This operation was repeated another two times (these correspond to Second step of the present measuring method).

A masking oligonucleotide M4 having the nucleotide sequence of SEQ ID NO: 167 capable of complementarily binding to a 5'-end biotin-labeled oligonucleotide B4 having the nucleotide sequence of SEQ ID NO: 162 was synthesized, and a 0.02 µM solution thereof in TE buffer was prepared.

### <5'-end biotin-labeled oligonucleotide>

B4: 5'Biotin-AGTACAGATCTGGCGTTCTCG-3' (SEQ ID NO: 162)

### <Masking oligonucleotide>

M4: 5'-CGAGAACGCCAGATCTGTACT-3' (SEQ ID NO: 167)

Each well was added with 100 µL of a methylated cytosine antibody [available from Aviva Systems Biology, a 0.5 µg/mL solution in a 0.1% BSA-containing phosphate buffer (1 mM KH₂PO₄, 3 mM Na₂HPO·7H₂O, 154 mM NaCl, pH 7.4)] and 1 µL of the aforementioned masking oligonucleotide solution, and left still for an hour at room temperature. Thereafter, each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Then each well was added with 100 µL of an Eu-N1 labeled mouse IgG antibody [available from Perkin Elmer, a 0.05 µg/mL solution in a 0.1% BSA-containing phosphate buffer (1 mM KH₂PO₄, 3 mM Na₂HPO·7H₂O, 154 mM NaCl, pH 7.4)], and left still for an hour at room temperature. Then, the solution was removed by decantation, and each well was washed three times with 200 µL of a washing buffer [0.05% Tween 20-containing phosphate buffer (1 mM KH₂PO₄, 3 mM Na₂HPO·7H₂O, 154 mM NaCl, pH 7.4)].

Each well was added with 150 µL of Enhancement Solution (available from Perkin Elmer), stirred for 5 minutes, and left still for 15 minutes at room temperature. Thereafter, the fluorescence was measured at excitation 340 nm/fluorescence 612 nm (these correspond to Third step of the present measuring method).

The result is shown in Fig. 43. In Solutions MA, MB and MC of the methylated DNA fragment MT, an increase in fluorescence intensity was observed and the intensity was concentration-dependent. In the negative control solution MD, an increase in fluorescence intensity was not observed. In Solutions A, B, C and D of the unmethylated DNA fragment T, an increase in fluorescence intensity was not observed.

These demonstrate that DNA containing a methylated target DNA region T' can be selected and immobilized by a biotin-labeled oligonucleotide and a methyl cytosine antibody, and methylated DNA can be detected and quantified with high sensitivity.

### Example 21

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

A 0.5 µg/mL solution of the synthetically obtained biotin-labeled methylated cytosine antibody was prepared, and each 100 µL of this solution was added to an 8-well strip coated with streptavidin (available from Perkin Elmer), and immobilized to wells by leaving the solution still for about an hour at room temperature. Then the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by decantation. This operation was repeated another two times (these correspond to preparation of an immobilized methylated DNA antibody for use in the present measuring method).

For genomic DNA derived from human blood purchased from Clontech, a DNA fragment (X, SEQ ID NO: 113, a region corresponding to the base numbers 25687390 to 25687775 shown in Genbank Accession No. NT_029419 and so on) to be used as a test sample was amplified by conducting PCR using oligonucleotide primers (PF1 and PR1) designed for PCR of SEQ ID NO: 111 and SEQ ID NO: 112 and the following reaction condition.

### <Oligonucleotide primers designed for PCR>

PF1: 5'-CTCAGCACCCAGGCGGCC-3' (SEQ ID NO: 111)
PR1: 5'-CTGGCCAAACTGGAGATCGC-3' (SEQ ID NO: 112)

### <DNA fragment>

As a reaction solution of PCR, 5 ng of genomic DNA which is a template, each 3 µL of oligonucleotide primer solutions prepared to 5 µM, each 5 µL of 2 mM dNTPs, 5 µL of a 10 × buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold, available from ABI) were mixed, and sterile ultrapure water was added to a liquid volume of 50 µL. The reaction solution was kept at 95°C for 10 minutes, and then subjected to PCR conducting 40 cycles of incubation each including 30 seconds at 95°C, 30 seconds at 61°C, and 45 seconds at 72°C.

After conducting PCR, amplification was checked by 1.5% agarose gel electrophoresis, and a DNA fragment X was purified with Wizard SV Gel/PCR Kit (PROMEGA).

For a part of the obtained DNA fragment solution, a reaction solution was prepared by mixing 1 µL of SssI methylase (available from NEB), 10 µL of 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MX, SEQ ID NO: 114).

### <DNA fragment> (N represents 5-methyl cytosine)

For the obtained DNA fragment X, the following solutions were prepared.
Solution A: 10 ng/10 µL solution in TE
Solution B: 1 ng/10 µL solution in TE
Solution C: 0.1 ng/10 µL solution in TE
Solution D: TE solution (negative control solution)

For the obtained DNA fragment MX, the following solutions were prepared.
Solution MA: 10 ng/10 µL solution in TE
Solution MB: 1 ng/10 µL solution in TE
Solution MC: 0.1 ng/10 µL solution in TE
Solution MD: TE solution (negative control solution)

Also a 5'-end FITC-labeled oligonucleotide F1 having the nucleotide sequence of SEQ ID NO: 168 capable of complementarily binding to a target DNA region X' having the nucleotide sequence of SEQ ID NO: 115 was synthesized, and a 0.02 µM solution thereof in a 10 mM Tris-HCl buffer was prepared.

<Target DNA region> (5-methyl cytosine is also denoted by C)

### <5'-end FITC-labeled oligonucleotide>

F1: 5'FITC-CTGGCCAAACTGGAGATCGC-3' (SEQ ID NO: 168)

Counter oligonucleotides C1 to C12 having the nucleotide sequences of SEQ ID NO: 117 to SEQ ID NO: 128 capable of complementarily binding to a minus strand of the target DNA region X' having the nucleotide sequence of SEQ ID NO: 115 were synthesized, and a solution in TE buffer wherein a concentration of each oligonucleotide is 0.01 µM was prepared.

<Target DNA region> (5-methyl cytosine is also denoted by C)

### <Counter oligonucleotides>

C1: 5'-GCCACCGCGAAAGTCACCGCCAGCACGGCG-3' (SEQ ID NO: 117)
C2: 5'-GCCAGCAGTACGCTGCTGTTGCCCAGCACG-3' (SEQ ID NO: 118)
C3: 5'-CGGGACGTCTTGCGCGGCGTCCGGTGCAGA-3' (SEQ ID NO: 119)
C4: 5'-AGGCTGAGGTGTCGGATGAAGAGGTGCATG-3' (SEQ ID NO: 120)
C5: 5'-ACCTGGAAGAATGCCACGGCCAGGTCGGCC-3' (SEQ ID NO: 121)
C6: 5'-TAGGTGATGTCCCAGCACATTTGCGGCAGC-3' (SEQ ID NO: 122)
C7: 5'-CGGCACAGCCAGTCGGGGCCGCGGAAGCGG-3' (SEQ ID NO: 123)
C8: 5'-ATGCCGAACACCTGCAGGTGCTTCACCACG-3' (SEQ ID NO: 124)
C9: 5'-ATGACTACCAGCATGTAGGCCGACGCAAAC-3' (SEQ ID NO: 125)
C10: 5'-TGGCACACCGCGATGTAGCGGTCGGCTGTC-3' (SEQ ID NO: 126)
C11: 5'-CGCGCGGGCTGTTGCAGAGTCTTGAGCGGG-3' (SEQ ID NO: 127)
C12: 5'-CAGGCGGCCGCGATCATGAGGCGCGAGCGG-3' (SEQ ID NO: 128)

For each of the solutions of the DNA fragment X and the solutions of the methylated DNA fragment MX, the following treatment was executed.

To a PCR tube, 10 µL of a DNA fragment solution prepared as described above, 10 µL of a 5'-end FITC-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

To an 8-well strip coated with streptavidin onto which the aforementioned biotin-labeled methyl cytosine antibody is immobilized, 100 µL of a reaction solution of a DNA fragment prepared as described above was added, and left still at room temperature for an hour. Then, the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and the buffer was removed by pipetting. This operation was repeated another two times (these correspond to Second step of the present measuring method).

Thereafter, 100 µL of an HRP-labeled FITC antibody solution [available from Jackson ImmunoResearch Laboratories, a 0.005 µg/100 µL solution in a 0.1% BSA-containing phosphate buffer (1 mM KH₂PO₄, 3 mM Na₂HPO·7H₂O, 154 mM NaCl, pH 7.4)] was added to each well, and left still for an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and then the buffer was removed by decantation. This operation was repeated another two times.

Then, 100 µL of a substrate (available from R&D, #DY999) was added and mixed to each well to start a reaction.

Then after being left still for about 30 minutes at room temperature, each well was added and mixed with 50 µL of a stop solution (1 N H₂SO₄ aqueous solution) to stop the reaction. The absorbance at 450 nm within 30 minutes after stop of the reaction was measured (these correspond to Third step of the present measuring method).

The result is shown in Fig. 44. In Solutions MA, MB and MC of the methylated DNA fragment MX, an increase in chromogenic intensity was observed, and the intensity was concentration-dependent. In the negative control solution MD, an increase in chromogenic intensity was not observed. In Solutions A, B, C and D of the unmethylated DNA fragment X, an increase in chromogenic intensity was not observed.

These demonstrate that DNA containing a methylated target DNA region can be selected by an immobilized methyl cytosine antibody, and methylated DNA can be detected and quantified with high sensitivity.

### Example 22

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

A 0.5 µg/mL solution of the synthetically obtained biotin-labeled methylated cytosine antibody was prepared, and each 100 µL of this solution was added to an 8-well strip coated with streptavidin (available from Perkin Elmer), and immobilized to wells by leaving the solution still for about an hour at room temperature. Then the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by decantation. This operation was repeated another two times (these correspond to preparation of an immobilized methylated DNA antibody for use in the present measuring method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment (S, SEQ ID NO: 142, a region corresponding to the base numbers 271743 to 272083 of yeast chromosome VII shown in Genbank Accession No. NC_001139 and so on) to be used as a test sample was amplified by conducting PCR using primers (PF3 and PR3) of SEQ ID NO: 140 and SEQ ID NO: 141 and the following reaction condition.
PF3: 5'-AGGTGAGCTACGTGTGTTTGG-3' (SEQ ID NO: 140)
PR3: 5'-AGACATGTGCTCACGTACGGT-3' (SEQ ID NO: 141)

### <DNA fragment>

As a reaction solution of PCR, 10 ng of genomic DNA which is a template, each 3 µL of 5 µM of the aforementioned primer solutions, each 5 µL of 2 mM dNTPs, 5 µL of a 10 × buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold) were mixed, and sterile ultrapure water was added to a liquid volume of 50 µL. The reaction solution was kept at 95°C for 10 minutes, and then subjected to PCR conducting 40 cycles of incubation each including 20 seconds at 95°C, 30 seconds at 58°C, and 30 seconds at 72°C.

After conducting PCR, amplification was checked by 2% agarose gel electrophoresis, and a DNA fragment S was purified with Wizard SV Gel/PCR Kit (PROMEGA).

For a part of the obtained DNA fragment solution, a reaction solution was prepared by mixing 1 µL of SssI methylase (available from NEB), 10 µL of 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MS, SEQ ID NO: 143).

<DNA fragment> (N represents 5-methyl cytosine)

For the obtained DNA fragment S, the following solutions were prepared in duplicate.
Solution A: 10 ng/10 µL solution in TE
Solution B: 1 ng/10 µL solution in TE
Solution C: 0.1 ng/10 µL solution in TE
Solution D: TE solution (negative control solution)

For the obtained DNA fragment MS, the following solutions were prepared in duplicate.
Solution MA: 10 ng/10 µL solution in TE
Solution MB: 1 ng/10 µL solution in TE
Solution MC: 0.1 ng/10 µL solution in TE
Solution MD: TE solution (negative control solution)

Also, a 5'-end FITC-labeled oligonucleotide F2 having the nucleotide sequence of SEQ ID NO: 169 capable of complementarily binding to a target DNA region S' having the nucleotide sequence of SEQ ID NO: 144 was synthesized, and a 0.02 µM solution thereof in a 10 mM Tris-HCl buffer was prepared.

<Target DNA region> (5-methyl cytosine is also denoted by C)

### <5'-end FITC-labeled oligonucleotide>

F2: 5'FITC-AGACATGTGCTCACGTACGGT-3' (SEQ ID NO: 169)

Also, counter oligonucleotides C16 to C25 having the nucleotide sequences of SEQ ID NO: 146 to SEQ ID NO: 155 capable of complementarily binding to a minus strand of the target DNA region S' having the nucleotide sequence of SEQ ID NO: 144 were synthesized, and a solution in TE buffer wherein a concentration of each oligonucleotide is 0.01 µM was prepared.

<Target DNA region> (5-methyl cytosine is also denoted by C)

### <Counter oligonucleotides>

C16: 5'-AGGTGAGCTACGTGTGTTTGG-3' (SEQ ID NO: 146)
C17: 5'-GCGTCGTGCACTGGCTCACTTGTACGCGCA-3' (SEQ ID NO: 147)
C18: 5'-CTTGTACGATTGGTGACCCGCCTTTTCGAC-3' (SEQ ID NO: 148)
C19: 5'-ACTGGACCGCTATGGACGTGGCGGCGGTGT-3' (SEQ ID NO: 149)
C20: 5'-GGCGGCGGCTCAATGACCTGTGGCGCCCGT-3' (SEQ ID NO: 150)
C21: 5'-TTGTGGCGTGCGATAGTCGAGCCGCCTGTC-3' (SEQ ID NO: 151)
C22: 5'-ACGTGCGCGGCCGCCCTGCTCCGTT-3' (SEQ ID NO: 152)
C23: 5'-TGACGCGATGCATAGCATGCGACCACCCAG-3' (SEQ ID NO: 153)
C24: 5'-ACTGCTGACGCTATTGGTCACGTGGTTATG-3' (SEQ ID NO: 154)
C25: 5'-CTGCTGTTGACTGCGGTGGCGTCCCGTTTC-3' (SEQ ID NO: 155)

For each of the solutions of the DNA fragment S and the solutions of the methylated DNA fragment MS, the following treatment was executed.

To a PCR tube, 10 µL of a DNA fragment solution prepared as described above, 10 µL of a 5'-end FITC-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

To an 8-well strip coated with streptavidin onto which the aforementioned biotin-labeled methyl cytosine antibody is immobilized, 100 µL of a reaction solution of a DNA fragment prepared as described above was added, and left still at room temperature for an hour. Then, the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and the buffer was removed by pipetting. This operation was repeated another two times (these correspond to Second step of the present measuring method).

Thereafter, 100 µL of an HRP-labeled FITC antibody solution [available from Jackson ImmunoResearch Laboratories, a 0.005 µg/100 µL solution in a 0.1% BSA-containing phosphate buffer (1 mM KH₂PO₄, 3 mM Na₂HPO·7H₂O, 154 mM NaCl, pH 7.4)] was added to each well, and left still for an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and then the buffer was removed by decantation. This operation was repeated another two times.

Then, 100 µL of a substrate (available from R&D, #DY999) was added and mixed to each well to start a reaction.

Then after being left still for about 15 minutes at room temperature, each well was added and mixed with 50 µL of a stop solution (1 N H₂SO₄ aqueous solution) to stop the reaction. The absorbance at 450 nm of the sample obtained within 30 minutes after stop of the reaction was measured (these correspond to Third step of the present measuring method).

The result is shown in Fig. 45. In Solutions MA, MB and MC of the methylated DNA fragment MS, an increase in chromogenic intensity was observed, and the intensity was concentration-dependent. In the negative control solution MD, an increase in chromogenic intensity was not observed. In Solutions A, B, C and D of the unmethylated DNA fragment S, an increase in chromogenic intensity was not observed.

These demonstrate that DNA containing a methylated target DNA region can be selected by an immobilized methyl cytosine antibody, and methylated DNA can be detected and quantified with high sensitivity.

### Example 23

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

A 0.5 µg/mL solution of the synthetically obtained biotin-labeled methylated cytosine antibody was prepared, and each 100 µL of this solution was added to an 8-well strip coated with streptavidin (available from Perkin Elmer), and immobilized to wells by leaving the solution still for about an hour at room temperature. Then the solution was removed by pipetting, and 200 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by decantation. This operation was repeated another two times (these correspond to preparation of an immobilized methylated DNA antibody for use in the present measuring method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment (T, SEQ ID NO: 159, a region corresponding to the base numbers 384569 to 384685 of yeast chromosome VII shown in Genbank Accession No. NC_001139 and so on) to be used as a test sample was amplified by conducting PCR using oligonucleotide primers (PF4 and PR4) designed for PCR of SEQ ID NO: 157 and SEQ ID NO: 158 and the following reaction condition.

### <Oligonucleotide primers designed for PCR>

PF4: 5'-GGACCTGTGTTTGACGGGTAT-3' (SEQ ID NO: 157)
PR4: 5'-AGTACAGATCTGGCGTTCTCG-3' (SEQ ID NO: 158)

### <DNA fragment>

As a reaction solution of PCR, 10 ng of genomic DNA which is a template, each 3 µL of oligonucleotide primer solutions prepared to 5 µM, each 5 µL of 2 mM dNTPs, 5 µL of a 10 x buffer (100 mM Tris-HCl pH 8.3, 500 mM KCl, 15 mM MgCl₂, 0.01% Gelatin), and 0.25 µL of a 5 U/µL thermostable DNA polymerase (AmpliTaq Gold, available from ABI) were mixed, and sterile ultrapure water was added to a liquid volume of 50 µL. The reaction solution was kept at 95°C for 10 minutes, and then subjected to PCR conducting 40 cycles of incubation each including 20 seconds at 95°C, 30 seconds at 58°C, and 30 seconds at 72°C.

After conducting PCR, amplification was checked by 1.5% agarose gel electrophoresis, and a DNA fragment T was purified with Wizard SV Gel/PCR Kit (available from PROMEGA).

A part of the obtained DNA fragment solution was mixed with 1 µL of SssI methylase (available from NEB), 10 µL of 10 × NEBuffer 2 (available from NEB), and 1 µL of S-adenosyl methionine (3.2 mM, available from NEB), and added with sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MT, SEQ ID NO: 160).

<DNA fragment> (N represents 5-methyl cytosine)

For the obtained DNA fragment T, the following solutions were prepared in duplicate.
Solution A: 10 ng/10 µL solution in TE
Solution B: 1 ng/10 µL solution in TE
Solution C: 0.1 ng/10 µL solution in TE
Solution D: TE solution (negative control solution)

For the obtained DNA fragment MT, the following solutions were prepared in duplicate.
Solution MA: 10 ng/10 µL solution in TE
Solution MB: 1 ng/10 µL solution in TE
Solution MC: 0.1 ng/10 µL solution in TE
Solution MD: TE solution (negative control solution)

Also, a 5'-end FITC-labeled oligonucleotide F3 having the nucleotide sequence of SEQ ID NO: 170 capable of complementarily binding to a target DNA region T' having the nucleotide sequence of SEQ ID NO: 161 was synthesized, and a 0.02 µM solution thereof in a 10 mM Tris-HCl buffer was prepared.

<Target DNA region> (5-methyl cytosine is also denoted by C)

### <5'-end FITC-labeled oligonucleotide>

F3: 5'FITC-AGTACAGATCTGGCGTTCTCG-3' (SEQ ID NO: 170)

Also, counter oligonucleotides C26, C27, C28 and C29 having the nucleotide sequences of SEQ ID NO: 163, SEQ ID NO: 164, SEQ ID NO: 165 and SEQ ID NO: 166 capable of complementarily binding to a minus strand of the target DNA region T' having the nucleotide sequence of SEQ ID NO: 161 were synthesized, and a solution in TE buffer wherein a concentration of each oligonucleotide is 0.01 µM was prepared.

<Target DNA region> (Methyl cytosine is also denoted by C)

### <Counter oligonucleotides>

C26: 5'-GGACCTGTGTTTGACGGGTAT-3' (SEQ ID NO: 163)
C27: 5'-AACACTAAGTTGCGCAATTTGCTGT-3' (SEQ ID NO: 164)
C28: 5'-ATTGCGAAATCCGCCCGGACGATAT-3' (SEQ ID NO: 165)
C29: 5'-CACTCTTGAGCGCATGTGCCGTTTC-3' (SEQ ID NO: 166)

For each of the solutions of the DNA fragment T and the solutions of the methylated DNA fragment MT, the following treatment was executed.

To a PCR tube, 10 µL of a DNA fragment solution prepared as described above, 10 µL of an FITC-labeled oligonucleotide solution prepared as described above, 10 µL of a counter oligonucleotide solution prepared as described above, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, and 10 µL of a 1 mg/mL BSA solution were added, and the resultant mixture was added with sterile ultrapure water to a liquid volume of 100 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to First step of the present measuring method).

To an 8-well strip coated with streptavidin onto which the aforementioned biotin-labeled methyl cytosine antibody is immobilized, 100 µL of a reaction solution of a DNA fragment prepared as described above was added, and left still at room temperature for an hour. Then, the solution was removed by pipetting, and 200 µL of a washing buffer [0.05% Tween 20-containing phosphate buffer (1 mM KH₂PO₄, 3 mM Na₂HPO·H₂O, 154 mM NaCl, pH 7.4)] was added, and the buffer was removed by pipetting. This operation was repeated another two times (these correspond to Second step of the present measuring method).

Thereafter, 100 µL of an HRP-labeled FITC antibody solution [available from Jackson ImmunoResearch Laboratories, a 0.005 µg/100 µL solution in a 0.1% BSA-containing phosphate buffer (1 mM KH₂PO₄, 3 mM Na₂HPO·7H₂O, 154 mM NaCl, pH 7.4)] was added to each well, and left still for an hour at room temperature. After being left still, each well was added with 200 µL of a washing buffer [0.05% Tween 20-containing phosphate buffer (1 mM KH₂PO₄, 3 mM Na₂HPO·7H₂O, 154 mM NaCl, pH 7.4)], and then the buffer was removed by decantation. This operation was repeated another two times.

Then, 100 µL of a substrate (available from R&D, #DY999) was added and mixed to each well to start a reaction.

Then after being left still for about an hour at room temperature, each well was added and mixed with 50 µL of a stop solution (1 N H₂SO₄ aqueous solution) to stop the reaction. The absorbance at 450 nm within 30 minutes after stop of the reaction was measured (these correspond to Third step of the present measuring method).

The result is shown in Fig. 46. In Solutions MA, MB and MC of the methylated DNA fragment MT, an increase in chromogenic intensity was observed, and the intensity was concentration-dependent. In the negative control solution MD, an increase in chromogenic intensity was not observed. In Solutions A, B, C and D of the unmethylated DNA fragment T, an increase in chromogenic intensity was not observed.

From the above, it was demonstrated that in forming a bound body of single-stranded DNA containing a target DNA region and the present oligonucleotide, a reaction system containing a magnesium ion is preferably used. Also from the above, it was confirmed that DNA containing a methylated target DNA region can be selected by an immobilized methyl cytosine antibody, and methylated DNA can be detected and quantified with high sensitivity.

### INDUSTRIAL APPLICABILITY

Based on the present invention, it becomes possible to provide a method of detecting or quantifying methylated DNA in an objective DNA region in a genomic DNA contained in a biological specimen in a simple and convenient manner, and so on.

## Claims

1. A method of detecting or quantifying methylated DNA in a target DNA region possessed by genomic DNA contained in a biological specimen, consisting of:
(1) First step of separating double-stranded DNA contained in a DNA sample derived from the genomic DNA contained in the biological specimen into single-stranded DNA,
(2) Second step of mixing (i) the single-stranded DNA separated in First step, (ii) a methylated DNA antibody, and (iii) an oligonucleotide (hereinafter, also referred to as the present oligonucleotide) capable of binding with the single-stranded DNA without inhibiting binding between the methylated DNA antibody and methylated DNA in a target DNA region, thereby forming a complex of the single-stranded DNA containing methylated DNA in the target DNA region, the methylated DNA antibody, and the oligonucleotide, and immobilizing the methylated DNA antibody or the oligonucleotide contained in the complex to a support followed by separating the complex simultaneously or after the formation; and
(3) Third step of detecting or quantifying either the methylated DNA antibody or the oligonucleotide contained in the separated complex, according to an identification function available for detection possessed by the methylated DNA antibody or the oligonucleotide,
wherein when the methylated DNA antibody contained in the complex is bound to a support said third step consists of detecting or quantifying said oligonucleotide, and wherein when the oligonucleotide contained in the complex is bound to a support said third step consists of detecting or quantifying said methylated DNA antibody,
thereby detecting or quantifying methylated DNA in the target DNA region contained in the biological specimen.

2. The method according to claim 1, wherein as the oligonucleotide that is mixed in Second step, and does not inhibit binding between the methylated DNA antibody and a methylated base existing in single-stranded DNA containing the target DNA region, two or more kinds of oligonucleotides are used.

3. The method according to claim 1, wherein the complex formed in Second step, or a bound body of the single-stranded DNA separated in First step and the oligonucleotide not inhibiting binding between the methylated DNA antibody and a methylated base existing in single-stranded DNA containing the target DNA region arising during formation of the complex in Second step is formed in a reaction system containing a bivalent positive ion.

4. The method according to claim 3, wherein the bivalent positive ion is a magnesium ion.

5. The method according to any one of claims 1 to 4, comprising as a separating operation of the complex in Second step,
(a) a step of making the methylated DNA antibody contained in the formed complex be bound to a support; or
(b) a step of making the oligonucleotide contained in the formed complex be bound to a support.

6. The method according to any one of claims 1 to 5, additionally comprising, between immediately after end of First step and immediately before start of Third step,
(a) a step of digesting the single-stranded DNA separated in First step by at least one kind of methylation sensitive restriction enzyme capable of digesting single-stranded DNA; or
(b) (i) a step of mixing the single-stranded DNA separated in First step, and a masking oligonucleotide having a recognition sequence of at least one kind of methylation sensitive restriction enzyme as its part, and (ii) digesting a mixture obtained by the previous step (single-stranded DNA existing therein in which DNA is not methylated in the target DNA region) by the methylation sensitive restriction enzyme.

7. The method according to claim 6, wherein the at least one kind of methylation sensitive restriction enzyme capable of digesting single-stranded DNA is HhaI which is a methylation sensitive restriction enzyme capable of digesting single-stranded DNA.

8. The method according to claim 6, wherein the at least one kind of methylation sensitive restriction enzyme is HpaII or HhaI which is a methylation sensitive restriction enzyme.

9. The method according to any one of claims 1 to 8, wherein the methylated DNA antibody is a methyl cytosine antibody.

10. The method according to any one of claims 1 to 9, wherein the biological specimen is
(a) serum or plasma of a mammal;
(b) blood or a bodily fluid of a mammal; or
(c) a cell lysate or a tissue lysate.

11. The method according to any one of claims 1 to 10, wherein the DNA sample derived from the genomic DNA contained in the biological specimen is a DNA sample preliminarily subjected to a digestion treatment by a restriction enzyme whose recognition cleaving site excludes a target DNA region possessed by the genomic DNA.

12. The method according to any one of claims 1 to 11, wherein the DNA sample derived from the genomic DNA contained in the biological specimen is
(a) a DNA sample preliminarily subjected to a digestion treatment by at least one kind of methylation sensitive restriction enzyme; or
(b) a DNA sample preliminarily subjected to a digestion treatment by at least one kind of methylation sensitive restriction enzyme after addition of the masking oligonucleotide.

13. The method according to claim 12, wherein the at least one kind of methylation sensitive restriction enzyme is HpaII or HhaI which is a methylation sensitive restriction enzyme.

14. The method according to any one of claims 1 to 13, wherein
(a) the DNA sample derived from the genomic DNA contained in the biological specimen is a preliminarily purified DNA sample; and/or
(b) the target DNA region possessed by the genomic DNA is a DNA region having a cleaving site recognized by at least one kind of methylation sensitive restriction enzyme.

15. The method according to any one of claims 1 to 14 wherein
(a) a counter oligonucleotide is added in separating double-stranded DNA contained in a DNA sample derived from the genomic DNA into single-stranded DNA in First step; and/or
(b) separation of double-stranded DNA contained in a DNA sample derived from the genomic DNA into single-stranded DNA in First step is conducted in a reaction system containing a bivalent positive ion or a magnesium ion.

## Patentansprüche

1. Verfahren zum Nachweisen oder Quantifizieren von methylierter DNA in einer DNA-Zielregion, die eine in einer biologischen Probe enthaltene genomische DNA hat, bestehend aus:
(1) einem ersten Schritt des Auftrennens doppelsträngiger DNA, die in einer DNA-Probe enthalten ist, die von der genomischen DNA stammt, die in der biologischen Probe enthalten ist, in einzelsträngige DNA,
(2) einem zweiten Schritt des Mischens (i) der einzelsträngigen DNA, die im ersten Schritt aufgetrennt wurde, (ii) eines Antikörpers gegen methylierte DNA und (iii) eines Oligonucleotids (nachstehend auch als das vorliegende Oligonucleotid bezeichnet), das in der Lage ist, mit der einzelsträngigen DNA zu binden, ohne die Bindung zwischen dem Antikörper gegen methylierte DNA und der methylierten DNA in einer DNA-Zielregion zu inhibieren, wodurch sich ein Komplex aus der einzelsträngigen DNA, die methylierte DNA in der DNA-Zielregion enthält, dem Antikörper gegen methylierte DNA und dem Oligonucleotid bildet, und des Immobilisierens des Antikörpers gegen methylierte DNA oder des Oligonucleotids, die in dem Komplex enthalten sind, auf einem Träger, gefolgt von dem Abtrennen des Komplexes, entweder gleichzeitig oder nach dessen Bildung; und
(3) einem dritten Schritt des Nachweisens oder Quantifizierens entweder des Antikörpers gegen methylierte DNA oder des Oligonucleotids, die in dem abgetrennten Komplex enthalten sind, mittels einer Funktion zur Identifizierung, die für den Nachweis vorliegt, die der Antikörper gegen methylierte DNA oder das Oligonucleotid hat,
wobei, wenn der in dem Komplex enthaltene Antikörper gegen methylierte DNA an einen Träger gebunden ist, der dritte Schritt aus dem Nachweisen oder dem Quantifizieren des Oligonucleotids besteht, und wobei, wenn das in dem Komplex enthaltene Oligonucleotid an einen Träger gebunden ist, der dritte Schritt aus dem Nachweisen oder dem Quantifizieren des Antikörpers gegen methylierte DNA besteht,
wodurch methylierte DNA in der DNA-Zielregion, die in der biologischen Probe enthalten ist, nachgewiesen oder quantifiziert wird.

2. Verfahren nach Anspruch 1, wobei für das Oligonucleotid, das im zweiten Schritt gemischt wird und das nicht die Bindung zwischen dem Antikörper gegen methylierte DNA und einer methylierten Base inhibiert, die auf der einzelsträngigen DNA vorliegt, die die DNA-Zielregion enthält, zwei oder mehr Oligonucleotidarten verwendet werden.

3. Verfahren nach Anspruch 1, wobei der Komplex, der im zweiten Schritt gebildet wird, oder ein gebundener Körper aus der einzelsträngigen DNA, die im ersten Schritt aufgetrennt wird, und dem Oligonucleotid, das nicht die Bindung zwischen dem Antikörper gegen methylierte DNA und einer methylierten Base inhibiert, die auf der einzelsträngigen DNA vorliegt, die die DNA-Zielregion enthält, die während der Bildung des Komplexes im zweiten Schritt entsteht, in einem Reaktionssystem gebildet wird, das ein zweiwertiges positives Ion enthält.

4. Verfahren nach Anspruch 3, wobei das zweiwertige positive Ion ein Magnesiumion ist.

5. Verfahren nach einem der Ansprüche 1 bis 4, umfassend als Abtrennungsvorgang des Komplexes im zweiten Schritt:
(a) einen Schritt, der den im gebildeten Komplex enthaltenen Antikörper gegen methylierte DNA an einen Träger binden lässt; oder
(b) einen Schritt, der das im gebildeten Komplex enthaltene Oligonucleotid an einen Träger binden lässt.

6. Verfahren nach einem der Ansprüche 1 bis 5, das zwischen unmittelbar nach dem Ende des ersten Schritts und unmittelbar vor Beginn des dritten Schritts zusätzlich umfasst:
(a) einen Schritt des Verdauens der im ersten Schritt aufgetrennten einzelsträngigen DNA mit mindestens einer Art, eines Restriktionsenzyms, das methylierungssensitiv ist, das in der Lage ist, einzelsträngige DNA zu verdauen; oder
(b) (i) einen Schritt des Mischens der im ersten Schritt aufgetrennten einzelsträngigen DNA und einem maskierenden Oligonucleotid, das selbst eine Erkennungssequenz für mindestens eine Art eines Restriktionsenzyms hat, das methylierungssensitiv ist, und (ii) des Verdauens des aus dem vorherigen Schritt erhaltenen Gemischs (darin vorhanden einzelsträngige DNA, in der DNA in der DNA-Zielregion nicht methyliert ist) mit dem methylierungssensitiven Restriktionsenzym.

7. Verfahren nach Anspruch 6, wobei die mindestens eine Art eines Restriktionsenzyms, das methylierungssensitiv ist, das in der Lage ist, einzelsträngige DNA zu verdauen, Hhal ist, das ein methylierungssensitives Restriktionsenzym ist und in der Lage ist, einzelsträngige DNA zu verdauen.

8. Verfahren nach Anspruch 6, wobei die mindestens eine Art eines Restriktionsenzyms, das methylierungssensitiv ist, Hpall oder Hhal ist, das ein methylierungssensitives Restriktionsenzym ist.

9. Verfahren nach einem der Ansprüche 1 bis 8, wobei der Antikörper gegen methylierte DNA ein Methylcytosin-Antikörper ist.

10. Verfahren nach einem der Ansprüche 1 bis 9, wobei die biologische Probe
(a) Serum oder Plasma eines Säugers ist;
(b) Blut oder eine Körperflüssigkeit eines Säugers ist; oder
(c) ein Zelllysat oder Gewebelysat ist.

11. Verfahren nach einem der Ansprüche 1 bis 10, wobei die DNA-Probe, die von der in der biologischen Probe enthaltenen genomischen DNA stammt, eine DNA-Probe ist, die zuvor einer Verdaubehandlung mit einem Restriktionsenzym unterzogen wird, dessen Erkennungsschnittstelle eine DNA-Zielregion ausschließt, die die genomische DNA hat.

12. Verfahren nach einem der Ansprüche 1 bis 11, wobei die DNA-Probe, die von der in der biologischen Probe enthaltenen genomischen DNA stammt
(a) eine DNA-Probe ist, die zuvor einer Verdaubehandlung mit mindestens einer Art eines Restriktionsenzyms unterzogen wird, das methylierungssensitiv ist; oder
(b) eine DNA-Probe ist, die zuvor einer Verdaubehandlung nach Hinzufügen des maskierenden Oligonucleotids mit mindestens einer Art eines Restriktionsenzyms unterzogen wird, das methylierungssensitiv ist.

13. Verfahren nach Anspruch 12, wobei die mindestens eine Art eines Restriktionsenzyms, das methylierungssensitiv ist, Hpall oder Hhal ist, das ein methylierungssensitives Restriktionsenzym ist.

14. Verfahren nach einem der Ansprüche 1 bis 13, wobei
(a) die DNA-Probe, die von der in der biologischen Probe enthaltenen genomischen DNA stammt, eine zuvor gereinigte DNA-Probe ist; und/oder
(b) die DNA-Zielregion, die die genomische DNA hat, eine DNA-Region ist, die eine Schnittstelle hat, die von mindestens einer Art eines Restriktionsenzyms, das methylierungssensitiv ist, erkannt wird.

15. Verfahren nach einem der Ansprüche 1 bis 14, wobei
(a) beim Auftrennen der in einer DNA-Probe, die von der genomischen DNA stammt, enthaltenen doppelsträngigen DNA in einzelsträngige DNA im ersten Schritt ein Gegenoligonucleotid hinzugefügt wird; und/oder
(b) das Auftrennen der in einer DNA-Probe, die von der genomischen DNA stammt, enthaltenen doppelsträngigen DNA in einzelsträngige DNA im ersten Schritt in einem Reaktionssystem durchgeführt wird, das ein zweiwertiges positives Ion oder ein Magnesiumion enthält.

## Revendications

1. Procédé de détection ou de quantification d'ADN méthylé dans une région d'ADN cible possédée par de l'ADN génomique contenu dans un échantillon biologique, qui consiste en :
(1) une première étape de séparation de l'ADN double brin contenu dans un échantillon d'ADN dérivé de l'ADN génomique contenu dans l'échantillon biologique en ADN simple brin,
(2) une deuxième étape de mélange (i) de l'ADN simple brin séparé lors de la première étape, (ii) d'un anticorps anti-ADN méthylé, et (iii) d'un oligonucléotide (également désigné ci-après le présent oligonucléotide) capable de se lier à l'ADN simple brin sans inhiber la liaison entre l'anticorps anti-ADN méthylé et l'ADN méthylé dans une région d'ADN cible, en formant ainsi un complexe entre l'ADN simple brin contenant l'ADN méthylé dans la région d'ADN cible, l'anticorps anti-ADN méthylé, et l'oligonucléotide, et l'immobilisation de l'anticorps anti-ADN méthylé ou de l'oligonucléotide contenu dans le complexe sur un support, suivie de la séparation du complexe simultanément ou après la formation ; et
(3) une troisième étape de détection ou de quantification de l'anticorps anti-ADN méthylé ou de l'oligonucléotide contenu dans le complexe séparé, selon une fonction d'identification disponible pour une détection qui est possédée par l'anticorps anti-ADN méthylé ou l'oligonucléotide,
où lorsque l'anticorps anti-ADN méthylé contenu dans le complexe est lié à un support, ladite troisième étape consiste à détecter ou à quantifier ledit oligonucléotide, et où lorsque l'oligonucléotide contenu dans le complexe est lié à un support, ladite troisième étape consiste à détecter ou à quantifier ledit anticorps anti-ADN méthylé,
en détectant ou en quantifiant ainsi l'ADN méthylé dans la région d'ADN cible contenue dans l'échantillon biologique.

2. Procédé selon la revendication 1, dans lequel, en tant qu'oligonucléotide qui est mélangé lors de la deuxième étape et qui n'inhibe pas la liaison entre l'anticorps anti-ADN méthylé et une base méthylée existant dans un ADN simple brin contenant la région d'ADN cible, deux types d'oligonucléotides ou plus sont utilisés.

3. Procédé selon la revendication 1, dans lequel le complexe formé lors de la deuxième étape, ou un corps lié entre l'ADN simple brin séparé lors de la première étape et l'oligonucléotide qui n'inhibe pas la liaison entre l'anticorps anti-ADN méthylé et une base méthylée existant dans un ADN simple brin contenant la région d'ADN cible survenant pendant la formation du complexe lors de la deuxième étape, est formé dans un système réactionnel contenant un ion positif bivalent.

4. Procédé selon la revendication 3, dans lequel l'ion positif bivalent est un ion magnésium.

5. Procédé selon l'une quelconque des revendications 1 à 4, comprenant, en tant qu'opération de séparation du complexe lors de la deuxième étape,
(a) une étape de liaison de l'anticorps anti-ADN méthylé contenu dans le complexe formé à un support ; ou
(b) une étape de liaison de l'oligonucléotide contenu dans le complexe formé à un support.

6. Procédé selon l'une quelconque des revendications 1 à 5, comprenant en outre, entre immédiatement après la fin de la première étape et immédiatement avant le début de la troisième étape,
(a) une étape de digestion de l'ADN simple brin séparé lors de la première étape par au moins un type d'enzyme de restriction sensible à la méthylation capable de digérer de l'ADN simple brin ; ou
(b) (i) une étape de mélange de l'ADN simple brin séparé lors de la première étape et d'un oligonucléotide de masquage comportant une séquence de reconnaissance d'au moins un type d'enzyme de restriction sensible à la méthylation qui en fait partie, et (ii) la digestion d'un mélange obtenu par l'étape précédente (ADN simple brin existant dans celui-ci dans lequel l'ADN n'est pas méthylé dans la région d'ADN cible) par l'enzyme de restriction sensible à la méthylation.

7. Procédé selon la revendication 6, dans lequel le au moins un type d'enzyme de restriction sensible à la méthylation capable de digérer de l'ADN simple brin est HhaI qui est une enzyme de restriction sensible à la méthylation capable de digérer de l'ADN simple brin.

8. Procédé selon la revendication 6, dans lequel le au moins un type d'enzyme de restriction sensible à la méthylation est HpaII ou HhaI qui est une enzyme de restriction sensible à la méthylation.

9. Procédé selon l'une quelconque des revendications 1 à 8, dans lequel l'anticorps anti-ADN méthylé est un anticorps anti-méthylcytosine.

10. Procédé selon l'une quelconque des revendications 1 à 9, dans lequel l'échantillon biologique est
(a) du sérum ou du plasma d'un mammifère ;
(b) du sang ou un fluide corporel d'un mammifère ; ou
(c) un lysat cellulaire ou un lysat tissulaire.

11. Procédé selon l'une quelconque des revendications 1 à 10, dans lequel l'échantillon d'ADN dérivé de l'ADN génomique contenu dans l'échantillon biologique est un échantillon d'ADN préalablement soumis à un traitement de digestion par une enzyme de restriction dont le site de clivage de reconnaissance exclut une région d'ADN cible possédée par l'ADN génomique.

12. Procédé selon l'une quelconque des revendications 1 à 11, dans lequel l'échantillon d'ADN dérivé de l'ADN génomique contenu dans l'échantillon biologique est
(a) un échantillon d'ADN préalablement soumis à un traitement de digestion par au moins un type d'enzyme de restriction sensible à la méthylation ; ou
(b) un échantillon d'ADN préalablement soumis à un traitement de digestion par au moins un type d'enzyme de restriction sensible à la méthylation après l'addition de l'oligonucléotide de masquage.

13. Procédé selon la revendication 12, dans lequel le au moins un type d'enzyme de restriction sensible à la méthylation est HpaII ou HhaI qui est une enzyme de restriction sensible à la méthylation.

14. Procédé selon l'une quelconque des revendications 1 à 13, dans lequel
(a) l'échantillon d'ADN dérivé de l'ADN génomique contenu dans l'échantillon biologique est un échantillon d'ADN préalablement purifié ; et/ou
(b) la région d'ADN cible possédée par l'ADN génomique est une région d'ADN comportant un site de clivage reconnu par au moins un type d'enzyme de restriction sensible à la méthylation.

15. Procédé selon l'une quelconque des revendications 1 à 14, dans lequel
(a) un oligonucléotide antisens est ajouté pour séparer l'ADN double brin contenu dans un échantillon d'ADN dérivé de l'ADN génomique en ADN simple brin lors de la première étape ; et/ou
(b) la séparation de l'ADN double brin contenu dans un échantillon d'ADN dérivé de l'ADN génomique en ADN simple brin lors de la première étape est réalisée dans un système réactionnel contenant un ion positif bivalent ou un ion magnésium.


### SEQUENCE LISTING

<110> Sumitomo Chemical Co., Ltd.
<120> Method for measuring DNA methylation
<130> S17332W001
<150> JP2007-078660
   <151> 2007-3-26
<160> 170
<210> 1
   <211> 2661
   <212> DNA
   <213> Homo sapiens
<400> 1
   acagacatgt gccaccatgc ccagctaatt ttttgtttgt ttgtttgttt gtttgtattt     60
   ttagtagaga tggggttttg ccatgttggc caggctggtc tcgaactcct gacctcgaat    120
   gataatgatc cgccgcttgg cctccaaagt gctaggatta caggtgtgag ccactgcgcc    180
   aggcctgggc actttcttta gtagtttgag gagcaacatt tttgacagtg tccttctgct    240
   caagattcaa gatcccagat aaaattaaac catctagaga gatggcttga ttggccaaac    300
   ctggatctca tgaccacttc ttgaagtggg taagtctcat aaatgctcag tccttccact    360
   atgcaactga gtggggtggg tgggaagccc ctcaaaggaa aatccggttg ttcttactag    420
   aaagaaaagg aaaatggatg tgaggcagtc aaaatcagca gaggtccacc acaccaccaa    480
   aatgtggtga ttaaatatgg agagacagag actaacagag gtatgtgaat attgaagtat    540
   gtctggacaa tagcccaatg atgagaccaa taaaatggtt accaaaatct ggttttgagt 600
   agtagtgtta aatcagacca tttagtaacc attttttgtt gcaaagtttc tagcactgcc 660
   caaaccctga gtggtatatg aataactcgt ccattatgta tctctttcca gtcagcataa 720
   tttatccccc acctatattc ttttctgacc actcctactt ccttctcttt accaaaatct 780
   aaactctaag gctgtttctt cagcaacttc tttgtttaga ttggaagata aattaaacag 840
   catgcgatgt tttactgact ttcagtattt aacagaggtg atttaatttt tttttaaatc 900
   caaagtcaaa cttctttata agatgaagga gaaaaatgtc ttataaaatg catatgtgaa 960
   gatgccttct gagtgctttc tcatgcagac ttgttctagt ctttaatgaa tcttccttgt 1020
   agacactgtg gagatgaaag atggttctcc acttctactc aaagtacaaa tcaggccggc 1080
   attttgaaaa agagacaggt ttattcatag ctgcagcgtt agctggcttt gttccctgta 1140
   caatttcact tttggttatt aaaatattca ctgtaggaaa taaatttgta acccatttct 1200
   catattacct acacacagaa aaacaaaatt tgatatcctg gggtttattt gctgagggcg 1260
   cttcccataa aagcgagaga gtgtgcgttg ggaaatgtgt ctggttaact cttttatgga 1320
   taaactttag tcacaatcct cccccgcccc cctctcaccc ccagcaccct cccaacctcc 1380
   cgacttcccg cctctcaagg gctggtgacc taatagcatt tttcttcgtg catattttgg 1440
   cgtcgcccca tggcctggct gccttcgcct gtctgagttt tttgaaattc ctgcatgttc 1500
   gccccagatt aagccagtgt gtctcaggat gtgtgttccg ttttgttctt tccccttaac 1560
   gctccctgtg caacgtgtct ggggggagga gggcagggac gggagagagg gaggggcaga 1620
   ggcgaggagc tgtccgcctt gcacgtttcc aatcgcatta cgtgaacaaa tagctgaggg 1680
   gcggccgggc cagaacggct tgtgtaactt tgcaaacgtg ccagaaagtt taaatctctc 1740
   ctccttcctt cactccagac actgcccgct ctccgggact gccgcgcggc tccccgttgc 1800
   cttccaggac tgagaaaggg gaaagggaag ggtgccacgt ccgagcagcc gccttgactg 1860
   gggaagggtc tgaatcccac ccttggcatt gcttggtgga gactgagata cccgtgctcc 1920
   gctcgcctcc ttggttgaag atttctcctt ccctcacgtg atttgagccc cgtttttatt 1980
   ttctgtgagc cacgtcctcc tcgagcgggg tcaatctggc aaaaggagtg atgcgcttcg 2040
   cctggaccgt gctcctgctc gggcctttgc agctctgcgc gctagtgcac tgcgcccctc 2100
   ccgccgccgg ccaacagcag cccccgcgcg agccgccggc ggctccgggc gcctggcgcc 2160
   agcagatcca atgggagaac aacgggcagg tgttcagctt gctgagcctg ggctcacagt 2220
   accagcctca gcgccgccgg gacccgggcg ccgccgtccc tggtgcagcc aacgcctccg 2280
   cccagcagcc ccgcactccg atcctgctga tccgcgacaa ccgcaccgcc gcggcgcgaa 2340
   cgcggacggc cggctcatct ggagtcaccg ctggccgccc caggcccacc gcccgtcact 2400
   ggttccaagc tggctactcg acatctagag cccgcgaacc tggcgcctcg cgcgcggaga 2460
   accagacagc gccgggagaa gttcctgcgc tcagtaacct gcggccgccc agccgcgtgg 2520
   acggcatggt gggcgacgac ccttacaacc cctacaagta ctctgacgac aacccttatt 2580
   acaactacta cgatacttat gaaaggccca gacctggggg caggtaccgg cccggatacg 2640
   gcactggcta cttccagtac g 2661
<210> 2
   <211> 1953
   <212> DNA
   <213> Homo sapie.s
<400> 2
   tataaattcc acgcaggcat tgaattgaat ttgttcttaa ccaaatgcgt tttatctata 60
   cctggcagga atctagaagt gaaattacaa gatttatttc attttaattc tattatgaag 120
   catttaatca caaataccct gaaaatgaaa agataattta tcattttacc ttgactgagc 180
   aactctcctc acttcacatt catgaatcca taacgcagag aggagactgg atgattaagt 240
   gtttgattag agaaaacaga ttaacctagc aaacataata aatttggctc ataagcagga 300
   tggctttata aatgctcaca atacctctcc tgtataaaat catgaaccac ttcctacagt 360
   gatgactcca tcgaaatagt tgagaaacat aaagcaaatg catgtttatg gctttctctt 420
   tgagacatta aaagggtatt gaaaggcata tctgattcag cttataactc tggatatata 480
   ttaaggaaca tgtaagaaaa tattaatgca taaaaaaagc tacaacttct caagtgttct 540
   agtttccact ttgtcaataa ttacgttttc aatgtccttc tgtggactgt ttccaaaggt 600
   gccaatccag acccaaagtt tcagatcact cagattcacc cttaaccttc ataacacaac 660
   ccaatagctt tacgaaaaaa gttgcatatt taggtagttg ttatcccatt atgacaaaat 720
   acataaaatt agcgagatat tttttagcct tcaaataagt gggaaaaaat ccttttagct 780
   gagattccat ttacatcaga ataaaaatct aagttatgac taggttgaag caacgtcctg 840
   tgcagcgctc cataaagttc acttagtctt caagggttcc ttacttagct aggttagtat 900
   tcctggcctc tttttttagc agtgagaaaa aggatactct ccctgcccca gctttatttt 960
   taaactcaca gccatatcct ggaggtctct gctggctatt tggcgcgtgg gggcggaggg 1020
   gggccggggg aggggggcgg ggcggggtct ggaggtctgt gctggctatc tggcgtgtgt 1080
   gtgtgtgtgt gtgtgtgtgt gtgtggttgg aggtctctgc tggctatctg gcgtgtgtgt 1140
   gtgtgtggtg tggtgtgtgt aagcagtgag gttgttttag ggccagtcct tcctccgcca 1200
   ctttgctgac tcaaagaccc agaggctttc ttggggtgca ggtaccatga ttccttgggc 1260
   cctaagggaa tttttgttag gctagaagag tgggtgtact catgatgggt gtacccgaac 1320
   attcctgggc tcaacaaaac cgattatctt tataaccgcg gcgcctagca cagcgcctgg 1380
   tgccctaaac gttggctgcg ggaacgtccg agacgcgggt gcggagccgg gggcggaata 1440
   actggttgcg cggcgctttg accgtaggcg ctggagcgcg tgcgttgcgt gcgcgcgcgg 1500
   aggcggctgc gtcggggcgc gagaaggtgc agttccccgg cgggcgggcg ggcgggcggg 1560
   cgaagctggg ctcggggcca agcgaggtct agccggagcg actgtgcccc gcctcctggg 1620
   cggagcgggc ggctccccat ggtcagagcc tcgtgccggc tcggcagcgc ccggacgccg 1680
   agcccagcgc gtcggccccc cggcgtgcgg gcgtctcaga gccgcggagg ggccgccggg 1740
   accgtttcag cgtggcggcg ctggtgctgg cgttggccct ggaggacggc cccgagtgat 1800
   ggctggcgcc tgcctcccgg gtgtctcccg ggtacagatg gagtcgtccc gcggccgccg 1860
   gcggcaaggt cggcagctgc gaggccaaga gagaccccag gacacacaca gctgcctccc 1920
   ggtgcgagaa gaagaccccg gcttgagagt gag 1953
<210> 3
   <211> 889
   <212> DNA
   <213> Homo saptens
<400> 3
   cggccccatg gctccgtgtc gtgtccaagg gatgggctgg cacctcttgg accaggctta 60
   ccaccagggc ccttctctga agccccagtc tgaccggcct gctgctggga atccccctct 120
   gcccccacac taacctctgc tggggctgag ccagggcgcg tcggacagtc agggcgaccc 180
   agccagggcg accgttggcc ccgctcctat ggggcagcag ggaccgacgt cagcagggtg 240
   gggcgggcac ccgagtggta tgccccgccc tgccccgcct gcccgccctg gtggccgtct 300
   gggggcgaca agtcctgaga gaaccagacg gaagcgcgct gggactgaca cgtggacttg 360
   ggcggtgctg cccgggtggg tcagcctggg ctgggaggca gccccgggac acagctgtgc 420
   ccacgccgtc tgagcacccc aagcccgatg cagccacccc cagacgaggc ccgcagggac 480
   atggccgggg acacccagtg gtccaggtgt ggcgggggtg aggggagggg gggtgggagc 540
   ggtggagatg gggccgtggg gagggagctg agatactgcc acgtgggacg atgctaggtg 600
   gggagggctg agctgggcgg gctcctctgg ctgtggggcc ccctgtgttc cttgtgggag 660
   gtggaaggaa gtgagtgccc tgtccttcct ccctgccatg agattccagg accggacctg 720
   gcaagtgccc tatcccagcc agtgttcctg gggctcttcc aggcagggct atgttcccca 780
   ggccaggggc attgtcctgg acagtcagga ggcatacccc tcgccaggtg gaaccaccct 840
   gtgtatgcat gaccctgaca agcaggcgcc aggacagtca ggaggccag 889
<210> 4
   <211> 863
   <212> DNA.
   <213> Homo sapiens
<400> 4
   gttgttgggt gtgaatggag aactgtgggc cctccccgac accttccagc gggacggcaa 60
   cgggggccca gggggtgggc gccatcaacc ccgtcccacc gccaggacgg cgcgggggag 120
   ggccggcggg ggcggggcgt cctgtaaggc gcggccccca cccgcgggcg gggcggcatt 180
   cctgggaggc cggcgctctg acgtggaccc gggggccgcg ggcacggcgg gggggcggcg 240
   gtccgggggc ttcttaaacc ccccgccccg gcccagcccg cacttcccga gcaccgctcc 300
   gaccctggag ggagagagag ccagagagcg gccgagcgcc taggaggccc gccgagcctc 360
   gccgagcccc gccagccccg gcgcgagaga agttggagag gagagcagcg cagcgcagcg 420
   agtcccgtgg tcgcgcccca acagcgcccg acagcccccg atagcccaaa ccgcggccct 480
   agccccggcc gcacccccag cccgcgccag catgatgaac aacagcggct actcagacgc 540
   cggcctcggc ctgggcgatg agacagacga gatgccgtcc acggagaagg acctggcgga 600
   ggacgcgccg tggaagaaga tccagcagaa cacattcacg cgctggtgca atgagcacct 660
   caagtgcgtg ggcaagcgcc tgaccgacct gcagcgcgac ctcagcgacg ggctccggct 720
   catcgcgctg ctcgaggtgc tcagccagaa gcgcatgtac cgcaagttcc atccgcgccc 780
   caacttccgc caaatgaagc tggagaacgt gtccgtggcc ctcgagttcc tcgagcgcga 840
   gcacatcaag ctcgtgtcca tag 863
<210> 5
   <211> 2198
   <212> DNA
   <213> Homo sapiens
<400> 5
   aagagaggca cactccctct accacaccga gggagggggc gttgagctga gaaaggttga 60
   gagaatgagg gacccaggta ggtggacatc ggccaagaaa ggaaccacag cgggaggtaa 120
   gaccgagagt ccccagcttg aagcgtcacc actccgggat tcccagattc caacgcgagc 180
   ctggggaaag cccacagtgg agagagtccg gctggcaggg aatggcccta cccccggggt 240
   gaaatctcgg agggtcgtgc agccgagtcg cgcctctgcg ctgatgcgtg agagatgccg 300
   gacgtcgcgt ttgcctgtgc gagcctcgcg gatgctgtgc agtcttggtc ccctctgcgt 360
   gtgtctaacg ccgaatgctg gtgtctcgag gtgtgagctt cggggccggt gtctttaaag 420
   aaccaaagat tcttaaggag tgatgatctg ggtagagcgg cccgacgtag ccgcgctccc 480
   aggtctcggt gcgagtcctg cggacagacc agaggagacc tgctggccag atgccccggg 540
   cccaaggcgg acgccagact gtctctgcgc cagccgggct ggccttcgga atggatcagg 600
   cacccgggag gccggagtgg atctcagacc ctcaagccgg gaacaaaccc gtcgatgccc 660
   gtgggcctgg agtccgcctc ctccttcccg ccccacccct acccctgcct ccgaaaggct 720
   tcttcgctgg tcagtagctg cgtgcccgtc tgcctgaggc tgggtcagaa ttggcgggct 780
   ggtaacgacc ccgtgcacaa gcggctccca gtctctccag aaagggccga tgactaaggg 840
   gtgggggtgg gggcggaggg ctggaaggtg ttagggaaga acgttagcgg cctatcctgt 900
   cttcagcagc gccctctcat cttctagctc tgacgccgag cagagcagtt ggagctcggg 960
   actgggaact gctggaattc ctatttagac ttctagacag tctagaaaca agaacctttc 1020
   tttccctggg cctcagtttc cttgtctgta aaatcaaaag gcgggctcta ggtgtaggcc 1080
   ttcttttcgc ttggtgattc tggattcctt tccttggatc cgtggggagg gggtggcagc 1140
   aacagtccag ggcgttggcc gtcctgtgcc tcaagtacgt agtccccgtg cccgccccct 1200
   caacaccccc agcagcccgc ccccctaagc ccgcagagca gggagctgag tgggaggggc 1260
   agaggcgggg ccggttccca gtccctgctg gcggactaga gtggcgcggg ctgagcgtaa 1320
   aacctgggat agccactccc ccttttcctt atccccgccc ccctgccatt ggctcccggg 1380
   agaggttgac atcaaagccg cggtcttata taagccagat ccgcagggga gtccgcagaa 1440
   gggttaaaca ggtctttggg cttcggcgac ctcgcccgcg gcagaaaccg gtaagaagac 1500
   agtgggctgc gcgtctcatt ttcagccttg cccggactct cccaaagccg gcgcccagta 1560
   gtggctccag agcccacagg tggcccccgg cagtctctgg ggcgcatgga gcggcgttaa 1620
   tagggctggc ggcgcaggcc agtagccgct ccaacatgaa cctcgtgggc agctacgcac 1680
   accatcacca ccatcaccac ccgcaccctg cgcaccccat gctccacgaa cccttcctct 1740
   tcggtccggc ctcgcgctgt catcaggaaa ggccctactt ccagagctgg ctgctgagcc 1800
   cggctgacgc tgccccggac ttccctgcgg gcgggccgcc gcccgcggcc gctgcagccg 1860
   ccaccgccta tggtcctgac gccaggcctg ggcagagccc cgggcggctg gaggcgcttg 1920
   gcggccgtct tggccggcgg aaaggctcag gacccaagaa ggagcggaga cgcactgaga 1980
   gcattaacag cgcattcgcg gagttgcgcg agtgcatccc caacgtgccg gccgacacca 2040
   agctctccaa gatcaagact ctgcgcctag ccaccagcta catcgcctac ctgatggacg 2100
   tgctggccaa ggatgcacag tctggcgatc ccgaggcctt caaggctgaa ctcaagaagg 2160
   cggatggcgg ccgtgagagc aagcggaaaa gggagctg 2198
<210> 6
   <211> 1945
   <212> DNA
   <213> Homo sapiens
<400> 6
   ctggatgaca gagtgagact ccgtctcaaa aaaaaagctc catttgggag gccgaggagg 60
   gtggattacc tgaggtcagg agtttgagac cagcctggcc cacataggga aaccccatct 120
   ctactaaaaa tacaaaaatt agtcaggtgt ggtggctgac acctataatc ccagctactt 180
   gggaagctga ggcagggaga atcacttgaa ccggggaggt ggaggttgca gtgagctgag 240
   atcatgccac tgcactccag cctgggcgac agggtgagat tctgtctcaa acaaacaaat 300
   ttaaaagctc cgaatcctcc aaaaatacca agattttcct gtcggtaact agagatgggt 360
   actgatgatt atttttaata ggtgattttc aaagatgtga acgttatcca tggagattta 420
   agtctccaaa aggaaaaaaa atgcatacct ttatactaaa acttcatcac cagtcaaatt 480
   tggatcatca ctaaattggc ttctacacct ctctcctaat ataaggtact tgtgtaagtt 540
   tgcagttgtg agacacttat ttcctcattt ttaatgtctt ctcagtaggg ccactgatat 600
   agtcactatt tgactgacca gaatggttgg cactggtgat tggctcataa agtgccctcg 660
   atttaggggg ctcaattatc aaaggtttaa atcctagccc aaaccattgc tgtgatgggg 720
   gttaatcaat gaaccactca gcttcacttg caaaagcggg atcacaatag ccgctttcgt 780
   catgacccag cctaggtgag atttagtact taagtacact gccaggcaca caaggttaat 840
   ttaacaattt aacacatttg tttcctcatc catttctcca aaccttccaa ctaatcctaa 900
   cgttcgttcg gccaaatggg ccaggaattc acttaaacaa aaacaaaaaa caaaacaaac 960
   aaaaaaacac tccctggggc ttggggaagg aggcaccgcc gcccatgtcg cagtctgggg 1020
   gtggctcagt cctcagcacc cagatctacg gccataatgc tcttcgaggc caaggagccc 1080
   ggatgcgggg cgttgccgaa ggcgtcttgc tcaggctgcg ggaaaggaga ggggtgggag 1140
   cggggtgggg gcatcgcgac ccagggcaag gcggcgagtc gccgtcttcg agtcccacct 1200
   gtccgaagcg gggtgagaaa aggcaaaaca tggcaaagcc atgcacctcc cagggtgggc 1260
   aactcacggc cggtgaacgc cggaccctta gcagtttcca gacctttgga accggaagcg 1320
   gagcctgaga gcgcgcccga gagggcgtga acgggaccgc tttcccggaa gtgcttgcgg 1380
   cctctgccca gcgagctgcc ccggggtctc tctggtttcc taatcagggc aacgccgcgg 1440
   gagagaacct ttaccttggc tgcactaagt tctcggtgcc actccctggc agggcgggac 1500
   cttgtttagg ccctgtgatc gcgcggttcg tagtagcgca aggcgcagag tggaccttga 1560
   cccgcctagg gcgggaagag tttggcccgc cgggtcccaa agggcagaat ggacgggctc 1620
   ctaaatccca gggaatcctc taaattcatt gcagaaaaca gtcgggatgt gtttattgac 1680
   agcggaggcg tacggagggt ggcagagctg ctgctggcca aggcggcggg gccagagctg 1740
   cgcgtggagg ggtggaaagc ccttcatgag ctgaacccca gggcggccga cgaggccgcg 1800
   gtcaactggg tgttcgtgac agacacgctc aacttctcct tttggtcgga gcaggacgag 1860
   cacaagtgtg tggtgaggta cagagggaaa acatacagtg ggtactggtc cctgtgcgcc 1920
   gccgtcaaca gagccctcga cgaag 1945
<210> 7
   <211> 2379
   <212> DNA
   <213> Homo sapiens
<400> 7
<210> 8
   <211> 933
   <212> DNA
   <213> Homo sapiens
<400> 8
   gagacgtact ctggctctgt cgcccaggct ggagcgcaat ggcgccatct cggcgcactg 60
   caacctccac ctcccgggtt caagcgattc tactgcctca gcctcccgag tagctgggac 120
   tacaggcgcg cactaccaag cccggctaat ttcttttgta tttttagtag agactgggtt 180
   tcacgatgtt ggccgggctg gtctggaagt cttgacctca agcgtgcgcc ctctccgcca 240
   ctgggtaagg cggggggcgg aatagggggc ttgcaatttc acactagagg cgggcgccgt 300
   gggggaaaga agagtcacgt ctcccacggt tcgtagagga aggcctgcct gagcctggag 360
   cgggggcggg agagccacag tttggcatcc ccagggcatc ccccagcccg cagactacca 420
   ggcctccaga ggacaggacc ccacccccgg ccacaggccc tgcccccagc actccccgca 480
   ccccgcctcc aagactcctc cgcccactcc gcacccaact tataaaaacc gtcctcgggc 540
   gcggcgggga gaagccgagc tgagcggatc ctcacacgac tgtgatccga ttctttccag 600
   cggcttctgc aaccaagcgg gtcttacccc cggtcctccg cgtctccagt cctcgcacct 660
   ggaaccccaa cgtccccgag agtccccgaa tccccgctcc caggctacct aagaggatga 720
   gcggtgctcc gacggccggg gcagccctga tgctctgcgc cgccaccgcc gtgctactga 780
   gcgctcaggg cggacccgtg cagtccaagt cgccgcgctt tgcgtcctgg gacgagatga 840
   atgtcctggc gcacggactc ctgcggctcg gccaggggct gcgcgaacac gcggagcgca 900
   cccgcagcca gctgagcgcg ctggagcggc gcc 933
<210> 9
   <211> 6096
   <212> DNA
   <213> Homo sapiens
<400> 9
   atctgcacct cctcatatag ggttgatcca agtttcacag acatcactga gttcttagtg 60
   gactcagcta ttggggctgt tctcacactt tttttttctt tgcaagaatc agcaatgggt 120
   gcaagtggac ctgtgtagga cgtccagtga aacattgtgt tggtgaatca gctagaatcc 180
   atccaagaac tcagccagcc tggtgtgggg tgagatctga tccttgaatg tccctcagtg 240
   gcttttaggg ctggcaggtt cagaagggcc ctctcatcac ccccccaggg cctcattcct 300
   tgtttaacac tttgctatca cagtcttgaa tccttgtaat tgaacaatgg accccacatt 360
   ttcactttgc actggtttct gattctgtaa ccgatcctgt ccccctctct tgtctcattc 420
   actctgggaa ttgtccccac attctgagac ctttcagcag tgccccaacg aggttcctgc 480
   ccttatctga agctccaccc tcacccccat ggcggcaccg caggcagccc tgcttttgcg 540
   tcccgcgtag gcaggctgtg caccggagtc acgaccccct gattcagcct aggcagccac 600
   agcttgactg ctcccgccgg acaagcccta ctgtgctatc tgccgctctt cccttcctct 660
   tcccaggggg tccgcgtcag gggaggcgca gctgtgtgca ttccgggagc ttcagacccc 720
   cgtgtccagc agctccttcg tttcctgggt gctggggcgg ccttcccagc gaagagctca 780
   actcagcggg acgtttggag gctctctgcc ccaaggcgct ggggagtgtg cggcgggaca 840
   gtcgtgcttg cctttttcac tttcagagtg tccacgcccc acccgtttgg tcactgcagg 900
   tcagtccagt ccagcccggc ccaccccacc ggtgcgtgtc tgtcgcacgt ggcagacgcc 960
   atactctctg ttcttgttta aagcccagga tctactgggc cctggaggca agaggtgaac 1020
   gcagcggaat ccacgctgag ctgcccggga acggagcttc caaccccaga aggaggactc 1080
   tgtgctccta caccttaacc ctttttagcc cgaaacttct ccaacttcct tggctttgtt 1140
   tagagctcga cagcgccgcc ccctggcgct cgttgtgagg acagtagagg agagaggcaa 1200
   gggtgttttt aaacagtttg cctctcacca ttatgggggc gacccgaggg ggagacccac 1260
   tcttccgcat tcccggtaag tgaaccaccg gaagaggtcg aaagtgacgg attcccatgt 1320
   cctcctccag cccccccccc accctgccca tccacaggac ggtggctctt cagtgccctt 1380
   tgccgagcaa gtggcgtttc tatgcacgtg ggtatcaatt cggactctgg acgaaatgga 1440
   aacctcctta gccgacccgg gtgggatcag ctgggatcct gcgcgctccc ctggggggtt 1500
   gccagccact ctgttggggt gcaagaagca ccatccttcg gaagctgggc cgaaactggc 1560
   caggctgact cgctcccacg cgcccgcccc tacccggcgc cgcagcaatt cacctgccac 1620
   cgcctctgag ccgggtccgg acttcggcgc cctgacagtg tccccgcgac ttccccaccc 1680
   gatgagatgg ggtctggcgt tggccagtgc gtgtccaggg actcgcgggt ccctggccag 1740
   ccatggggca gagggcgctg gtgttaggcc agtcttcccc accctgcccc gtcaccccag 1800
   ccacacccac tgtcctgtga ggccaagcgc gctccgctgg tttcctgagc caggcacctt 1860
   ggccgcggac aggatccagc tgtctctcct tgcgatcctg tcttcgggga agtccacgtc 1920
   ctaggcaggt cctcccaaag tgcccttggt gccgatcacc cctcccagcg tcttgcaggt 1980
   cctgtgcacc acctccccca ctccccattc aaagccctct tctctgaagt ctccggttcc 2040
   cagagctctt gcaatccagg ctttccttgg aagtggctgt aacatgtatg aaaagaaaga 2100
   aaggaggacc aagagatgaa agagggctgc acgcgtgggg gcccgagtgg tgggcgggga 2160
   cagtcgtctt gttacagggg tgctggcctt ccctggcgcc tgcccctgtc ggccccgccc 2220
   gagaacctcc ctgcgccagg gcagggttta ctcatcccgg cgaggtgatc ccatgcgcga 2280
   gggcgggcgc aagggcggcc agagaaccca gcaatccgag tatgcggcat cagcccttcc 2340
   caccaggcac ttccttcctt ttcccgaacg tccagggagg gagggccggg cacttataaa 2400
   ctcgagccct ggccgatccg catgtcagag gctgcctcgc aggggctgcg cgcagcggca 2460
   agaagtgtct gggctgggac ggacaggaga ggctgtcgcc atcggcgtcc tgtgcccctc 2520
   tgctccggca cggccctgtc gcagtgcccg cgctttcccc ggcgcctgca cgcggcgcgc 2580
   ctgggtaaca tgcttggggt cctggtcctt ggcgcgctgg ccctggccgg cctggggttc 2640
   cccgcacccg cagagccgca gccgggtggc agccagtgcg tcgagcacga ctgcttcgcg 2700
   ctctacccgg gccccgcgac cttcctcaat gccagtcaga tctgcgacgg actgcggggc 2760
   cacctaatga cagtgcgctc ctcggtggct gccgatgtca tttccttgct actgaacggc 2820
   gacggcggcg ttggccgccg gcgcctctgg atcggcctgc agctgccacc cggctgcggc 2880
   gaccccaagc gcctcgggcc cctgcgcggc ttccagtggg ttacgggaga caacaacacc 2940
   agctatagca ggtgggcacg gctcgacctc aatggggctc ccctctgcgg cccgttgtgc 3000
   gtcgctgtct ccgctgctga ggccactgtg cccagcgagc cgatctggga ggagcagcag 3060
   tgcgaagtga aggccgatgg cttcctctgc gagttccact tcccagccac ctgcaggcca 3120
   ctggctgtgg agcccggcgc cgcggctgcc gccgtctcga tcacctacgg caccccgttc 3180
   gcggcccgcg gagcggactt ccaggcgctg ccggtgggca gctccgccgc ggtggctccc 3240
   ctcggcttac agctaatgtg caccgcgccg cccggagcgg tccaggggca ctgggccagg 3300
   gaggcgccgg gcgcttggga ctgcagcgtg gagaacggcg gctgcgagca cgcgtgcaat 3360
   gcgatccctg gggctccccg ctgccagtgc ccagccggcg ccgccctgca ggcagacggg 3420
   cgctcctgca ccgcatccgc gacgcagtcc tgcaacgacc tctgcgagca cttctgcgtt 3480
   cccaaccccg accagccggg ctcctactcg tgcatgtgcg agaccggcta ccggctggcg 3540
   gccgaccaac accggtgcga ggacgtggat gactgcatac tggagcccag tccgtgtccg 3600
   cagcgctgtg tcaacacaca gggtggcttc gagtgccact gctaccctaa ctacgacctg 3660
   gtggacggcg agtgtgtgga gcccgtggac ccgtgcttca gagccaactg cgagtaccag 3720
   tgccagcccc tgaaccaaac tagctacctc tgcgtctgcg ccgagggctt cgcgcccatt 3780
   ccccacgagc cgcacaggtg ccagatgttt tgcaaccaga ctgcctgtcc agccgactgc 3840
   gaccccaaca cccaggctag ctgtgagtgc cctgaaggct acatcctgga cgacggtttc 3900
   atctgcacgg acatcgacga gtgcgaaaac ggcggcttct gctccggggt gtgccacaac 3960
   ctccccggta ccttcgagtg catctgcggg cccgactcgg cccttgcccg ccacattggc 4020
   accgactgtg actccggcaa ggtggacggt ggcgacagcg gctctggcga gcccccgccc 4080
   agcccgacgc ccggctccac cttgactcct ccggccgtgg ggctcgtgca ttcgggcttg 4140
   ctcataggca tctccatcgc gagcctgtgc ctggtggtgg cgcttttggc gctcctctgc 4200
   cacctgcgca agaagcaggg cgccgccagg gccaagatgg agtacaagtg cgcggcccct 4260
   tccaaggagg tagtgctgca gcacgtgcgg accgagcgga cgccgcagag actctgagcg 4320
   gcctccgtcc aggagcctgg ctccgtccag gagcctgtgc ctcctcaccc ccagctttgc 4380
   taccaaagca ccttagctgg cattacagct ggagaagacc ctccccgcac cccccaagct 4440
   gttttcttct attccatggc taactggcga gggggtgatt agagggagga gaatgagcct 4500
   cggcctcttc cgtgacgtca ctggaccact gggcaatgat ggcaattttg taacgaagac 4560
   acagactgcg atttgtccca ggtcctcact accgggcgca ggagggtgag cgttattggt 4620
   cggcagcctt ctgggcagac cttgacctcg tgggctaggg atgactaaaa tatttatttt 4680
   ttttaagtat ttaggttttt gtttgtttcc tttgttctta cctgtatgtc tccagtatcc 4740
   actttgcaca gctctccggt ctctctctct ctacaaactc ccacttgtca tgtgacaggt 4800
   aaactatctt ggtgaatttt tttttcctag ccctctcaca tttatgaagc aagccccact 4860
   tattccccat tcttcctagt tttctcctcc caggaactgg gccaactcac ctgagtcacc 4920
   ctacctgtgc ctgaccctac ttcttttgct cttagctgtc tgctcagaca gaacccctac 4980
   atgaaacaga aacaaaaaca ctaaaaataa aaatggccat ttgctttttc accagatttg 5040
   ctaatttatc ctgaaatttc agattcccag agcaaaataa ttttaaacaa aggttgagat 5100
   gtaaaaggta ttaaattgat gttgctggac tgtcatagaa attacaccca aagaggtatt 5160
   tatctttact tttaaacagt gagcctgaat tttgttgctg ttttgatttg tactgaaaaa 5220
   tggtaattgt tgctaatctt cttatgcaat ttcctttttt gttattatta cttatttttg 5280
   acagtgttga aaatgttcag aaggttgctc tagattgaga gaagagacaa acacctccca 5340
   ggagacagtt caagaaagct tcaaactgca tgattcatgc caattagcaa ttgactgtca 5400
   ctgttccttg tcactggtag accaaaataa aaccagctct actggtcttg tggaattggg 5460
   agcttgggaa tggatcctgg aggatgccca attagggcct agccttaatc aggtcctcag 5520
   agaatttcta ccatttcaga gaggcctttt ggaatgtggc ccctgaacaa gaattggaag 5580
   ctgccctgcc catgggagct ggttagaaat gcagaatcct aggctccacc ccatccagtt 5640
   catgagaatc tatatttaac aagatctgca gggggtgtgt ctgctcagta atttgaggac 5700
   aaccattcca gactgcttcc aattttctgg aatacatgaa atatagatca gttataagta 5760
   gcaggccaag tcaggccctt attttcaaga aactgaggaa ttttctttgt gtagctttgc 5820
   tctttggtag aaaaggctag gtacacagct ctagacactg ccacacaggg tctgcaaggt 5880
   ctttggttca gctaagctag gaatgaaatc ctgcttcagt gtatggaaat aaatgtatca 5940
   tagaaatgta acttttgtaa gacaaaggtt ttcctcttct attttgtaaa ctcaaaatat 6000
   ttgtacatag ttatttattt attggagata atctagaaca caggcaaaat ccttgcttat 6060
   gacatcactt gtacaaaata aacaaataac aatgtg 6096
<210> 10
   <211> 2500
   <212> DNA
   <213> Homo sapiens
<400> 10
   acccacttct gtgtgtggat agtatcctgc aggagagatg ttgtctgcag tgtgagctgg 60
   gcccaccgga gtgtgtgaat aggatcctgc aggagaaatg gaatccggag tgtgagctgc 120
   atccgctgta gagggtggat aaaatcctgc aggaaagatg gcatctggaa tgtcagcggg 180
   agccaccgac ctctgaggat gcaccccgca ggtgtgatgc ggggccagtt ccaaggctgg 240
   gttaggtttt accctggctt ctgtgttgta ctctcattct cttcctcttt cttctaatac 300
   ctgctctggg aggcatcagg ccatgtccag tgtgcaggcc atggagaccc acacggcaag 360
   gaactggaac cccctgccag cagcctcggg ggtccagtcc ttagatggtg ccctgtggtc 420
   agcaatgcac ctgtgacctc cgggctatgt ctcgtggtag ttgcttttgt gttttaacat 480
   agcaacagga aactagccta ttacccacca atcccattcc aggctgcttt caaacgcagc 540
   tcaggctaga acaccagcac ggggacacag ctgagacttg gggtttgcga cgggaacacg 600
   cccatgctgt gcctctgaat ctggcaccgt caccctgtgg cctgggttca gcaacttggc 660
   ctcaccttcc ttgtctgtga aattcagact gggtccttgt gagatgattg gagagaatgt 720
   atgaactatg tgagaacgcc acctttgtgc gtatctcacg cagtgtcttc cctcctttcc 780
   aaagtcttct gctgtctcta gacacacccg acgtgggggg ggggggttcc ctgggtctcc 840
   tcctaggtct gtcccaggag ggcacgcact gaaggccgcg agaatcccgg gggctgcatt 900
   gcgccgcgcc aaggactcca cacaggacct ttcattttcc caactgtgct gagccaggcg 960
   gccggcagag agcaggtggc tgacaggccc cggggagccg gaccgcctgg gtctaatctt 1020
   cccgcagact cccttgctgt gcgctttggg gcttgggcct cagtttcctc aaaaggaatg 1080
   aggggctttt ttggaacgtt aaataatttc ctacgtggtt gcgggtaggg agaaggagaa 1140
   agagaggagc gcgcctgcgc gcctggaatc gtgcccggat cagagcaagc gctctaaaag 1200
   tgttacaaac attaaggcgc caactaaaaa acccgtagtg agcgcaggca gaaaccacgg 1260
   gtaagagaag tggagaagct tcgcgtaggc cccagggtcc cgagccccga gtctcgagcg 1320
   cagaatcagg ggtgccaatg ctctcctccg cgcccccgag cgctcgcctt ggccatgcgg 1380
   gccgccccac cgggatgagg gcgctcaggc cggacgctgg ggccccgggt tctcgccccg 1440
   ccccgccctc ggggattcag aggggccggg aggagcctcg cgcatgtgca cagctggcgc 1500
   cccccgcccc ccgcgcacag ctgggacgtg ggccgcggcc gggcgggcgc agtcgggagc 1560
   cggccgtggt ggctccgtgc gtccgagcgt ccgtccgcgc cgtcggccat ggccaagcgc 1620
   tccaggggcc ccgggcgccg ctgcctgttg gcgctcgtgc tgttctgcgc ctgggggacg 1680
   ctggccgtgg tggcccagaa gccgggcgca gggtgtccga gccgctgcct gtgcttccgc 1740
   accaccgtgc gctgcatgca tctgctgctg gaggccgtgc ccgccgtggc gccgcagacc 1800
   tccatcctgt gagtgccgcg ggggacgccg ggggcgcggg gtccggggct tcgtggagat 1860
   ccgggagcgc aggggtgatc ggaggtgggg ggcgcggagg gtggaggggg catcgggcgc 1920
   gcggggggcc tggggacttg ggacgcagaa gggaacctcc gaagggggac gtggggggac 1980
   ctgggcgcgg ggacccgctg ggcctttgtt cgccctgcgg gagacgccga ggggcggaac 2040
   agagcgctgt gcgcgcggcc ttcgtagccg cctttgttcg gaactcggaa tccccgcagg 2100
   actgggaagt tgttggagcc tccggggctc cccccgctcg cctcccgccg ccccctctca 2160
   tgctccgccg gcctcccgct tccccctggt tcgcggcccc tcctccgctc acctttcccc 2220
   cgctcaggac ccctcggtcc ccctccgctc cccgagcgcg gcgcagcccc ctccgtcctc 2280
   ccagccccct ccgccccgtt cctcgtcctg ttcgctcccc tcctccgctc ctcttcctcc 2340
   tccccttcct cctcctcctc cccttcctcc tcctcctccc cttcctcctc ctcctccctt 2400
   cccctcctcc tccccccctt ccttctcctc ccccagcctc cgccctctcc ccctcccccg 2460
   ccccttggag cgcagtgccc accccatccc cccgcgccgg 2500
<210> 11
   <211> 2200
   <212> DNA
   <213> Homo sapiens
<400> 11
   cctcgccccc tccagccggc cccccgggcc cctcctctcg gcgcccggac cttggccctc 60
   cctctccttt cccacttctc tctttgccct aacttcgccc ccatcccccg ctcatttcct 120
   ctcgcacccg ggctcgccaa tccctctttc caagtccctc ttccagcccg gccttcctct 180
   cgggttcgcc ccccttctcc ccaatctccg tcctcttccc tcccttcgcc ctccccccct 240
   tccttcctct tcccctcacc caaccctggt tcccctcgtt cctcagtccc gatctctccc 300
   ttactctgtc cccgcccact ctgcgccggc ctctcagtcc gggttgagcc ccacgtgtgg 360
   acggccgcgc ccccactgac agccgccgcc cgccggcccg ccccgcgccc cgccgggcct 420
   ctaaaacccc cgcgccgcgc cctccaccgc cgcatcttct ccagcgccca gcctcccgcc 480
   ctctctcttg ctggccgcac gccccggccc cgcgcacctc cgcccggctc cgcagccgct 540
   acccgcgctt cgttgccctg tgggactccg agcgagcccg gagggaaccc tcctcttctt 600
   ctgggggcga cttttgtttg cttgcctgtt tctttctggt gacttttgca gctttccaat 660
   atccgtcttc ggagcgcacg ggaatccgcc gagctctgcg tgcaggccct tttttctttt 720
   gaggttcaca ttttttgaaa ttttacgcca gggcttttgt aatttcctcc cccgcccgct 780
   gacggtcctg gagtcgctcg gggctttagg ccggttatgc aacgtgtacc gctcggggct 840
   gccggctgca cctccgccgc gcctcgccgc tcactgcgct agacccggcg ccccgcgtct 900
   cgcttcgcgg gcagtcaggg ggccggcgct ctgtcgaggt ctccagctag agcagggagc 960
   ccgagcccga gggagtcccc ggagccgacg aagggcttat tagaccctga ctcttttctg 1020
   aggcgcgcag attttgtctt tgatcactcc ctctccgcgg gtctacggcc gcgcgctttc 1080
   ggcgccggcg atggggagaa gacggaggct gtgtctccag ctctacttcc tgtggctggg 1140
   ctgtgtggtg ctctgggcgc agggcacggc cggccagcct cagcctcctc cgcccaagcc 1200
   gccccggccc cagccgccgc cgcaacaggt tcggtccgct acagcaggct ctgaaggcgg 1260
   gtttctagcg cccgagtatc gcgaggaggg tgccgcagtg gccagccgcg tccgccggcg 1320
   aggacagcag gacgtgctcc gagggtaagt gggcaagcgg ctccgcacct agggctccgg 1380
   cttgggggag gggggaatcc tcagtttggc ggctttctgg cccactccgt cccagaccct 1440
   ttagctggag cctagagctg cagccccctt tgccagaata tccaaagacc cccaggagcg 1500
   cgtccccctt ttccttccca accccgcagc tcagcgggcg gaaagccctc tctccggggg 1560
   ttgggcggcg ggtggttagg gggtccaggg gtgccgatcg cagagcgtgt gcagagctcg 1620
   cgctgcggga acaggttctg aatgtccggc ggcaggcggg cctgggtccg cctgctgcag 1680
   gggccagaga agcctgcttg ctccccacgt cggggccgcc gctcgtgagc cttttgtttg 1740
   aggacgtgtg cagggttcac agctcacctt ctcatcgtca acccgagcgc tccaccttgc 1800
   gacgcgcttt ccttgacacg tcggggccaa agtaacagtt gaccaaggag gaatggattt 1860
   gggaaggagg gcaaggattc tttggaacgg aatggtccct ttgttctctg catctggaag 1920
   ctagaatagt agcaaattat atgtttccat gcctcttttc gccctttaaa aaggcaggca 1980
   agggacgaca gatgaaaggc agtgtttaga catttctgac cctcctgcat tccagcatct 2040
   agctcttttg cttccacgtc tgcctcccga tctccaataa tttgaagtgt aattttgatt 2100
   tgtttgttgt cctgaaatct actcgctcgg ggcattgctt acgaagaccg tttatatgtt 2160
   gctgcatccc tctacctatc tgttacgtga ccgcgcttgt 2200
<210> 12
   <211> 2000
   <212> DNA
   <213> Homo sapiens
<400> 12
   ttggaagaaa aggatctccg aggaaggggc tgagagaagg gcagggtgaa ctggactaaa 60
   ggccagagta ggaaggagaa gaggggccaa aaaagaaggg gatgaaatta agcacagaag 120
   atgggtaaag aaaaaagtat cagggaaagg gcaaaataag agaaagcctt gaggataaga 180
   gggtagaagg ctaaagaaca aggggaccac tgggtcgggg aagcgctgcc tgaacggcgg 240
   gacagtgaca aagaaagggc gctggcgata ttcgcaccaa gggtgcgaaa cgcaatcggg 300
   aggtgagaaa tggaaagaag gcgaatgccc ggctacaagt agcctgggac tgaaagggga 360
   cctgggggag gggctgggcc cagggcagaa aagtccaggt tcccatgcgg cctgggccca 420
   cgtggagcgg gcgctgaatc accgttcagc cgcccccctc ccctcctccc cgaccggtgc 480
   ccgcagtccc cgcctcctcg gccgccgcct ccacggggcg gggccctggc ccgggaccag 540
   cgccgcggct ataaatgggc tgcggcgagg ccggcagaac gctgtgacag ccacacgccc 600
   caaggcctcc aagatgagct acacgttgga ctcgctgggc aacccgtccg cctaccggcg 660
   ggtaaccgag acccgctcga gcttcagccg cgtcagcggc tccccgtcca gtggcttccg 720
   ctcgcagtcg tggtcccgcg gctcgcccag caccgtgtcc tcctcctata agcgcagcat 780
   gctcgccccg cgcctcgctt acagctcggc catgctcagc tccgccgaga gcagccttga 840
   cttcagccag tcctcgtccc tgctcaacgg cggctccgga cccggcggcg actacaagct 900
   gtcccgctcc aacgagaagg agcagctgca ggggctgaac gaccgctttg ccggctacat 960
   agagaaggtg cactacctgg agcagcagaa taaggagatt gaggcggaga tccaggcgct 1020
   gcggcagaag caggcctcgc acgcccagct gggcgacgcg tacgaccagg agatccgcga 1080
   gctgcgcgcc accctggaga tggtgaacca cgagaaggct caggtgcagc tggactcgga 1140
   ccacctggag gaagacatcc accggctcaa ggagcgcttt gaggaggagg cgcggttgcg 1200
   cgacgacact gaggcggcca tccgcgcgct gcgcaaagac atcgaggagg cgtcgctggt 1260
   caaggtggag ctggacaaga aggtgcagtc gctgcaggat gaggtggcct tcctgcggag 1320
   caaccacgag gaggaggtgg ccgaccttct ggcccagatc caggcatcgc acatcacggt 1380
   ggagcgcaaa gactacctga agacagacat ctcgacggcg ctgaaggaaa tccgctccca 1440
   gctcgaaagc cactcagacc agaatatgca ccaggccgaa gagtggttca aatgccgcta 1500
   cgccaagctc accgaggcgg ccgagcagaa caaggaggcc atccgctccg ccaaggaaga 1560
   gatcgccgag taccggcgcc agctgcagtc caagagcatc gagctagagt cggtgcgcgg 1620
   caccaaggag tccctggagc ggcagctcag cgacatcgag gagcgccaca accacgacct 1680
   cagcagctac caggtaggaa ccgcggctgc gcggccagcc tgcgccagcg ccagcgccgc 1740
   gcgcccccga cacttgggct cgtgcccagg cgccctctcc gccgcgctcc ctggtggccg 1800
   ctcgctagag cacgcgcgcc gcagacctag ggtatttgcg gatcagcgtc ctcgcccatc 1860
   tcatcctcca cactccgccc ccacccacct gccccagctg ctaagggtct tgaccttttt 1920
   cagaaacgtg catcttttcc agttctaatt ttgcacgctt gcacgtttaa agcaggaggg 1980
   atgaattcgg tagtggataa 2000
<210> 13
   <211> 2300
   <212> DNA
   <213> Homo sapiens
<400> 13
   tcagattgtc attgggaggg tgaataaatg aatgcttgca ttatgagagt ttgggggcag 60
   aaatatgcca cagactctta tctgaagcca tcagatttag tggctgcgaa cccaccgaag 120
   tcagggattt acatttttta cagcaacgag agaaaacttc ccctttcctc tgcagaagtc 180
   aggactggat ctcaaaaata gaaatgtgtc ctcctaaatg tgtgcccatc cccgtggttg 240
   acaaacaacg gatttcccaa gatagctgcc acacacttgg tttctaatct ctgtattgct 300
   tccccgccag aatgtcgaag tccttcccga atatgcccag tcatactttc tgaacttttg 360
   agcaaacacc gtccggcttc ttgtgctttc ctcaaagacc ccaggcaccg gcagggagga 420
   cacaggccgg ggcagagcgc ccctgcgcgg gggattcctg ccactccgcg ccagcctgcg 480
   gcgcaaacgc tcttctcagc cgcagtccca cccgctgctg gcaatctgaa tgaggagccg 540
   cgctattttt acctccccgg ctgcaatcct ttatatttac atgcaggaag caaatatata 600
   agggattaag aaggagatgc gtggccttag tttatccaga gcaggaagag gttggaatag 660
   gagagggtat gtgaagtctg gggtggtgga aaaggcaggt ggacttcggc tggttgtttt 720
   ctcccgatca tccctgtctc tggcctggaa acccccgtac tctctttctt ctggcttatc 780
   cgtgactgcc ggctccccct ccaccgcccc catcttttga ggtaccaccc gtcacctccg 840
   atgctgcttg ggctgctgca tcactctgct gctttacccc cttccccgcc ccccaacaaa 900
   gcatgcgcag tgcgttccgg gccaggcaac agcagcagca cagcatccag caacagcatc 960
   agcacccgaa gccccgctcg ggcgcgctct cggggggcgg ggcgcacgcc cgctccgcgc 1020
   gtccccgcgc cgctcgctcc cgcgcgtccc cgcgccgctc gctcccgcgc gccgcctcag 1080
   catcctcagg cccggcggca gcccccgcag tcgctgaagc ggccgcgccc gccgggggag 1140
   ggagtagccg ctggggaggc tccaagttgg cggagcggcg aggacccctg gactcctctg 1200
   cgtcccgccc cgggagtggc tgcgaggcta ggcgagccgg gaaagggggc gccgcccagc 1260
   cccgagcccc gcgccccgtg ccccgagccc ggagccccct gcccgccgcg gcaccatgcg 1320
   cgccgagccg gcgtgaccgg ctccgcccgc ggccgccccg cagctagccc ggcgctctcg 1380
   ccggccacac ggagcggcgc ccgggagcta tgagccatga agccgcccgg cagcagctcg 1440
   cggcagccgc ccctggcggg ctgcagcctt gccggcgctt cctgcggccc ccaacgcggc 1500
   cccgccggct cggtgcctgc cagcgccccg gcccgcacgc cgccctgccg cctgcttctc 1560
   gtccttctcc tgctgcctcc gctcgccgcc tcgtcccggc cccgcgcctg gggggctgct 1620
   gcgcccagcg gtgggtatgg ccccgtgccc tttgcgttgg ctttcccgcg gggccctgca 1680
   gaggaaagcg aagggcgcgc gggtccgtgt gctccgggct tgtccccggc tcggcctttc 1740
   cttccctccc tgcctgtctt tccacccttc tcgttcccaa acccccattc atcccagttc 1800
   acttttggaa gtccatttct gttgcattcg cgaaaaaccc attccaattc ttgttggttc 1860
   cactgggagg tgtttagtgg atcctgggtc cctcagcgat ctctgtgcaa cttgcggagg 1920
   ggcaaccagt ggatgggaaa tacagcgagg gagcaagttg ctacttgcgt ggtggaacct 1980
   taatgtgaat gcggggagga tgtagtgata atagtggtaa tgggctgttt cctcaaattt 2040
   cgtatccggc gcattcagtg cggttggaat taaggtgggg gaggcacact tcggggacca 2100
   aagaattaag gtgctgaaga catacttcat gcacgacctt tggttctgat ttctcaaagt 2160
   gcttgtcatt ataatgaaca attaatataa taccatcttc tatatattga tgattggaag 2220
   tcactgaaag cagaaagctg gctttgtcag gaaaataaaa agaaattggg aagctgccag 2280
   catctgtatc cctacatggc 2300
<210> 14
   <211> 3000
   <212> DNA
   <213> Homo sapiens
<400> 14
   tactgccgac tttaggtctc tctggatctc aggccccctt ctctaagatg catcctagag 60
   gaccaaaaat acactttatt tgggcttcgc ctgcttttgt ggaagggtag tttactagag 120
   gatataatct cgtgttttaa tttgctctct ctcctaaagg aaatgtggag aaaaaaaaaa 180
   agcagaaatt ggaaataacc aatatttagt ttatttcatt cgattcttag gggaactggt 240
   gaggagccta agatgatttt cccttcctag agaaagaatc caaagtccag ggaaatagcg 300
   acaggggagt tcaagactgc ccctgctagt ccttccttgg ctactctccg ctgcgatcgc 360
   aggatagctc tcattagcag gagaatcggg caagtgtgtg gataagtaga gagtgtgttg 420
   aacaacttgt aacgttttat gaaatacgca ttgtcatggt tccctaaaag gctttgcgga 480
   agccgtttgt ctttactaat caagtcttta cttacacaaa agtagaagta gaagtagttt 540
   tagaaaacat actaacaatc ttctatcccc ttgaagacca gagtagcaga aaacaggtga 600
   tttgcattat aaaattgcac tcactttttc ctcctttcag atttcacatt acattagccc 660
   atttgtgtta cggtgtataa aaaatggaac aggcgcctcc actgcattgt tctcctttaa 720
   aaatagatca cttacaccct aactttgttt tccttaaatt cgattcttaa caggagagct 780
   ttctattatt tcagatggag tgaggttgca cgactgggat ggaagaaagg aatcccttaa 840
   atttggggga atttctgttc tctgttccaa gaccatttta cttggggtgt gggggtgggc 900
   gcggcggtca gggcagtgga acgcagtcgc ggctgcgcca tccctgcact tccaggcgcg 960
   cgggagggac cggcggggac gcgagctgcg gactctggcg aactcggggg aggcagacag 1020
   ggggaggcgg acacccagcc ggcaggcgtc tcagcctccc cgcagccggc gggcttttct 1080
   cctgacagct ccaggaaagg cagacccctt ccccagccag ccaggtaagg taaagactgc 1140
   tgttgagctt gctgttactg agggcgcaca gaccctgggg agaccgaagc ttgccactgc 1200
   gggattctgt ggggtaacct gggtctacgg aagtttcctg aaagagggga gaagggtttg 1260
   catttttcct atggaggatt cttctctctc tagcatttcg tttgatgtat tcaactggta 1320
   gaagtgagat ttcaacaggt agcagagagc gctcacgtgg aggaggtttg gggcgccgcg 1380
   gcgccacccc cacccctcct cgggaccgcg cctatttcta aagttacacg tcgacgaact 1440
   aacctatgct ttaaattcct ctttccagcc ccgtgagtcc gcggcgacat tgggccgtgg 1500
   ggtggctggg aacggtcccc tcctccggaa aaaccagaga acggcttgga gagctgaaac 1560
   gagcgtccgc gagcaggtcc gtgcagaacc gggcttcagg accgctgagc tccgtagggc 1620
   gtccttgggg gacgccaggt cgccggctcc tctgccctcg ttgagatgga caacgcctcg 1680
   ttctcggagc cctggcccgc caacgcatcg ggcccggacc cggcgctgag ctgctccaac 1740
   gcgtcgactc tggcgccgct gccggcgccg ctggcggtgg ctgtaccagt tgtctacgcg 1800
   gtgatctgcg ccgtgggtct ggcgggcaac tccgccgtgc tgtacgtgtt gctgcgggcg 1860
   ccccgcatga agaccgtcac caacctgttc atcctcaacc tggccatcgc cgacgagctc 1920
   ttcacgctgg tgctgcccat caacatcgcc gacttcctgc tgcggcagtg gcccttcggg 1980
   gagctcatgt gcaagctcat cgtggctatc gaccagtaca acaccttctc cagcctctac 2040
   ttcctcaccg tcatgagcgc cgaccgctac ctggtggtgt tggccactgc ggagtcgcgc 2100
   cgggtggccg gccgcaccta cagcgccgcg cgcgcggtga gcctggccgt gtgggggatc 2160
   gtcacactcg tcgtgctgcc cttcgcagtc ttcgcccggc tagacgacga gcagggccgg 2220
   cgccagtgcg tgctagtctt tccgcagccc gaggccttct ggtggcgcgc gagccgcctc 2280
   tacacgctcg tgctgggctt cgccatcccc gtgtccacca tctgtgtcct ctataccacc 2340
   ctgctgtgcc ggctgcatgc catgcggctg gacagccacg ccaaggccct ggagcgcgcc 2400
   aagaagcggg tgaccttcct ggtggtggca atcctggcgg tgtgcctcct ctgctggacg 2460
   ccctaccacc tgagcaccgt ggtggcgctc accaccgacc tcccgcagac gccgctggtc 2520
   atcgctatct cctacttcat caccagcctg agctacgcca acagctgcct caaccccttc 2580
   ctctacgcct tcctggacgc cagcttccgc aggaacctcc gccagctgat aacttgccgc 2640
   gcggcagcct gactccccca gcgtccggct ccgcaactgc ccgccactcc tggccagcga 2700
   gggaggagcc ggcgccagag tgcgggacca gacaggccgc ctaggcctcc tggggaaacc 2760
   gactcgcgcc ccatacccga cctagcagat cggaagcgct gcgactgtgc ccgcaggttg 2820
   accttgccaa gccctccagg tgatgcgcgg ccatgccggg tgaggagaac tgaggctgag 2880
   atcgccacac tgagggctcc ctaaagccga ggtggaggaa gaggagggta gaggaggagg 2940
   gcggtattgc tgggaaccgc cccctccctg ccctgctccc tgctgcccca cccgagccct 3000
<210> 15
   <211> 3000
   <212> DNA
   <213> Homo sapiens
<400> 15
   gaatacatta aagtaggggc aacccttgag cccagacttc tgccatgtga agaccctttg 60
   aaaatcctga caaacacagg tactgcgtaa gtggtcagct aattaaagag gggaggtgga 120
   gctgtccttt gtgtatccaa taagtaccca ttatctcatt tgagcatgaa aagaggccac 180
   tgttattact ttcaagaagg aaagtaagca ggatagctca tatttttaga accattcctc 240
   accaaatgga ataattccgg tgaaaagtgg gagtgaggaa gaaagaaaaa aaaaacttct 300
   aatcataatg tttgggaata agaaaggaag aagaaactca cgtcaaagcc gactttctcc 360
   tgcagctgta aaataaactc ttaagaccct tcctgctgaa actctggaga ggaaaactgg 420
   agtggcgggt gggctttgcc tgcagctcaa ctctccctcg cggcgcgggc gcggctgggt 480
   tcagcacctc ggaaagcgcc cctcgcggcg ccccgggatt acgcatgctc cttggggccc 540
   gccgccttgg ccgtgcaagt gccaccgtaa ctggtgagag ccgctggcaa cccacccgga 600
   gttgacaacc gcggagagac gcagacaccc actgacctcc aggaagctga gcgtggtgga 660
   tggaactcta cgatctcttt ctctccaagg acggaaacct catccaagca gtcccagagg 720
   aaacggataa aggtatttga aagggagcga gcggccccaa atcgcacaat tgagcggctg 780
   ggggagttat gcgccagtgc cccagtgacc gcgggacacg gagaggggaa gtctgcgttg 840
   tacataagga cctagggact ccgagcttgg cctgagaacc cttggacgcc gagtgcttgc 900
   cttacgggct gcactcctca actctgctcc aaagcagccg ctgagctcaa ctcctgcgtc 960
   cagggcgttc gctgcgcgcc aggacgcgct tagtacccag ttcctgggct ctctcttcag 1020
   tagctgcttt gaaagctccc acgcacgtcc cgcaggctag cctggcaaca aaactggggt 1080
   aaaccgtgtt atcttaggtc ttgtccccca gaacatgacc tagaggtacc tgcgcatgca 1140
   gatggccgat gcagccacga tagccaccat gaataaggca gcaggcgggg acaagctagc 1200
   agaactcttc agtctggtcc cggaccttct ggaggcggcc aacacgagtg gtaacgcgtc 1260
   gctgcagctt ccggacttgt ggtgggagct ggggctggag ttgccggacg gcgcgccgcc 1320
   aggacatccc ccgggcagcg gcggggcaga gagcgcggac acagaggccc gggtgcggat 1380
   tctcatcagc gtggtgtact gggtggtgtg cgccctgggg ttggcgggca acctgctggt 1440
   tctctacctg atgaagagca tgcagggctg gcgcaagtcc tctatcaacc tcttcgtcac 1500
   caacctggcg ctgacggact ttcagtttgt gctcaccctg cccttctggg cggtggagaa 1560
   cgctcttgac ttcaaatggc ccttcggcaa ggccatgtgt aagatcgtgt ccatggtgac 1620
   gtccatgaac atgtacgcca gcgtgttctt cctcactgcc atgagtgtga cgcgctacca 1680
   ttcggtggcc tcggctctga agagccaccg gacccgagga cacggccggg gcgactgctg 1740
   cggccggagc ctgggggaca gctgctgctt ctcggccaag gcgctgtgtg tgtggatctg 1800
   ggctttggcc gcgctggcct cgctgcccag tgccattttc tccaccacgg tcaaggtgat 1860
   gggcgaggag ctgtgcctgg tgcgtttccc ggacaagttg ctgggccgcg acaggcagtt 1920
   ctggctgggc ctctaccact cgcagaaggt gctgctgggc ttcgtgctgc cgctgggcat 1980
   cattatcttg tgctacctgc tgctggtgcg cttcatcgcc gaccgccgcg cggcggggac 2040
   caaaggaggg gccgcggtag ccggaggacg cccgaccgga gccagcgccc ggagactgtc 2100
   gaaggtcacc aaatcagtga ccatcgttgt cctgtccttc ttcctgtgtt ggctgcccaa 2160
   ccaggcgctc accacctgga gcatcctcat caagttcaac gcggtgccct tcagccagga 2220
   gtatttcctg tgccaggtat acgcgttccc tgtgagcgtg tgcctagcgc actccaacag 2280
   ctgcctcaac cccgtcctct actgcctcgt gcgccgcgag ttccgcaagg cgctcaagag 2340
   cctgctgtgg cgcatcgcgt ctccttcgat caccagcatg cgccccttca ccgccactac 2400
   caagccggag cacgaggatc aggggctgca ggccccggcg ccgccccacg cggccgcgga 2460
   gccggacctg ctctactacc cacctggcgt cgtggtctac agcgggggcc gctacgacct 2520
   gctgcccagc agctctgcct actgacgcag gcctcaggcc cagggcgcgc cgtcggggca 2580
   aggtggcctt ccccgggcgg taaagaggtg aaaggatgaa ggagggctgg ggggggcccc 2640
   atttaagaag taggtgggag gaggatgggc agagcatgga ggaggagcct gtggataggc 2700
   cgaggacctt ctctggagag gagatgcttc gaaatcaggt ggagagagga aattggcaaa 2760
   gggatagaga cgagccccac gggccagaca gccaacctcc gctccgcacc ccacagcctc 2820
   tccttactct tcccacgctg agtagtgtgg gggcgcccag aagcgaagac aagcagcaaa 2880
   aatgtagaga aattggcacg gggagcgggg cttagccaaa tgatgcacag acaattgtgc 2940
   ccgtttattc cagcgacttc tgcggagagg gcagccgtcg gcacaaacac tcctttgcgt 3000
<210> 16
   <211> 2200
   <212> DNA
   <213> Homo sapiens
<400> 16
   gtcccccgat tccctcaccc atcatataac gtgtgtattt attatgtttc ccgtttcctc 60
   tgtctccgcc agcagaatgt aaactccatg aggtcaggaa tctccgagtt atgttgcgcc 120
   agtgtaatcc aagagcccgg aacagtgcct ggcacacagc gggcatatgg aagaacaaat 180
   gtgtgaaggt gtgaatgaat gaataattga aagaataaat agtagttctc agcctcacag 240
   aacacgggtc acaacctcaa atgacctgct accctgccca taaataacag agatgcagga 300
   gtaagtgctg ggctgtgacc tgtcaacatg ctaagccgct caaacaaaac tgcccaacag 360
   cccgctggcc gcctatttgc agcactgggc cctgagccgc acattcccat ttcgttgata 420
   aagaaactga ccagatagtt taagtggcct gctgcggaag acagagctgg tgctgcaccg 480
   gtcgctgctt ccccagtcct tttttggcct cctttctgac gcgacgcaga ccccagttct 540
   ggagagtctg tcactcgctc cccgtggtgg gagatcagag gcctggtgtc cttgggagcg 600
   gcgagcggtg ctcggcgcag gatagaaagg gagtgcgcgc ccgagtcccc cagatccctg 660
   ggaacccgcg ccaccctccc gcccctgccc atccccggcc gcgctgtcag tctccattag 720
   cgctaacagg ctccagacgg agcgggccgg gcgctgggtt aatgcaatcg gcgcgttacc 780
   tggggcgcag gctacattac cagcccggcc cccgccaggc acggccagaa ccagtcagcc 840
   cgcgccctgc cggccgcccc gcgcctccag ctcttccccg gccccgcccg aacgccacac 900
   ggcggagccc agccccagcc cgcgccctag agcctgccaa ggcgccgccg gtcgggggcc 960
   ggcagggcgc aaggcaccag ggatcccctc gccgccggac acgtgagtgc gccctgagcg 1020
   cgggacaggg ctaggtctgc ctgggaggcc cgggccgaga cgcgccagca gagggctagc 1080
   gagtttgtag tgcagtgacg ttaagtgtcc gagaaggctc ctgtggctgt tgaagtgtcg 1140
   cggacctgag ctggggaggg ggtcggcacg ctgccctcag cctcggtgag ttcaatccca 1200
   gccatttggg gcaggcgaga gtgggtgaac gaggaaaagt gctgcagggt cttcagccgc 1260
   ccccagaggg ctgtcagaag tctccaactc ttgagttccg gcgtgcccca acctctgttt 1320
   ccaaattttt ccagcggacg cgcgctcttt tctgggaacc ctgcgtccgc tcagcgcgcg 1380
   ctcatcccag tgtctaaggc gctcccgggt ggtcttggga gttgcaagta gggaggaacg 1440
   gccgggtaac cacctctttt ccctttatcc aagcagagcc tcggcgtgcc cccaggaccg 1500
   gtaaagttcc tctcgccagc cgcatccatg cttctggcgc ggatgaaccc gcaggtgcag 1560
   cccgagaaca acggggcgga cacgggtcca gagcagcccc ttcgggcgcg caaaactgcg 1620
   gagctgctgg tggtgaagga gcgcaacggc gtccagtgcc tgctggcgcc ccgcgacggc 1680
   gacgcgcagc cccgggagac ctggggcaag aagatcgact tcctgctgtc cgtagtcggc 1740
   ttcgcagtgg acctggccaa cgtgtggcgc ttcccctacc tctgctacaa gaacggcggc 1800
   ggtgagcgtg gggtcgggct gggaatttga atctgggagg tccactgtct gcagcggtgg 1860
   ctgggacagg agctggaata cacacggaag ggaggcgagg agacaggggc aaatctgggg 1920
   cgcagaaaga actggacagg gctaacggga aaaaaaaaag attggagtcc tctggaaggt 1980
   cattttccca ggctctttgc agagtacctc gagctcattc cagcggaagt gtcaggattg 2040
   ggcaccctgg aagcaaaaca gcagaagagt gaaatcgagt catgacccta aagtcatggt 2100
   aggggtatgg atggaaagga cagaatctgg ggtgccaggt tgggtggggg agcctgacct 2160
   tttgatggtc tgctggaagg gaggtggaga ttccaagagc 2200
<210> 17
   <211> 16
   <212> DNA
   <213> Artificial Sequence
<220>
   <223> Designed biotinated oligonucleotide for fixation
<220>
   <223> n stands for m5c
<400> 17
   angaangtan ggangc 16
<210> 18
   <211> 16
   <212> DNA
   <213> Artificial Sequence
<220>
   <223> Designed biotinated oligonucleotide for fixation
<220>
   <223> n stands for m5c
<400> 18
   tngatngttn ggtngc 16
<210> 19
   <211> 16
   <212> DNA
   <213> Artificial Sequence
<220>
   <223> Designed biotinated oligonucleotide for fixation
<220>
   <223> n stands for m5c
<400> 19
   gngagngtgn gggngc 16
<210> 20
   <211> 16
   <212> DNA
   <213> Artificial Sequence
<220>
   <223> Designed biotinated oligonucleotide for fixation
<220>
   <223> n stands for m5c
<400> 20
   cngacngtcn ggcngc 16
<210> 21
   <211> 16
   <212> DNA
   <213> Artificial Sequence
<220>
   <223> Designed biotinated oligonucleotide for fixation
<400> 21
   acgaacgtac ggacgc 16
<210> 22
   <211> 16
   <212> DNA
   <213> Artificial Sequence
<220>
   <223> Designed biotinated oligonucleotide for fixation
<400> 22
   tcgatcgttc ggtcgc 16
<210> 23
   <211> 16
   <212> DNA
   <213> Artificial Sequence
<220>
   <223> Designed biotinated oligonucleotide for fixation
<400> 23
   gcgagcgtgc gggcgc 16
<210> 24
   <211> 16
   <212> DNA
   <213> Artificial Sequence
<220>
   <223> Designed biotinated oligonucleotide for fixation
<400> 24
   ccgaccgtcc ggccgc 16
<210> 25
   <211> 54
   <212> DNA
   <213> Artificial Sequence
<220>
   <223> Designed oligonucleotide
<220>
   <223> n stands for m5c
<400> 25
   angaangtan ggangctnga tngttnggtn gcctgtctga ctacaacatc caga 54
<210> 26
   <211> 54
   <212> DNA
   <213> Artificial Sequence
<220>
   <223> Designed oligonucleotide
<400> 26
   acgaacgtac ggacgctcga tcgttcggtc gcctgtctga ctacaacatc caga 54
<210> 27
   <211> 22
   <212> DNA
   <213> Artificial Sequence
<220>
   <223> Designed biotinated oligonucleotide for fixation
<400> 27
   tctggatgtt gtagtcagac ag 22
<210> 28
   <211> 80
   <212> DNA
   <213> Artificial Sequence
<220>
   <223> Designed oligonucleotide
<220>
   <223> n stands for m5c
<400> 28
   agtgacacca tngagaatgt cagatcngga tcagagngcc atctagatgg acatgtcact 60
   gtctgactac aacatccaga 80
<210> 29
   <211> 80
   <212> DNA
   <213> Artificial Sequence
<220>
   <223> Designed oligonucleotide
<220>
   <223> n stands for m5c
<400> 29
   agtgacacca tngagaatgt cagatccgga tcagagcgcc atctagatgg acatgtcact 60
   gtctgactac aacatccaga 80
<210> 30
   <211> 80
   <212> DNA
   <213> Artificial Sequence
<220>
   <223> Designed oligonucleotide
<400> 30
   agtgacacca tcgagaatgt cagatccgga tcagagcgcc atctagatgg acatgtcact 60
   gtctgactac aacatccaga 80
<210> 31
   <211> 12
   <212> DNA
   <213> Artificial Sequence
<220>
   <223> Designed oligonucleotide
<400> 31
   gatggcgctc tg 12
<210> 32
   <211> 22
   <212> DNA
   <213> Artificial Sequence
<220>
   <223> Designed biotinated oligonucleotide for fixation
<400> 32
   tctggatgtt gtagtcagac ag 22
<210> 33
   <211> 86
   <212> DNA
   <213> Artificial Sequence
<220>
   <223> Designed oligonucleotide
<220>
   <223> n stands for m5c
<400> 33
   agtgacacca tngagaatgt cagatcngga tcagagngcc atctacngga tggacatgng 60
   ctcactgtct gactacaaca tccaga 86
<210> 34
   <211> 86
   <212> DNA
   <213> Artificial Sequence
<220>
   <223> Designed oligonucleotide
<220>
   <223> n stands for m5c
<400> 34
<210> 35
   <211> 12
   <212> DNA
   <213> Artificial Sequence
<220>
   <223> Designed oligonucleotide
<400> 35
   gatggcgctc tg 12
<210> 36
   <211> 22
   <212> DNA
   <213> Artificial Sequence
<220>
   <223> Designed biotinated oligonucleotide for fixation
<400> 36
   tctggatgtt gtagtcagac ag 22
<210> 37
   <211> 54
   <212> DNA
   <213> Artificial Sequence
<220>
   <223> Designed oligonucleotide
<220>
   <223> n stands for m5c
<400> 37
   angaangtan ggangctnga tngttnggtn gcctgtctga ctacaacatc caga 54
<210> 38
   <211> 54
   <212> DNA
   <213> Artificial Sequence
<220>
   <223> Designed oligonucleotide
<220>
   <223> n stands for m5c
<400> 38
   angaangtan ggangctnga tngttnggtn gccagccgac gaagggctta ttag 54
<210> 39
   <211> 54
   <212> DNA
   <213> Artificial Sequence
<220>
   <223> Designed oligonucleotide
<220>
   <223> n stands for m5c
<400> 39
   angaangtan ggangctnga tngttnggtn gcctgccgag aacgaggcgt tgtc 54
<210> 40
   <211> 54
   <212> DNA
   <213> Artificial Sequence
<220>
   <223> Designed oligonucleotide
<400> 40
   acgaacgtac ggacgctcga tcgttcggtc gcctgtctga ctacaacatc caga 54
<210> 41
   <211> 54
   <212> DNA
   <213> Artificial Sequence
<220>
   <223> Designed-oligonucleotide
<400> 41
   acgaacgtac ggacgctcga tcgttcggtc gccagccgac gaagggctta ttag 54
<210> 42
   <211> 54
   <212> DNA
   <213> Artificial Sequence
<220>
   <223> Designed oligonucleotide
<400> 42
   acgaacgtac ggacgctcga tcgttcggtc gcctgccgag aacgaggcgt tgtc 54
<210> 43
   <211> 22
   <212> DNA
   <213> Artificial Sequence
<220>
   <223> Designed biotinated oligonucleotide for fixation
<400> 43
   tctggatgtt gtagtcagac ag 22
<210> 44
   <211> 21
   <212> DNA
   <213> Artificial Sequence
<220>
   <223> Designed biotinated oligonucleotide for fixation
<400> 44
   ctaataagcc cttcgtcggc t 21
<210> 45
   <211> 19
   <212> DNA
   <213> Artificial Sequence
<220>
   <223> Designed biotinated oligonucleotide for fixation
<400> 45
   gacaacgcct cgttctcgg 19
<210> 46
   <211> 54
   <212> DNA
   <213> Artificial Sequence
<220>
   <223> Designed oligonucleotide
<220>
   <223> n stands for m5c
<400> 46
   angaangtan ggangctnga tngttnggtn gcctgtctga ctacaacatc caga 54
<210> 47
   <211> 54
   <212> DNA
   <213> Artificial Sequence
<220>
   <223> Designed oligonucleotide
<220>
   <223> n stands for m5c
<400> 47
   angaangtan ggangctnga tngttnggtn gcctgccgag aacgaggcgt tgtc 54
<210> 48
   <211> 54
   <212> DNA
   <213> Artificial Sequence
<220>
   <223> Designed oligonucleotide
<220>
   <223> n stands for m5c
<400> 48
   angaangtan ggangctnga tngttnggtn gccagccgac gaagggctta ttag 54
<210> 49
   <211> 54
   <212> DNA
   <213> Artificial Sequence
<220>
   <223> Designed oligonucleotide
<400> 49
   acgaacgtac ggacgctcga tcgttcggtc gcctgtctga ctacaacatc caga 54
<210> 50
   <211> 54
   <212> DNA
   <213> Artificial Sequence
<220>
   <223> Designed oligonucleotide
<400> 50
   acgaacgtac ggacgctcga tcgttcggtc gcctgccgag aacgaggcgt tgtc 54
<210> 51
   <211> 54
   <212> DNA
   <213> Artificial Sequence
<220>
   <223> Designed oligonucleotide
<400> 51
   acgaacgtac ggacgctcga tcgttcggtc gccagccgac gaagggctta ttag 54
<210> 52
   <211> 22
   <212> DNA
   <213> Artificial Sequence
<220>
   <223> Designed FITC labeled oligonucleotide
<400> 52
   tctggatgtt gtagtcagac ag 22
<210> 53
   <211> 19
   <212> DNA
   <213> Artificial Sequence
<220>
   <223> Designed FITC labeled oligonucleotide for fixation
<400> 53
   gacaacgcct cgttctcgg 19
<210> 54
   <211> 21
   <212> DNA
   <213> Artificial Sequence
<220>
   <223> Designed FITC labeled oligonucleotide for fixation
<400> 54
   ctaataagcc cttcgtcggc t 21
<210> 55
   <211> 80
   <212> DNA
   <213> Artificial Sequence
<220>
   <223> Designed oligonucleotide
<220>
   <223> n stands for m5c
<400> 55
   agtgacacca tngagaatgt cagatcngga tcagagngcc atctagatgg acatgtcact 60
   gtctgactac aacatccaga 80
<210> 56
   <211> 80
   <212> DNA
   <213> Artificial Sequence
<220>
   <223> Designed oligonucleotide
<220>
   <223> n stands for m5c
<400> 56
   agtgacacca tngagaatgt cagatccgga tcagagcgcc atctagatgg acatgtcact 60
   gtctgactac aacatccaga 80
<210> 57
   <211> 80
   <212> DNA
   <213> Artificial Sequence
<220>
   <223> Designed oligonucleotide
<400> 57
   agtgacacca tcgagaatgt cagatccgga tcagagcgcc atctagatgg acatgtcact 60
   gtctgactac aacatccaga 80
<210> 58
   <211> 12
   <212> DNA
   <213> Artificial Sequence
<220>
   <223> Designed oligonucleotide
<400> 58
   gatggcgctc tg 12
<210> 59
   <211> 22
   <212> DNA
   <213> Artificial Sequence
<220>
   <223> Designed FITC labeled oligonucleotide
<400> 59
   tctggatgtt gtagtcagac ag 22
<210> 60
   <211> 66
   <212> DNA
   <213> Artificial Sequence
<220>
   <223> Designed oligonucleotide
<220>
   <223> n stands for m5c
<400> 60
   ctgtctgact acaacatcca gangccgaga acgaggcgtt gtcngagccg acgaagggct 60
   tattag 66
<210> 61
   <211> 80
   <212> DNA
   <213> Artificial Sequence
<220>
   <223> Designed oligonucleotide
<400> 61
<210> 62
   <211> 22
   <212> DNA
   <213> Artificial Sequence
<220>
   <223> Designed FITC labeled oligonucleotide for fixation
<400> 62
   tctggatgtt gtagtcagac ag 22
<210> 63
   <211> 19
   <212> DNA
   <213> Artificial Sequence
<220>
   <223> Designed FITC labeled oligonucleotide for fixation
<400> 63
   gacaacgcct cgttctcgg 19
<210> 64
   <211> 21
   <212> DNA
   <213> Artificial Sequence
<220>
   <223> Designed FITC labeled oligonucleotide for fixation
<400> 64
   ctaataagcc cttcgtcggc t 21
<210> 65
   <211> 54
   <212> DNA
   <213> Artificial Sequence
<220>
   <223> Designed oligonucleotide
<220>
   <223> n stands for m5c
<400> 65
   angaangtan ggangctnga tngttnggtn gcctgtctga ctacaacatc caga 54
<210> 66
   <211> 54
   <212> DNA
   <213> Artificial Sequence
<220>
   <223> Designed oligonucleotide
<220>
   <223> n stands for m5c
<400> 66
   angaangtan ggangctnga tngttnggtn gccagccgac gaagggctta ttag 54
<210> 67
   <211> 54
   <212> DNA
   <213> Artificial Sequence
<220>
   <223> Designed oligonucleotide
<220>
   <223> n stands for m5c
<400> 67
   angaangtan ggangctnga tngttnggtn gcctgccgag aacgaggcgt tgtc 54
<210> 68
   <211> 54
   <212> DNA
   <213> Artificial Sequence
<220>
   <223> Designed oligonucleotide
<400> 68
   acgaacgtac ggacgctcga tcgttcggtc gcctgtctga ctacaacatc caga 54
<210> 69
   <211> 54
   <212> DNA
   <213> Artificial Sequence
<220>
   <223> Designed oligonucleotide
<400> 69
   acgaacgtac ggacgctcga tcgttcggtc gccagccgac gaagggctta ttag 54
<210> 70
   <211> 54
   <212> DNA
   <213> Artificial Sequence
<220>
   <223> Designed oligonucleotide
<400> 70
   acgaacgtac ggacgctcga tcgttcggtc gcctgccgag aacgaggcgt tgtc 54
<210> 71
   <211> 22
   <212> DNA
   <213> Artificial Sequence
<220>
   <223> Designed FITC labeled oligonucleotide
<400> 71
   tctggatgtt gtagtcagac ag 22
<210> 72
   <211> 19
   <212> DNA
   <213> Artificial Sequence
<220>
   <223> Designed FITC labeled oligonucleotide
<400> 72
   gacaacgcct cgttctcgg 19
<210> 73
   <211> 21
   <212> DNA
   <213> Artificial Sequence
<220>
   <223> Designed FITC labeled oligonucleotide
<400> 73
   ctaataagcc cttcgtcggc t 21
<210> 74
   <211> 54
   <212> DNA
   <213> Artificial Sequence
<220>
   <223> Designed oligonucleotide
<220>
   <223> n stands for m5c
<400> 74
   angaangtan ggangctnga tngttnggtn gcctgtctga ctacaacatc caga 54
<210> 75
   <211> 54
   <212> DNA
   <213> Artificial Sequence
<220>
   <223> Designed oligonucleotide
<400> 75
   acgaacgtac ggacgctcga tcgttcggtc gcctgtctga ctacaacatc caga 54
<210> 76
   <211> 22
   <212> DNA
   <213> Artificial Sequence
<220>
   <223> Designed-FITC labeled oligonucleotide
<400> 76
   tctggatgtt gtagtcagac ag 22
<210> 77
   <211> 12
   <212> DNA
   <213> Artificial Sequence
<220>
   <223> Designed oligonucleotide
<400> 77
   gatggcgctc tg 12
<210> 78
   <211> 54
   <212> DNA
   <213> Artificial Sequence
<220>
   <223> Designed oligonucleotide
<220>
   <223> n stands for m5c
<400> 78
   angaangtan ggangctnga tngttnggtn gcctgtctga ctacaacatc caga 54
<210> 79
   <211> 54
   <212> DNA
   <213> Artificial Sequence
<220>
   <223> Designed oligonucleotide
<220>
   <223> n stands for m5c
<400> 79
   angaangtan ggangctnga tngttnggtn gccagccgac gaagggctta ttag 54
<210> 80
   <211> 54
   <212> DNA
   <213> Artificial Sequence
<220>
   <223> Designed oligonucleotide
<220>
   <223> n stands for m5c
<400> 80
   angaangtan ggangctnga tngttnggtn gcctgccgag aacgaggcgt tgtc 54
<210> 81
   <211> 54
   <212> DNA
   <213> Artificial Sequence
<220>
   <223> Designed oligonucleotide
<400> 81
   acgaacgtac ggacgctcga tcgttcggtc gcctgtctga ctacaacatc caga 54
<210> 82
   <211> 54
   <212> DNA
   <213> Artificial Sequence
<220>
   <223> Designed oligonucleotide
<400> 82
   acgaacgtac ggacgctcga tcgttcggtc gccagccgac gaagggctta ttag 54
<210> 83
   <211> 54
   <212> DNA
   <213> Artificial Sequence
<220>
   <223> Designed oligonucleotide
<400> 83
   acgaacgtac ggacgctcga tcgttcggtc gcctgccgag aacgaggcgt tgtc 54
<210> 84
   <211> 22
   <212> DNA
   <213> Artificial Sequence
<220>
   <223> Designed FITC labeled oligonucleotide
<400> 84
   tctggatgtt gtagtcagac ag 22
<210> 85
   <211> 21
   <212> DNA
   <213> Artificial Sequence
<220>
   <223> Designed FITC labeled oligonucleotide
<400> 85
   ctaataagcc cttcgtcggc t 21
<210> 86
   <211> 19
   <212> DNA
   <213> Artificial Sequence
<220>
   <223> Designed FITC labeled oligonucleotide
<400> 86
   gacaacgcct cgttctcgg 19
<210> 87
   <211> 12
   <212> DNA
   <213> Artificial Sequence
<220>
   <223> Designed oligonucleotide
<400> 87
   gatggcgctc tg 12
<210> 88
   <211> 54
   <212> DNA
   <213> Artificial Sequence
<220>
   <223> Designed oligonucleotide
<220>
   <223> n stands for m5c
<400> 88
   angaangtan ggangctnga tngttnggtn gcctgtctga ctacaacatc caga 54
<210> 89
   <211> 54
   <212> DNA
   <213> Artificial Sequence
<220>
   <223> Designed oligonucleotide
<220>
   <223> n stands for m5c
<400> 89
   angaangtan ggangctnga tngttnggtn gccagccgac gaagggctta ttag 54
<210> 90
   <211> 54
   <212> DNA
   <213> Artificial Sequence
<220>
   <223> Designed oligonucleotide
<220>
   <223> n stands for m5c
<400> 90
   angaangtan ggangctnga tngttnggtn gcctgccgag aacgaggcgt tgtc 54
<210> 91
   <211> 54
   <212> DNA
   <213> Artificial Sequence
<220>
   <223> Designed oligonucleotide
<400> 91
   acgaacgtac ggacgctcga tcgttcggtc gcctgtctga ctacaacatc caga 54
<210> 92
   <211> 54
   <212> DNA
   <213> Artificial Sequence
<220>
   <223> Designed oligonucleotide
<400> 92
   acgaacgtac ggacgctcga tcgttcggtc gccagccgac gaagggctta ttag 54
<210> 93
   <211> 54
   <212> DNA
   <213> Artificial Sequence
<220>
   <223> Designed oligonucleotide
<400> 93
   acgaacgtac ggacgctcga tcgttcggtc gcctgccgag aacgaggcgt tgtc 54
<210> 94
   <211> 22
   <212> DNA
   <213> Artificial Sequence
<220>
   <223> Designed FITC labeled oligonucleotide
<400> 94
   tctggatgtt gtagtcagac ag 22
<210> 95
   <211> 21
   <212> DNA
   <213> Artificial Sequence
<220>
   <223> Designed FITC labeled oligonucleotide
<400> 95
   ctaataagcc cttcgtcggc t 21
<210> 96
   <211> 19
   <212> DNA
   <213> Artificial Sequence
<220>
   <223> Designed FITC labeled oligonucleotide
<400> 96
   gacaacgcct cgttctcgg 19
<210> 97
   <211> 12
   <212> DNA
   <213> Artificial Sequence
<220>
   <223> Designed oligonucleotide
<400> 97
   gatggcgctc tg 12
<210> 98
   <211> 54
   <212> DNA
   <213> Artificial Sequence
<220>
   <223> Designed oligonucleotide
<220>
   <223> n stands for m5c
<400> 98
   angaangtan ggangctnga tngttnggtn gccagccgac gaagggctta ttag 54
<210> 99
   <211> 54
   <212> DNA
   <213> Artificial Sequence
<220>
   <223> Designed oligonucleotide
<400> 99
   acgaacgtac ggacgctcga tcgttcggtc gccagccgac gaagggctta ttag 54
<210> 100
   <211> 21
   <212> DNA
   <213> Artificial Sequence
<220>
   <223> Designed FITC labeled oligonucleotide
<400> 100
   ctaataagcc cttcgtcggc t 21
<210> 101
   <211> 12
   <212> DNA
   <213> Artificial Sequence
<220>
   <223> Designed oligonucleotide
<400> 101
   gatggcgctc tg 12
<210> 102
   <211> 54
   <212> DNA
   <213> Artificial Sequence
<220>
   <223> Designed oligonucleotide
<220>
   <223> n stands for m5c
<400> 102
   angaangtan ggangctnga tngttnggtn gcctgtctga ctacaacatc caga 54
<210> 103
   <211> 54
   <212> DNA
   <213> Artificial Sequence
<220>
   <223> Designed oligonucleotide
<400> 103
   acgaacgtac ggacgctcga tcgttcggtc gcctgtctga ctacaacatc caga 54
<210> 104
   <211> 22
   <212> DNA
   <213> Artificial Sequence
<220>
   <223> Designed biotinated oligonucleotide for fixation
<400> 104
   tctggatgtt gtagtcagac ag 22
<210> 105
   <211> 54
   <212> DNA
   <213> Artificial Sequence
<220>
   <223> Designed oligonucleotide
<220>
   <223> n stands for m5c
<400> 105
   angaangtan ggangctnga tngttnggtn gccagccgac gaagggctta ttag 54
<210> 106
   <211> 54
   <212> DNA
   <213> Artificial Sequence
<220>
   <223> Designed oligonucleotide
<400> 106
   acgaacgtac ggacgctcga tcgttcggtc gccagccgac gaagggctta ttag 54
<210> 107
   <211> 21
   <212> DNA
   <213> Artificial Sequence
<220>
   <223> Designed biotinated oligonucleotide for fixation
<400> 107
   ctaataagcc cttcgtcggc t 21
<210> 108
   <211> 54
   <212> DNA
   <213> Artificial Sequence
<220>
   <223> Designed oligonucleotide
<220>
   <223> n stands for m5c
<400> 108
   angaangtan ggangctnga tngttnggtn gccagccgac gaagggctta ttag 54
<210> 109
   <211> 54
   <212> DNA
   <213> Artificial Sequence
<220>
   <223> Designed oligonucleotide
<400> 109
   acgaacgtac ggacgctcga tcgttcggtc gccagccgac gaagggctta ttag 54
<210> 110
   <211> 21
   <212> DNA
   <213> Artificial Sequence
<220>
   <223> Designed biotinated oligonucleotide for fixation
<400> 110
   ctaataagcc cttcgtcggc t 21
<210> 111
   <211> 18
   <212> DNA
   <213> Artificial Sequence
<220>
   <223> Designed oligonucleotide primer for PCR
<400> 111
   ctcagcaccc aggcggcc 18
<210> 112
   <211> 20
   <212> DNA
   <213> Artificial Sequence
<220>
   <223> Designed oligonucleotide primer for PCR
<400> 112
   ctggccaaac tggagatcgc 20
<210> 113
   <211> 386
   <212> DNA
   <213> Artificial Sequence
<220>
   <223> Amplified oligonucleotide consist of objective DNA domain (Genbank
   Accession No.NT029419 25687390-25687775 Homo sapiens)
<400> 113
   ctcagcaccc aggcggccgc gatcatgagg cgcgagcggc gcgcgggctg ttgcagagtc 60
   ttgagcgggt ggcacaccgc gatgtagcgg tcggctgtca tgactaccag catgtaggcc 120
   gacgcaaaca tgccgaacac ctgcaggtgc ttcaccacgc ggcacagcca gtcggggccg 180
   cggaagcggt aggtgatgtc ccagcacatt tgcggcagca cctggaagaa tgccacggcc 240
   aggtcggcca ggctgaggtg tcggatgaag aggtgcatgc gggacgtctt gcgcggcgtc 300
   cggtgcagag ccagcagtac gctgctgttg cccagcacgg ccaccgcgaa agtcaccgcc 360
   agcacggcga tctccagttt ggccag 386
<210> 114
   <211> 386
   <212> DNA
   <213> Artificial Sequence
<220>
   <223> Designed oligonucleotide consist of objective DNA domain ( Genbank
   Accession No.NT029419 25687390-25687775 Homo sapiens ) n=m5c
<400> 114
   ctcagcaccc aggnggcngn gatcatgagg ngngagnggn gngngggctg ttgcagagtc 60
   ttgagngggt ggcacacngn gatgtagngg tnggctgtca tgactaccag catgtaggcn 120
   gangcaaaca tgcngaacac ctgcaggtgc ttcaccangn ggcacagcca gtnggggcng 180
   nggaagnggt aggtgatgtc ccagcacatt tgnggcagca cctggaagaa tgccanggcc 240
   aggtnggcca ggctgaggtg tnggatgaag aggtgcatgn gggangtctt gngnggngtc 300
   nggtgcagag ccagcagtan gctgctgttg cccagcangg ccacngngaa agtcacngcc 360
   agcanggnga tctccagttt ggccag 386
<210> 115
   <211> 386
   <212> DNA
   <213> Artificial Sequence
<220>
   <223> Designed oligonucleotide consist of objective DNA domain ( Genbank
   Accession No.NT029419 2568.7390-25687775 Homo sapiens )
<400> 115
   ctcagcaccc aggcggccgc gatcatgagg cgcgagcggc gcgcgggctg ttgcagagtc 60
   ttgagcgggt ggcacaccgc gatgtagcgg tcggctgtca tgactaccag catgtaggcc 120
   gacgcaaaca tgccgaacac ctgcaggtgc ttcaccacgc ggcacagcca gtcggggccg 180
   cggaagcggt aggtgatgtc ccagcacatt tgcggcagca cctggaagaa tgccacggcc 240
   aggtcggcca ggctgaggtg tcggatgaag aggtgcatgc gggacgtctt gcgcggcgtc 300
   cggtgcagag ccagcagtac gctgctgttg cccagcacgg ccaccgcgaa agtcaccgcc 360
   agcacggcga tctccagttt ggccag 386
<210> 116
   <211> 20
   <212> DNA
   <213> Artificial Sequence
<220>
   <223> Designed biotinated oligonucleotide for fixation
<400> 116
   ctggccaaac tggagatcgc 20
<210> 117
   <211> 30
   <212> DNA
   <213> Artificial Sequence
<220>
   <223> Designed counter oligonucleotide for making a objective DNA domain a
   single strand DNA
<400> 117
   gccaccgcga aagtcaccgc cagcacggcg 30
<210> 118
   <211> 30
   <212> DNA
   <213> Artificial Sequence
<220>
   <223> Designed counter oligonucleotide for making a objective DNA domain a
   single strand DNA
<400> 118
   gccagcagta cgctgctgtt gcccagcacg 30
<210> 119
   <211> 30
   <212> DNA
   <213> Artificial Sequence
<220>
   <223> Designed counter oligonucleotide for making a objective DNA domain a
   single strand DNA
<400> 119
   cgggacgtct tgcgcggcgt ccggtgcaga 30
<210> 120
   <211> 30
   <212> DNA
   <213> Artificial Sequence
<220>
   <223> Designed counter oligonucleotide for making a objective DNA domain a
   single strand DNA
<400> 120
   aggctgaggt gtcggatgaa gaggtgcatg 30
<210> 121
   <211> 30
   <212> DNA
   <213> Artificial Sequence
<220>
   <223> Designed counter oligonucleotide for making a objective DNA domain a
   single strand DNA
<400> 121
   acctggaaga atgccacggc caggtcggcc 30
<210> 122
   <211> 30
   <212> DNA
   <213> Artificial Sequence
<220>
   <223> Designed counter oligonucleotide for making a objective DNA domain a
   single strand DNA
<400> 122
   taggtgatgt cccagcacat ttgcggcagc 30
<210> 123
   <211> 30
   <212> DNA
   <213> Artificial Sequence
<220>
   <223> Designed counter oligonucleotide for making a objective DNA domain a
   single strand DNA
<400> 123
   cggcacagcc agtcggggcc gcggaagcgg 30
<210> 124
   <211> 30
   <212> DNA
   <213> Artificial Sequence
<220>
   <223> Designed counter oligonucleotide for making a objective DNA domain a
   single strand DNA
<400> 124
   atgccgaaca cctgcaggtg cttcaccacg 30
<210> 125
   <211> 30
   <212> DNA
   <213> Artificial Sequence
<220>
   <223> Designed counter oligonucleotide for making a objective DNA domain a
   single strand DNA
<400> 125
   atgactacca gcatgtaggc cgacgcaaac 30
<210> 126
   <211> 30
   <212> DNA
   <213> Artificial Sequence
<220>
   <223> Designed counter oligonucleotide for making a objective DNA domain a
   single strand DNA
<400> 126
   tggcacaccg cgatgtagcg gtcggctgtc 30
<210> 127
   <211> 30
   <212> DNA
   <213> Artificial Sequence
<220>
   <223> Designed counter oligonucleotide for making a objective DNA domain a
   single strand DNA
<400> 127
   cgcgcgggct gttgcagagt cttgagcggg 30
<210> 128
   <211> 30
   <212> DNA
   <213> Artificial Sequence
<220>
   <223> Designed counter oligonucleotide for making a objective DNA domain a
   single strand DNA
<400> 128
   caggcggccg cgatcatgag gcgcgagcgg 30
<210> 129
   <211> 17
   <212> DNA
   <213> Artificial Sequence
<220>
   <223> Designed oligonucleotide
<400> 129
   atctccagtt tggccag 17
<210> 130
   <211> 19
   <212> DNA
   <213> Artificial Sequence
<220>
   <223> Designed oligonucleotide primer for PCR
<400> 130
   tgagctccgt agggcgtcc 19
<210> 131
   <211> 17
   <212> DNA
   <213> Artificial Sequence
<220>
   <223> Designed oligonucleotide primer for PCR
<400> 131
   gcgccgggtc cgggccc 17
<210> 132
   <211> 121
   <212> DNA
   <213> Artificial Sequence
<220>
   <223> Amplified oligonucleotide consist of objective DNA domain ( Genbank
   Accession No.AC009800 76606-76726 Homo sapiens )
<400> 132
   gcgccgggtc cgggcccgat gcgttggcgg gccagggctc cgagaacgag gcgttgtcca 60
   tctcaacgag ggcagaggag ccggcgacct ggcgtccccc aaggacgccc tacggagctc 120
   a 121
<210> 133
   <211> 121
   <212> DNA
   <213> Artificial Sequence
<220>
   <223> Designed oligonucleotide consist of objective DNA domain ( Genbank
   Accession No.AC009800 76606-76726 Homo sapiens ) n=m5c
<400> 133
   gngcngggtc ngggccngat gngttggngg gccagggctc ngagaangag gngttgtcca 60
   tctcaangag ggcagaggag cnggngacct ggngtccccc aaggangccc tanggagctc 120
   a 121
<210> 134
   <211> 121
   <212> DNA
   <213> Artificial Sequence
<220>
   <223> Designed oligonucleotide consist of objective DNA domain ( Genbank
   Accession No.AC009800 76606-76726 Homo sapiens )
<400> 134
   gcgccgggtc cgggcccgat gcgttggcgg gccagggctc cgagaacgag gcgttgtcca 60
   tctcaacgag ggcagaggag ccggcgacct ggcgtccccc aaggacgccc tacggagctc 120
   a 121
<210> 135
   <211> 19
   <212> DNA
   <213> Artificial Sequence
<220>
   <223> Designed biotinated oligonucleotide for fixation
<400> 135
   gacaacgcct cgttctcgg 19
<210> 136
   <211> 30
   <212> DNA
   <213> Artificial Sequence
<220>
   <223> Designed counter oligonucleotide for making a objective DNA domain a
   single strand DNA
<400> 136
   gcgtccccca aggacgccct acggagctca 30
<210> 137
   <211> 30
   <212> DNA
   <213> Artificial Sequence
<220>
   <223> Designed counter oligonucleotide for making a objective DNA domain a
   single strand DNA
<400> 137
   ctcaacgagg gcagaggagc cggcgacctg 30
<210> 138
   <211> 30
   <212> DNA
   <213> Artificial Sequence
<220>
   <223> Designed counter oligonucleotide for making a objective DNA domain a
   single strand DNA
<400> 138
   cgccgggtcc gggcccgatg cgttggcggg 30
<210> 139
   <211> 20
   <212> DNA
   <213> Artificial Sequence
<220>
   <223> Designed oligonucleotide
<400> 139
   ccgagaacga ggcgttgtct 20
<210> 140
   <211> 21
   <212> DNA
   <213> Artificial Sequence
<220>
   <223> Designed oligonucleotide primer for PCR
<400> 140
   aggtgagcta cgtgtgtttg g 21
<210> 141
   <211> 21
   <212> DNA
   <213> Artificial Sequence
<220>
   <223> Designed oligonucleotide primer for PCR
<400> 141
   agacatgtgc tcacgtacgg t 21
<210> 142
   <211> 331
   <212> DNA
   <213> Artificial Sequence
<220>
   <223> Amplified oligonucleotide consist of objective DNA domain ( Genbank
   Accession No.NC001139 271743-272083 Saccharomyces cereviciae chromosome VII)
<400> 142
   aggtgagcta cgtgtgtttg ggcgtcgtgc actggctcac ttgtacgcgc agaaatggca 60
   gcttgtacga ttggtgaccc gccttttcga cactggaccg ctatggacgt ggcggcggtg 120
   tggcggcggc tcaatgacct gtggcgcccg tttgtggcgt gcgatagtcg agccgcctgt 180
   cacgtgcgcg gccgccctgc tccgtttgac gcgatgcata gcatgcgacc acccagtaat 240
   catactgctg acgctattgg tcacgtggtt atggcagctg ctgttgactg cggtggcgtc 300
   ccgtttccac accgtacgtg agcacatgtc t 331
<210> 143
   <211> 331
   <212> DNA
   <213> Artificial Sequence
<220>
   <223> Designed oligonucleotide consist of objective DNA domain ( Genbank Accession No.NC001139 271743-272083 Saccharomyces cereviciae chromosome VII) n=m5c
<400> 143
   aggtgagcta ngtgtgtttg ggngtngtgc actggctcac ttgtangngc agaaatggca 60
   gcttgtanga ttggtgaccn gccttttnga cactggacng ctatggangt ggnggnggtg 120
   tggnggnggc tcaatgacct gtggngccng tttgtggngt gngatagtng agcngcctgt 180
   cangtgngng gcngccctgc tcngtttgan gngatgcata gcatgngacc acccagtaat 240
   catactgctg angctattgg tcangtggtt atggcagctg ctgttgactg nggtggngtc 300
   cngtttccac acngtangtg agcacatgtc t 331
<210> 144
   <211> 331
   <212> DNA
   <213> Artificial Sequence
<220>
   <223> Designed oligonucleotide consist of objective DNA domain ( Genbank
   Accession No.NC001139 271743-272083 Saccharomyces cereviciae chromosome VII)
<400> 144
   aggtgagcta cgtgtgtttg ggcgtcgtgc actggctcac ttgtacgcgc agaaatggca 60
   gcttgtacga ttggtgaccc gccttttcga cactggaccg ctatggacgt ggcggcggtg 120
   tggcggcggc tcaatgacct gtggcgcccg tttgtggcgt gcgatagtcg agccgcctgt 180
   cacgtgcgcg gccgccctgc tccgtttgac gcgatgcata gcatgcgacc acccagtaat 240
   catactgctg acgctattgg tcacgtggtt atggcagctg ctgttgactg cggtggcgtc 300
   ccgtttccac accgtacgtg agcacatgtc t 331
<210> 145
   <211> 21
   <212> DNA
   <213> Artificial Sequence
<220>
   <223> Designed biotinated oligonucleotide for fixation
<400> 145
   agacatgtgc tcacgtacgg t 21
<210> 146
   <211> 21
   <212> DNA
   <213> Artificial Sequence
<220>
   <223> Designed counter oligonucleotide for making a objective DNA domain a
   single strand DNA
<400> 146
   aggtgagcta cgtgtgtttg g 21
<210> 147
   <211> 30
   <212> DNA
   <213> Artificial Sequence
<220>
   <223> Designed counter oligonucleotide for making a objective DNA domain a
   single strand DNA
<400> 147
   gcgtcgtgca ctggctcact tgtacgcgca 30
<210> 148
   <211> 30
   <212> DNA
   <213> Artificial Sequence
<220>
   <223> Designed counter oligonucleotide for making a objective DNA domain a
   single strand DNA
<400> 148
   cttgtacgat tggtgacccg ccttttcgac 30
<210> 149
   <211> 30
   <212> DNA
   <213> Artificial Sequence
<220>
   <223> Designed counter oligonucleotide for making a objective DNA domain a
   single strand DNA
<400> 149
   actggaccgc tatggacgtg gcggcggtgt 30
<210> 150
   <211> 30
   <212> DNA
   <213> Artificial Sequence
<220>
   <223> Designed counter oligonucleotide for making a objective DNA domain a
   single strand DNA
<400> 150
   ggcggcggct caatgacctg tggcgcccgt 30
<210> 151
   <211> 30
   <212> DNA
   <213> Artificial Sequence
<220>
   <223> Designed counter oligonucleotide for making a objective DNA domain a
   single strand DNA
<400> 151
   ttgtggcgtg cgatagtcga gccgcctgtc 30
<210> 152
   <211> 25
   <212> DNA
   <213> Artificial Sequence
<220>
   <223> Designed counter oligonucleotide for making a objective DNA domain a
   single strand DNA
<400> 152
   acgtgcgcgg ccgccctgct ccgtt 25
<210> 153
   <211> 30
   <212> DNA
   <213> Artificial Sequence
<220>
   <223> Designed counter oligonucleotide for making a objective DNA domain a
   single strand DNA
<400> 153
   tgacgcgatg catagcatgc gaccacccag 30
<210> 154
   <211> 30
   <212> DNA
   <213> Artificial Sequence
<220>
   <223> Designed counter oligonucleotide for making a objective DNA domain a
   single strand DNA
<400> 154
   actgctgacg ctattggtca cgtggttatg 30
<210> 155
   <211> 30
   <212> DNA
   <213> Artificial Sequence
<220>
   <223> Designed counter oligonucleotide for making a objective DNA domain a
   single strand DNA
<400> 155
   ctgctgttga ctgcggtggc gtcccgtttc 30
<210> 156
   <211> 21
   <212> DNA
   <213> Artificial Sequence
<220>
   <223> Designed oligonucleotide
<400> 156
   accgtacgtg agcacatgtc t 21
<210> 157
   <211> 21
   <212> DNA
   <213> Artificial Sequence
<220>
   <223> Designed oligonucleotide primer for PCR
<400> 157
   ggacctgtgt ttgacgggta t 21
<210> 158
   <211> 21
   <212> DNA
   <213> Artificial Sequence
<220>
   <223> Designed oligonucleotide primer for PCR
<400> 158
   agtacagatc tggcgttctc g 21
<210> 159
   <211> 116
   <212> DNA
   <213> Artificial Sequence
<220>
   <223> Amplified oligonucleotide consist of objective DNA domain ( Genbank
   Accession No.NC001139 384569-384685 Saccharomyces cereviciae chromosome VII )
<400> 159
<210> 160
   <211> 116
   <212> DNA
   <213> Artificial Sequence
<220>
   <223> Designed oligonucleotide consist of objective DNA domain ( Genbank
   Accession No.NC001139 384569-384685 Saccharomyces cereviciae chromosome VII )
n=m5c
<400> 160
<210> 161
   <211> 116
   <212> DNA
   <213> Artificial Sequence
<220>
   <223> Designed oligonucleotide consist of objective DNA domain ( Genbank
   Accession No.NC001139 384569-384685 Saccharomyces cereviciae chromosome VII )
<400> 161
<210> 162
   <211> 21
   <212> DNA
   <213> Artificial Sequence
<220>
   <223> Designed biotinated oligonucleotide for fixation
<400> 162
   agtacagatc tggcgttctc g 21
<210> 163
   <211> 21
   <212> DNA
   <213> Artificial Sequence
<220>
   <223> Designed counter oligonucleotide for making a objective DNA domain a
   single strand DNA
<400> 163
   ggacctgtgt ttgacgggta t 21
<210> 164
   <211> 25
   <212> DNA
   <213> Artificial Sequence
<220>
   <223> Designed counter oligonucleotide for making a objective DNA domain a
   single strand DNA
<400> 164
   aacactaagt tgcgcaattt gctgt 25
<210> 165
   <211> 25
   <212> DNA
   <213> Artificial Sequence
<220>
   <223> Designed counter oligonucleotide for making a objective DNA domain a
   single strand DNA
<400> 165
   attgcgaaat ccgcccggac gatat 25
<210> 166
   <211> 25
   <212> DNA
   <213> Artificial Sequence
<220>
   <223> Designed counter oligonucleotide for making a objective DNA domain a
   single strand DNA
<400> 166
   cactcttgag cgcatgtgcc gtttc 25
<210> 167
   <211> 21
   <212> DNA
   <213> Artificial Sequence
<220>
   <223> Designed oligonucleotide
<400> 167
   cgagaacgcc agatctgtac t 21
<210> 168
   <211> 20
   <212> DNA
   <213> Artificial Sequence
<220>
   <223> Designed FITC labeled oligonucleotide
<400> 168
   ctggccaaac tggagatcgc 20
<210> 169
   <211> 21
   <212> DNA
   <213> Artificial Sequence
<220>
   <223> Designed FITC labeled oligonucleotide
<400> 169
   agacatgtgc tcacgtacgg t 21
<210> 170
   <211> 21
   <212> DNA
   <213> Artificial Sequence
<220>
   <223> Designed FITC labeled oligonucleotide
<400> 170
   agtacagatc tggcgttctc g 21